(19) Europäisches Patentamt
European Patent Office
Office européen des brevets

(11) EP 4 342 490 A1

(12) EUROPEAN PATENT APPLICATION
published in accordance with Art. 153(4) EPC

(43) Date of publication:
**27.03.2024 Bulletin 2024/13**

(21) Application number: **22804735.3**

(22) Date of filing: **18.05.2022**

(51) International Patent Classification (IPC):
***A61K 39/145*** *(2006.01)* ***A61K 9/127*** *(2006.01)*
***A61K 47/10*** *(2017.01)* ***A61K 47/18*** *(2017.01)*
***A61K 47/24*** *(2006.01)* ***A61K 47/28*** *(2006.01)*
***A61K 48/00*** *(2006.01)* ***A61P 31/16*** *(2006.01)*
***A61P 37/04*** *(2006.01)* ***C12N 15/44*** *(2006.01)*
***C12N 15/88*** *(2006.01)*

(52) Cooperative Patent Classification (CPC):
**A61K 9/127; A61K 39/145; A61K 47/10; A61K 47/18; A61K 47/24; A61K 47/28; A61K 48/00; A61P 31/16; A61P 37/04; C07K 14/11; C12N 15/88**

(86) International application number:
**PCT/JP2022/020745**

(87) International publication number:
**WO 2022/244825 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.05.2021 JP 2021084669**

(71) Applicant: **Daiichi Sankyo Company, Limited**
**Tokyo 103-8426 (JP)**

(72) Inventors:
- **TOMOZAWA, Takanori**
  **Tokyo 103-8426 (JP)**
- **NIWA, Takako**
  **Tokyo 103-8426 (JP)**
- **NUMATA, Yukinobu**
  **Tokyo 103-8426 (JP)**
- **KOIZUMI, Makoto**
  **Tokyo 103-8426 (JP)**

(74) Representative: **Vossius & Partner Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) INFLUENZA VIRUS NUCLEIC ACID LIPID PARTICLE VACCINE

(57) Provided is a vaccine for preventing and/or treating an infection with an influenza virus. The vaccine comprises lipid particles containing a nucleic acid capable of expressing a haemagglutinin (HA) protein of the influenza virus, wherein a lipid is a cationic lipid having general formula (Ia), or a pharmaceutically acceptable salt thereof.

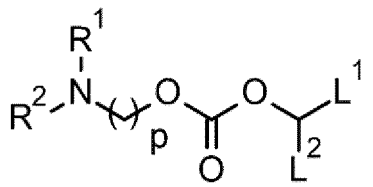

(Ia)

[In the formula, $R^1$, $R^2$, p, $L^1$ and $L^2$ are as defined in the specification.]

EP 4 342 490 A1

[Figure 1]

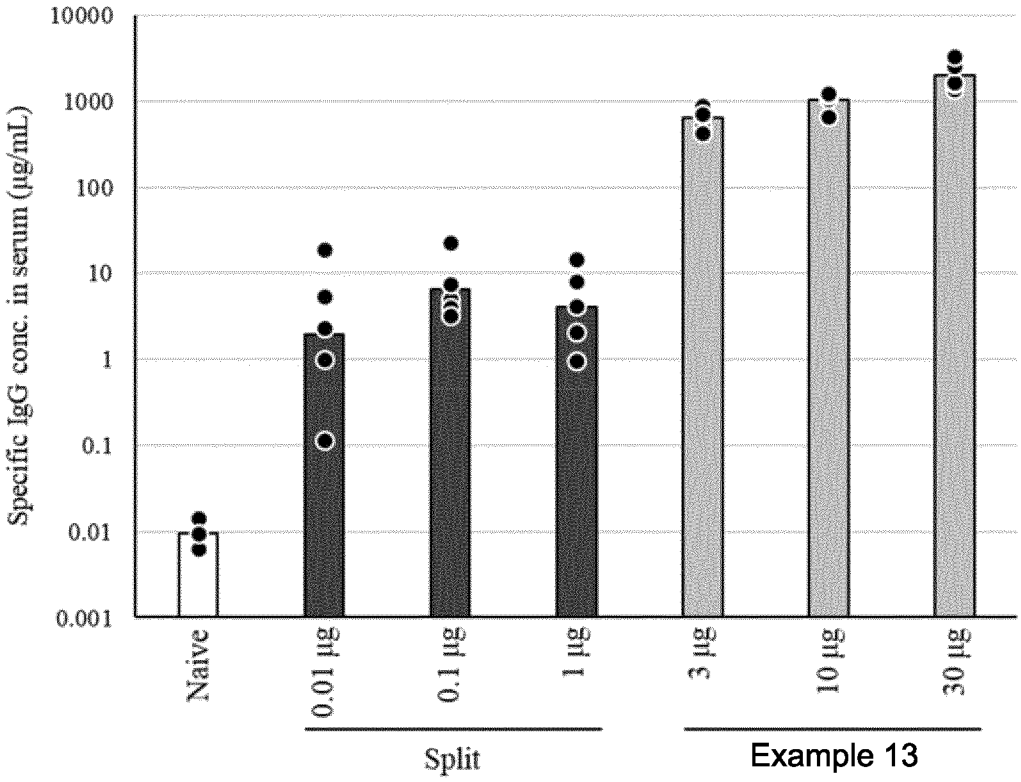

Specific IgG conc. in serum (µg/mL)
10000
1000
100
10
1
0.1
0.01
0.001
Naive
0.01 µg
0.1 µg
1 µg
3 µg
10 µg
30 µg
Split
Example 13

## Description

### Technical Field

The present invention relates to lipid particles encapsulating a nucleic acid capable of expressing haemagglutinin (HA) of an influenza virus, and a vaccine composition using the lipid particles.

### Background Art

Seasonal influenza infections are respiratory tract infections caused by type-A and type-B influenza viruses, and typically prevail in certain seasons. Type-A and type-B influenza viruses are envelope viruses having eight negative-strand RNA segments in the genome. One of RNA segments encodes HA which is a glycoprotein on the viral surface. HA binds to sialic acid on the surfaces of airway epithelial cells, so that the virus can invade host cells. A seasonal epidemic of influenza affects one billion people, severely affects three to five million people, and kills three to five hundred thousand people (Non Patent Literature 1).

Vaccines that are used for prevention of seasonal influenza and that contain HA as a main component include chicken egg-derived inactivation split influenza vaccines, cultured cell-derived inactivation split influenza vaccines, and recombinant HA vaccines (Non Patent Literature 2).

The vaccine induces an antibody response mainly against immunodominant regions of HA, but a virus can continuously change the antigenicity of HA by mutation (antigen drift) to readily evade a protective immune response of a host (Non Patent Literature 3).

A seasonal influenza vaccine is produced from a recommended strain that is annually announced by the World Health Organization (WHO), or a virus strain similar in antigenicity to the recommended strain. If the antigenicity of the vaccine strain is matched with the epidemic strain, the vaccine has a moderate efficacy ratio, otherwise the vaccine has a low efficacy ratio (Non Patent Literature 4). Analysis of the antigenicity of a vaccine strain using ferret standard antiserum has shown that with respect to the efficacy ratio of the vaccine for the type-A influenza H3N2 subtype virus, the vaccine virus strain acquires mutations in HA for efficiently proliferating in chicken egg, and as a result, the antigenicity against the endemic strain decreases (Non Patent Literature 5). For this reason, development of a more effective seasonal influenza vaccine is required.

In recent years, lipid nanoparticles (LNPs) comprising mRNA encoding HA (LNP-mRNA) have been reported as a seasonal influenza vaccine having a novel action mechanism (for example, Non Patent Literature 6, and Patent Literatures 1 and 2).

### Citation List

#### Patent Literature

Patent Literature 1: International Publication No. WO 2015/164674
Patent Literature 2: International Publication No. WO 2018/078053

#### Non Patent Literature

Non Patent Literature 1: Nature Reviews Disease Primers, 4, Article number: 3, 2018.
Non Patent Literature 2: American Family Physician, 102(8), 505-507, 2020.
Non Patent Literature 3: Nature Reviews Drug Discovery, 14, 167-182, 2015.
Non Patent Literature 4: PLOS ONE, 12(1), e0169528, 2017.
Non Patent Literature 5: The New England Journal of Medicine, 378, 7-9, 2018.
Non Patent Literature 6: Nature Communications, 9, Article number: 3361, 2018.

### Summary of Invention

#### Technical Problem

None of conventional influenza vaccines is satisfactory from the standpoint of side effects and efficacy. In the

case of quadrivalent split vaccines, vaccines that are most commonly used today, it is known that if the vaccine is matched with the antigenicity of the endemic virus strain, a moderate efficacy ratio is exhibited, otherwise the efficacy ratio significantly decreases. Thus, weakness in immunogenicity, high dependency on prediction of an endemic virus strain, and vulnerability to mutation of an influenza virus are the problems. Lipid particles encapsulating a nucleic acid capable of expressing HA of an influenza virus according to the present invention can efficiently express HA in cells of an individual recipient, and exhibit a high prophylactic effect and/or therapeutic effect by activating the immune system. Further, the lipid particles are also effective against influenza viruses which are not matched in antigenicity, and mutant viruses.

An object of the present invention is to provide an effective vaccine for preventing and/or treating infection with an influenza virus as described above.

Solution to problem

The present inventors have found that lipid particles encapsulating a nucleic acid capable of expressing HA of an influenza virus solve the above-described problems, leading to completion of the present invention.

That is, the present invention relates to the following [1] to [56].

[1] A lipid particle encapsulating a nucleic acid capable of expressing a haemagglutinin (HA) protein of an influenza virus, wherein a lipid comprises a cationic lipid having general formula (Ia), or a pharmaceutically acceptable salt thereof:

[Formula 1]

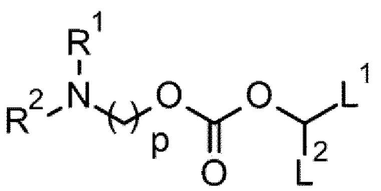

(Ia)

wherein

$R^1$ and $R^2$ each independently represent a C1-C3 alkyl group;
$L^1$ represents a C17-C19 alkenyl group optionally having one or more C2-C4 alkanoyloxy groups;
$L^2$ represents a C10-C19 alkyl group optionally having one or more C2-C4 alkanoyloxy groups, or a C10-C19 alkenyl group optionally having one or more C2-C4 alkanoyloxy groups; and
p is 3 or 4.

[2] The particle according to [1], wherein each of $R^1$ and $R^2$ in general formula (Ia) is a methyl group.
[3] The particle according to [1] or [2], wherein p in general formula (Ia) is 3.
[4] The particle according to any one of [1] to [3], wherein $L^1$ in general formula (Ia) is a C17-C19 alkenyl group optionally having one or more acetyloxy groups.
[5] The particle according to any one of [1] to [4], wherein $L^2$ in general formula (Ia) is a C10-C12 alkyl group optionally having one or more acetyloxy groups, or a C10-C19 alkenyl group optionally having one or more acetyloxy groups.
[6] The particle according to any one of [1] to [4], wherein $L^2$ in general formula (Ia) is a C10-C12 alkyl group optionally having one or more acetyloxy groups, or a C17-C19 alkenyl group optionally having one or more acetyloxy groups.
[7] The particle according to any one of [1] to [6], wherein $L^1$ in general formula (Ia) is a (R)-11-acetyloxy-cis-8-heptadecenyl group, a cis-8-heptadecenyl group, or a (8Z,11Z)-heptadecadienyl group.
[8] The particle according to any one of [1] to [7], wherein $L^2$ in general formula (Ia) is a decyl group, a cis-7-decenyl group, a dodecyl group, or a (R)-11-acetyloxy-cis-8-heptadecenyl group.
[9] The particle according to [1], wherein the cationic lipid has the following structural formula:

[Formula 2]

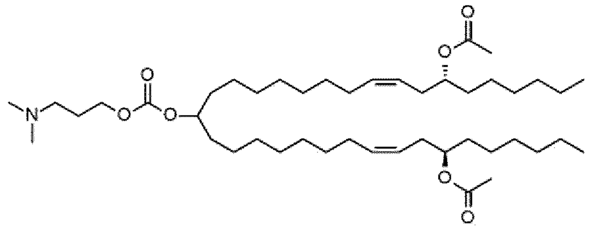

[10] The particle according to [1], wherein the cationic lipid has the following structural formula:

[Formula 3]

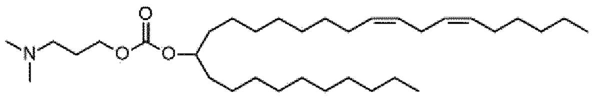

[11] The particle according to [1], wherein the cationic lipid has the following structural formula:

[Formula 4]

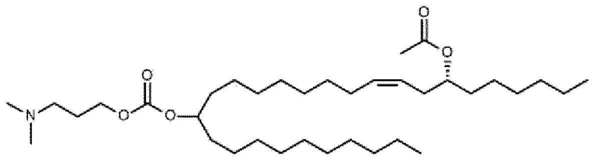

[12] The particle according to [9] or [10], wherein the lipid further comprises an amphipathic lipid, a sterol and a PEG lipid.

[13] The particle according to [11], wherein the lipid further comprises an amphipathic lipid, a sterol and a PEG lipid.

[14] The particle according to [12], wherein the amphipathic lipid is at least one selected from the group consisting of distearoyl phosphatidylcholine, dioleoyl phosphatidylcholine and dioleoyl phosphatidylethanolamine.

[15] The particle according to [13], wherein the amphipathic lipid is at least one selected from the group consisting of distearoyl phosphatidylcholine, dioleoyl phosphatidylcholine and dioleoyl phosphatidylethanolamine.

[16] The particle according to [12] or [14], wherein the sterol is cholesterol.

[17] The particle according to [13] or [15], wherein the sterol is cholesterol.

[18] The particle according to any one of [12], [14] and [16], wherein the PEG lipid is 1,2-dimyristoyl-sn-glycelol methoxypolyethylene glycol and/or N-[methoxy poly(ethylene glycol) 2000]carbamoyl]-1,2-dimyristyloxypropyl-3-amine.

[19] The particle according to any one of [13], [15] and [17], wherein the PEG lipid is 1,2-dimyristoyl-sn-glycelol methoxypolyethylene glycol and/or N-[methoxy poly(ethylene glycol) 2000]carbamoyl]-1,2-dimyristyloxypropyl-3-amine.

[20] The particle according to any one of [12] to [19], wherein the lipid composition of the amphipathic lipid, the sterol, the cationic lipid and the PEG lipid is amphipathic lipid: 5 to 25%, sterol: 10 to 55%, cationic lipid: 40 to 65% and PEG lipid: 1 to 5% on a molar amount basis.

[21] The particle according to [20], wherein the proportion of the amphipathic lipid is 10 to 25%.

[22] The particle according to any one of [12], [14], [16] and [18], wherein the lipid composition of the amphipathic lipid, the sterol, the cationic lipid and the PEG lipid is amphipathic lipid: 5 to 15%, sterol: 35 to 50%, cationic lipid: 40 to 55% and PEG lipid: 1 to 3% on a molar amount basis.

[23] The particle according to [22], wherein the proportions of the amphipathic lipid, the sterol, the cationic lipid and the PEG lipid are 10 to 15%, 35 to 45%, 40 to 50% and 1 to 2.5%, respectively.

[24] The particle according to [23], wherein the proportion of the PEG lipid is 1 to 2%.

[25] The particle according to any one of [13], [15], [17] and [19], wherein the lipid composition of the amphipathic lipid, the sterol, the cationic lipid and the PEG lipid is amphipathic lipid: 10 to 25%, sterol: 10 to 50%, cationic lipid: 40 to 65% and PEG lipid: 1 to 3% on a molar amount basis.

[26] The particle according to [25], wherein the proportions of the sterol, the cationic lipid and the PEG lipid are 10

to 45%, 42.5 to 65% and 1 to 2.5%, respectively.

[27] The particle according to [26], wherein the proportion of the PEG lipid is 1 to 2%.

[28] The particle according to any one of [20] to [27], wherein the ratio of the total weight of lipids to the weight of the nucleic acid is from 15 to 30.

[29] The particle according to [28], wherein the ratio of the total weight of lipids to the weight of the nucleic acid is from 15 to 25.

[30] The particle according to [29], wherein the ratio of the total weight of lipids to the weight of the nucleic acid is from 17.5 to 22.5.

[31] The particle according to any one of [1] to [30], wherein the HA protein of the influenza virus is a fusion protein having an amino acid sequence in which two or more different HA proteins are bound to each other by a linker.

[32] The particle according to [31], wherein the linker has a sequence comprising a protease cleavage site.

[33] The particle according to any one of [1] to [32], wherein the influenza virus is a type-A or type-B influenza virus.

[34] The particle according to any one of [1] to [33], wherein the influenza virus is a type-A or type-B influenza virus, and the HA protein comprises an amino acid sequence having an identity of at least 85% with one amino acid sequence selected from the group consisting of SEQ ID NOS: 20 to 24, 50, 54 and 58.

[35] The particle according to [34], wherein the influenza virus is a type-A or type-B influenza virus, and the HA protein comprises an amino acid sequence having an identity of at least 90% with one amino acid sequence selected from the group consisting of SEQ ID NOS: 20 to 24, 50, 54 and 58.

[36] The particle according to any one of [31] to [35], wherein the nucleic acid capable of expressing a HA protein of an influenza virus is an mRNA comprising a cap structure (Cap), a 5' untranslated region (5'-UTR), a translated region of a HA protein, a 3' untranslated region (3'-UTR) and a poly A tail (polyA).

[37] The particle according to [36], wherein the sequence of the nucleic acid capable of expressing a HA protein consists of a nucleotide sequence having an identity of at least 90% with any of the sequences of SEQ ID NOS: 2, 7, 10, 13, 16, 19, 38 to 49 and 53.

[38] The particle according to any one of [31] to [35], wherein the nucleic acid capable of expressing a HA protein of an influenza virus is an mRNA having a structure comprising a cap structure (Cap), a 5' untranslated region (5'-UTR), a translated region of a HA protein and a 3' untranslated region (3'-UTR).

[39] The particle according to [38], wherein the structure comprising a cap structure (Cap), a 5' untranslated region (5'-UTR), a translated region of a HA protein and a 3' untranslated region (3'-UTR) consists of a nucleotide sequence having an identity of at least 90% with any of SEQ ID NO: 2, the sequence of residues 1 to 1900 in SEQ ID NO: 7, the sequence of residues 1 to 1903 in SEQ ID NO: 10, the sequence of residues 1 to 1903 in SEQ ID NO: 13, the sequence of residues 1 to 1957 in SEQ ID NO: 16, the sequence of residues 1 to 1954 in SEQ ID NO: 19, the sequence of residues 1 to 1903 in SEQ ID NO: 38, the sequence of residues 1 to 1957 in SEQ ID NO: 44 and the sequence of residues 1 to 1906 in SEQ ID NO: 53.

[40] The particle according to any one of [1] to [39], wherein the nucleic acid comprises at least one modified nucleotide.

[41] The particle according to [40], wherein the modified nucleotide comprises at least one of pyrimidine nucleotide substituted at the 5-position and/or pseudouridine optionally substituted at the 1-position.

[42] The particle according to [41], wherein the modified nucleotide comprises at least one selected from the group consisting of 5-methylcytidine, 5-methoxyuridine, 5-methyluridine, pseudouridine and 1-alkylpseudouridine.

[43] The particle according to any one of [1] to [42], wherein the average particle size of the particles is 30 to 300 nm.

[44] The particle according to any one of [1] to [43], comprising two or more nucleic acids capable of expressing different HA proteins in one lipid particle.

[45] Use of the particle according to any one of [1] to [44], for producing a composition for preventing and/or treating infection with an influenza virus.

[46] A composition comprising the particle according to any one of [1] to [44].

[47] A composition comprising two or more types of the particles according to any of [1] to [46], which are capable of expressing different HA proteins.

[48] The composition according to [46] or [47], for expressing a HA protein of an influenza virus *in vivo* or *in vitro.*

[49] The composition according to [46] to [48] for use as a medicament.

[50] The composition according to [49] for inducing an immune reaction against an influenza virus.

[51] The composition according to [49] or [50] for preventing and/or treating infection with an influenza virus.

[52] A method for expressing a HA protein of an influenza virus in *vitro,* comprising introducing the composition according to any one of [46] to [48] into cells.

[53] A method for expressing a HA protein of an influenza virus *in vivo,* comprising administering the composition according to any one of [46] to [51] to a mammal.

[54] A method for inducing an immune reaction against an influenza virus, comprising administering the composition according to [49] or [50] to a mammal.

[55] A method for preventing and/or treating infection with an influenza virus, comprising administering the composition according to any one of [49] to [51] to a mammal.
[56] The method according to any one of [53] to [55], wherein the mammal is a human.

Advantageous Effects of Invention

The present invention provides lipid particles that enable prevention and/or treatment of infection by an influenza virus. By the present invention, influenza can be prevented and/or treated. The particles of the present invention have excellent properties in terms of efficacy against influenza viruses which are not matched in the amount of antigen expression or antigenicity, and mutant viruses, metabolic stability, *in vitro* activity, *in vivo* activity, rapidity of onset of a drug effect, persistence of a drug effect, physical stability, drug interaction, safety and the like, and are useful as a medicament for preventing and/or treating influenza.

Brief Description of Drawings

[Figure 1] Figure 1 is a diagram obtained by evaluating the ability to induce production of IgG specific to HA of A/Puerto Rico/8/34 (H1 subtype) by the lipid particles of the present invention.
[Figure 2] Figure 2 is a diagram obtained by evaluating the protective effect as measured by virus titer in the lung as an indicator against challenge infection with A/Puerto Rico/8/34 by the lipid particles of the present invention.
[Figure 3] Figure 3 is a diagram obtained by evaluating the life-extending effect and the protective effect as measured by body weight change as an indicator against challenge infection with A/Puerto Rico/8/34 by the lipid particles of the present invention.
[Figure 4] Figure 4 is a diagram obtained by evaluating the life-extending effect and the protective effect as measured by body weight change as an indicator against challenge infection with A/Puerto Rico/8/34 by the lipid particles of the present invention.
[Figure 5] Figure 5 is a diagram obtained by evaluating the ability to produce a T cell cytokine specific to HA of A/Puerto Rico/8/34 by the lipid particles of the present invention.
[Figure 6] Figure 6 is a diagram obtained by evaluating the ability to produce a T cell cytokine specific to HA of A/Puerto Rico/8/34 by the lipid particles of the present invention.
[Figure 7] Figure 7 is a diagram obtained by evaluating the ability to produce a CD4-positive or CD8-positive T cell cytokine specific to HA of A/Puerto Rico/8/34 by the lipid particles of the present invention.
[Figure 8] Figure 8 is a diagram obtained by evaluating the ability to produce a CD4-positive or CD8-positive T cell cytokine specific to HA of A/Puerto Rico/8/34 by the lipid particles of the present invention.
[Figure 9] Figure 9 is a diagram obtained by evaluating the ability to induce production of IgG specific to HA after administration of the lipid particles of the present invention to mice infected with A/Puerto Rico/8/34.
[Figure 10] Figure 10 is a diagram obtained by evaluating the ability to induce production of a HI antibody after administration of the lipid particles of the present invention to mice infected with A/Puerto Rico/8/34.
[Figure 11] Figure 11 is a diagram obtained by evaluating the ability to induce production of a HI antibody against A/Singapore/GP1908/2015 (H1 subtype) by the lipid particles of the present invention.
[Figure 12] Figure 12 is a diagram obtained by evaluating the ability to induce production of a HI antibody against A/Singapore/INFIMH-16-0019/2016 (H3 subtype) by the lipid particles of the present invention.
[Figure 13] Figure 13 is a diagram obtained by evaluating the ability to induce production of a HI antibody against B/Phuket/3073/2013 (Yamagata lineage) by the lipid particles of the present invention.
[Figure 14] Figure 14 is a diagram obtained by evaluating the ability to induce production of a HI antibody against B/Maryland/15/2016 (Victoria lineage) by the lipid particles of the present invention.
[Figure 15] Figure 15 is a diagram obtained by evaluating the ability to induce production of an antibody against a H3 subtype antigen drift strain HA by the particles of the present invention.
[Figure 16] Figure 16 is a diagram obtained by evaluating the ability to induce production of an antibody against a H3 subtype antigen drift strain HA by the particles of the present invention.
[Figure 17] Figure 17 is a diagram obtained by evaluating the protective effect as measured by virus titer in the lung as an indicator against challenge infection with a H3 subtype antigen drift strain A/Guizhou/54/89 by the particles of the present invention.
[Figure 18] Figure 18 is a diagram obtained by evaluating the ability to induce production of a HI antibody against A/Singapore/GP1908/2015 by the particles of the present invention (monovalent vaccine or quadrivalent vaccine).
[Figure 19] Figure 19 is a diagram evaluating the ability to induce production of a HI antibody against A/Singapore/INFIMH-16-0019/2016 by the particles of the present invention (monovalent vaccine or quadrivalent vaccine).

[Figure 20] Figure 20 is a diagram obtained by evaluating the ability to induce production of a HI antibody against B/Phuket/3073/2013 by the particles of the present invention (monovalent vaccine or quadrivalent vaccine).

[Figure 21] Figure 21 is a diagram obtained by evaluating the ability to induce production of a HI antibody against B/Maryland/15/2016 by the particles of the present invention (monovalent vaccine or quadrivalent vaccine).

[Figure 22] Figure 22 is a diagram obtained by evaluating the ability to induce production of a HI antibody against A/Singapore/GP1908/2015 by the lipid particles of the present invention (quadrivalent vaccine) which are administered through different routes.

[Figure 23] Figure 23 is a diagram obtained by evaluating the ability to induce production of a HI antibody against A/Singapore/INFIMH-16-0019/2016 by the lipid particles of the present invention (quadrivalent vaccine) which are administered through different routes.

[Figure 24] Figure 24 is a diagram obtained by evaluating the ability to induce production of a HI antibody against B/Phuket/3073/2013 by the lipid particles of the present invention (quadrivalent vaccine) which are administered through different routes.

[Figure 25] Figure 25 is a diagram obtained by evaluating the ability to induce production of a HI antibody against B/Maryland/15/2016 by the lipid particles of the present invention (quadrivalent vaccine) which are administered through different routes.

[Figure 26] Figure 26 is a diagram obtained by evaluating the ability to induce production of a HI antibody against Singapore/GP1908/2015 by the lipid particles of the present invention which contain different cationic lipids.

[Figure 27] Figure 27 is a diagram obtained by evaluating the ability to induce production of IgG specific to HA of A/Michigan/45/2015 by the lipid particles of the present invention which contain different cationic lipids.

[Figure 28] Figure 28 is a diagram obtained by evaluating the ability to induce production of IgG specific to HA of A/Michigan/45/2015 by the lipid particles of the present invention which have different lipid compositions.

[Figure 29] Figure 29 is a diagram obtained by evaluating the ability to induce production of IgG specific to HA of A/Michigan/45/2015 by the lipid particles of the present invention which have different lipid compositions.

[Figure 30] Figure 30 is a diagram obtained by evaluating the ability to induce production of IgG specific to HA of A/Michigan/45/2015 by the lipid particles of the present invention which have different lipid compositions.

[Figure 31] Figure 31 is a diagram obtained by evaluating the ability to induce production of IgG specific to HA of A/Puerto Rico/8/34 by the lipid particles of the present invention with mRNAs having different chemically modified bases.

[Figure 32] Figure 32 is a diagram obtained by evaluating the protective effect as measured by virus titer in the lung as an indicator against challenge infection with A/Puerto Rico/8/34 by the lipid particles of the present invention with mRNAs having different chemically modified bases.

[Figure 33] Figure 33 is a diagram obtained by evaluating the life-extending effect and the protective effect as measured by body weight change as an indicator against challenge infection with A/Puerto Rico/8/34 by the lipid particles of the present invention with mRNAs having different chemically modified bases.

[Figure 34] Figure 34 is a diagram obtained by evaluating the life-extending effect and the protective effect as measured by body weight change as an indicator against challenge infection with A/Puerto Rico/8/34 by the lipid particles of the present invention with mRNAs having different chemically modified bases.

[Figure 35] Figure 35 is a diagram obtained by evaluating the immunogenicity and the vaccine interference of quadrivalent vaccines by the lipid particles of the present invention with mRNAs having different chemically modified bases.

[Figure 36] Figure 36 is a diagram obtained by evaluating the immunogenicity and the vaccine interference of quadrivalent vaccines by the lipid particles of the present invention with mRNAs having different chemically modified bases.

[Figure 37] Figure 37 is a diagram obtained by evaluating the immunogenicity and the vaccine interference of quadrivalent vaccines by the lipid particles of the present invention with mRNAs having different chemically modified bases.

[Figure 38] Figure 38 is a diagram obtained by evaluating the immunogenicity and the vaccine interference of quadrivalent vaccines by the lipid particles of the present invention with mRNAs having different chemically modified bases.

[Figure 39] Figure 39 shows a nucleotide sequence of template DNA for IVT of A/Puerto Rico/8/34 (H1 subtype) HA mRNA-001 (SEQ ID NO: 1).

[Figure 40] Figure 40 shows a nucleotide sequence of A/Puerto Rico/8/34 (H1 subtype) HA mRNA-001 (SEQ ID NO: 2) .

[Figure 41] Figure 41 shows a nucleotide sequence of a DNA fragment containing A/Puerto Rico/8/34 (H1 subtype) HA (SEQ ID NO: 3).

[Figure 42] Figure 42 shows nucleotide sequences of a sense primer (SEQ ID NO: 4) and an antisense primer (SEQ ID NO: 5).

[Figure 43] Figure 43 shows a nucleotide sequence of template DNA of A/Puerto Rico/8/34 (H1 subtype) HA (SEQ ID NO: 6).

[Figure 44] Figure 44 shows a nucleotide sequence of A/Puerto Rico/8/34 (H1 subtype) HA mRNA-002 and 003 (SEQ ID NO: 7).

[Figure 45] Figure 45 shows a nucleotide sequence of a DNA fragment containing A/Singapore/GP1908/2015 (H1 subtype) HA (SEQ ID NO: 8).

[Figure 46] Figure 46 shows a nucleotide sequence of template DNA of A/Singapore/GP1908/2015 (H1 subtype) HA (SEQ ID NO: 9).

[Figure 47] Figure 47 shows a nucleotide sequence of A/Singapore/GP1908/2015 (H1 subtype) HA mRNA-001, 002 and 003 (SEQ ID NO: 10).

[Figure 48] Figure 48 shows a nucleotide sequence of a DNA fragment containing A/Singapore/INFIMH-16-0019/2016 (H3 subtype) HA (SEQ ID NO: 11).

[Figure 49] Figure 49 shows a nucleotide sequence of template DNA of A/Singapore/INFIMH-16-0019/2016 (H3 subtype) HA (SEQ ID NO: 12).

[Figure 50] Figure 50 shows a nucleotide sequence of A/Singapore/INFIMH-16-0019/2016 (H3 subtype) HA mRNA-001 and 002 (SEQ ID NO: 13).

[Figure 51] Figure 51 shows a nucleotide sequence of a DNA fragment containing B/Phuket/3073/2013 (Yamagata lineage) HA (SEQ ID NO: 14).

[Figure 52] Figure 52 shows a nucleotide sequence of template DNA of B/Phuket/3073/2013 (Yamagata lineage) HA (SEQ ID NO: 15).

[Figure 53] Figure 53 shows a nucleotide sequence of B/Phuket/3073/2013 (Yamagata lineage) HA mRNA-001 and 002 (SEQ ID NO: 16).

[Figure 54] Figure 54 shows a nucleotide sequence of a DNA fragment containing B/Maryland/15/2016 (Victoria lineage) HA (SEQ ID NO: 17).

[Figure 55] Figure 55 shows a nucleotide sequence of template DNA of B/Maryland/15/2016 (Victoria lineage) HA (SEQ ID NO: 18).

[Figure 56] Figure 56 shows a nucleotide sequence of B/Maryland/15/2016 (Victoria lineage) HA mRNA-001 and 002 (SEQ ID NO: 19).

[Figure 57] Figure 57 shows an amino acid sequence of A/Puerto Rico/8/34 (H1 subtype) HA (SEQ ID NO: 20).

[Figure 58] Figure 58 shows an amino acid sequence of A/Singapore/GP1908/2015 (H1 subtype) HA (SEQ ID NO: 21).

[Figure 59] Figure 59 shows an amino acid sequence of A/Singapore/INFIMH-16-0019/2016 (H3 subtype) HA (SEQ ID NO: 22).

[Figure 60] Figure 60 shows an amino acid sequence of B/Phuket/3,073/2013 (Yamagata lineage) HA (SEQ ID NO: 23) .

[Figure 61] Figure 61 shows an amino acid sequence of B/Maryland/15/2016 (Victoria lineage) HA (SEQ ID NO: 24).

[Figure 62] Figure 62 shows an amino acid sequence of a protease cleavage sequence (SEQ ID NO: 25).

[Figure 63] Figure 63 shows a template DNA sequence of A/Guangdong-Maonan/SWL 1536/2019, polyA110 (SEQ ID NO: 26) .

[Figure 64] Figure 64 shows a template DNA sequence of A/Guangdong-Maonan/SWL 1536/2019, polyA95 (SEQ ID NO: 27) .

[Figure 65] Figure 65 shows a template DNA sequence of A/Guangdong-Maonan/SWL 1536/2019, polyA80 (SEQ ID NO: 28) .

[Figure 66] Figure 66 shows a template DNA sequence of A/Guangdong-Maonan/SWL 1536/2019, polyA60 (SEQ ID NO: 29) .

[Figure 67] Figure 67 shows a template DNA sequence of A/Guangdong-Maonan/SWL 1536/2019, polyA40 (SEQ ID NO: 30) .

[Figure 68] Figure 68 shows a template DNA sequence of A/Guangdong-Maonan/SWL 1536/2019, polyA20 (SEQ ID NO: 31) .

[Figure 69] Figure 69 shows a template DNA sequence of B/Phuket/3073/2013 (Yamagata lineage), polyA110 (SEQ ID NO: 32).

[Figure 70] Figure 70 shows a template DNA sequence of B/Phuket/3073/2013 (Yamagata lineage), polyA95 (SEQ ID NO: 33).

[Figure 71] Figure 71 shows a template DNA sequence of B/Phuket/3073/2013 (Yamagata lineage), polyA80 (SEQ ID NO: 34).

[Figure 72] Figure 72 shows a template DNA sequence of B/Phuket/3073/2013 (Yamagata lineage), polyA60 (SEQ ID NO: 35).

[Figure 73] Figure 73 shows a template DNA sequence of B/Phuket/3073/2013 (Yamagata lineage), polyA40 (SEQ

ID NO: 36).

[Figure 74] Figure 74 shows a template DNA sequence of B/Phuket/3073/2013 (Yamagata lineage), polyA20 (SEQ ID NO: 37).

[Figure 75] Figure 75 shows an mRNA sequence of A/Guangdong-Maonan/SWL 1536/2019, polyA110 (SEQ ID NO: 38) .

[Figure 76] Figure 76 shows an mRNA sequence of A/Guangdong-Maonan/SWL 1536/2019, polyA95 (SEQ ID NO: 39) .

[Figure 77] Figure 77 shows an mRNA sequence of A/Guangdong-Maonan/SWL 1536/2019, polyA80 (SEQ ID NO: 40) .

[Figure 78] Figure 78 shows an mRNA sequence of A/Guangdong-Maonan/SWL 1536/2019, polyA60 (SEQ ID NO: 41) .

[Figure 79] Figure 79 shows an mRNA sequence of A/Guangdong-Maonan/SWL 1536/2019, polyA40 (SEQ ID NO: 42) .

[Figure 80] Figure 80 shows an mRNA sequence of A/Guangdong-Maonan/SWL 1536/2019, polyA20 (SEQ ID NO: 43) .

[Figure 81] Figure 81 shows an mRNA sequence of B/Phuket/3073/2013 (Yamagata lineage), polyA110 (SEQ ID NO: 44).

[Figure 82] Figure 82 shows an mRNA sequence of B/Phuket/3073/2013 (Yamagata lineage), polyA95 (SEQ ID NO: 45).

[Figure 83] Figure 83 shows an mRNA sequence of B/Phuket/3073/2013 (Yamagata lineage), polyA80 (SEQ ID NO: 46) .

[Figure 84] Figure 84 shows an mRNA sequence of B/Phuket/3073/2013 (Yamagata lineage), polyA60 (SEQ ID NO: 47).

[Figure 85] Figure 85 shows an mRNA sequence of B/Phuket/3073/2013 (Yamagata lineage), polyA40 (SEQ ID NO: 48).

[Figure 86] Figure 86 shows an mRNA sequence of B/Phuket/3073/2013 (Yamagata lineage), polyA20 (SEQ ID NO: 49) .

[Figure 87] Figure 87 shows an amino acid sequence of A/Guangdong-Maonan/SWL 1536/2019 HA (SEQ ID NO: 50).

[Figure 88] Figure 88 shows DNA containing A/Astrakhan/3212/2020 (H5 subtype) HA (SEQ ID NO: 51).

[Figure 89] Figure 89 shows template DNA of A/Astrakhan/3212/2020 (H5 subtype) HA (SEQ ID NO: 52).

[Figure 90] Figure 90 shows A/Astrakhan/3212/2020 (H5 subtype) HA mRNA-001 (SEQ ID NO: 53).

[Figure 91] Figure 91 shows an amino acid sequence of A/Astrakhan/3212/2020 (H5 subtype) HA (SEQ ID NO: 54).

[Figure 92] Figure 92 shows a sequence in which a KOZAK sequence and an A/Astrakhan/3212/2020 (H5N8) HA gene translated region are connected (SEQ ID NO: 55).

[Figure 93] Figure 93 shows a sequence in which a KOZAK sequence and an A/Laos/2121/2020 (H5N1) HA gene translated region are connected (SEQ ID NO: 56).

[Figure 94] Figure 94 shows a base sequence of a translated region of A/Laos/2121/2020 (H5N1) HA (SEQ ID NO: 57).

[Figure 95] Figure 95 shows an amino acid sequence of a translated region of A/Laos/2121/2020 (H5N1) HA (SEQ ID NO: 58).

[Figure 96] Figure 96 shows the results of detecting HA (A/Guangdong-Maonan/SWL 1536/2019), which is expressed in cells to which the lipid particles of the present invention are applied, by an ELISA method.

[Figure 97] Figure 97 shows the result of measuring the neutralization antibody titer of an antibody induced by the lipid particles of the present invention using a microneutralization assay (MN) method using a pseudovirus.

## Description of Embodiments

Hereinafter, the present invention will be described in detail.

The terms used herein are described below.

The "influenza virus" is classified into the four types of type A, type B, type C and type D by a difference in antigenicity between a virus nucleoprotein (NP) and a structural protein (M1). The term "influenza virus" as used herein denotes all these types of viruses.

The term "influenza" denotes an infection with any of the above-described viruses.

The term "haemagglutinin (HA)" denotes an antigenic glycoprotein present on the surface of an influenza virus. For the type-A influenza virus, HA is classified into the 18 subtypes of H1 to H18, and for the type-B influenza virus, the two lineages of Victoria lineage and Yamagata lineage are known. As vaccine antigens for humans, H1 to H9, Victoria lineage and Yamagata lineage are preferable, H1 to H3, H5, H7, H9, Victoria lineage and Yamagata lineage are more preferable, H1, H3, Victoria lineage and Yamagata lineage are further more preferable.

The term "lipid particle" as used herein denotes a particle comprising an amphipathic lipid, a sterol, a cationic

lipid and a PEG lipid as a constituent lipid.

The term "capable of expressing" as used herein means that a target protein can be produced in cells *in vitro* or *in vivo*.

The term "C1-C3 alkyl group" denotes a linear or branched alkyl group having 1 to 3 carbon atoms. Examples thereof include a methyl group, an ethyl group, a propyl group, and an isopropyl group.

The term "C2-C4 alkanoyl group" denotes an alkanoyl group having 2 to 4 carbon atoms. Examples thereof include an acetyl group, a propionyl group, a butyryl group, and an isobutyryl group.

The term "C2-C4 alkanoyloxy group" denotes a group in which the C2-C4 alkanoyl group is bonded to an oxygen atom. Examples thereof include an acetyloxy group, a propionyloxy group, a butyryloxy group, and an isobutyryloxy group.

The term "C17-C19 alkenyl group" denotes a linear or branched alkenyl group having 17 to 19 carbon atoms. The C17-C19 alkenyl group herein includes all of a C17-C19 alkadienyl group, a C17-C19 alkatrienyl group and a C17-C19 alkatetraenyl group. Examples thereof include a heptadecenyl group, an octadecenyl group, a nonadecenyl group, a heptadecadienyl group, an octadecadienyl group, a nonadecadienyl group, a heptadecatrienyl group, an octadecatrienyl group, and a nonadecatrienyl group.

The term "C17-C19 alkenyl group optionally having one or more C2-C4 alkanoyloxy groups" denotes a group in which a hydrogen atom at any position on the C17-C19 alkenyl group is replaced with the C2-C4 alkanoyloxy group. Examples thereof include a 11-acetyloxy-8-heptadecenyl group, and a 11-propionyloxy-8-heptadecenyl group.

The term "C10-C19 alkyl group" denotes a linear or branched alkyl group having 10 to 19 carbon atoms. Examples thereof include a decyl group, an undecyl group, a dodecyl group, a tridecyl group, a tetradecyl group, a pentadecyl group, a hexadecyl group, a heptadecyl group, an octadecyl group, a nonadecyl group.

The term "C10-C19 alkyl group optionally having one or more C2-C4 alkanoyloxy groups" denotes a group in which a hydrogen atom at any position on the C10-C19 alkyl group is replaced with the C2-C4 alkanoyloxy group.

The term "C10-C19 alkenyl group" denotes a linear or branched alkenyl group having 10 to 19 carbon atoms. The C10-C19 alkenyl group herein includes all of a C10-C19 alkadienyl group, a C10-C19 alkatrienyl group and a C10-C19 alkatetraenyl group. Examples thereof include a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, a decadienyl group, an undecadienyl group, a dodecadienyl group, a tridecadienyl group, a tetradecadienyl group, a pentadecadienyl group, a hexadecadienyl group, a heptadecadienyl group, an octadecadienyl group, a nonadecadienyl group, a decatrienyl group, an undecatrienyl group, a dodecatrienyl group, a tridecatrienyl group, a tetradecatrienyl group, a pentadecatrienyl group, a hexadecatrienyl group, a heptadecatrienyl group, an octadecatrienyl group, and a nonadecatrienyl group.

The term "C10-C19 alkenyl group optionally having one or more C2-C4 alkanoyloxy groups" denotes a group in which a hydrogen atom at any position on the C10-C19 alkenyl group is replaced with the C2-C4 alkanoyloxy group. Examples thereof include a 11-acetyloxy-8-heptadecenyl group, and a 11-propionyloxy-8-heptadecenyl group.

The term "C17-C19 alkenyl group optionally having one or more acetyloxy groups" denotes a group in which a hydrogen atom at any position on the C17-C19 alkenyl group is replaced with an acetyloxy group. Examples thereof include a 11-acetyloxy-8-heptadecenyl group, and a 11-propionyloxy-8-heptadecenyl group.

The term "C10-C12 alkyl group optionally having one or more acetyloxy groups" denotes a group in which a hydrogen atom at any position on the C10-C12 alkyl group is replaced with an acetyloxy group.

The term "C10-C19 alkenyl group optionally having one or more acetyloxy groups" denotes a group in which a hydrogen atom at any position on the C10-C19 alkenyl group is replaced with an acetyloxy group. Examples thereof include a 11-acetyloxy-8-heptadecenyl group, and a 11-propionyloxy-8-heptadecenyl group.

The term "treatment" as used herein means that in a patient having developed an infection with a virus, bacteria or the like, or a disease caused by the infection (for example, influenza, precancerous lesion or cancer), the clinical symptoms of such a disease are cured, caused to remit, alleviated and/or delayed from worsening.

The term "prevention" as used herein means reducing incidence of a disease caused by an infection with a virus, bacteria or the like. The prevention includes lowering the risk of progression of a disease caused by an infection with a virus, bacteria or the like, or reducing aggravation of such a disease. The particles of the present invention are effective in prevention and/or treatment of the disease because they induce a preventive immune reaction.

The term "identity" as used herein refers to a relationship between two or more nucleotide sequences or amino acid sequences which is determined by comparison between the sequences as known in the art. In the art, the identity also means, as the case may be, a degree of sequence relatedness between nucleic acid molecules or polypeptides when determined by a match between two or more nucleotide sequences or two or more amino acid sequences in one row. The identity can be evaluated by calculating a percentage of the exact match between the smallest of two or more sequences and a gap alignment (if present) addressed by a specific mathematical model or computer program (i.e., an algorithm). Specifically, software such as Clustal W2 provided by European Molecular Biology Laboratory-European Bioinformatics Institute (EMBL-EBI) may be used to evaluate the identity, but tools for calculation of identity are not

limited thereto as long as they are used by those skilled in the art.

The present invention provides lipid particles encapsulating a nucleic acid capable of expressing haemagglutinin (HA) which is an antigen on the surface of an influenza virus, wherein the lipid comprises a cationic lipid having general formula (Ia), or a pharmaceutical acceptable salt thereof.

[Formula 5]

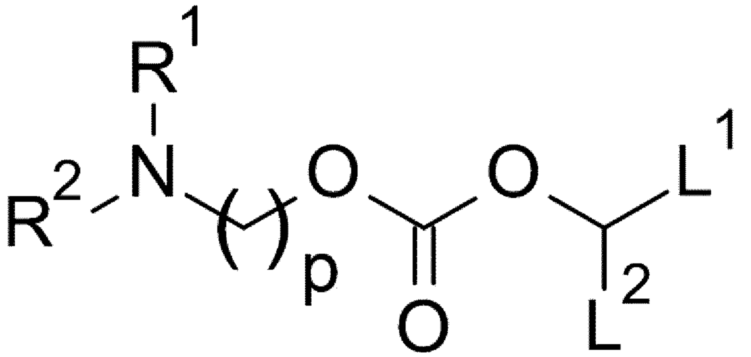

(Ia)

wherein

- $R^1$ and $R^2$ each independently represent a C1-C3 alkyl group;
- $L^1$ represents a C17-C19 alkenyl group optionally having one or more C2-C4 alkanoyloxy groups;
- $L^2$ represents a C10-C19 alkyl group optionally having one or more C2-C4 alkanoyloxy groups, or a C10-C19 alkenyl group optionally having one or more C2-C4 alkanoyloxy groups; and
- p is 3 or 4.

In general formula (Ia), $R^1$ and $R^2$ each independently represent a C1-C3 alkyl group, preferably a methyl group.

In general formula (Ia), p is 3 or 4, preferably 3.

In general formula (Ia), $L^1$ represents a C17-C19 alkenyl group optionally having one or more C2-C4 alkanoyloxy groups, preferably a C17-C19 alkenyl group optionally having one or more acetyloxy groups. Specifically, a (R)-11-acetyloxy-cis-8-heptadecenyl group, a cis-8-heptadecenyl group, a (8Z,11Z)-heptadecadienyl group or the like can be exemplified as L1.

In general formula (Ia), $L^2$ represents a C10-C19 alkyl group optionally having one or more C2-C4 alkanoyloxy groups, or a C10-C19 alkenyl group optionally having one or more C2-C4 alkanoyloxy groups, preferably a C10-C12 alkyl group optionally having one or more acetyloxy groups, or a C10-C19 alkenyl group optionally having one or more acetyloxy groups. It is also preferable that $L^2$ in general formula (Ia) be a C10-C12 alkyl group optionally having one or more acetyloxy groups, or a C17-C19 alkenyl group optionally having one or more acetyloxy groups. Specifically, a decyl group, a cis-7-decenyl group, a dodecyl group, a (R)-11-acetyloxy-cis-8-heptadecenyl group or the like can be exemplified as $L^2$.

Specifically, (7R,9Z,26Z,29R)-18-({[3-(dimethylamino)propoxy]carbonyl}oxy)pentatriaconta-9,26-diene-7,29-diyl diacetate, 3-dimethylaminopropyl(9Z,12Z)-octaoctacosa-19,22-dien-11-yl carbonate, and (7R,9Z)-18-({ [3-(dimethylamino)propyloxy]carbonyl}oxy)octacos-9-en-7-yl acetate having the following structural formulae:

[Formula 6]

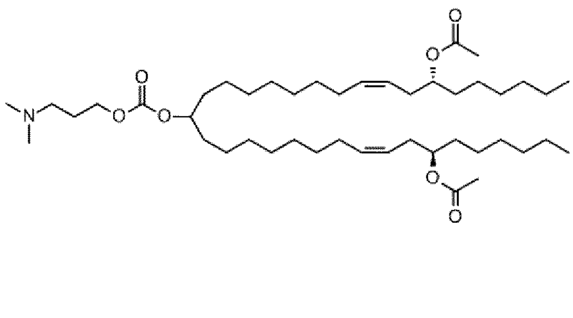

[Formula 7]

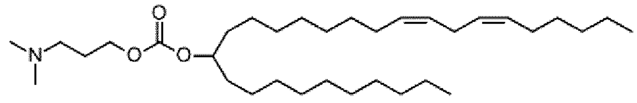

[Formula 8]

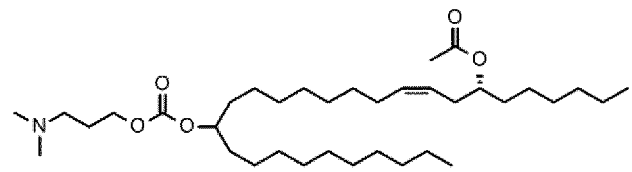

, respectively, are examples of cationic lipids which are components forming the particles of the present invention.

The cationic lipid having general formula (Ia) may be one type of compound, or a combination of two or more types of compounds.

A method for producing a cationic lipid having general formula (Ia) is described in International Publication No. WO 2015/005253.

The lipid according to the present invention may further comprise an amphipathic lipid, a sterol and a PEG lipid.

The amphipathic lipid has affinity for both polar and non-polar solvents, and specifically, distearoyl phosphatidylcholine (DSPC), dioleoyl phosphatidylcholine (DOPC), dioleoyl phosphatidylethanolamine (DOPE), a combination thereof or the like are examples for amphipathic lipids. The amphipathic lipid used for the particles of the present invention is preferably distearoyl phosphatidylcholine and/or dioleoyl phosphatidylethanolamine, preferably distearoyl phosphatidylcholine.

The sterol is a sterol having a hydroxy group, and specifically, cholesterol or the like can be exemplified.

The PEG lipid is a lipid modified with PEG (polyethylene glycol), and specifically, 1,2-dimyristoyl-sn-glycelol methoxypolyethylene glycol and/or N-[methoxy poly(ethylene glycol) 2000]carbamoyl]-1,2-dimyristyloxypropyl-3-amine, a combination thereof, or the like can be exemplified, with 1,2-dimyristoyl-sn-glycelol methoxypolyethylene glycol being preferable. The average molecular weight of PEG lipid is not particularly limited, and is, for example, 1,000 to 5,000, preferably 1,500 to 3,000, more preferably 1,800 to 2,200.

The lipid composition of the amphipathic lipid, the sterol, the cationic lipid and the PEG lipid is not particularly limited, and is, for example, amphipathic lipid: 5 to 25%, sterol: 10 to 55%, cationic lipid: 40 to 65% and PEG lipid: 1 to 5% on a molar amount basis. Preferably, the lipid composition of the amphipathic lipid, the sterol, the cationic lipid and the PEG lipid is amphipathic lipid: 10 to 25%, sterol: 10 to 55%, cationic lipid: 40 to 65% and PEG lipid: 1 to 5% on a molar amount basis. More preferably, the lipid composition of the amphipathic lipid, the sterol, the cationic lipid and the PEG lipid is amphipathic lipid: 10 to 22.5%, sterol: 15 to 55%, cationic lipid: 40 to 65% and PEG lipid: 1 to 5% on a molar amount basis. In the lipid composition, the proportion of the PEG lipid is more preferably 1 to 3%, further more preferably 1 to 2%, further more preferably 1.2 to 2%, further more preferably 1.25 to 2%, further more preferably 1.3 to 2%, further more preferably 1.5 to 2% on a molar amount basis. In the lipid composition, the ratio of the total weight of lipids to the weight of the nucleic acid is not particularly limited, may be from 15 to 30, and is preferably from 15 to 25, more preferably from 15 to 22.5, further more preferably from 17.5 to 22.5.

When 3-dimethylaminopropyl(9Z,12Z)-octaoctacosa-19,22-dien-11-yl carbonate or (7R,9Z,26Z,29R)-18-({[3-(dimethylamino)propoxy]carbonyl}oxy)pentatriaconta-9,26-diene-7,29-diyl diacetate is used as the cationic lipid, the lipid composition of the amphipathic lipid, the sterol, the cationic lipid and the PEG lipid is not particularly limited, and is, for example, amphipathic lipid: 5 to 25%, sterol: 10 to 55%, cationic lipid: 40 to 65% and PEG lipid: 1 to 5%, preferably amphipathic lipid: 5 to 15%, sterol: 20 to 55%, cationic lipid: 40 to 65% and PEG lipid: 1 to 5%, on a molar amount basis. More preferably, the lipid composition of the amphipathic lipid, the sterol, the cationic lipid and the PEG lipid is amphipathic lipid: 5 to 15%, sterol: 35 to 50%, cationic lipid: 40 to 55% and PEG lipid: 1 to 3% on a molar amount basis. Further more preferably, the lipid composition of the amphipathic lipid, the sterol, the cationic lipid and the PEG lipid is amphipathic lipid: 10 to 15%, sterol: 35 to 45%, cationic lipid: 40 to 50% and PEG lipid: 1 to 2.5% on a molar amount basis. Further more preferably, the lipid composition of the amphipathic lipid, the sterol, the cationic lipid and the PEG lipid is amphipathic lipid: 10 to 15%, sterol: 35 to 45%, cationic lipid: 40 to 50% and PEG lipid: 1 to 2% on a molar amount basis. In the lipid composition, the PEG lipid is more preferably 1.2 to 2%, further more preferably 1.25 to 2%, further more preferably 1.3 to 2%, further more preferably 1.5 to 2%. In the lipid composition, the ratio of the total

weight of lipids to the weight of the nucleic acid is not particularly limited, may be from 15 to 30, and is preferably from 15 to 25, more preferably from 15 to 22.5, further more preferably from 17.5 to 22.5.

When (7R,9Z)-18-({ [3-(dimethylamino)propyloxy]carbonyl}oxy)octacos-9-en-7-yl acetate is used as the cationic lipid, the lipid composition of the amphipathic lipid, the sterol, the cationic lipid and the PEG lipid is not particularly limited, and is, for example, amphipathic lipid: 5 to 25%, sterol: 10 to 55%, cationic lipid: 40 to 65% and PEG lipid: 1 to 5%, preferably amphipathic lipid: 10 to 25%, sterol: 10 to 50%, cationic lipid: 40 to 65% and PEG lipid: 1 to 3%, on a molar amount basis. More preferably, the lipid composition of the amphipathic lipid, the sterol, the cationic lipid and the PEG lipid is amphipathic lipid: 10 to 25%, sterol: 10 to 45%, cationic lipid: 42.5 to 65% and PEG lipid: 1 to 2.5% on a molar amount basis. Further more preferably, the lipid composition of the amphipathic lipid, the sterol, the cationic lipid and the PEG lipid is amphipathic lipid: 15 to 22.5%, sterol: 15 to 40%, cationic lipid: 45 to 65% and PEG lipid: 1 to 2% on a molar amount basis. Further more preferably, the lipid composition of the amphipathic lipid, the sterol, the cationic lipid and the PEG lipid is amphipathic lipid: 17.5 to 22.5%, sterol: 15 to 40%, cationic lipid: 45 to 65% and PEG lipid: 1 to 2% on a molar amount basis. In the lipid composition, the PEG lipid is more preferably 1.2 to 2%, further more preferably 1.25 to 2%, further more preferably 1.3 to 2%, further more preferably 1.5 to 2%. In the lipid composition, the ratio of the total weight of lipids to the weight of the nucleic acid is not particularly limited, may be from 15 to 30, and is preferably from 15 to 25, more preferably from 15 to 22.5, further more preferably from 17.5 to 22.5.

As a specific combination of lipids in the present invention, distearoyl phosphatidylcholine, dioleoyl phosphatidylcholine or dioleoyl phosphatidylethanolamine as the amphipathic lipid, cholesterol as the sterol, (7R,9Z,26Z,29R)-18-({[3-(dimethylamino)propoxy]carbonyl}oxy)pentatriaconta-9,26-diene-7,29-diyl diacetate, 3-dimethylaminopropyl(9Z,12Z)-octaoctacosa-19,22-dien-11-yl carbonate or (7R,9Z)-18-({[3-(dimethylamino)propyloxy]carbonyl}oxy)octacos-9-en-7-yl acetate as the cationic lipid, and 1,2-dimyristoyl-sn-glycelol methoxypolyethylene glycol or N-[methoxy poly(ethylene glycol) 2000]carbamoyl]-1,2-dimyristyloxypropyl-3-amine as the PEG lipid may be used in combination. A combination of distearoyl phosphatidylcholine or dioleoyl phosphatidylethanolamine as the amphipathic lipid, cholesterol as the sterol, (7R,9Z,26Z,29R)-18-({[3-(dimethylamino)propoxy]carbonyl}oxy)pentatriaconta-9,26-diene-7,29-diyl diacetate or (7R,9Z)-18-({[3-(dimethylamino)propyloxy]carbonyl}oxy)octacos-9-en-7-yl acetate as the cationic lipid and 1,2-dimyristoyl-sn-glycelol methoxypolyethylene glycol as the PEG lipid is preferable. The specific combination of lipids in the present invention is more preferably a combination of distearoyl phosphatidylcholine as the amphipathic lipid, cholesterol as the sterol, (7R,9Z,26Z,29R)-18-({[3-(dimethylamino)propoxy]carbonyl}oxy)pentatriaconta-9,26-diene-7,29-diyl diacetate or (7R,9Z)-18-({[3-(dimethylamino)propyloxy]carbonyl}oxy)octacos-9-en-7-yl acetate as the cationic lipid and 1,2-dimyristoyl-sn-glycelol methoxypolyethylene glycol as the PEG lipid.

In the present invention, the nucleic acid encapsulated in the lipid particles is capable of expressing a haemagglutinin (HA) protein of an influenza virus. The HA protein of an influenza virus may be one type of HA protein, two or more different types of HA proteins, or a fusion protein in which two or more different types of HA proteins are bound to each other through a linker. When the HA protein is two or more types of HA proteins, separately prepared nucleic acids that each express one type of HA protein may be encapsulated in the lipid particles such that the number types of nucleic acids is identical to the number of types of HA proteins. When the HA protein of an influenza virus which is expressed by the nucleic acid encapsulated in the lipid particles is a fusion protein in which two or more types of HA proteins are bound to each other through a linker, the fusion protein may be one in which two or more types of HA proteins are fused by a protease cleavage sequence, and examples thereof include linkers comprising an amino acid sequence recognized and cleaved by Furin which is a protease. Examples of the amino acid sequence recognized and cleaved by Furin include sequences comprising a sequence R-X-X/R-R (R represents arginine, K represents lysin, and X represents an arbitrary amino acid) (J. Biol. Chem. 1992, 267, 16396; J. Biol. Chem. 1991, 266, 12127). Examples of the protease cleavage sequence for use in the present invention include the sequence of SEQ ID NO: 25. The type of the HA protein is not particularly limited. Examples of HA proteins include HA proteins H1 to H18 in type-A influenza viruses, and HA proteins of the two lineages of Victoria lineage and Yamagata Lineage in type-B influenza viruses. H1 to H9, Victoria lineage and Yamagata lineage are preferable, H1 to H3, H5, H7, H9, Victoria lineage and Yamagata lineage are more preferable, and H1, H3, H5, Victoria lineage and Yamagata lineage are further more preferable.

The amino acid sequences of A/Puerto Rico/8/34 (H1 subtype), A/Singapore/GP1908/2015 (H1 subtype), A/Singapore/INFIMH-16-0019/2016 (H3 subtype), B/Phuket/3073/2013 (Yamagata lineage), B/Maryland/15/2016 (Victoria lineage), A/Guangdong-Maonan/SWL 1536/2019 (H1 subtype), A/Astrakhan/3212/2020 (H5 subtype) and A/Laos/2121/2020 (H5 subtype) as examples of a HA protein of an influenza virus are set forth as SEQ ID NOS: 20 to 24, 50, 54 and 58. The nucleic acid encapsulated in the lipid particles encodes the HA protein of an influenza virus which comprises an amino acid sequence having an identity of preferably at least 85%, more preferably at least 90%, further more preferably at least 95%, further more preferably at least 96%, further more preferably at least 97% with the amino acid sequence of a HA protein of a targeted influenza virus.

The identity of amino acid sequences is a quantified ratio of matched amino acids with respect to the full-length sequence where amino acids are considered identical to corresponding amino acids when completely matched therewith. The identity of the sequence in the present invention is calculated using sequence analysis software GENETYX-SV/RC

(manufactured by GENETYX Corporation), and its algorithm is commonly used in the art. Amino acids encoded by the nucleic acid encapsulated in the lipid particles of the present invention may undergo mutation (substitution), deletion, insertion and/or addition of amino acids as long as the amino acids maintain a certain level of identity with the HA protein of a targeted influenza virus. The same applies to the HA proteins of an influenza virus which are set forth in SEQ ID NOS: 20 to 24, 50, 54 and 58.

The amino acids encoded by the nucleic acid encapsulated in the lipid particles of the present invention maintain the sequence identity described above, and at several locations (preferably 5 or less locations, more preferably 3, 2 or 1 location) in an amino acid sequence of a HA protein of a targeted influenza virus, several (preferably 10 or less, more preferably 7 or less, further more preferably 5, 4, 3, 2 or 1) amino acids per location may be substituted, deleted, inserted and/or added. The same applies to the HA proteins of an influenza virus which are set forth in SEQ ID NOS: 20 to 24, 50, 54 and 58.

In addition to HA proteins exemplified herein, HA proteins of viruses selected as candidate strains for influenza vaccines which are announced by WHO can be used for the lipid particles of the present invention. Examples thereof include HA proteins shown in Table 1-1, but the present invention is not limited thereto. It is possible to refer to nucleotide sequences and amino acid sequences of full-length HAs by the Accession number of GISAID (Global Initiative on Sharing All Influenza Data) in the table, and for influenza viruses of subtypes or lineages other than those shown in Table 1-1, a similar approach can be taken to refer to the sequences.

[Table 1-1]

| Strain | Type | Subtype or lineage | GISAID Accession No. |
|---|---|---|---|
| A/California/07/2009 | A | H1N1 | EPI1736465 |
| A/Singapore/GP1908/2015 | A | H1N1 | EPI848715 |
| A/Brisbane/02/2018 | A | H1N1 | EPI1384208 |
| A/Guangdong-Maonan/SWL1536/2019 | A | H1N1 | EPI1719956 |
| A/Switzerland/9715293/2013 | A | H3N2 | EPI696955 |
| A/Hong Kong/4801/2014 | A | H3N2 | EPI1727415 |
| A/Singapore/INFIMH-16-0019/2016 | A | H3N2 | EPI1259099 |
| A/Kansas/14/2017 | A | H3N2 | EPI1444535 |
| A/Hong Kong/2671/2019 | A | H3N2 | EPI1735141 |
| A/Hong Kong/5923/2012 | A | H5N1 | EPI375432 |
| A/Guizhou/1/2012 | A | H5N1 | EPI352222 |
| A/Alberta/1/2014 | A | H5N1 | EPI500771 |
| A/Egypt/MOH-NRC-7305/2014 | A | H5N1 | EPI574132 |
| A/Cambodia/Y0314301/2014 | A | H5N1 | EPI957382 |
| A/Indonesia/NIHRD15023/2015 | A | H5N1 | EPI1377750 |
| A/Indonesia/NIHRD17109/2017 | A | H5N1 | EPI1079019 |
| A/Nepal/19FL1997/2019 | A | H5N1 | EPI1449522 |
| A/Laos/2121/2020 | A | H5N1 | EPI1842225 |
| A/Guangdong/18SF020/2018 | A | H5N6 | EPI1352813 |
| A/Astrakhan/3212/2020 | A | H5N8 | EPI1846961 |
| A/Zhejiang/1/2016 | A | H7N9 | EPI1102973 |
| A/Changsha/70/2017 | A | H7N9 | EPI1036702 |
| A/Tianjin/31940/2017 | A | H7N9 | EPI1102925 |
| A/Yunnan/wenshan01/2017 | A | H7N9 | EPI1360558 |
| A/Sichuan/29764/2017 | A | H7N9 | EPI1102893 |
| A/Fujian/27871/2017 | A | H7N9 | EPI1101479 |
| A/Fujian/QZ-Th002/2017 | A | H7N9 | EPI1054102 |
| A/Hunan/25356/2017 | A | H7N9 | EPI1103967 |
| A/Gansu/23447/2019 | A | H7N9 | EPI1431536 |
| A/Hong Kong/69955/2008 | A | H9N2 | EPI470960 |
| A/Oman/2747/2019 | A | H9N2 | EPI1431480 |
| A/Guangdong/SF16348/2020 | A | H9N2 | EPI1841250 |
| A/Guangdong/20SF15010/2020 | A | H9N2 | EPI1841242 |
| B/Phuket/3073/2013 | B | Yamagata | EPI1799824 |
| B/Texas/2/2013 | B | Victoria | EPI1605916 |
| B/Maryland/15/2016 | B | Victoria | EPI1203490 |
| B/Victoria/705/2018 | B | Victoria | EPI1618364 |

The HA protein used for the lipid particles of the present invention may be a full-length protein or a partial protein, and is preferably a full-length protein.

The nucleic acid capable of expressing a HA protein of an influenza virus is an mRNA comprising a cap structure (Cap), a 5' untranslated region (5'-UTR), a HA protein encoding region, a 3' untranslated region (3'-UTR) and a poly A tail (polyA). The cap structure (Cap) is a site which is present at the 5'-end of mRNA in many eukaryotes and has a 7-methylguanosine structure. Examples of the cap structure include cap structures associated with the use of cap 0, cap 1, cap 2 or ARCA (Anti-reverse Cap Analog). The cap structures have the structural formulae shown below.

[Formula 9]

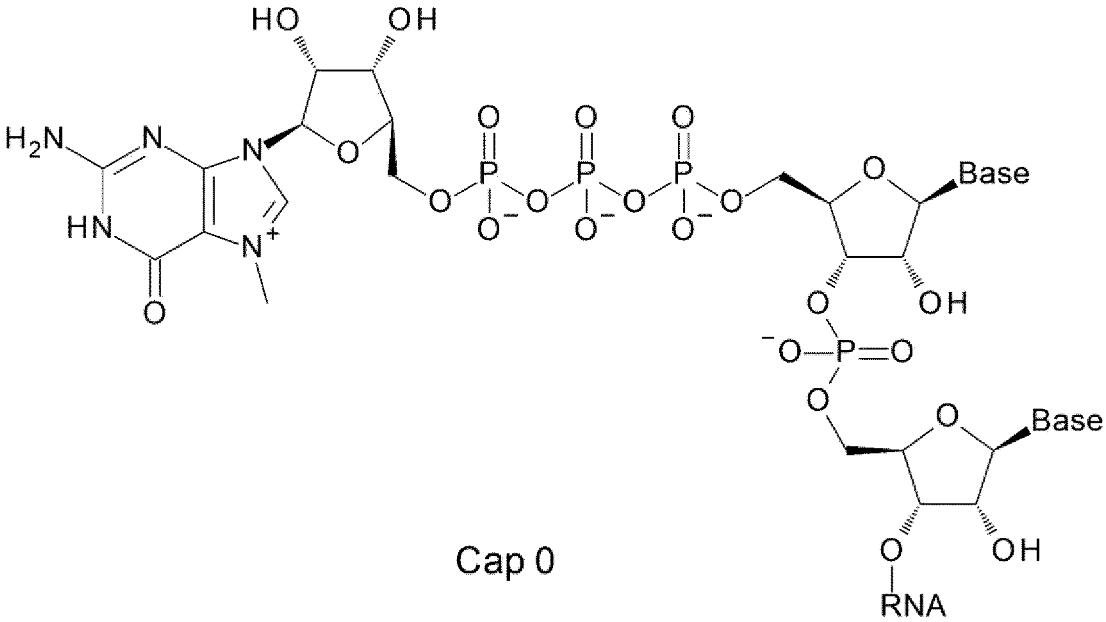

Cap 0

wherein Base represents any unmodified or modified nucleic acid base, and RNA represents any polynucleotide.

[Formula 10]

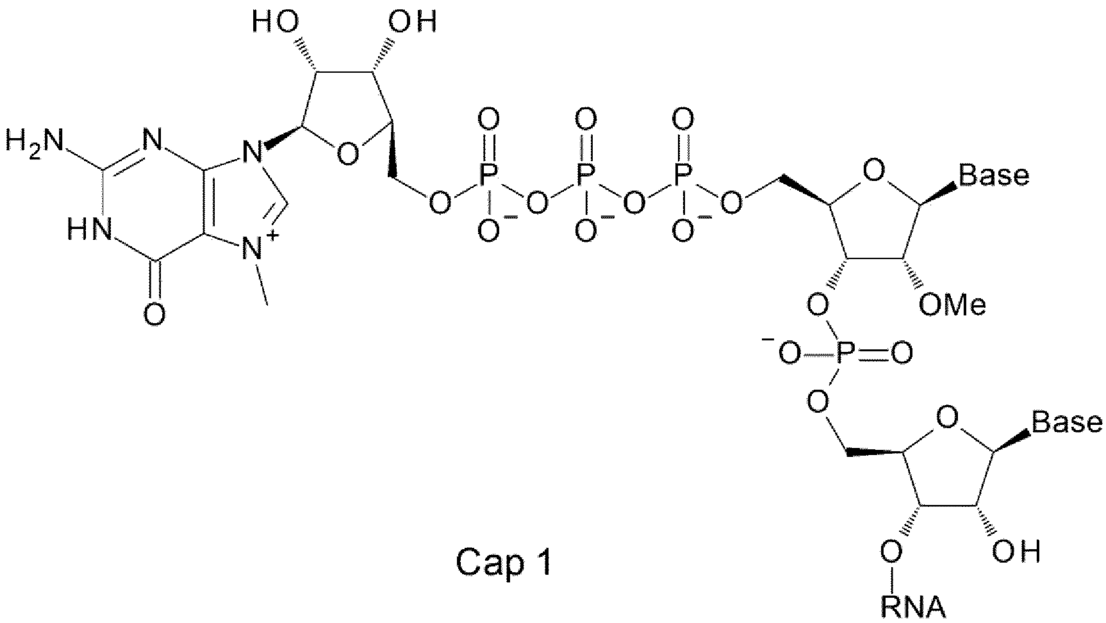

Cap 1

wherein Base represents any unmodified or modified nucleic acid base, and RNA represents any polynucleotide.

[Formula 11]

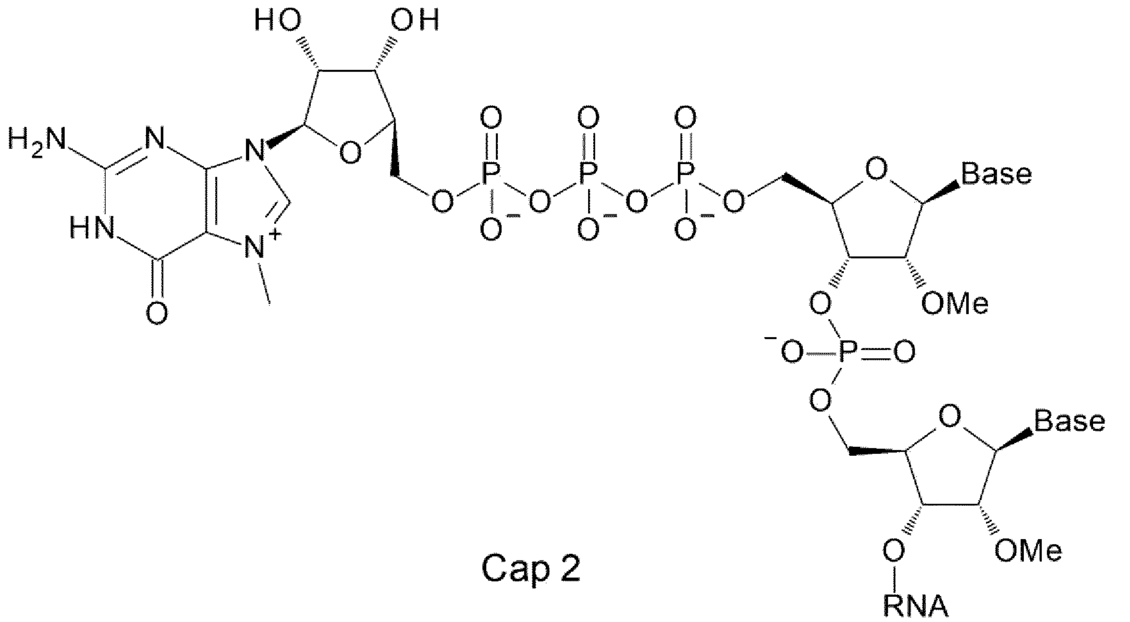

Cap 2

wherein Base represents any unmodified or modified nucleic acid base, and RNA represents any polynucleotide.

[Formula 12]

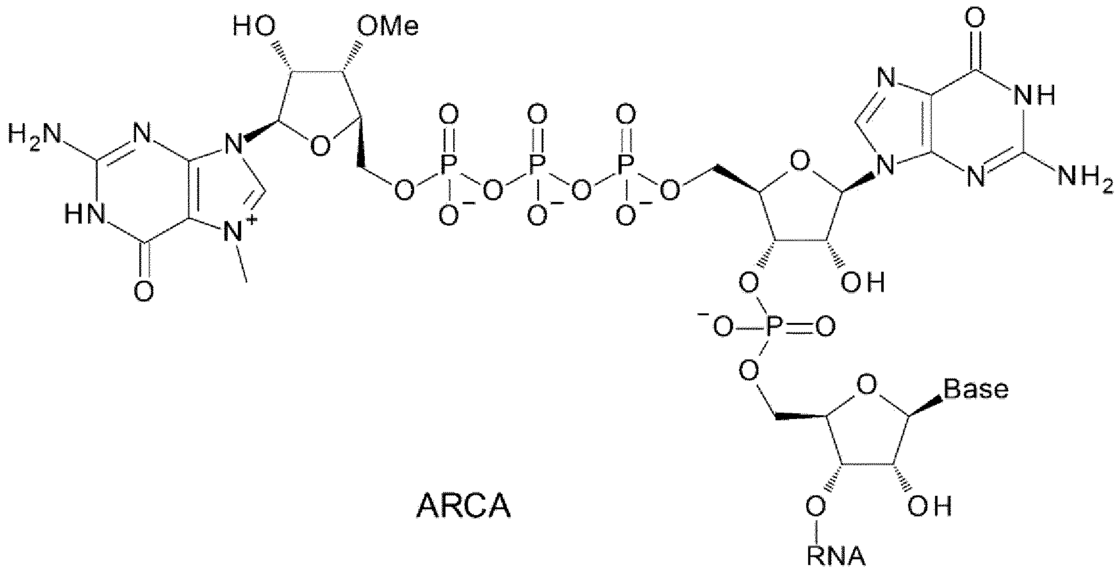

ARCA

wherein Base represents any unmodified or modified nucleic acid base, and RNA represents any polynucleotide.

The cap structure of mRNA in the present invention is preferably cap 0 or cap 1, more preferably cap 1.

The sequences of the 5' untranslated region and the 3' untranslated region are not particularly limited, and an untranslated region of stable mRNA such as α-globin, β-globin, actin or GAPDH can be used. The untranslated region used for the lipid particles of the present invention is preferably an untranslated region of β-globin. For example, a sequence comprising base Nos. 15 to 64 in the sequence of SEQ ID NO: 2 can be used as the 5' untranslated region, and a sequence comprising base Nos. 1769 to 1900 in the sequence of SEQ ID NO: 2 can be used as the 3' untranslated region.

The sequence of the 5' untranslated region (5'-UTR) is, for example, a sequence of base Nos. 1 to 70 in the sequence of SEQ ID NO: 2, a sequence of base Nos. 1 to 70 in the sequence of SEQ ID NO: 7, a sequence of base Nos. 1 to 70 in the sequence of SEQ ID NO: 10, a sequence of base Nos. 1 to 70 in the sequence of SEQ ID NO: 13, a sequence of base Nos. 1 to 70 in each of the sequences of SEQ ID NOS: 16 and 44 to 49, a sequence of base Nos. 1 to 70 in the sequence of SEQ ID NO: 19, a sequence of base Nos. 1 to 70 in each of the sequences of SEQ ID NOS:

38 to 43, or a sequence of base Nos. 1 to 70 in the sequence of SEQ ID NO: 53.

The sequence of the translated region of a HA protein of an influenza virus is capable of expressing the whole or a part of the amino acid sequence of the HA protein, and may comprise a start codon and/or a stop codon. The sequence of the translated region of a HA protein of an influenza virus may be a nucleic acid region using any of codons encoding the same amino acid (degenerate codons) as long as it is a nucleic acid sequence capable of expressing an amino acid sequence of a desired HA protein. The sequence of the translated region of a HA protein of an influenza virus is, for example, a sequence of base Nos. 71 to 1768 in the sequence of SEQ ID NO: 2, a sequence of base Nos. 71 to 1768 in the sequence of SEQ ID NO: 7, a sequence of base Nos. 71 to 1771 in the sequence of SEQ ID NO: 10, a sequence of base Nos. 71 to 1771 in the sequence of SEQ ID NO: 13, a sequence of base Nos. 71 to 1825 in each of the sequences of SEQ ID NOS: 16 and 44 to 49, a sequence of base Nos. 71 to 1822 in the sequence of SEQ ID NO: 19, a sequence of base Nos. 71 to 1771 in each of the SEQ ID NOS: 38 to 43, or a sequence of base Nos. 71 to 1774 in the sequence of SEQ ID NO: 53.

The sequence of the 3' untranslated region (3'-UTR) is a sequence of base Nos. 1769 to 1905 in the sequence of SEQ ID NO: 2, a sequence of base Nos. 1769 to 1900 in the sequence of SEQ ID NO: 7, a sequence of base Nos. 1772 to 1903 in the sequence of SEQ ID NO: 10, a sequence of base Nos. 1772 to 1903 in the sequence of SEQ ID NO: 13, a sequence of base Nos. 1826 to 1957 in each of the sequences of SEQ ID NOS: 16 and 44 to 49, a sequence of base Nos. 1823 to 1954 in the sequence of SEQ ID NO: 19, a sequence of base Nos. 1823 to 1954 in the sequence of SEQ ID NO: 19, a sequence of base Nos. 1772 to 1903 in each of the sequence of SEQ ID NOS: 38 to 43, or a sequence of base Nos. 1775 to 1906 in the sequence of SEQ ID NO: 53. The sequence of the poly A tail (polyA) is a sequence of base Nos. 1901 to 2000 in the sequence of SEQ ID NO: 7, a sequence of base Nos. 1904 to 2003 in the sequence of SEQ ID NO: 10, a sequence of base Nos. 1904 to 2003 in the sequence of SEQ ID NO: 13, a sequence of base Nos. 1958 to 2057 in the sequence of SEQ ID NO: 16, a sequence of base Nos. 1958 to 2067 in the sequence of SEQ ID NO: 44, a sequence of base Nos. 1958 to 2052 in the sequence of SEQ ID NO: 45, a sequence of base Nos. 1958 to 2037 in the sequence of SEQ ID NO: 46, a sequence of base Nos. 1958 to 2017 in the sequence of SEQ ID NO: 47, a sequence of base Nos. 1958 to 1997 in the sequence of SEQ ID NO: 48, a sequence of base Nos. 1958 to 1977 in the sequence of SEQ ID NO: 49, a sequence of base Nos. 1955 to 2054 in the sequence of SEQ ID NO: 19, a sequence of base Nos. 1904 to 2013 in the sequence of SEQ ID NO: 38, a sequence of base Nos. 1904 to 1998 in the sequence of SEQ ID NO: 39, a sequence of base Nos. 1904 to 1983 in the sequence of SEQ ID NO: 40, a sequence of base Nos. 1904 to 1963 in the sequence of SEQ ID NO: 41, a sequence of base Nos. 1904 to 1943 in the sequence of SEQ ID NO: 42, a sequence of base Nos. 1904 to 1923 in the sequence of SEQ ID NO: 43, or a sequence of base Nos. 1907 to 2006 in the sequence of SEQ ID NO: 53.

The sequences of the cap structure (Cap), the 5' untranslated region (5'-UTR), the translated region of a HA protein, the 3' untranslated region (3'-UTR) and the poly A tail (polyA) may be altered, and the sequence of the nucleic acid capable of expressing a HA protein of an influenza virus consists of a nucleotide sequence having an identity of at least 90%, preferably 95%, more preferably 97% with any of the sequences of SEQ ID NOS: 2, 7, 10, 13, 16, 19, 38-49 and 53.

The length of the poly A tail is not particularly limited, and is, for example, a length of 10 to 250 bases, preferably a length of 15 to 120 bases, more preferably a length of 15 to 115 bases, further more preferably a length of 20 to 110 bases, particularly preferably a length of 50 to 110 bases.

The mRNA according to the present invention may be an mRNA comprising a nucleotide sequence in which the sequence comprises a cap structure (Cap), a 5' untranslated region (5'-UTR), a translated region of a HA protein and a 3' untranslated region (3'-UTR), and a part consisting of the cap structure (Cap), the 5' untranslated region (5'-UTR), the translated region of a HA protein and the 3' untranslated region (3'-UTR) has an identity of at least 90%, preferably 95%, more preferably 97% with SEQ ID NO: 2, the sequence of residues 1 to 1900 in SEQ ID NO: 7, the sequence of residues 1 to 1903 in SEQ ID NO: 10, the sequence of residues 1 to 1903 in SEQ ID NO: 13, the sequence of residues 1 to 1957 in SEQ ID NO: 16, the sequence of residues 1 to 1954 in SEQ ID NO: 19, the sequence of residues 1 to 1903 in SEQ ID NO: 38, the sequence of residues 1 to 1957 in SEQ ID NO: 44 and the sequence of residues 1 to 1906 in SEQ ID NO: 53.

The nucleic acid encapsulated in the lipid particles may be in any form as long as it is capable of expressing a HA protein of an influenza virus. Examples thereof include single-stranded DNA, single-stranded RNA (for example, mRNA), single-stranded polynucleotide in which DNA and RNA are mixed, double-stranded DNA, double-stranded RNA, hybrid polynucleotide of DNA-RNA, and double-stranded polynucleotide comprising two polynucleotides in which DNA and RNA are mixed. mRNA is preferred.

The nucleotide forming a nucleic acid encapsulated in the lipid particles may be natural or modified nucleotide, and at least one modified nucleotide is preferably included.

The modified part in the modified nucleotide may be any of a base, a sugar and a phosphoric acid diester bond. There may be one or more modification sites.

Examples of the modification of a base include 5-methylation of cytosine, 5-fluoridation, N4-methylation, 5-methylation of uracil (thymine), 5-fluoridation, N6-methylation of adenine, and N2-methylation of guanine.

Examples of the modification of sugar include 2'-O-methylation of D-ribofuranose.

Examples of the modification of a phosphoric acid diester bond include phosphorothioate bond.

The modified nucleotide is preferably one that is modified at a base part. Examples thereof include pyrimidine nucleotide substituted at the 5-position, and pseudouridine optionally substituted at the 1-position. Specifically, 5- methylcytidine, 5-methoxyuridine, 5-methyluridine, pseudouridine, 1-alkylpseudouridine can be exemplified. The 1-alkylpseudouridine may be 1-(C1-C6 alkyl)pseudouridine, and is preferably 1-methylpseudouridine or 1-ethylpsedouridine. Examples of the more preferred modified nucleotide include 5-methylcytidine, 5-methyluridine, and 1-methylpseudouridine. Examples of the particularly preferred modified nucleotide include a combination of 5-methylcytidine and 5-methyluridine, and a combination of 5-methylcytidine and 1-methylpseudouridine.

The nucleic acid capable of expressing a HA protein of an influenza virus according to the present invention can be produced by an *in vitro* transcription reaction from DNA having a desired base sequence. An enzyme, a buffer solution and a nucleoside-5'-triphosphoric acid mixture (adenosine-5'-triphosphoric acid (ATP), guanosine-5'-triphosphoric acid (GTP), cytidine-5'-triphosphoric acid (CTP) and uridine-5'-triphosphoric acid (UTP)) that are necessary for *in vitro* transcription are commercially available (for example, AmpliScribeT7 High Yield Transcription Kit (Epicentre), or mMESSAGE mMACHINE T7 Ultra Kit (Life technologies). DNA for use in production of single-stranded RNA is cloned DNA, for example, plasmid DNA or a DNA fragment. The plasmid DNA or DNA fragment used may be a commercial product, or may be produced by a method generally known in the art (for example, a method described in Sambrook, J. et al., Molecular Cloning a Laboratory Manual second edition (1989), Rashtchian, A., Current Opinion in Biotechnology, 1995, 6(1), 30-36, Gibson D.G. et al., Science, 2008, 319 (5867), 1215-1220).

For obtaining mRNA having improved stability and/or safety, some or all of natural nucleotides in mRNA can also be replaced with modified nucleotides by replacing some or all of natural nucleoside-5'-triphosphoric acids with modified nucleoside-5'-triphosphoric acids in the *in vitro* transcription reaction (Kormann, M., Nature Biotechnology, 2011, 29, 154-157).

For obtaining mRNA having improved stability and/or safety, a cap structure (the Cap 0 structure described above) can be introduced to the 5'-end of mRNA by a method in which a capping enzyme is used after the *in vitro* transcription reaction. Further, Cap 0 can be converted into Cap 1 by a method in which 2'-O-methyltransferase is applied to mRNA having Cap 0. The capping enzyme and the 2'-O-methyltransefrase used may be commercial products (for example, Vaccinia Capping System, N2080; mRNA Cap 2'-O-Methyltransferase, M0366 both manufactured by New England Biolab, Inc.). When a commercial product is used, mRNA having a cap structure can be produced in accordance with a protocol accompanying the product.

The cap structure at the 5'-end of mRNA can also be introduced by a method other than a method in which an enzyme is used. For example, a structure of a cap analog of ARCA or a Cap 1 structure derived from CleanCap (registered trademark) can be introduced into mRNA by adding ARCA or CleanCap (registered trademark) to the *in vitro* transcription reaction. ARCA and CleanCap (registered trademark) used may be commercial products (ARCA, N-7003; CleanCap Reagent AG, N-7113 both manufactured by TriLink BioTechnologies, LLC). When a commercial product is used, mRNA having a cap structure can be produced in accordance with a protocol accompanying the product.

In the present invention, the nucleic acid encapsulated in the lipid particles may be purified by any a method such as desalting, HPLC (reverse phase, gel permeation, ion exchange, affinity), PAGE or ultrafiltration. Removal of impurities by purification treatment can reduce production of inflammatory cytokines in a living body receiving the nucleic acid.

The nucleic acid lipid particles of the present invention can be produced by a method such as a thin-film method, a reverse phase evaporation method, an ethanol injection method, an ether injection method, a dehydration-rehydration method, a surfactant dialysis method, a hydration method or a freezing and thawing method. The nucleic acid lipid particles can be produced by, for example, a method disclosed in International Publication No. WO 2015/005253. The nucleic acid lipid particles of the present invention can also be produced by mixing a nucleic acid solution and a lipid solution in a microchannel. For example, using Nano Assemblr (registered trademark) from Precision Nanosystems Inc., the nucleic acid lipid particles can be produced by the method described in the accompanying protocol.

The particles of the present invention have an average particle size of 30 to 300 nm, preferably 30 to 200 nm, more preferably 30 to 150 nm, further more preferably 30 to 100 nm. The average particle size can be obtained by measuring a volume average particle size on the basis of the principle of a dynamic light scattering method or the like using equipment such as Zeta Potential/Particle Sizer NICOMP (registered trademark) 380ZLS (PARTICLE SIZING SYSTEMS).

The particles of the present invention can be used for producing a composition for preventing and/or treating a disease caused by influenza virus infection. The infection is caused by type-A, type-B, type-C and/or type-D influenza viruses, preferably type-A and/or type-B influenza viruses.

The particles of the present invention can be used for expressing a HA protein of an influenza virus *in vivo* or *in vitro.* Accordingly, the present invention provides a method for expressing a HA protein of an influenza virus *in vitro,*

comprising introducing a composition containing the particles into cells. The present invention also provides a method for expressing a HA protein of an influenza virus *in vivo,* comprising administering a composition containing the particles to a mammal. By expressing a HA protein of an influenza virus *in vivo,* an immune reaction against the influenza virus can be induced. In particular, the cross-reactivity is higher in the immune reaction induced by the particles of the present invention than in the immune reaction in an inactivation split vaccine administration group, and therefore the particles of the present invention can induce an effective immune reaction. As a result, influenza virus infection can be prevented and/or treated. The present invention provides a vaccine composition that can be used in prevention and/or treatment of an influenza virus. Accordingly, the present invention provides a method for inducing an immune reaction against an influenza virus, comprising administering a composition containing the particles. The present invention provides a method for preventing and/or treating influenza virus infection, comprising administering a composition containing the particles to a mammal.

The particles of the present invention can be used as a monovalent or polyvalent vaccine composition. The term "polyvalent" vaccine composition refers to a vaccine capable of expressing two or more different types of HA proteins. The polyvalent vaccine may be a mixture of a plurality of monovalent vaccine compositions, or lipid particles which comprise a plurality of types of nucleic acids and are capable of expressing different types of HA proteins. The polyvalent vaccine may be lipid particles comprising a nucleic acid capable of expressing a fusion protein in which different types of HA proteins are fused through a linker.

The particles of the present invention can be used as a medicament and as a laboratory reagent. The particles of the present invention are typically added to a carrier such as water, a buffer solution or physiological saline. The resulting formulation (composition) can be introduced into cells *(in vitro),* or administered to a mammal (*in vivo*). In the case of administration to a mammal, the carrier is a pharmaceutically acceptable carrier (for example, physiological saline). The particles of the present invention may be formulated into a dosage form such as a cream, a paste, an ointment, a gel or a lotion made using fat, fatty oil, lanolin, vaseline, paraffin, wax, resin, plastic, glycol, a higher alcohol, glycerin, water, an emulsifier, a suspension agent or the like as a substrate material.

The particles of the present invention can be administered to mammals such as humans, mice, rats, hamsters, guinea pigs, rabbits, pigs, monkeys, cats, dogs, horses, goats, sheep and bovines orally, or parenterally by a method such as intramuscular administration, intravenous administration, intrarectal administration, transdermal administration, transmucosal administration, subcutaneous administration or intracutaneous administration.

When the particles of the present invention are administered to a human, for example, the particles are intramuscularly injected, subcutaneously injected, intracutaneously injected, drip-injected intravenously, or intravenously injected once or several times in a dosage amount of 0.001 to 1 mg, preferably 0.01 to 0.2 mg of mRNA per administration per adult, but the dosage amount and the frequency of administration can be appropriately changed depending on the type of disease, the symptom, the age and the administration method.

When the particles of the present invention are used as a laboratory reagent, a HA protein of an influenza virus can be expressed *in vitro* by introducing the particles into cells in which the HA protein of an influenza virus is to be expressed (for example, HEK 293 cells and derived cells thereof (HEK 293T cells, FreeStyle 293 cells and Expi 293 cells), CHO cells, C2C12 mouse myoblast cells, immortalized mouse dendritic cells (MutuDC1940)). Expression of the HA protein of an influenza virus can be analyzed by detecting the HA protein of an influenza virus in a sample by a Western-blot method, or detecting a peptide fragment specific to the HA protein of an influenza virus by mass spectrometry.

Examples

Hereinafter, the present invention will be described in detail by way of Examples, which are intended to illustrate the present invention, and should not be construed as limiting the scope of the present invention.

In Examples, the following terms may be used.

LNP-mRNA: lipid nanoparticles containing mRNA
ARCA: Anti-Reverse Cap Analog
UTR: Untranslated Region, untranslated region
HPLC: High performance Liquid Chromatography, high-performance liquid chromatography
Chol: Cholesterol
DSPC: Distearoyl phosphatidylcholine
LP: Cationic lipid
PEG-DMG: 1,2-Dimyristoyl-sn-glycelol methoxypolyethylene glycol

[Example 1]

Preparation of A/Puerto Rico/8/34 (H1 subtype) HA mRNA-001

(1) Production of template DNA for *in vitro* transcription (IVT) of A/Puerto Rico/8/34 (H1 subtype) HA

A plasmid was constructed for producing template DNA for use in *in vitro* transcription (IVT). Specifically, a plasmid (A/Puerto Rico/8/34-opt1) containing a DNA fragment (SEQ ID NO: 1) comprising a sequence in which GCTAGC (NheI site), a T7 promoter sequence, a 5'-UTR sequence of human β-globin, a KOZAK sequence, a translated region of A/Puerto Rico/8/34 (H1 subtype) HA, a 3'-UTR sequence of human β-globin and GAATTC (EcoRI site) are connected in this order was produced.

(2) Linearization of template DNA

To nuclease-free water (2640 μL) in which the plasmid (300 μg) obtained in Example 1-(1) was dissolved, 10× CutSmart Buffer (300 μL, New England Biolabs catalog # B7204S), and EcoRI-HF (60 μL, New England Biolabs catalog # R3101S) were added. The mixture was incubated at 37°C for 2 hours, and then at 65°C for 20 minutes. 7.5 M ammonium acetate (1,500 μL) and ethanol (9,000 μL) were mixed therewith, the mixture was stored at -78°C for 3 hours, and centrifuged (-10°C, 15,000×g, 10 min). The supernatant was then discarded, 70% ethanol was added, the mixture was centrifuged (-10°C, 15,000×g, 10 min), and the supernatant was then discarded, followed by drying in air. A 500 μg/mL solution of the obtained residue was prepared with TE-Buffer.

(3) Preparation of A/Puerto Rico/8/34 (H1 subtype) HA mRNA-001 by *in vitro* transcription

The 500 μg/mL template (400 μL) obtained in Example 1-(2), 100 mM ARCA (240 μL, TriLink catalog # N-7003), 100 mM ATP (300 μL, Hongene catalog # R1331), 100 mM GTP (60 μL, Hongene catalog # R2331), 100 mM 5-Me-CTP (300 μL, Hongene catalog # R3-029), 100 mM 5-methyluridine triphosphate (300 μL), Nuclease-free water (1,200 μL), T7 Transcription 5× buffer (800 μL, Promega catalog # P140X), an enzyme mix and T17 RNA Polymerase (400 μL, Promega catalog # P137X) were mixed, and incubated at 37°C for 2 hours. RQ1 PNase-free DNase (200 μL, Promega catalog # M6101) was mixed, and the mixture was incubated at 37°C for 15 minutes. A 8 M LiCl solution (2,000 μL, Sigma-Aldrich catalog # L7026) was mixed, the mixture was stored overnight at -20°C, and centrifuged (4°C, 5,200×g, 30 min). The supernatant was then discarded, 70% ethanol was added, the mixture was centrifuged (4°C, 5,200×g, 10 min), and the supernatant was then discarded, followed by drying in air. The obtained residue was dissolved in nuclease-free water to obtain desired mRNA.

The obtained mRNA has the sequence of SEQ ID NO: 2, while having an ARCA structure at the 5'-end and having cytidine and uridine replaced with 5-methylcytidine and 5-methyluridine. The mRNA was analyzed with Experion RNA StdSens (BIO-RAD catalog #7007103JA), and confirmed to have a desired length.

A part of the obtained solution was applied to a 25 mM ATP solution and Yeast polyA polymerase (Affymetrix catalog # 74225Z) to extend polyA as in the accompanying protocol, followed by treatment with rApid Alkaline Phosphatase (Roche catalog # 04 898 141 001). The obtained solution was purified in accordance with the attached manual using RNeasy Midi kit (Qiagen catalog # 75144), thereby obtaining desired mRNA.

The obtained mRNA was analyzed with Experion RNA StdSens (BIO-RAD catalog # 7007103JA), and confirmed to have extended polyA.

[Example 2]

Preparation of A/Puerto Rico/8/34 (H1 subtype) HA mRNA-002

(1) Production of template DNA for IVT of A/Puerto Rico/8/34 (H1 subtype)

For producing template DNA for use *in vitro* transcription (IVT), A/Puerto Rico/8/34 (H1 subtype) HA DNA was amplified, and then purified. A DNA fragment (SEQ ID NO: 3) comprising a sequence in which a T7 promoter sequence, a 5'-UTR sequence of human β-globin, a KOZAK sequence, an A/Puerto Rico/8/34 (H1 subtype) HA sequence, a 3'-UTR sequence of human β-globin and a PolyA sequence are connected in this order was introduced into a plasmid (A/Puerto Rico/8/34-opt2). To nuclease-free water (849.6 μL) in which 6 ng of the plasmid was dissolved, 10× Buffer for KOD-Plus- Ver. 2 (120 μL, Toyobo Co., Ltd. catalog # KOD-211), 2 mM dNTP mix (120 μL, Toyobo Co., Ltd. catalog # KOD-211), 25 mM MgSO4 (72 μL, Toyobo Co., Ltd. catalog # KOD-211), a 50 μM sense primer (7.2 μL, SEQ ID NO: 4), a 50 μM antisense primer (7.2 μL, SEQ ID NO: 5) and KOD Plus polymerase (24 μL, Toyobo Co., Ltd. catalog #

KOD-211) were added. The mixture was incubated at 98°C for 1 minute, followed by 20 cycles of incubation at 98°C for 5 seconds, 55°C for 15 seconds and 68°C for 2 minutes, followed by a further 1 minute incubation at 68°C to amplify Puerto Rico-opt2 DNA. After the reaction, template DNA (SEQ ID NO: 6) was purified with Wizard SV Gel and PCR Clean-Up System (Promega catalog # A9281).

(2) Preparation of A/Puerto Rico/8/34 (H1 subtype) HA mRNA-002 by *in vitro* transcription

The 391 μg/mL template DNA (25.6 μL) obtained in Example 2-(1), 100 mM CleanCap AG (50 pL, TriLink catalog # N-7113), 100 mM ATP (50 μL, Hongene catalog # R1331), 100mM GTP (50 μL, Hongene catalog # R2331), 100 mM CTP (50 μL, Hongene catalog # R3331), 100 mM N1-methyl pseudouridine-5'-triphosphate (50 μL, Hongene catalog # R5-027), nuclease-free water (424.4 μL, thermo Fisher catalog # AM9937), T7 Transcription 5× buffer (200 μL, Promega catalog # P140X), an enzyme mix and T7 RNA Polymerase (100 μL, Promega catalog # P137X) were mixed, and incubated at 37°C for 2 hours. RQ1 RNase-free DNase (10 μL, Promega catalog # N6101) was mixed, and the mixture was incubated at 37°C for 15 minutes. A M LiCl solution (500 μL, Sigma-Aldrich catalog # L7026) was mixed, and the mixture was stored at -20°C for 2 hours, and centrifuged (4°C, 5,250×g, 30 min). The supernatant was then discarded, 70% ethanol was added, the mixture was centrifuged (4°C, 5,250×g, 10 min), and the supernatant was then discarded, followed by drying in air. The obtained residue was dissolved in nuclease-free water, and then purified in accordance with the attached manual using RNeasy Maxi Kit (Qiagen catalog # 75162), thereby obtaining desired mRNA.
The obtained mRNA has the sequence of SEQ ID NO: 7, while having a cap 1 structure at the 5'-end and having uridine replaced with N1-pseudouridine. The mRNA was analyzed with Experion RNA StdSens (BIO-RAD catalog # 7007103JA), and confirmed to have a desired length.

[Example 3]

Preparation of A/Singapore/GP1908/2015 (H1 subtype) HA mRNA-001

(1) Production of template DNA for IVT of A/Singapore/GP1908/2015 (H1 subtype)

Template DNA (SEQ ID NO: 9) was produced in the same manner as in Example 2-(1) while a plasmid (A/Singapore/GP1908/2015-opt1) containing a DNA fragment (SEQ ID NO: 8) comprising a sequence in which a T7 promoter sequence, a 5'-UTR sequence of human β-globin, a KOZAK sequence, an A/Singapore/GP1908/2015 (H1 subtype) HA sequence, a 3'-UTR sequence of human β-globin and a polyA sequence are connected in this order was used instead of the plasmid obtained in Example 2-(1).

(2) Preparation of A/Singapore/GP1908/2015 (H1 subtype) HA mRNA-001 by *in vitro* transcription

mRNA was produced in the same manner as in Example 2-(2) while the template DNA obtained in Example 3-(1) was used instead of the template DNA of Example 2-(1).
The obtained mRNA has the sequence of SEQ ID NO: 10. The mRNA was analyzed with Experion RNA StdSens, and confirmed to have a desired length.

[Example 4]

Preparation of A/Singapore/IMFIMH-16-0019/2016 (H3 subtype) HA mRNA-001

(1) Production of template DNA for IVT of A/Singapore/IMFIMH-16-0019/2016 (H3 subtype) HA

Template DNA (SEQ ID NO: 12) was produced in the same manner as in Example 2-(1) while a plasmid (A/Singapore/INFIMH-16-0019/2016-opt1) containing a DNA fragment (SEQ ID NO: 11) comprising a sequence in which a T7 promoter sequence, a 5'-UTR sequence of human β-globin, a KOZAK sequence, an A/Singapore/INFIMH-16-0019/2016 (H3 subtype) HA sequence, a 3'-UTR sequence of human β-globin and a polyA sequence are connected in this order was used instead of the plasmid obtained in Example 2-(1).

(2) Preparation of A/Singapore/IMFIMH-16-0019/2016 (H3 subtype) HA mRNA-001 by *in vitro* transcription

mRNA was produced in the same manner as in Example 2-(2) while the template DNA obtained in Example 4-(1) was used instead of the template DNA of Example 2-(1).
The obtained mRNA has the sequence of SEQ ID NO: 13, while having a cap 1 structure at the 5'-end and

having uridine replaced with N1-methylpseudouridine. The mRNA was analyzed with Experion RNA StdSens (BIO-RAD catalog # 7007103JA) and confirmed to have a desired length.

[Example 5]

Preparation of B/Phuket/3073/2013 (Yamagata lineage) HA mRNA-001

(1) Production of template DNA for IVT of B/Phuket/3073/2013 (Yamagata lineage) HA

Template DNA (SEQ ID NO: 15) was produced in the same manner as in Example 2-(1) while a plasmid (B/Phuket/3073/2013-opt1) containing a DNA fragment (SEQ ID NO: 14) comprising a sequence in which a T7 promoter sequence, a 5'-UTR sequence of human β-globin, a KOZAK sequence, a B/Phuket/3073/2013 (Yamagata lineage) HA sequence, a 3'-UTR sequence of human β-globin and a polyA sequence are connected in this order was used instead of the plasmid obtained in Example 2-(1).

(2) Preparation of B/Phuket/3073/2013 (Yamagata lineage) HA mRNA-001 by *in vitro* transcription

mRNA was produced in the same manner as in Example 2-(2) while the template DNA obtained in Example 5-(1) was used instead of the template DNA of Example 2-(1).

The obtained mRNA has the sequence of SEQ ID NO: 16, while having a cap 1 structure at the 5'-end and having uridine replaced with N1-methylpseudouridine. The mRNA was analyzed with Experion RNA StdSens (BIO-RAD catalog # 7007103JA) and confirmed to have a desired length.

[Example 6]

Preparation of B/Maryland/15/2016 (Victoria lineage) HA mRNA-001

(1) Production of template DNA for IVT of B/Maryland/15/2016 (Victoria lineage)

Template DNA (SEQ ID NO: 18) was produced in the same manner as in Example 2-(1) while a plasmid (B/Maryland/15/2016-opt1) containing a DNA fragment (SEQ ID NO: 17) comprising a sequence in which a T7 promoter sequence, a 5'-UTR sequence of human β-globin, a KOZAK sequence, a B/Maryland/15/2016 (Victoria lineage) HA sequence, a 3'-UTR sequence of human β-globin and a polyA sequence are connected in this order was used instead of the plasmid obtained in Example 2-(1).

(2) Preparation of B/Maryland/15/2016 (Victoria lineage) HA mRNA-001 by *in vitro* transcription

mRNA was produced in the same manner as in Example 2-(2) while the template DNA obtained in Example 6-(1) was used instead of the template DNA of Example 2-(1).

The obtained mRNA has the sequence of SEQ ID NO: 19, while having a cap 1 structure at the 5'-end and having uridine replaced with N1-methylpseudouridine. The mRNA was analyzed with Experion RNA StdSens (BIO-RAD catalog # 7007103JA) and confirmed to have a desired length.

[Example 7]

Preparation of A/Singapore/GP1908/2015 (H1 subtype) HA mRNA-002

The 449.5 μg/mL template DNA (4.4 μL) obtained in Example 2-(1), 100 mM Clean Cap AG (10 μL, TriLink catalog # N-7113), 100 mM ATP (10 μL, Hongene catalog # R1331), 100 mM GTP (10 μL, Hongene catalog # R2331), 100 mM CTP (10 μL, Hongene catalog # R3331), 100 mM N1-methylpseudoUTP (10 μL, Hongene catalog # R5-027), nuclease-free water (85.6 μL, Thermo Fisher catalog # AM9937), T7 transcription 5× buffer (40 μL, Promega catalog # P140X), an enzyme mix and T7 RNA Polymerase (20 μL, Promega catalog # P137X) were mixed, and incubated at 37°C for 4 hours. An 8 M LiCl solution (100 μL, Sigma-Aldrich catalog # L7026) was mixed, the mixture was stored at -30°C, and centrifuged (4°C, 5,200×g, 35 min). The supernatant was then discarded, 70% ethanol was added, the mixture was centrifuged (4°C, 5,200×g, 10 min), the supernatant was then discarded, followed by drying in air. The obtained residue was dissolved in nuclease-free water (500 μL), and the solution was then purified in accordance with the attached manual using RNeasy Midi kit (Qiagen catalog # 75144). The obtained solution (750 μL), a buffer solution of rApid Alkaline Phosphatase (Roche catalog # 04 898 141 001) (85 μL) and an enzyme (40 μL) were mixed, incubated

at 37°C for 30 minutes, and then at 75°C for 2 minutes. The obtained solution was purified in accordance with the attached manual using RNeasy Midi kit (Qiagen catalog # 75144). Similar experiment operations were carried out using a total of 21 Eppendorf tubes, and the obtained mRNA solutions were combined to obtain desired mRNA.

The obtained mRNA has the sequence of SEQ ID NO: 10, while having a cap 1 structure at the 5'-end and having uridine replaced with N1-methylpseudouridine. The mRNA was analyzed with LabChip GX Touch Standard RNA Reagent Kit (PerkinElmer catalog #CLS960010) and confirmed to have a desired length.

[Example 8]

Preparation of A/Puerto Rico/8/34 (H1 subtype) HA mRNA-003

mRNA was produced in the same manner as in Example 2-(2) while the template DNA obtained in Example 2-(1) was used, 100 mM 5-Me-CTP (Hongene catalog #R3-029) was used instead of 100 mM CTP and 100 mM 5-methyluridine triphosphate was used instead of 100 mM N1-methylpseudouridine-5'-triphosphate (TriLink catalog # N-1081).

The obtained mRNA has the sequence of SEQ ID NO: 7, while having a cap 1 structure at the 5'-end and having cytidine and uridine replaced with 5-methylcytidine and 5-methyluridine, respectively. The mRNA was analyzed with LabChip GX Touch Standard RNA Reagent Kit (PerkinElmer catalog #CLS960010) and confirmed to have a desired length.

[Example 9]

Preparation of A/Singapore/GP1908/2015 (H1 subtype) HA mRNA-003

mRNA was produced in the same manner as in Example 2-(2) while the template DNA obtained in Example 3-(1) was used instead of the template DNA of Example 2-(1), 100 mM 5-Me-CTP (Hongene catalog #R3-029) was used instead of 100 mM CTP and 100 mM 5-methyluridine triphosphate was used instead of 100 mM N1-methylpseudouridine-5'-triphosphate (TriLink catalog # N-1081).

The obtained mRNA has the sequence of SEQ ID NO: 7, while having a cap 1 structure at the 5'-end and having cytidine and uridine replaced with 5-methylcytidine and 5-methyluridine, respectively. The mRNA was analyzed with LabChip GX Touch Standard RNA Reagent Kit (PerkinElmer catalog #CLS960010) and confirmed to have a desired length.

[Example 10]

Preparation of A/Singapore/INFIMH-16-0019/2016 (H3 subtype) HA mRNA-002

mRNA was produced in the same manner as in Example 2-(2) while the template DNA obtained in Example 4-(1) was used instead of the template DNA of Example 2-(1), 100 mM 5-Me-CTP (Hongene catalog #R3-029) was used instead of 100 mM CTP and 100 mM 5-methyluridine triphosphate was used instead of 100 mM N1-methylpseudouridine-5'-triphosphate (TriLink catalog # N-1081).

The obtained mRNA has the sequence of SEQ ID NO: 13, while having a cap 1 structure at the 5'-end and having cytidine and uridine replaced with 5-methylcytidine and 5-methyluridine, respectively. The mRNA was analyzed with LabChip GX Touch Standard RNA Reagent Kit (PerkinElmer catalog #CLS960010) and confirmed to have a desired length.

[Example 11]

Preparation of B/Phuket/3073/2013 (Yamagata lineage) HA mRNA-002

mRNA was produced in the same manner as in Example 2-(2) while the template DNA obtained in Example 5-(1) was used instead of the template DNA of Example 2-(1), 100 mM 5-Me-CTP (Hongene catalog #R3-029) was used instead of 100 mM CTP and 100 mM 5-methyluridine triphosphate was used instead of 100 mM N1-methylpseudouridine-5'-triphosphate (TriLink catalog # N-1081).

The obtained mRNA has the sequence of SEQ ID NO: 16, while having a cap 1 structure at the 5'-end and having cytidine and uridine replaced with 5-methylcytidine and 5-methyluridine, respectively. The mRNA was analyzed with LabChip GX Touch Standard RNA Reagent Kit (PerkinElmer catalog #CLS960010) and confirmed to have a desired length.

[Example 12]

Preparation of B/Maryland/15/2016 (Victoria lineage) HA mRNA-002

mRNA was produced in the same manner as in Example 2-(2) while the template DNA obtained in Example 6-(1) was used instead of the template DNA of Example 2-(1), 100 mM 5-Me-CTP (Hongene catalog #R3-029) was used instead of 100 mM CTP and 100 mM 5-methyluridine triphosphate was used instead of 100 mM N1-methylpseudouridine-5'-triphosphate (TriLink catalog # N-1081).

The obtained mRNA has the sequence of SEQ ID NO: 19, while having a cap 1 structure at the 5'-end and having cytidine and uridine replaced with 5-methylcytidine and 5-methyluridine, respectively. The mRNA was analyzed with LabChip GX Touch Standard RNA Reagent Kit (PerkinElmer catalog #CLS960010) and confirmed to have a desired length.

[Example 13]

Preparation of nucleic acid lipid particles encapsulating A/Puerto Rico/8/34 (H1 subtype) HA mRNA-001 described in Example 1

(1) Preparation of mRNA-encapsulating nucleic acid lipid particles

Distearoyl phosphatidylcholine (1,2-distearoyl-sn-glycero-3-phosphocholine: hereinafter referred to as DSPC, NOF CORPORATION), cholesterol (hereinafter referred to as Chol, Sigma-Aldrich, Inc.), (7R,9Z,26Z,29R)-18-({[3-(dimethylamino)propoxy]carbonyl}oxy)pentatriaconta-9,26-diene-7,29-diyl diacetate (compound described in WO 2015/005253, Example 23) (hereinafter referred to as LP1), and 1,2-dimyristoryl-sn-glycero-3-methoxypolyethylene glycol having a polyethylene glycol molecular weight of about 2,000 (hereinafter referred to as PEG-DMG, NOF CORPORATION, SUNBRIGHT GM-020) were dissolved at a molar ratio of DSPC : Chol : LP1 : PEG-DMG = 10 : 43.5 : 45 : 1.5 in ethanol so that the total lipid concentration was 10 mM.

Meanwhile, the A/Puerto Rico/8/34 (H1 subtype) HA mRNA-001 obtained in Example 1 was adjusted to 104 μg/mL with a citrate buffer solution (20 mM citrate buffer, pH 4.0).

Using NanoAssemblr BenchTop (Precision Nanosystems Inc.), the lipid solution and the mRNA solution were mixed at a volume ratio of 1 : 3 in a microchannel to obtain a crude dispersion liquid of nucleic acid particles. The dispersion liquid of nucleic acid lipid particles was dialyzed overnight with a phosphate buffer solution (pH 7.4) in an amount about 25 to 50 times the amount of the dispersion liquid (Float-A-Lyzer G2, MWCO: 1,000 kD, Spectra/Por) to remove ethanol, thereby obtaining a purified dispersion liquid of mRNA-encapsulating nucleic acid lipid particles.

The LP1 was synthesized in accordance with the method described in WO 2015/005253, Example 23.

(2) Evaluation of characteristics of mRNA-encapsulating nucleic acid lipid particles

The characteristics of the dispersion liquid of nucleic acid lipid particles, which had been prepared in (1), were evaluated. Methods for evaluating respective characteristics will be described.

(2-1) mRNA encapsulation percentage

Using Quant-iT RiboGreen RNA Assay Kit (Invitrogen), the mRNA encapsulation percentage was measured in accordance with the attached document. That is, the amount of mRNA in the dispersion liquid of nucleic acid lipid particles was determined in the presence and absence of a 0.0165% Triton X-100 surfactant, and the encapsulation percentage was calculated from the following expression:

$$\{([\text{amount of mRNA in the presence of surfactant}] - [\text{amount of mRNA in the absence of surfactant}]) / [\text{amount of mRNA in the presence of surfactant}]\} \times 100\ (\%).$$

(2-2) Ratio between mRNA and lipid

The amount of mRNA in the dispersion liquid of nucleic acid lipid particles was measured by reverse phase

chromatography (system: Agilent 1100 series, Column: BIO shell A400 Protein C4 (10 cm × 4.6 mm, 3.4 μm) (SUPELCO), buffer A: 0.1 M triethylamine acetate (pH 7.0), buffer B: acetonitrile, (B%): 0-30% (0-20 min), flow rate: 1 mL/min, temperature: 70°C, detection: 260 nm) .

Using Phospholipid C-Test Wako (FUJIFILM Wako Pure Chemical Corporation), the amount of phospholipid in the dispersion liquid of nucleic acid lipid particles was measured in accordance with the attached document. That is, the amount of phospholipid in the sample was measured in the presence of a 2% Triton X-100 surfactant. The amounts of cholesterol and LP1 in the dispersion liquid of nucleic acid lipid particles were measured (system: DIONEX UltiMate 3000, column: Chromolith Performance RP-18 endcapped 100-4.6 HPLC-column (Merck, Cat.#: 1021290001), buffer A: 0.01% trifluoroacetic acid, buffer B: 0.01% trifluoroacetic acid, methanol, (B%): 82-97% (0-17 mi,), flow rate: 2 mL/min, temperature: 50°C, detection: Corona CAD (charged Aerosol detector).

The total lipid amount was calculated from the measured values of phospholipid, cholesterol and LP1 and the composition ratio of lipid components forming the nucleic acid lipid particles.

The ratio of the total lipid amount to mRNA was calculated from the following expression:

[total lipid concentration]/[mRNA concentration] (wt/wt).

(2-3) Average particle size

The particle size of the nucleic acid lipid particle was measured with Zeta Potential/Particle Sizer NICOMPTM 380ZLS (PARTICLE SIZING SYSTEMS). The average particle size in the table represents a volume average particle size, and the number following ± represents a deviation.

Table 1-2 shows the results.

[Example 14]

Preparation of nucleic acid lipid particles encapsulating A/Puerto Rico/8/34 (H1 subtype) HA mRNA-002 described in Example 2

(1) Preparation of mRNA-encapsulating nucleic acid lipid particles

In the same manner as in Example 13-(1), nucleic acid lipid particles encapsulating the A/Puerto Rico/8/34 (H1 subtype) HA mRNA-002 described in Example 2 were prepared, and the characteristics thereof were evaluated. However, the constituent lipid composition was set to DSPC:Chol:LP1:PEG-DMG = 12.5:41:45:1.5 in terms of a molar ratio.

(2) Evaluation of characteristics of mRNA-encapsulating nucleic acid lipid particles

(2-1) mRNA encapsulation percentage and average particle size

The mRNA encapsulation percentage and the average particle size were measured in the same manner as in Example 13-(2). However, the ratio between mRNA and the lipid was measured by the following method.

(2-2) Ratio between mRNA and lipid

The amount of mRNA in the dispersion liquid of nucleic acid lipid particles was measured by reverse phase chromatography (system: Agilent 1100 series, Column: BIO shell A400 Protein C4 (10 cm × 4.6 mm, 3.4 μm) (SUPELCO), buffer A: 0.1 M triethylamine acetate (pH 7.0), buffer B: acetonitrile, (B%): 5-50% (0-15 min), flow rate: 1 mL/min, temperature: 70°C, detection: 260 nm) .

The amounts of the lipids in the dispersion liquid of nucleic acid lipid particles were measured by reverse phase chromatography (system: DIONEX UltiMate 3000, column: XSelect CSH (50 mm × 3 mm, 5 μm) (Waters), buffer A: 0.2% formic acid, buffer B: 0.2% formic acid, methanol, (B%): 75-95% (0-15 min), 95% (15-17 min), flow rate: 0.45 mL/min, temperature: 50°C, detection: Corona CAD (charged aerosol detector)).

The ratio of the total lipid amount to mRNA was calculated from the following expression:

[total lipid concentration]/[mRNA concentration] (wt/wt).

Table 1-2 shows the results.

[Example 15]

Preparation of nucleic acid lipid particles encapsulating A/Singapore/GP1908/2015 (H1 subtype) HA mRNA-001 described in Example 3

(1) Preparation of mRNA-encapsulating nucleic acid lipid particles

DSPC, Chol, LP1 and PEG-DMG were dissolved at a molar ratio of DSPC : Chol : LP1 : PEG-DMG = 12.5 : 41 : 45 . 1.5 in ethanol so that the total lipid concentration was 5 mM.

Meanwhile, the A/Singapore/GP1908/2015 (H1 subtype) HA mRNA-001 obtained in Example 3 was adjusted to 53 $\mu$g/mL with a citrate buffer solution (20 mM citrate buffer, pH 4.0).

Using NanoAssemblr BenchTop (Precision Nanosystems Inc.), the lipid solution and the mRNA solution were mixed at a volume ratio of 1:3 in a microchannel to obtain a crude dispersion liquid of nucleic acid particles. The dispersion liquid of nucleic acid lipid particles was dialyzed overnight (Float-A-Lyzer G2, MWCO: 1,000 kD, Spectra/Por) to remove ethanol, thereby obtaining a purified dispersion liquid of mRNA-encapsulating nucleic acid lipid particles.

(2) Evaluation of characteristics of mRNA-encapsulating nucleic acid lipid particles

(2-1) mRNA encapsulation percentage and average particle size

The mRNA encapsulation percentage and the average particle size were measured in the same manner as in Example 13-(2). However, the ratio between mRNA and the lipid was measured by the following method.

(2-2) Ratio between mRNA and lipid

In the same manner as in Example 14-(2), the amount of mRNA in the dispersion liquid of nucleic acid lipid particles was measured by reverse phase chromatography.

The amounts of the lipids in the dispersion liquid of nucleic acid lipid particles were measured by reverse phase chromatography under the following conditions (system: DIONEX UltiMate 3000, column: XSelect CSH (150 mm $\times$ 3 mm, 3.5 $\mu$m) (Waters), buffer A: 0.2% formic acid, buffer B: 0.2% formic acid, methanol, (B%): 75-95% (0-6 min), 100% (6-15 min), flow rate: 0.45 mL/min, temperature: 50°C, detection: Corona CAD (charged aerosol detector)).

The ratio of the total lipid amount to mRNA was calculated from the following expression:

[total lipid concentration]/[mRNA concentration] (wt/wt).

Table 1-2 shows the results.

[Examples 16 to 19]

Preparation of nucleic acid lipid particles encapsulating HA mRNA (1)

In the same manner as in Example 15, nucleic acid lipid particles encapsulating the mRNAs described in Examples 4, 5 and 6 were prepared, and the characteristics thereof were evaluated. Table 1-2 shows the results.

[Examples 20 to 27]

Preparation of nucleic acid lipid particles encapsulating HA mRNA (2)

In the same manner as in Example 15, nucleic acid lipid particles encapsulating the mRNAs described in Examples 4, 5 and 6 were prepared, and the characteristics thereof were evaluated. Table 2 shows the results.

[Example 28]

Preparation of nucleic acid lipid particles encapsulating A/Singapore/GP1908/2015 (H1 subtype) HA mRNA-002 described in Example 7 (1)

In the same manner as in Example 15, nucleic acid lipid particles encapsulating the mRNA described in Example

7 were prepared, and the characteristics thereof were evaluated. Table 3 shows the results.

[Example 29]

Preparation of nucleic acid lipid particles encapsulating A/Singapore/GP1908/2015 (H1 subtype) HA mRNA-002 described in Example 7 (2)

In the same manner as in Example 15, nucleic acid lipid particles encapsulating the mRNA described in Example 7 were prepared, and the characteristics thereof were evaluated. However, (7R,9Z)-18-({[3-(dimethylamino)propyloxy]carbonyl}oxy)octacos-9-en-7-yl acetate (compound described in WO 2015/005253, Example 28) (hereinafter referred to as LP2) was used instead of LP1, and the constituent lipid composition was set to a molar ratio shown in Table 3. Table 3 shows the results. LP2 was synthesized in accordance with the method described in WO 2015/005253, Example 28.

[Examples 30 to 43]

Preparation of nucleic acid lipid particles encapsulating A/Singapore/GP1908/2015 (H1 subtype) HA mRNA-002 described in Example 7 (3)

In the same manner as in Example 15, nucleic acid lipid particles encapsulating the mRNA described in Example 7 were prepared, and the characteristics thereof were evaluated. However, LP2 was used instead of LP1, and the constituent lipid composition was set to a molar ratio shown in Table 4 or 5. Table 4 or 5 shows the results.

[Examples 44 to 48]

Preparation of nucleic acid lipid particles encapsulating HA mRNA (3)

In the same manner as in Example 15, nucleic acid lipid particles encapsulating the mRNAs described in Examples 8, 9, 10, 11 and 12 were prepared, and the characteristics thereof were evaluated. However, LP2 was used instead of LP1, and the constituent lipid composition was set to a molar ratio shown in Table 6. Table 6 shows the results.

[Table 1-2]

| **Example** | mRNA | DSPC/Chol/LP1/PEG-DMG (mol%) | **mRNA encapsulation percentage** | lipid/ mRNA (wt/wt) | **Average particle size** (nm) |
|---|---|---|---|---|---|
| 13 | **Example 1** | 10/43.5/45/1.5 | 97.9% | 18 | 111±12 |
| 14 | **Example 2** | 12.5/41/45/1.5 | 97.3% | 20 | 101±7 |
| 15 | **Example 3** | 12.5/41/45/1.5 | 97.8% | 24 | 110±24 |
| 16 | **Example 4** | 12.5/41/45/1.5 | 97.4% | 24 | 95±27 |
| 17 | **Example 5** | 12.5/41/45/1.5 | 97.4% | 20 | 84±28 |
| 18 | **Example 6** | 12.5/41/45/1.5 | 97.7% | 21 | 90±29 |
| 19 | **Example 4** | 12.5/41/45/1.5 | 98.0% | 21 | 104±18 |

The above results reveal that in these nucleic acid lipid particles, 90% or more of mRNA is encapsulated in lipid particles, and the average particle size is about 80 nm to about 120 nm.

[Table 2]

| Example | mRNA | DSPC/Chol/LP1/PEG-DMG (mol%) | **mRNA encapsulation percentage** | lipid/ mRNA (wt/wt) | **Average particle size** (nm) |
|---|---|---|---|---|---|
| 20 | **Example 3** | 12.5/41/45/1.5 | 98.9% | 19 | 102±38 |
| 21 | **Example 4** | 12.5/41/45/1.5 | 99.3% | 19 | 105±34 |
| 22 | **Example 5** | 12.5/41/45/1.5 | 99.0% | 18 | 116±10 |
| 23 | **Example 6** | 12.5/41/4-5/1.5 | 99.0% | 18 | 107±35 |
| 24 | **Example 3** | 12.5/41/45/1.5 | 99.6% | 20 | 111±24 |
| 25 | **Example 4** | 12.5/41/45/1.5 | 99.5% | 20 | 115±25 |
| 26 | **Example 5** | 12.5/41/45/1.5 | 99.4% | 18 | 101±18 |
| 27 | **Example 6** | 12.5/41/45/1.5 | 99.4% | is | 103±31 |

The above results reveal that in these nucleic acid lipid particles, 90% or more of mRNA is encapsulated in lipid particles, and the average particle size is about 90 nm to about 120 nm.

[Table 3]

| Example | mRNA | IDSPC/Chol/LP/PEG-DMG (mol%) | **mRNA encapsulation percentage** | lipid/ mRNA (wt/wt) | **Average particle size** (nm) |
|---|---|---|---|---|---|
| 28 | **Example 7** | 12.5/41/45(LP1)/1.5 | 99.8% | 19 | 116±40 |
| 29 | **Example 7** | 12.5/41/45(LP2)/1.5 | 99.4% | 21 | 146±29 |

The above results reveal that in these nucleic acid lipid particles, 90% or more of mRNA is encapsulated in lipid particles, and the average particle size is about 110 nm to about 120 nm.

[Table 4]

| Example | mRNA | DSPC/Chol/LP2/PEG-DMG (mol%) | **mRNA encapsulation percentage** | lipid/mRNA (wt/wt) | **Average particle size (nm)** |
|---|---|---|---|---|---|
| 30 | **Example 7** | 12.5/41/45/1.5 | 99.4% | 22 | 157±37 |
| 31 | **Example 7** | 15/38.5/45/1.5 | 99.4% | 20 | 147±23 |
| 32 | **Example 7** | 17.5/36/45/1.5 | 99.4% | 23 | 138±16 |
| 33 | **Example 7** | 20/33.5/45/1.5 | 99.5% | 19 | 115±13 |
| 34 | **Example 7** | 22.5/31/45/1.5 | 99.5% | 20 | 110±32 |
| 35 | **Example 7** | 17.5/21/60/1.5 | 99.3% | 21 | 104±9 |
| 36 | **Example 7** | 17.5/26/55/1.5 | 99.5% | 19 | 111±45 |

(continued)

| Example | mRNA | DSPC/Chol/LP2/PEG-DMG (mol%) | **mRNA encapsulation percentage** | lipid/mRNA (wt/wt) | **Average particle size (nm)** |
|---|---|---|---|---|---|
| 37 | **Example 7** | 17.5/31/50/1.5 | 99.6% | 21 | 120±24 |
| 38 | **Example 7** | 17.5/38.5/42.5/1.5 | 99.4% | 21 | 134±32 |

The above results reveal that in these nucleic acid lipid particles, 90% or more of mRNA is encapsulated in lipid particles, and the average particle size is about 90 nm to about 160 nm.

[Table 5]

| Example | mRNA | DSPC/Chol/LP2/PEG-DMG (mol%) | **mRNA encapsulation percentage** | lipid/ mRNA (wt/wt) | **Average particle size** (nm) |
|---|---|---|---|---|---|
| 39 | **Example 7** | 12.5/41/45/1.5 | 98.7% | 17 | 177±83 |
| 40 | **Example 7** | 10/38.5/50/1.5 | 98.6% | 17 | 146±64 |
| 41 | **Example 7** | 12. 5/36/50/1.5 | 98.4% | 17 | 138±63 |
| 42 | **Example 7** | 15/33.5/50/1.5 | 97.7% | 17 | 119±45 |
| 43 | **Example 7** | 20/28.5/50/1.5 | 98.4% | 17 | 100±25 |

The above results reveal that in these nucleic acid lipid particles, 90% or more of mRNA is encapsulated in lipid particles, and the average particle size is about 90 nm to about 180 nm.

[Table 6]

| Example | mRNA | DSPC/Chol/LP2/PEG-DMG (mol%) | **mRNA encapsulation percentage** | lipid/mRNA (wt/wt) | **Average particle size** (nm) |
|---|---|---|---|---|---|
| 44 | **Example 8** | 17.5/21/60/1.5 | 95.7% | 28 | 130±35 |
| 45 | **Example 9** | 17.5/21/60/1.5 | 97.3% | 25 | 127±32 |
| 46 | **Example 10** | 17.5/21/60/1.5 | 95.9% | 23 | 137±57 |
| 47 | **Example 11** | 17.5/21/60/1.5 | 96.7% | 18 | 123±42 |
| 48 | **Example 12** | 1.5/21/60/1.5 | 96.4% | 18 | 132±45 |

The above results reveal that in these nucleic acid lipid particles, 90% or more of mRNA is encapsulated in lipid particles, and the average particle size is about 110 nm to about 140 nm.

[Examples 49 to 54]

Preparation of A/Guangdong-Maonan/SWL 1536/2019 (H1 subtype) HA mRNA

(1) Production of template DNA for *in vitro* transcription (IVT) of A/Guangdong-Maonan/SWL 1536/2019 (H1 subtype) HA mRNA

A plasmid was constructed for producing template DNA for use *in vitro* transcription (IVT). Specifically, a plasmid containing template DNA (each of SEQ ID NOS: 26 to 31) comprising a sequence in which a T7 promoter sequence, a 5'-UTR sequence of human β-globin, a KOZAK sequence, a translated region of A/Guangdong-Maonan/SWL 1536/2019 (H1 subtype) HA, a 3'-UTR sequence of human β-globin and a polyA sequence are connected in this order was produced (mRNAs of Examples 49 to 54 were prepared using plasmids containing SEQ ID NOS: 26 to 31, respectively).

(2) Linearization of template DNA

To nuclease-free water (268 μL) in which the plasmid (300 μg) obtained in (1) was dissolved, 10× NE Buffer 3.1, BspQI (32 μL, New England Biolabs catalog # R0712L) was added. The mixture was incubated at 50°C for 16 hours, and then at 80°C for 20 minutes. Ethanol (900 μL) and a 3 mol/L sodium acetate solution (30 μL) were therewith, the mixture was stored at -80°C for 4 hours, and centrifuged (4°C, 15,000×g, 30 min). The supernatant was then discarded, 70% ethanol was added, the mixture was centrifuged (4°C, 15,000×g, 10 min), the supernatant was then discarded, followed by drying in air. A 500 μg/mL solution of the obtained residue was prepared with nuclease-free water.

(3) Preparation of A/Guangdong-Maonan/SWL 1536/2019 (H1 subtype) HA mRNA and B/Phuket/3073/2013 (By subtype) HA mRNA by *in vitro* transcription

The 500 μg/mL template DNA (15 μL) obtained in (2), 100 mM CleanCap AG (15 μL, TriLink catalog # N-7113), 100 mM ATP (15 μL, Hongene catalog # R1331), 100 mM GTP (15 μL, Hongene catalog # R2331), 100 mM 5-Me-CTP (15 μL, Hongene catalog # R3-029), 100 mM 5-Me-UTP (15 μL, Hongene catalog # R5-104), nuclease-free water (113 μL), 5×IVT buffer (60 μL, 400 mM HEPES-KOH pH 7.5, 400 mM DTT, 120 mM MgCl2, 10 mM Spermidine), T7 RNA Polymerase (15 μL, Promega catalog # P407X), RNase Inhibitor (15 μL, Promega catalog # P261X) and Pyrophosphatase (7.5 μL, Hongene catalog # ON-025) were mixed, and incubated at 37°C for 30 minutes. A 10 M ammonium acetate solution (100 μL) was mixed, the mixture was stored at -20°C for 2 hours, and centrifuged (4°C, 15,000×g, 30 min). The supernatant was then discarded, 70% ethanol was added, the mixture was centrifuged (4°C, 15,000×g, 10 min), the supernatant was then discarded, followed by drying in air. The obtained residue was dissolved in nuclease-free water, and the solution was purified with NucleoSpin (Macherey-Nagel catalog # 740948.50) to obtain desired mRNA.

The obtained mRNAs have the sequences of SEQ ID NOS: 38 to 43, respectively, while having a cap 1 structure at the 5'-end and having cytidine and uridine replaced with 5-methylcytidine and 5-methyluridine, respectively. The mRNAs were analyzed with LabChip GX touch Standard RNA Reagent Kit (PerkinElmer catalog #CLS960010), and confirmed to have a desired length.

[Examples 55 to 60]

Preparation of nucleic acid lipid particles encapsulating A/Guangdong-Maonan/SWL 1536/2019 (H1 subtype) HA mRNA obtained in Examples 49 to 54.

(1) Preparation of mRNA-encapsulating nucleic acid lipid particles

DSPC, Chol, LP2, PEG-DMG were dissolved at a molar ratio of DSPC : Chol : LP2 : PEG-DMG = 17.5 : 21 : 60 : 1.5 in ethanol so that the total lipid concentration was 5 mM.

Meanwhile, the A/Guangdong-Maonan/SWL 1536/2019 (H1 subtype) HA mRNA obtained in Examples 49 to 54 was adjusted to 41 μg/mL with a citrate buffer solution (20 mM citrate buffer, pH 4.0).

Using NanoAssemblr BenchTop (Precision Nanosystems Inc.), the lipid solution and the mRNA solution were mixed at a volume ratio of 1 : 3 in a microchannel to obtain a crude dispersion liquid of nucleic acid particles. The dispersion liquid of nucleic acid lipid particles was dialyzed overnight with a histidine buffer solution (pH 7.0) in an amount about 25 to 50 times the amount of the dispersion liquid (Float-A-Lyzer G2, MWCO: 1,000 kD, Spectra/Por) to remove ethanol, thereby obtaining a purified dispersion liquid of mRNA-encapsulating nucleic acid lipid particles.

(2) Evaluation of characteristics of mRNA-encapsulating nucleic acid lipid particles

(2-1) mRNA encapsulation percentage and average particle size

The mRNA encapsulation percentage and the average particle size were measured in the same manner as in Example 13-(2). However, the ratio between mRNA and the lipid was measured by the following method.

(2-2) Ratio between mRNA and lipid

The amount of mRNA in the dispersion liquid of nucleic acid lipid particles was measured with an ultraviolet and visible spectrophotometer (LAMBDA™ 465 manufactured by PerkinElmer, Inc.) by diluting the dispersion liquid of nucleic acid lipid particles to a solution with 90% methanol. The mRNA concentration was calculated from the following expression:

$$\{[\text{absorbance at 260 nm}] - [\text{absorbance at 350 nm}]\} \times 40 \times \text{dilution ratio } (\mu g/mL).$$

The amounts of the lipids in the dispersion liquid of nucleic acid lipid particles were measured by reverse phase chromatography (system: DIONEX UltiMate 3000, column: XSelect CSH (150 mm × 3 mm, 3.5 μm) (Waters), buffer A: 0.2% formic acid, buffer B: 0.2% formic acid, methanol, (B%): 75-100% (0-6 min), 100% (6-15 min), flow rate: 0.45 mL/min, temperature: 50°C, detection: Corona CAD (charged aerosol detector)).

The ratio of the total lipid amount to mRNA was mRNA was calculated from the following expression:

$$[\text{total lipid concentration}]/[\text{mRNA concentration}] \text{ (wt/wt)}.$$

Table 7 shows the results.

[Table 7]

| **Example** | mRNA | DSPC/Chol/LP2/PEG-DMG (mol%) | **mRNA encapsulation percentage** | lipid/mRNA (wt/wt) | **Average particle size** (nm) |
|---|---|---|---|---|---|
| 55 | **Example 49** | 17.5/21/60/1.5 | 94% | 22.9 | 149±64 |
| 56 | **Example 50** | 17.5/21/60/1.5 | 97% | 22.2 | 123±58 |
| 57 | **Example 51** | 17.5/21/60/1.5 | 97% | 22.2 | 108±49 |
| 58 | **Example 52** | 17.5/21/60/1.5 | 97% | 22.7 | 111±48 |
| 59 | **Example 53** | 17.5/21/60/1.5 | 97% | 22.5 | 128±32 |
| 60 | **Example 54** | 17.5/21/60/1.5 | 96% | 22.9 | 123±55 |

[Example 61]

Preparation of A/Astrakhan/3212/2020 (H5 subtype) HA mRNA-001

(1) Production of template DNA for IVT of A/Astrakhan/3212/2020 (H5 subtype) HA

Template DNA (SEQ ID NO: 52) was produced in the same manner as in Example 2-(1) while a plasmid (A/Astrakhan/3212/2020-opt1) containing a DNA fragment (SEQ ID NO: 51) comprising a sequence in which a T7 promoter sequence, a 5'-UTR sequence of human β-globin, a KOZAK sequence, an A/Astrakhan/3212/2020 (H5 subtype)

HA sequence and a 3'-UTR sequence of human β-globin are connected in this order was used instead of the plasmid obtained in Example 2-(1).

(2) mRNA was produced in the same manner as in Example 2-(2) while the template DNA obtained in Example 61-(1) was used instead of the template DNA of Example 2-(1), 100 mM 5-Me-CTP (Hongene catalog # R3-029) was used instead of 100 mM CTP and 100 mM 5-methyluridine triphosphate was used instead of 100 mM N1-methylpsuedouridine-5'-triphosphate (TriLink catalog # N-1081).

The obtained mRNA has the sequence of SEQ ID NO: 53, while having a cap 1 structure at the 5'-end and having cytidine and uridine replaced with 5-methylcytidine and 5-methyluridine, respectively. The mRNA was analyzed with DynaMarker (registered trademark) RNA High for Easy Electrophoresis (BioDynamics Laboratory Inc., catalog # DM170), and confirmed to have a desired length.

[Example 62]

Preparation of nucleic acid lipid particles encapsulating A/Astrakhan/3212/2020 (H5 subtype) HA mRNA-001 (1)

In the same manner as in Example 15, nucleic acid lipid particles encapsulating the mRNA described in Example 61 were prepared, and the characteristics thereof were evaluated. Table 8 shows the results.

[Table 8]

| **Example** | mRNA | DSPC/Chol/LP1/PEG-DMG (mol%) | **mRNA encapsulation percentage** | **Average particle size** (nm) |
|---|---|---|---|---|
| 62 | **Example 61** | 12.5/41/45/1.5 | 99.1% | 124.7 |

The above results reveal that in these nucleic acid lipid particles, 90% or more of mRNA is encapsulated in lipid particles, and the average particle size is about 80 nm to about 125 nm.

[Example 63]

Production of A/Astrakhan/3212/2020 (H5N8) HA protein expression plasmid

A plasmid was produced in which a sequence with a KOZAK sequence connected to an A/Astrakhan/3212/2020 (H5N8) HA gene translation region (SEQ ID NO: 55) was inserted into multicloning sites (SalI and NotI sites) of a protein expression plasmid (pCAG-Neo) (FUJIFILM Wako Pure Chemical Corporation, #163-25601).

[Example 64]

Production of A/Laos/2121/2020 (H5N1) HA protein expression plasmid

A plasmid was produced in which a sequence with a KOZAK sequence connected to A/Laos/2121/2020 (H5N1) HA gene translation region was inserted into multicloning sites (SalI and NotI sites) of a protein expression plasmid (pCAG-Neo) (FUJIFILM Wako Pure Chemical Corporation, #163-25601).

[Example 65]

Production of A/Astrakhan/3212/2020 (H5N8) HA protein-carrying pseudovirus

Lenti-X 293T cells (Takara, # 632180) were transfected with three types of plasmids: A/Astrakhan/3212/2020 (H5N8) HA protein expression plasmid (Example 63); HIV-derived lentivirus packaging plasmid; and reporter protein (coGFP, luciferase) expression plasmid (pGreenFire Transcriptional Reporter Lentivector) (SBI, #TR000PA-1) using Lipofectamine 3000 (ThermoFisher Scientific, #L3000001). As a cell culturing medium after the transfection, Opti-MEM I (gibco, #11058-021) was used. Neuraminidase (Nacalai, #24229-74) was added 24 hours after the transfection. After 48 hours, the culture supernatant was collected, filtered through a 0.45 μm filter, and then frozen at - 80°C. Transfection with only two types of plasmids: HIV-derived lentivirus packaging plasmid; and reporter protein (coGFP, luciferase) expression plasmid (pGreenFire Transcriptional Reporter Lentivector) (SBI, #TR000PA-1) was performed to produce HA protein-free pseudoviruses. Each pseudovirus was treated with trypsin (37°C, 30 min), then serially diluted with Opti-MEMI, and infected with MDCK-SIAT1 cells (96-well plate) at 50 μl/well. After 48 hours, 50 μl of a luciferase luminescent reagent (Bright-Glo reagent) (Promega, # E2610) was added, the mixture was stirred for 20 seconds, and then subjected to photometry using a luminometer. A cut-off value was set using the luciferase activity value of HA protein-free pseudovirus as a benchmark, and whether infection occurred or not was determined. The 50% tissue culture infectious dose ($TCID_{50}$) of each pseudovirus was calculated.

[Example 66]

Production of A/Laos/2121/2020 (H5N1) HA protein-carrying pseudovirus

Lenti-X 293T cells (Takara, # 632180) were transfected with three types of plasmids: A/Laos/2121/2020 (H5N1) HA protein expression plasmid (Example 64); HIV-derived lentivirus packaging plasmid; and reporter protein (coGFP, luciferase) expression plasmid (pGreenFire Transcriptional Reporter Lentivector) (SBI, #TR000PA-1) using Lipofectamine 3000 (ThermoFisher Scientific, #L3000001). As a cell culturing medium after the transfection, Opti-MEM I (gibco, #11058-021) was used. Neuraminidase (Nacalai, #24229-74) was added 24 hours after the transfection. After 48 hours, the culture supernatant was collected, filtered through a 0.45 μm filter, and then frozen at -80°C. Transfection with only two types of plasmids: HIV-derived lentivirus packaging plasmid; and reporter protein (coGFP, luciferase) expression plasmid (pGreenFire Transcriptional Reporter Lentivector) (SBI, #TR000PA-1) was performed to produce HA protein-free pseudoviruses.

Each pseudovirus was treated with trypsin (37°C, 30 min), then serially diluted with Opti-MEMI, and infected with MDCK-SIAT1 cells (96-well plate) at 50 μl/well. After 48 hours, 50 μl of a luciferase luminescent reagent (Bright-Glo reagent) (Promega, # E2610) was added, the mixture was stirred for 20 seconds, and then subjected to photometry. A cut-off value was set using the luciferase activity value of HA protein-free pseudovirus as a benchmark, and whether infection occurred or not was determined. The 50% tissue culture infectious dose ($TCID_{50}$) of each pseudovirus was calculated.

(Test Example 1)

Immunogenicity of monovalent LNP-mRNA: Ability to induce production of IgG specific to HA of A/Puerto Rico/8/34 (H1 subtype) (Figure 1)

To the femoral area of six-week-old BALB/c mice, an inactivation split vaccine was intramuscularly administered in amounts of 0.01, 0.1 and 1 μg of HA twice at an interval of two weeks. Alternatively, the mRNA-encapsulating nucleic acid lipid particles in Example 13 were administered in amounts of 3, 10 and 30 μg of mRNA twice at an interval of two weeks. Two weeks after the final administration, the blood was collected, and serum was prepared. Serum IgG specific to HA was detected by an enzyme-linked immunosorbent assay (ELISA) method. For immobilization treatment in the ELISA method, 0.5 μg/mL HA of A/Puerto Rico/8/34 was added to a 96-well plate at 25 μL/well, and immobilized overnight at 4°C. At the same time, a mouse IgG whose concentration was known was serially diluted and similarly immobilized for preparing a standard curve. The immobilization solution was removed, and Dulbecco phosphate buffer saline (DPBS) containing 1% bovine serum albumin (BSA) and 0.05% Tween 20 (ELISA solution) was then added at 120 μL/well to perform blocking treatment. The serum was serially diluted with the ELISA solution. After the blocking treatment, washing was performed three times using DPBS containing 0.05% Tween 20 (washing solution), and the diluted serum was added at 25 μL/well To the well for a standard curve, the ELISA solution was added instead of the diluted serum. The mixture was reacted at room temperature for 1 hour, then washed three times with a washing solution, and horse radish peroxidase (HRP)-labeled anti-mouse IgG was added. The mixture was reacted at room temperature for 1 hour, and then washed with three times, and a 3,3',5,5'-tetramethylbenzidine (TMB) peroxidase substrate was added at 30 μL/well. The mixture was incubated at room temperature for 10 minutes to cause color development, a color development stop solution was then added at 30 μL/well, and the absorbance was measured at 450 nm. The serum concentration of specific IgG was calculated from the standard curve.

(Test Example 2)

Protective effect of monovalent LNP-mRNA: Protective effect as measured by virus titer in the lung as indicator against challenge infection with A/Puerto Rico/8/34 (Figure 2)

The mRNA-encapsulating nucleic acid lipid particles in Example 13 were administered in the same manner as in Test Example 1. Two weeks after the final administration, the mouse lung was subjected to challenge infection with 100 PFU (plaque-forming unit) of A/Puerto Rico/8/34. Two days after the challenge infection, the mice were exsanguinated, the lung was harvested, and a lung homogenate was prepared in DPBS containing 0.02% BSA. The cells were confluently proliferated in a 6-well plate. Madin-Darby Canine Kidney (MDCK) cells were washed once with DPBS, 200 μL of the lung homogenate was serially diluted by 10-fold and allowed to contact the cells for 1 hour in an incubator set at 37°C and 5% $CO_2$. Washing was performed with DPBS, and modified Eagle medium containing 0.2% BSA, a 25 mM HEPES buffer solution, 0.01% DEAE-dextrin, 1 μg/mL trypsin, 0.001% phenol red, 50 U/mL penicillin, 50 μg/mL streptomycin, 0.1% Fungizone, 0.001% phenol red and 0.6% agar was added at 3 mL/well. After solidification, the cells were cultured for 2 days in an incubator set at 37°C and 5% $CO_2$. The agar medium was removed, and a 19% methanol solution containing 0.1% crystal violet was added to fix and stain the cells. The staining solution was washed off with tap water, plaques were then counted, and the virus titer in the lung homogenate was calculated.

(Test Example 3)

Protective effect of monovalent LNP-mRNA: Life-extending effect and protective effect as measured by body weight change as indicator against challenge infection with A/Puerto Rico/8/34 (Figures 3 and 4)

The mRNA-encapsulating nucleic acid lipid particles in Example 13 were administered in the same manner as in Test Example 1. Two weeks after the final administration, mice were subjected to challenge infection with 200 PFU of A/Puerto Rico/8/34 intranasally. The body weight was measured six days after the challenge infection, and survival over time was observed until 21 days after the challenge infection.

(Test Example 4)

Immunogenicity of monovalent LNP-mRNA: Ability to produce T cell cytokines specific to HA of A/Puerto Rico/8/34 (Figures 5 and 6)

The mRNA-encapsulating nucleic acid lipid particles in Example 13 were administered in the same manner as in Test Example 1. Two weeks after the final administration, the spleen was harvested, and spleen cells were prepared. The spleen cells were stimulated with HA of A/Puerto Rico/8/34 and cultured for 24 hours in an incubator set at 37°C and 5% $CO_2$. The concentrations of Th1 cytokines interferon-γ (IFN-γ), interleukin-2 (IL-2) and tumor necrosis factor-α (TNF-α) , and the concentrations of Th2 cytokines IL-4, IL-6 and IL-10 secreted in the culture supernatant were measured by multiplex assay using flow cytometry.

(Test Example 5)

Immunogenicity of monovalent LNP-mRNA: Ability to produce CD4-positive or CD8-positive T cell cytokines specific to HA of A/Puerto Rico/8/34 (Figures 7 and 8)

From 1 μg of the inactivation split vaccine prepared in Test Example 4 or 30 μg of spleen cells derived from cells receiving LNP-mRNA, CD4-positive T cells or CD8-positive T cells were removed by a cell separation method using a magnetic column. The spleen cells after removal of CD4-positive T cells or CD8-positive T cells were stimulated with HA of A/Puerto Rico/8/34, cultured in an incubator set at 37°C and 5% $CO_2$. The concentrations of IFN-γ, IL-2 and TNF-α that are Th1 cytokines secreted in the culture supernatant, and the concentrations of IL-4, IL-6 and IL-10 that are Th2 cytokines were measured by multiplex assay using flow cytometry.

(Test Example 6)

Immunogenicity of monovalent LNP-mRNA in mice infected with A/Puerto Rico/8/34: Ability to induce production of A/Puerto Rico/8/34 HA-specific IgG and hemagglutination inhibition (HI) antibody (Figures 9 and 10)

Six-week-old BALB/c mice were infected intranasally with 1,000 PFU of A/Puerto Rico/8/34 or intrapulmonary with 30 PFU of A/Puerto Rico/8/34. To the intrapulmonary infected mice, laninamivir octanoic acid ester hydrate was therapeutically administered seven hours after the infection. Thirteen weeks after the infection, an inactivation split vaccine was subcutaneously administered to the back at a maximum reaction dose of 3 μg, or the mRNA-encapsulating nucleic acid lipid particles in Example 14 were administered into the gastrocnemius muscle at a maximum reaction dose of 30 μg of mRNA. The blood was collected from the time of infection, and serum was prepared. The serum concentration of IgG specific to HA was measured by an ELISA method, and the serum HI antibody titer 20 weeks after the infection was measured.

The HI antibody titer was measured using chicken erythrocytes. Three volumes of a receptor destroying enzyme

were added to one volume of serum, and the mixture was reacted overnight at 37°C. Further, 6 volumes of physiological saline were added, followed by addition of 1 volume of packed erythrocytes. The mixture was reacted at room temperature for 60 minutes, the supernatant was separated by centrifugation, and a 10-fold diluted serum was prepared. The 10-fold diluted serum was serially diluted with DPBS to obtain a serum specimen. To 25 μL of the serum specimen, 25 μL of an inactivation split vaccine of A/Puerto Rico/8/34 adjusted to have the HA value increased by 8 times in concentration, and the mixture was incubated at room temperature for 60 minutes. To this, 50 μL of erythrocyte suspension liquid adjusted to a hematocrit value of 0.5% with DPBS was added. The mixture was incubated at room temperature for 45 to 60 minutes, aggregation was then visually evaluated, and the HI antibody titer was determined.

(Test Example 7)

Immunogenicity of monovalent LNP-mRNA: Ability to induce production of a HI antibody against A/Singapore/GP1908/2015 (H1 subtype), A/Singapore/INFIMH-16-0019/2016 (H3 subtype), B/Phuket/3073/2013 (Yamagata lineage) or B/Maryland/15/2016 (Victoria lineage) (Figures 11, 12, 13 and 14)

To the femoral area of six-week-old BALB/c mice, an A/Singapore/GP1908/2015, A/Singapore/INFIMH-16-0019/2016, B/Phuket/3073/2013 or B/Maryland/15/2016 inactivation split vaccine was intramuscularly administered in amounts of 3, 10 and 30 μg of HA twice at an interval of two weeks. Alternatively, the mRNA-encapsulating nucleic acid lipid particles in Examples 15, 16, 17 or 18 were administered in amounts of 3, 10 and 30 μg of mRNA twice at an interval of two weeks. Two weeks after the final administration the blood was collected and serum was prepared. The serum HI antibody titer was measured against an inactivation split vaccine corresponding to each subtype or lineage of the administered vaccine.

(Test Example 8)

Immunogenicity of monovalent LNP-mRNA: Ability to induce production of an antibody against H3 subtype antigen drift strain HA (Figures 15 and 16)

Using the A/Singapore/INFIMH-16-0019/2016 inactivation split vaccine prepared in Test Example 7 and the serum of mice receiving mRNA-encapsulating nucleic acid lipid particles in Example 16, the total length of HA, and the serum concentration of IgG specific to HA1 was measured by an ELISA method. For immobilization treatment in the ELISA method, 0.5 μg/mL HA of A/Hong Kong/4801/2014 or A/Aichi/2/68 (H3 subtype) was added to a 96-well plate at 25 μL/well, and immobilized overnight at 4°C. For the immobilization antigen, full-length HA of A/Hong Kong/4801/2014 or A/Aichi/2/68 and HA1 were used. At the same time, a mouse IgG whose concentration was known was serially diluted and similarly immobilized for preparing a standard curve. The immobilization solution was removed, and washing was then performed three times with a washing solution, followed by blocking treatment with an ELISA solution at 120 μL/well. The serum was serially diluted with the ELISA solution. After the blocking treatment, washing was performed three times with a washing solution, and the diluted serum was added at 25 μL/well. To the well for a standard curve, the ELISA solution was added instead of the diluted serum. The mixture was reacted at room temperature for 1 hour, then washed three times with a washing solution, and a TMB peroxidase substrate was added at 30 μL/well. The mixture was incubated at room temperature for 10 minutes to cause color development, a color development stop solution was then added at 30 μL/well, and the absorbance was measured at 450 nm. The serum concentration of specific IgG was calculated from the standard curve.

(Test Example 9)

Protective effect of monovalent LNP-mRNA: Protective effect as measured by virus titer in the lung as indicator against challenge infection with H3 subtype antigen drift strain A/Guizhou/54/89 (Figure 17)

To the groin of six-week-old BALB/c mice, an A/Singapore/INFIM-H-16-0019/2016 inactivation split vaccine was subcutaneously administered in amounts of 1 and 10 μg of HA twice at an interval of two weeks. Alternatively, the mRNA-encapsulating nucleic acid lipid particles in Example 19 were administered into the gastrocnemius muscle in amounts of 1 and 10 μg of mRNA twice at an interval of two weeks. Two weeks after the final administration, the mice were subjected to challenge infection with A/Guizhou/54/89 as an antigen drift strain of A/Singapore/INFIMH-16-0019/2016 intranasally. The virus titer in the lung homogenate harvested two days after the challenge infection was calculated.

(Test Example 10)

Immunogenicity and vaccine interference of quadrivalent LNP-mRNA: Ability to induce production of a HI antibody against A/Singapore/GP1908/2015, A/Singapore/INFIMH-16-0019/2016, B/Phuket/3073/2013 or B/Maryland/15/2016 (Figures 18, 19, 20 and 21)

To the groin of 7-week-old BALB/c mice, an A/Singapore/GP1908/2015, A/Singapore/INFIMH-16-0019/2016, B/Phuket/3073/2013 or B/Maryland/15/2016 a monovalent or quadrivalent inactivation split vaccine was subcutaneously administered in an amount of 10 μg/strain of HA twice in at an interval of two weeks. Alternatively, the mRNA-encapsulating nucleic acid lipid particles in Examples 20, 21, 22 and 23, which are LNP-mRNA containing mRNA encoding HA of A/Singapore/GP1908/2015, A/Singapore/INFIMH-16-0019/2016, B/Phuket/3073/2013 or B/Maryland/15/2016, were administered into the gastrocnemius muscle in amounts of 0.25, 1 and 4 μg/strain of monovalent or quadrivalent mRNA twice at an interval of two weeks. Two weeks after the final administration, the blood was collected, and serum was prepared. The serum HI antibody titers were measured against the four inactivation split vaccines.

(Test Example 11)

Immunogenicity of quadrivalent LNP-mRNA administered through different routes: Ability to induce production of a HI antibody against A/Singapore/GP1908/2015, A/Singapore/INFIMH-16-0019/2016, B/Phuket/3073/2013 or B/Maryland/15/2016 (Figures 22, 23, 24 and 25)

The mRNA-encapsulating nucleic acid lipid particles Examples 24, 25, 26 and 27, which are LNP-mRNA containing mRNA encoding HA of A/Singapore/GP1908/2015, A/Singapore/INFIMH-16-0019/2016, B/Phuket/3073/2013 or B/Maryland/15/2016, were subcutaneously administered to the groin or administered into the gastrocnemius muscle in amounts of 0.1, 0.3 and 1 μg/strain of monovalent or quadrivalent mRNA twice at an interval of two weeks. Two weeks after the final administration, the blood was collected, and serum was prepared. The serum HI antibody titers were measured against the four inactivation split vaccines.

(Test Example 12)

Immunogenicity of monovalent LNP-mRNAs containing different cationic lipids: Ability to induce production of IgG specific to HA of A/Michigan/45/2015 and a HI antibody against Singapore/GP1908/2015 (Figures 26 and 27)

To the groin of 6- or 7-week-old BALB/c mice, an A/Singapore/GP1908/2015 inactivation split vaccine was administered in an amount of 10 μg of HA twice at an interval of two weeks. Alternatively, the mRNA-encapsulating nucleic acid lipid particles in Examples 28 and 29 were administered into the gastrocnemius muscle in amounts of 0.3, 1 and 3 μg of mRNA twice at an interval of two weeks. Two weeks after the final administration, the blood was collected, and serum was prepared. The serum concentration of IgG specific to HA of A/Michigan/45/2015 was measured by an ELISA method, and the serum HI antibody titer was measured against the A/Singapore/GP1908/2015 inactivation split vaccine.

(Test Example 13)

Immunogenicity of monovalent LNP-mRNAs with LNPs having different lipid compositions: Ability to induce production of IgG specific to HA of A/Michigan/45/2015 (Figures 28, 29 and 30)

The mRNA-encapsulating nucleic acid lipid particles in Examples 30, 31, 32, 33, 34, 35, 36, 37, 38, 39, 40, 41, 42 and 43, which are LNP-mRNA containing mRNA encoding HA of A/Singapore/GP1908/2015, were administered into the gastrocnemius muscle of 6-week-old BALB/c mice in amounts of 0.04, 0.2 and 1.0 μg of mRNA twice at an interval of two weeks. Two weeks after the final administration, the blood was collected, and serum was prepared. The serum concentration of IgG specific to HA of A/Michigan/45/2015 was measured by an ELISA method.

(Test Example 14)

Immunogenicity of monovalent LNP-mRNAs with LNPs having different modified bases: Ability to induce production of IgG specific to HA of A/Puerto Rico/8/34 (Figure 31)

To the femoral area of 6-week-old BALB/c mice, an inactivation split vaccine was intramuscularly administered

in amounts of 0.3, 1 and 3 μg of HA twice at an interval of two weeks. Alternatively, the mRNA-encapsulating nucleic acid lipid particles in Example 44 were administered in amounts of 0.3, 1 and 3 μg of mRNA twice at an interval of two weeks. Two weeks after the final administration, the blood was collected, and serum was prepared. Serum IgG specific to HA was detected by an ELISA method.

(Test Example 15)

Protective effect of monovalent LNP-mRNAs with LNPs having different modified bases: Protective effect as measured by virus titer in the lung against challenge infection with A/Puerto Rico/8/34 (Figure 32)

The mRNA-encapsulating nucleic acid lipid particles in Example 44 were administered in the same manner as in Test Example 14. Two weeks after the final administration, the mouse lung was subjected to challenge infection with 100 PFU of A/Puerto Rico/8/34. The virus titer in the lung homogenate harvested two days after the challenge infection was calculated.

(Test Example 16)

Protective effect of monovalent LNP-mRNAs with LNPs having different modified bases: Life-extending effect and protective effect as measured by body weight change as an indicator against challenge infection with A/Puerto Rico/8/34 (Figures 33 and 34).

The mRNA-encapsulating nucleic acid lipid particles in Example 44 were administered in the same manner as in Test Example 15. Two weeks after the final administration, the mice were subjected to challenge infection with 200 PFU of A/Puerto Rico/8/34 intranasally. The body weight was measured seven days after the challenge infection, and survival over time was observed until 14 days after the challenge infection.

(Test Example 17)

Immunogenicity and vaccine interference of quadrivalent LNP-mRNA with mRNAs having different modified bases (Figures 35, 36, 37 and 38)

The mRNA-encapsulating nucleic acid lipid particles in Examples 45, 46, 47 and 48, which are LNP-mRNA containing mRNA encoding HA of A/Singapore/GP1908/2015, A/Singapore/INFIMH-16-0019/2016, B/Phuket/3073/2013 and B/Maryland/15/2016, were administered in amounts of 0.1, 0.3 and 1 μg/strain of monovalent or quadrivalent mRNA twice at an interval of two weeks. Two weeks after the final administration, the blood was collected, and serum was prepared. The serum HI antibody titers were measured against the four inactivation split vaccines.

(Test Example 18)

Expression of HA from cultured cells containing monovalent LNP-mRNA encapsulating mRNAs having different polyA lengths (Figures 96 and 97)

HA expressed on the surfaces of HEK 293 cells by introducing the LNP-mRNAs of Example 55, 56, 57, 58, 59 and 60, which contain 12.5, 25, 50 and 100 ng of mRNA encoding HAs of A/Guangdong-Maonan/SWL 1536/2019 and having different polyA lengths (polyA lengths of 110, 95, 80, 60, 40 and 20 bases, respectively) and in which the ratio of the total lipid weight to the nucleic acid weight is 25, into the cells was detected by an ELISA method. The HEK 293 cells prepared at a concentration of $4.0 \times 10^5$ cells/mL in the minimum essential medium containing a 1% non-essential amino acid, 50 units/mL penicillin, 50 μg/mL streptomycin and 10% inactivated fetal bovine serum (FBS) were seeded in a collagen type I-coated 96-well plate at 100 μL/well and cultured for 1 day in an incubator set at 37°C and 5% $CO_2$. Further, LNP-mRNA serially diluted with a 10 mM histidine buffer solution (pH 7.0) containing 300 mM sucrose was added at 5 μL/well and cultured for 40.5 hours in an incubator set at 37°C and 5% $CO_2$. The culture supernatant was removed, the surfaces of the cells were washed with DPBS containing 100 μL/well of 1% bovine serum albumin (BSA) at (DPBS/BSA), a phosphate buffer solution containing 4% paraformaldehyde (PFA) was then added at 100 μL/well, the cells were incubated at room temperature for 30 minutes, and fixed. The PFA was removed, the surfaces of the cells were then washed twice with 100 μL/well of DPBS/BSA, and DPBS/BSA was added at 200 μL/well, followed by storage at room temperature. The DPBS/BSA was removed, and the cells were then washed three times with 200 μL/well of DPBS containing 0.05% Tween 20(DPBST). Next, DPBS containing 0.3% hydrogen peroxide was added at 100 μL/well, and the cells were incubated at room temperature for 30 minutes. Further, the cells were washed three times with 200

μL/well of DPBST, a human anti-type A influenza virus HA stalk region antibody diluted with DPBST (DPBST/BSA) containing 1% BSA was then added, and the cells were incubated at room temperature for 1 hour. The cells were washed three times with 200 μL/well of DPBST, a HRP-labeled anti-human IgG antibody diluted with DPBST/BSA was added, and the cells were incubated for 1 hour. The cells were washed three times with 200 μL/well of PBST, and a TMB peroxidase substrate was then added at 50 μL/well. The cells were incubated at room temperature for 10 minutes to cause color development, a color development stop solution containing hydrochloric acid at 50 μL/well was added, and the absorbance was measured at 450 nm.

(Test Example 19)

Immunogenicity of LNP-mRNA-HA (H5): Measurement of neutralization antibody titer in blood of mouse receiving LNP-mRNA-HA (H5) by pseudovirus-based microneutralization assay (MN) method (Figure 97)

To the femoral area of six-week-old BALB/c mice, nucleic acid lipid particles encapsulating mRNA encoding A/Astrakhan/3212/2020(H5N8)-derived HA (Example 62) were intramuscularly administered in an amount of 30 μg of mRNA twice at an interval of two weeks. Two weeks after the final administration, blood was collected, and serum was prepared. The neutralization antibody titer of the serum was measured by a microneutralization assay (MN) using HA protein-carrying pseudovirus. MDCK-SIAT1 cells were seeded in a 96-well plate at $2 \times 10^6$ cells/well and cultured overnight in an incubator set at 37°C and 5% $CO_2$. As a cell culture solution, Dulbecco MEM liquid medium (DMEM) containing 10% feral bovine serum (FBS), penicillin (50 units/ml), streptomycin (50 μg/ml) and geneticin (1 mg/ml) (Sigma-Aldrich, #D5796) was used. Each mouse serum was treated at 37°C for 20 hours with RDE (II) "Seiken" (Denka, #340122), and heated at 56°C for 45 minutes to remove non-specific hemagglutination inhibition substances. For each RDE (II)-treated serum, 80-fold to 10,240-fold diluted solutions were prepared by serial doubling dilution using Opti-MEM I (gibco, #11058-021), and 50 μl of serum with each concentration was mixed with 64 $TCID_{50}$/50 μl of HA protein-carrying pseudovirus (carrying A/Astrakhan/3212/2020 (H5N8) HA protein or A/Laos/2121/2020 (H5N1) HA protein) (Example 65 or 66). The mixed solution was incubated at 37°C for 30 minutes, then added to MDCK-SIAT1 cells (96-well plate) washed with Dulbecco phosphate buffer saline (DPBS), and the cells were cultured for 48 hours in an incubator set at 37°C and 5% $CO_2$. After the cells were cultured for 48 hours, 50 μl of a luciferase luminescent reagent (Bright-Glo reagent) (Promega, # E2610) was added, and the mixture was stirred. The cells were shielded from light, incubated at room temperature for 2 minutes, subjected to photometry using a luminometer, and the neutralization antibody titer was calculated.

(Results of Test Example 1)

Immunogenicity of monovalent LNP-mRNA: Ability to induce production of IgG specific to HA of A/Puerto Rico/8/34 (Figure 1)

Example 13 was intramuscularly administered to BALB/c mice, and the serum specific IgG induction level two weeks after the final administration was examined. The results are shown in Figure 1. The inactivation split vaccine reached the maximum drug effect at 0.1 μg, whereas Example 13 induced specific IgG up to 30 μg in a dose-dependent manner, and at a higher level as compared to the inactivation split vaccine.

(Results of Test Example 2)

Protective effect of monovalent LNP-mRNA: Protective effect as measured by virus titer in the lung as indicator against challenge infection with A/Puerto Rico/8/34 (Figure 2)

Example 13 was intramuscularly administered to BALB/c mice, and two weeks after the final administration, the mice were subjected to challenge infection with a virus homologous to the vaccine. The protective effect was examined as measured by virus titer in the lung two days after the challenge infection as an indicator. The results are shown in Figure 2. In the group receiving Example 13, the virus titer in the lung was below the detection limit at all doses, and an protective effect was exhibited against challenge infection with the homologous virus.

(Results of Test Example 3)

Protective effect of monovalent LNP-mRNA: Life-extending effect and protective effect as measured by body weight change as indicator against challenge infection with A/Puerto Rico/8/34 (Figures 3 and 4)

Example 13 was intramuscularly administered to BALB/c mice, and two weeks after the final administration, the mice were subjected to challenge infection with a virus homologous to the vaccine. The body weight change six days after the challenge infection and the life-extending effect until 21 days after the challenge infection were examined. The results are shown in Figure 3 (body weight change) and Figure 4 (life-extending effect). In the group receiving Example 13, neither body weight loss nor death was observed, and an protective effect was exhibited against challenge infection with the homologous virus.

(Results of Test Example 4)

Immunogenicity of monovalent LNP-mRNA: Ability to produce T cell cytokines specific to HA of A/Puerto Rico/8/34 (Figures 5 and 6)

Example 13 was intramuscularly administered to BALB/c mice, and two weeks after the final administration, spleen cells were harvested. The spleen cells were stimulated with HA, and the amounts of Th1-type and Th2-type cytokines secreted in the culture supernatant were examined. The results are shown in Figure 5 (Th1-type cytokines) and Figure 6 (Th2-type cytokines). By administering Example 13, production of Th1-type cytokines IFN-$\gamma$, IL-2 and TNF-$\alpha$ and Th2-type cytokines IL-4, IL-6 and IL-10 and high cellular immune responses of Th1 type and Th2 type to HA were induced.

(Results of Test Example 5)

Immunogenicity of monovalent LNP-mRNA: Ability to produce CD4-positive or CD8-positive T cell cytokines specific to HA of A/Puerto Rico/8/34 (Figures 7 and 8)

From the spleen cells prepared in Test Example 4, CD4-positive T cells or CD8-positive T cells were removed. The spleen cells were then stimulated with HA, the spleen cells after removal of CD4-positive T cells or CD8-positive T cells were stimulated with HA of A/Puerto Rico/8/34, and the amounts of Th1-type and Th2-type cytokines secreted in the culture supernatant were examined. The results are shown in Figure 7 (Th-type cytokine) and Figure 8 (Th2-type cytokine). The amount of the cytokines whose production was induced by administering Example 13 did not decrease even when CD8-positive T cells were removed, regardless of whether the cytokine was of Th1 type or Th2 type, whereas the amount of the cytokines significantly decreased when CD4-positive T cells were removed. Thus, it was shown that CD4-positive T cells specific to HA were induced at a high level.

(Results of Test Example 6)

Immunogenicity of monovalent LNP-mRNA in mice infected with A/Puerto Rico/8/34: Ability to induce production of A/Puerto Rico/8/34 HA-specific IgG and hemagglutination inhibition (HI) antibody (Figures 9 and 10)

BALB/c mice were infected with A/Puerto Rico/8/34, and after 13 weeks, Example 14 was intramuscularly administered at a maximum reaction dose. The serum level of induction of specific IgG over time after the administration and the HI antibody seven weeks after the administration were examined. The results are shown in Figure 9 (concentration of specific IgG) and Figure 10 (HI antibody titer). In both the intranasally and intrapulmonary infected mice, the booster effects of the inactivation split vaccine and Example 14 were observed, but the level of induction of the antibody as well as the concentration of specific IgG and the HI antibody titer tended to be higher in the group receiving Example B than in the group receiving the inactivation split vaccine.

(Results of Test Example 7)

Immunogenicity of monovalent LNP-mRNA: Ability to induce production of a HI antibody against A/Singapore/GP1908/2015, A/Singapore/INFIMH-16-0019/2016, B/Phuket/3073/2013 or B/Maryland/15/2016 (Figures 11, 12, 13 and 14)

Monovalent Example 15 (H1 subtype), Example 16 (H3 subtype), Example 17 (B Yamagata lineage) or Example

18 (B Victoria lineage) was intramuscularly administered to BALB/c mice, and the serum level of induction of the HI antibody two weeks after the final administration was examined. The results of Examples 15, 16, 17 and 18 are shown in Figures 11, 12, 13 and 14, respectively. The inactivation split vaccine reached the maximum drug effect at 3 μg, whereas Examples 15, 16, 17 and 18 induced the HI antibody at a higher level as compared to the maximum drug effect of the inactivation split vaccine.

(Results of Test Example 8)

Immunogenicity of monovalent LNP-mRNA: Ability to induce production of an antibody against H3 subtype antigen drift strain HA (Figures 15 and 16)

The total length of HA and the serum concentration of IgG specific to HA1 in the serum of mice receiving A/Singapore/INFIMH-16-0019/2016 inactivation split vaccine and Example 16 were measured by an ELISA method. The results are shown in Figures 15 and 16. As ELISA immobilization antigens, A/Hong Kong/4801/2014 similar in antigenicity to the vaccine strain and A/Aichi/2/68 different in antigenicity from the vaccine strain were used. In both the groups receiving the inactivation split vaccine and LNP-mRNA, IgG specific to both immobilization antigens was produced over the full-length HA (Figure 15). In the group receiving LNP-mRNA, a higher concentration of IgG specific to both immobilization antigens was exhibited as compared to the group receiving the inactivation split vaccine. On the other hand, with respect to HA1 (the antibody against this region is capable of neutralizing a virus), IgG specific to both immobilization antigens was produced in the group receiving LNP-mRNA, whereas the ability to bind to HA1 of A/Aichi/2/68 was lost in the group receiving the inactivation split vaccine (Figure 16). These results show that the antibody induced by administration of LNP-mRNA has higher cross reactivity over the group receiving the inactivation split vaccine.

(Results of Test Example 9)

Protective effect of monovalent LNP-mRNA: Protective effect virus titer in lung as indicator against challenge infection with H3 subtype antigen drift strain A/Guizhou/54/89 (Figure 17)

Example 19 was intramuscularly administered to BALB/c mice, and two weeks after the final administration, the mice were subjected to challenge infection with a virus that is an antigen drift strain of the vaccine strain. The protective effect was examined as measured by virus titer in the lung two days after the challenge infection as an indicator. The results are shown in Figure 17. At all the doses, the group receiving Example 19 had a lower virus titer in the lung as compared to the group receiving the inactivation split vaccine at 10 μg (maximum reaction dose). These results show that LMP-mRNA induces a higher level of cross-protection as compared to the group receiving inactivation split vaccine.

(Results of Test Example 10)

Immunogenicity and vaccine interference of quadrivalent LNP-mRNA: Ability to induce production of a HI antibody against A/Singapore/GP1908/2015, A/Singapore/INFIMH-16-0019/2016, B/Phuket/3073/2013 or B/Maryland/15/2016 (Figures 18, 19, 20 and 21)

To BALB/c mice, Examples 20, 21, 22 and 23 were intramuscularly administered twice monovalently or quadrivalently at an interval of two weeks. Two weeks after the final administration, the blood was collected, and the serum HI antibody titer was measured against the inactivation split vaccine in the serum. The results are shown in Figures 18, 19, 20 and 21. For any of the antigens, the groups receiving quadrivalent Examples 20, 21, 22 and 23 were comparable in HI antibody titer to the groups receiving the monovalent counterparts, and vaccine interference in administration of quadrivalent LNP-mRNA was not observed.

(Results of Test Example 11)

Immunogenicity of quadrivalent LNP-mRNA administered through different routes: Ability to induce production of a HI antibody against A/Singapore/GP1908/2015, A/Singapore/INFIMH-16-0019/2016, B/Phuket/3073/2013 or B/Maryland/15/2016 (Figures 22, 23, 24 and 25)

Quadrivalent Examples 24, 25, 26 and 27 were subcutaneously or intramuscularly administered to BALB/c mice. Two weeks after the final administration, the blood was collected, and serum HI antibody titers against four inactivation split vaccines were measured. The results are shown in Figures 22, 23, 24 and 25. When quadrivalent Examples 24, 25, 26 and 27 were administered, the subcutaneous administration group and the intramuscular administration group were comparable in HI antibody titer at respective doses, and A difference in immunogenicity depending on the administration route was not observed.

(Results of Test Example 12)

Immunogenicity of monovalent LNP-mRNAs containing different cationic lipids: Ability to induce production of IgG specific to HA of A/Michigan/45/2015 and a HI antibody against Singapore/GP1908/2015 (Figures 26 and 27)

Example 28 or 29 was intramuscularly administered to BALB/c mice twice at an interval of two weeks. Two weeks after the final administration, the blood was collected, and the HI antibody titer against the inactivation split vaccine of A/Singapore/GP1908/2015 and the serum concentration of IgG specific to HA of A/Michigan/45/2015 were measured. The results are shown in Figures 26 and 27. The groups receiving Examples 28 and 29 were comparable in HI antibody titer, and also comparable in concentration of specific IgG.

(Results of Test Example 13)

Immunogenicity of monovalent LNP-mRNAs with LNPs having different lipid compositions: Ability to induce production of IgG specific to HA of A/Michigan/45/2015 (Figures 28, 29 and 30)

Examples 30, 31, 32, 33, 34, 35, 36, 37, 38, 39, 40, 41, 42 and 43 were intramuscularly administered to BALB/c mice twice at an interval of two weeks. Two weeks after the final administration, the blood was collected, and the serum concentration of IgG specific to HA of A/Michigan/45/2015 was measured. The results are shown in Figures 28, 29 and 30. When the ratio of the phospholipid was changed to 12.5-22.5 mol% and the ratio of cholesterol was changed to 31-41 mol% while the ratio of the cationic lipid was fixed to 45 mol% and the ratio of the PEG lipid was fixed to 1.5 mol%, all the Examples induced production of specific IgG (Figure 28). When the ratio of cholesterol was changed to 21-38.5 mol% and the ratio of the cationic lipid was changed to 42.5-60 mol% while the ratio of the phospholipid was fixed to 17.5 mol% and the ratio of the PEG lipid was fixed to 1.5 mol%, all the Examples induced production of specific IgG (Figure 29). When the ratio of the phospholipid was changed to 10-20 mol% and the ratio of cholesterol was changed to 28.5-38.5 mol% while the ratio of the cationic lipid was fixed to 50 mol% and the ratio of the PEG lipid was fixed to 1.5 mol%, all the Examples induced production of specific IgG (Figure 30).

(Results of Test Example 14)

Immunogenicity of monovalent LNP-mRNAs with mRNAs having different modified bases: Ability to induce production of IgG specific to HA of A/Puerto Rico/8/34 (Figure 31)

Example 44 was intramuscularly administered to BALB/c mice, and the serum level of induction of specific IgG two weeks after the final administration was examined. The results are shown in Figure 31. The inactivation split vaccine reached the maximum drug effect at 0.3 μg, whereas Example 44 induced specific IgG up to 3 μg in a dose-dependent manner, and at a higher level as compared to the inactivation split vaccine.

(Results of Test Example 15)

Immunogenicity of monovalent LNP-mRNAs with mRNAs having different modified bases: Protective effect as measured by virus titer in the lung as indicator against challenge infection with A/Puerto Rico/8/34 (Figure 32)

Example 44 was intramuscularly administered to BALB/c mice, and the mice were subjected to challenge infection with a virus homologous to the vaccine two weeks after the final administration. The protective effect as measured by virus titer in the lung two days after the challenge infection was examined. The results are shown in Figure 32. In the group receiving Example 44, the virus titer in the lung was below the detection limit at all doses, and an protective effect was exhibited against challenge infection with the homologous virus.

(Results of Test Example 16)

Immunogenicity of monovalent LNP-mRNAs with mRNAs having different modified bases: Life-extending effect and protective effect as measured by body weight change as indicator against challenge infection with A/Puerto Rico/8/34 (Figures 33 and 34)

Example 44 was intramuscularly administered to BALB/c mice, and the mice were subjected to challenge infection with a virus homologous to the vaccine two weeks after the final administration. The body weight change seven days after the challenge infection and the life-extending effect until 14 days after the challenge infection were examined. The results are shown in Figure 33 (body weight change) and Figure 34 (life-extending effect). In the group receiving Example 44, neither a body weight loss nor a death was observed, and an protective effect was exhibited against challenge infection with the homologous virus.

(Results of Test Example 17)

Immunogenicity and vaccine interference of quadrivalent LNP-mRNAs with mRNAs having different modified bases (Figures 35, 36, 37 and 38)

To BALB/c mice, monovalent or quadrivalent Examples 45, 46, 47 and 48 were intramuscularly administered twice at an interval of two weeks. Two weeks after the final administration, the blood was collected, and the serum HI antibody titer was measured against the inactivation split vaccine in the serum. The results are shown in Figures 35, 36, 37 and 38. For any of the antigens, the groups receiving quadrivalent Examples 45, 46, 47 and 48 were comparable in HI antibody titer to the groups receiving the monovalent counterparts, and evident vaccine interference in administration of quadrivalent LNP-mRNA was not observed.

The above results show that the lipid particles of the present invention can be used not only as a vaccine with one type of HA antigen (monovalent vaccine) but also as a vaccine with two or more types of HA antigens (polyvalent vaccine) for preventing and/or treating influenza.

(Results of Test Example 18)

Expression of HA from cultured cells containing monovalent LNP-mRNAs encapsulating mRNAs having different polyA lengths (Figure 96)

Examples 55, 56, 57, 58, 59 or 60 were introduced into HEK293 cells, and HA expressed on the surfaces of the cells was detected by an ELISA method. The results are shown in Figure 96 (A/Guangdong-Maonan/SWL1536/2019). HA was expressed about 40.5 hours after introduction of the LNP-mRNAs of Examples 55 to 60.

(Results of Test Example 19)

Ability to induce neutralization antibody in blood of mouse receiving LNP-mRNA-HA(H5) by pseudovirus-based microneutralization assay (MN) method (Figure 97)

The antiserum of BALB/c mice, to which nucleic acid lipid particles encapsulating mRNA encoding A/Astrakhan/3212/2020(H5N8)-derived HA (Example 62) had been administered twice at an interval of two weeks, had neutralization activity until 1,280-fold dilution for pseudovirus carrying A/Astrakhan/3212/2020(H5N8)-derived HA that is a homologous antigen. On the other hand, the antiserum did not have neutralization activity even at 80-fold dilution as a minimum dilution ratio for pseudovirus carrying A/Laos/2121/2020(H5N1)-derived HA that belongs to a different subclade. (The A/Astrakhan/3212/2020 strain HA gene belongs to subclade 2.3.4.4b and the A/Laos/2121/2020 strain HA gene belongs to subclade 2.3.2.1c). The results show that nucleic acid lipid particles encapsulating mRNA encoding A/Astrakhan/3212/2020(H5N8)-derived HA have neutralization antibody inducing ability for homologous strains.

Industrial Applicability

The present invention can be used for preventing and/or treating influenza.

Sequence Listing Free Text

SEQ ID NO: 1: template DNA for IVT of A/Puerto Rico/8/34 (H1 subtype) HA mRNA-001
SEQ ID NO: 2: A/Puerto Rico/8/34 (H1 subtype) HA mRNA-001
SEQ ID NO: 3: DNA fragment containing A/Puerto Rico/8/34 (H1 subtype) HA
SEQ ID NO: 4: sense primer
SEQ ID NO: 5: antisense primer
SEQ ID NO: 6: template DNA of A/Puerto Rico/8/34 (H1 subtype)
SEQ ID NO: 7: A/Puerto Rico/8/34 (H1 subtype) HA mRNA-002 and 003
SEQ ID NO: 8: DNA fragment containing A/Singapore/GP1908/2015 (H1 subtype) HA
SEQ ID NO: 9: template DNA of A/Singapore/GP1908/2015 (H1 subtype) HA
SEQ ID NO: 10: A/Singapore/GP1908/2015 (H1 subtype) HA mRNA-001, 002 and 003
SEQ ID NO: 11: DNA fragment containing A/Singapore/INFIMH-16-0019/2016 (H3 subtype) HA
SEQ ID NO: 12: template DNA of A/Singapore/INFIMH-16-0019/2016 (H3 subtype) HA
SEQ ID NO: 13: A/Singapore/INFIMH-16-0019/2016 (H3 subtype) HA mRNA-001 and 002
SEQ ID NO: 14: DNA fragment containing B/Phuket/3073/2013 (Yamagata lineage) HA
SEQ ID NO: 15: template DNA of B/Phuket/3073/2013 (Yamagata lineage) HA
SEQ ID NO: 16: B/Phuket/3073/2013 (Yamagata lineage) HA mRNA-001 and 002
SEQ ID NO: 17: DNA fragment containing B/Maryland/15/2016 (Victoria lineage) HA
SEQ ID NO: 18: template DNA of B/Maryland/15/2016 (Victoria lineage) HA
SEQ ID NO: 19: B/Maryland/15/2016 (Victoria lineage) HA mRNA-001 and 002
SEQ ID NO: 20: amino acid sequence of A/Puerto Rico/8/34 (H1 subtype) HA
SEQ ID NO: 21: amino acid sequence of A/Singapore/GP1908/2015 (H1 subtype) HA
SEQ ID NO: 22: amino acid sequence of A/Singapore/INFIMH-16-0019/2016 (H3 subtype) HA
SEQ ID NO: 23: amino acid sequence of B/Phuket/3073/2013 (Yamagata lineage) HA
SEQ ID NO: 24: amino acid sequence of B/Maryland/15/2016 (Victoria lineage) HA
SEQ ID NO: 25: amino acid sequence of protease cleavage sequence
SEQ ID NO: 26: template DNA sequence of A/Guangdong-Maonan/SWL1536/2019, polyA110
SEQ ID NO: 27: template DNA sequence of A/Guangdong-Maonan/SWL1536/2019, polyA95
SEQ ID NO: 28: template DNA sequence of A/Guangdong-Maonan/SWL1536/2019, polyA80
SEQ ID NO: 29: template DNA sequence of A/Guangdong-Maonan/SWL1536/2019, polyA60
SEQ ID NO: 30: template DNA sequence of A/Guangdong-Maonan/SWL1536/2019, polyA40
SEQ ID NO: 31: template DNA sequence of A/Guangdong-Maonan/SWL1536/2019, polyA20
SEQ ID NO: 32: template DNA sequence of B/Phuket/3073/2013 (Yamagata lineage), polyA110
SEQ ID NO: 33: template DNA sequence of B/Phuket/3073/2013 (Yamagata lineage), polyA95
SEQ ID NO: 34: template DNA sequence of B/Phuket/3073/2013 (Yamagata lineage), polyA80
SEQ ID NO: 35: template DNA sequence of B/Phuket/3073/2013 (Yamagata lineage), polyA60
SEQ ID NO: 36: template DNA sequence of B/Phuket/3073/2013 (Yamagata lineage), polyA40
SEQ ID NO: 37: template DNA sequence of B/Phuket/3073/2013 (Yamagata lineage), polyA20
SEQ ID NO: 38: mRNA sequence of A/Guangdong-Maonan/SWL 1536/2019, polyA110
SEQ ID NO: 39: mRNA sequence of A/Guangdong-Maonan/SWL 1536/2019, polyA95
SEQ ID NO: 40: mRNA sequence of A/Guangdong-Maonan/SWL 1536/2019, polyA80
SEQ ID NO: 41: mRNA sequence of A/Guangdong-Maonan/SWL 1536/2019, polyA60
SEQ ID NO: 42: mRNA sequence of A/Guangdong-Maonan/SWL 1536/2019, polyA40
SEQ ID NO: 43: mRNA sequence of A/Guangdong-Maonan/SWL 1536/2019, polyA20
SEQ ID NO: 44: mRNA sequence of B/Phuket/3073/2013 (Yamagata lineage), polyA110
SEQ ID NO: 45: mRNA sequence of B/Phuket/3073/2013 (Yamagata lineage), polyA95
SEQ ID NO: 46: mRNA sequence of B/Phuket/3073/2013 (Yamagata lineage), polyA80
SEQ ID NO: 47: mRNA sequence of B/Phuket/3073/2013 (Yamagata lineage), polyA60
SEQ ID NO: 48: mRNA sequence of B/Phuket/3073/2013 (Yamagata lineage), polyA40
SEQ ID NO: 49: mRNA sequence of B/Phuket/3073/2013 (Yamagata lineage), polyA20
SEQ ID NO: 50: amino acid sequence of A/Guangdong-Maonan/SWL1536/2019 HA
SEQ ID NO: 51: DNA fragment containing A/Astrakhan/3212/2020 (H5 subtype) HA
SEQ ID NO: 52: template DNA of A/Astrakhan/3212/2020 (H5 subtype) HA
SEQ ID NO: 53: A/Astrakhan/3212/2020 (H5 subtype) HA mRNA-001
SEQ ID NO: 54: amino acid sequence of A/Astrakhan/3212/2020 (H5 subtype) HA
SEQ ID NO: 55: sequence in which KOZAK sequence and A/Astrakhan/3212/2020 (H5N8) HA gene translation region are connected
SEQ ID NO: 56: sequence in which KOZAK sequence and A/Laos/2121/2020 (H5N1) HA gene translation region are connected

SEQ ID NO: 57: base sequence of translation region of A/Laos/2121/2020 (H5N1) HA
SEQ ID NO: 58: amino acid sequence of translation region of A/Laos/2121/2020 (H5N1) HA

## Claims

1. A lipid particle encapsulating a nucleic acid capable of expressing a haemagglutinin (HA) protein of an influenza virus, wherein

a lipid comprises a cationic lipid having general formula (Ia), or a pharmaceutically acceptable salt thereof:

[Formula 1]

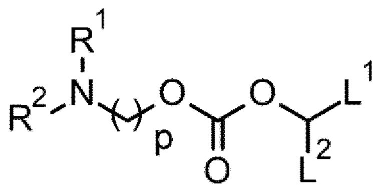

(Ia)

wherein

$R^1$ and $R^2$ each independently represent a C1-C3 alkyl group;
$L^1$ represents a C17-C19 alkenyl group optionally having one or more C2-C4 alkanoyloxy groups;
$L^2$ represents a C10-C19 alkyl group optionally having one or more C2-C4 alkanoyloxy groups, or a C10-C19 alkenyl group optionally having one or more C2-C4 alkanoyloxy groups; and
p is 3 or 4.

2. The particle according to claim 1, wherein each of $R^1$ and $R^2$ in general formula (Ia) is a methyl group.

3. The particle according to claim 1 or 2, wherein p in general formula (Ia) is 3.

4. The particle according to any one of claims 1 to 3, wherein $L^1$ in general formula (Ia) is a C17-C19 alkenyl group optionally having one or more acetyloxy groups.

5. The particle according to any one of claims 1 to 4, wherein $L^2$ in general formula (Ia) is a C10-C12 alkyl group optionally having one or more acetyloxy groups, or a C10-C19 alkenyl group optionally having one or more acetyloxy groups.

6. The particle according to any one of claims 1 to 4, wherein $L^2$ in general formula (Ia) is a C10-C12 alkyl group optionally having one or more acetyloxy groups, or a C17-C19 alkenyl group optionally having one or more acetyloxy groups.

7. The particle according to any one of claims 1 to 6, wherein $L^1$ in general formula (Ia) is a (R)-11-acetyloxy-cis-8-heptadecenyl group, a cis-8-heptadecenyl group, or a (8Z,11Z)-heptadecadienyl group.

8. The particle according to any one of claims 1 to 7, wherein $L^2$ in general formula (Ia) is a decyl group, a cis-7-decenyl group, a dodecyl group, or a (R)-11-acetyloxy-cis-8-heptadecenyl group.

9. The particle according to claim 1, wherein the cationic lipid has the following structural formula:

[Formula 2]

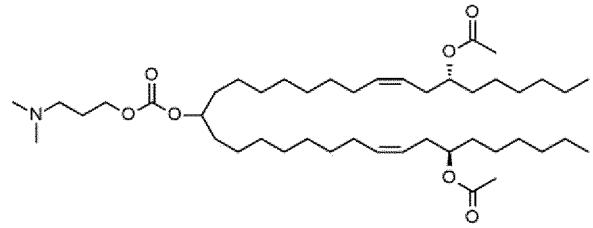

10. The particle according to claim 1, wherein the cationic lipid has the following structural formula:

[Formula 3]

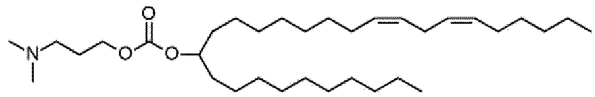

11. The particle according to claim 1, wherein the cationic lipid has the following structural formula:

[Formula 4]

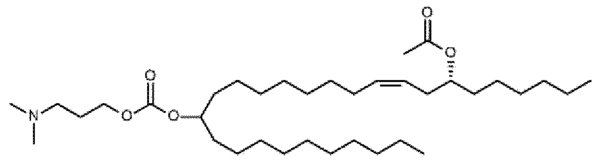

12. The particle according to claim 9 or 10, wherein the lipid further comprises an amphipathic lipid, a sterol and a PEG lipid.

13. The particle according to claim 11, wherein the lipid further comprises an amphipathic lipid, a sterol and a PEG lipid.

14. The particle according to claim 12, wherein the amphipathic lipid is at least one selected from the group consisting of distearoyl phosphatidylcholine, dioleoyl phosphatidylcholine and dioleoyl phosphatidylethanolamine.

15. The particle according to claim 13, wherein the amphipathic lipid is at least one selected from the group consisting of distearoyl phosphatidylcholine, dioleoyl phosphatidylcholine and dioleoyl phosphatidylethanolamine.

16. The particle according to claim 12 or 14, wherein the sterol is cholesterol.

17. The particle according to claim 13 or 15, wherein the sterol is cholesterol.

18. The particle according to any one of claims 12, 14 and 16, wherein the PEG lipid is 1,2-dimyristoyl-sn-glycelol methoxypolyethylene glycol and/or N-[methoxy poly(ethylene glycol) 2000]carbamoyl]-1,2-dimyristyloxypropyl-3-amine.

19. The particle according to any one of claims 13, 15 and 17, wherein the PEG lipid is 1,2-dimyristoyl-sn-glycelol methoxypolyethylene glycol and/or N-[methoxy poly(ethylene glycol) 2000]carbamoyl]-1,2-dimyristyloxypropyl-3-amine.

20. The particle according to any one of claims 12 to 19, wherein the lipid composition of the amphipathic lipid, the sterol, the cationic lipid and the PEG lipid is amphipathic lipid: 5 to 25%, sterol: 10 to 55%, cationic lipid: 40 to 65% and PEG lipid: 1 to 5% on a molar amount basis.

**21.** The particle according to claim 20, wherein the proportion of the amphipathic lipid is 10 to 25%.

**22.** The particle according to any one of claims 12, 14, 16 and 18, wherein the lipid composition of the amphipathic lipid, the sterol, the cationic lipid and the PEG lipid is amphipathic lipid: 5 to 15%, sterol: 35 to 50%, cationic lipid: 40 to 55% and PEG lipid: 1 to 3% on a molar amount basis.

**23.** The particle according to claim 22, wherein the proportions of the amphipathic lipid, the sterol, the cationic lipid and the PEG lipid are 10 to 15%, 35 to 45%, 40 to 50% and 1 to 2.5%, respectively.

**24.** The particle according to claim 23, wherein the proportion of the PEG lipid is 1 to 2%.

**25.** The particle according to any one of claims 13, 15, 17 and 19, wherein the lipid composition of the amphipathic lipid, the sterol, the cationic lipid and the PEG lipid is amphipathic lipid: 10 to 25%, sterol: 10 to 50%, cationic lipid: 40 to 65% and PEG lipid: 1 to 3% on a molar amount basis.

**26.** The particle according to claim 25, wherein the proportions of the sterol, the cationic lipid and the PEG lipid are 10 to 45%, 42.5 to 65% and 1 to 2.5%, respectively.

**27.** The particle according to claim 26, wherein the proportion of the PEG lipid is 1 to 2%.

**28.** The particle according to any one of claims 20 to 27, wherein the ratio of the total weight of lipids to the weight of the nucleic acid is from 15 to 30.

**29.** The particle according to claim 28, wherein the ratio of the total weight of lipids to the weight of the nucleic acid is from 15 to 25.

**30.** The particle according to claim 29, wherein the ratio of the total weight of lipids to the weight of the nucleic acid is from 17.5 to 22.5.

**31.** The particle according to any one of claims 1 to 30, wherein the HA protein of the influenza virus is a fusion protein having an amino acid sequence in which two or more different HA proteins are bound to each other by a linker.

**32.** The particle according to claim 31, wherein the linker has a sequence comprising a protease cleavage site.

**33.** The particle according to any one of claims 1 to 32, wherein the influenza virus is a type-A or type-B influenza virus.

**34.** The particle according to any one of claims 1 to 33, wherein the influenza virus is a type-A or type-B influenza virus, and the HA protein comprises an amino acid sequence having an identity of at least 85% with one amino acid sequence selected from the group consisting of SEQ ID NOS: 20 to 24, 50, 54 and 58.

**35.** The particle according to claim 34, wherein the influenza virus is a type-A or type-B influenza virus, and the HA protein comprises an amino acid sequence having an identity of at least 90% with one amino acid sequence selected from the group consisting of SEQ ID NOS: 20 to 24, 50, 54 and 58.

**36.** The particle according to any one of claims 31 to 35, wherein the nucleic acid capable of expressing a HA protein of an influenza virus is mRNA comprising a cap structure (Cap), a 5' untranslated region (5'-UTR), a translated region of a HA protein, a 3' untranslated region (3'-UTR) and a poly A tail (polyA).

**37.** The particle according to claim 36, wherein the sequence of the nucleic acid capable of expressing a HA protein consists of a nucleotide sequence having an identity of at least 90% with any of the sequences of SEQ ID NOS: 2, 7, 10, 13, 16, 19, 38 to 49 and 53.

**38.** The particle according to any one of claims 31 to 35, wherein the nucleic acid capable of expressing a HA protein of an influenza virus is mRNA having a structure comprising a cap structure (Cap), a 5' untranslated region (5'-UTR), a translated region of a HA protein and a 3' untranslated region (3'-UTR).

**39.** The particle according to claim 38, wherein the structure comprising a cap structure (Cap), a 5' untranslated region (5'-UTR), a translated region of a HA protein and a 3' untranslated region (3'-UTR) comprises a nucleotide sequence

having an identity of at least 90% with any of SEQ ID NO: 2, the sequence of residues 1 to 1900 in SEQ ID NO: 7, the sequence of residues 1 to 1903 in SEQ ID NO: 10, the sequence of residues 1 to 1903 in SEQ ID NO: 13, the sequence of residues 1 to 1957 in SEQ ID NO: 16, the sequence of residues 1 to 1954 in SEQ ID NO: 19, the sequence of residues 1 to 1903 in SEQ ID NO: 38, the sequence of residues 1 to 1957 in SEQ ID NO: 44 and the sequence of residues 1 to 1906 in SEQ ID NO: 53.

**40.** The particle according to any one of claims 1 to 39, wherein the nucleic acid comprises at least one modified nucleotide.

**41.** The particle according to claim 40, wherein the modified nucleotide comprises at least one of pyrimidine nucleotide substituted at the 5-position and/or pseudouridine optionally substituted at the 1-position.

**42.** The particle according to claim 41, wherein the modified nucleotide comprises at least one selected from the group consisting of 5-methylcytidine, 5-methoxyuridine, 5-methyluridine, pseudouridine and 1-alkylpseudouridine.

**43.** The particle according to any one of claims 1 to 42, wherein the average particle size of the particles is 30 to 300 nm.

**44.** The particle according to any one of claims 1 to 43, comprising two or more nucleic acids capable of expressing different HA proteins in one lipid particle.

**45.** Use of the particle according to any one of claims 1 to 44, for producing a composition for preventing and/or treating infection with an influenza virus.

**46.** A composition comprising the particle according to any one of claims 1 to 44.

**47.** A composition comprising two or more types of the particles according to any of claims 1 to 46, which are capable of expressing different HA proteins.

**48.** The composition according to claim 46 or 47, for expressing a HA protein of an influenza virus *in vivo* or *in vitro.*

**49.** The composition according to claim 46 to 48 for use as a medicament.

**50.** The composition according to claim 49 for inducing an immune reaction against an influenza virus.

**51.** The composition according to claim 49 or 50 for preventing and/or treating infection with an influenza virus.

**52.** A method for expressing a HA protein of an influenza virus *in vitro,* comprising introducing the composition according to any one of claims 46 to 48 into cells.

**53.** A method for expressing a HA protein of an influenza virus *in vivo,* comprising administering the composition according to any one of claims 46 to 51 to a mammal.

**54.** A method for inducing an immune reaction against an influenza virus, comprising administering the composition according to claim 49 or 50 to a mammal.

**55.** A method for preventing and/or treating infection with an influenza virus, comprising administering the composition according to any one of claims 49 to 51 to a mammal.

**56.** The method according to any one of claim 53 to 55, wherein the mammal is a human.

[Figure 1]

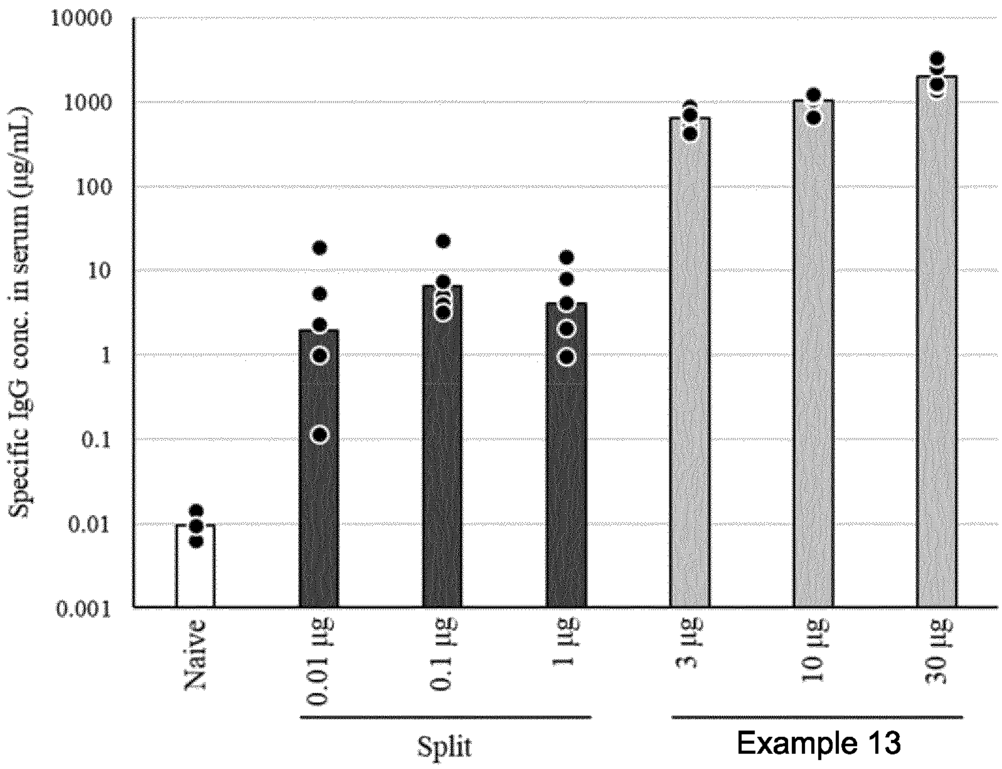


[Figure 2]

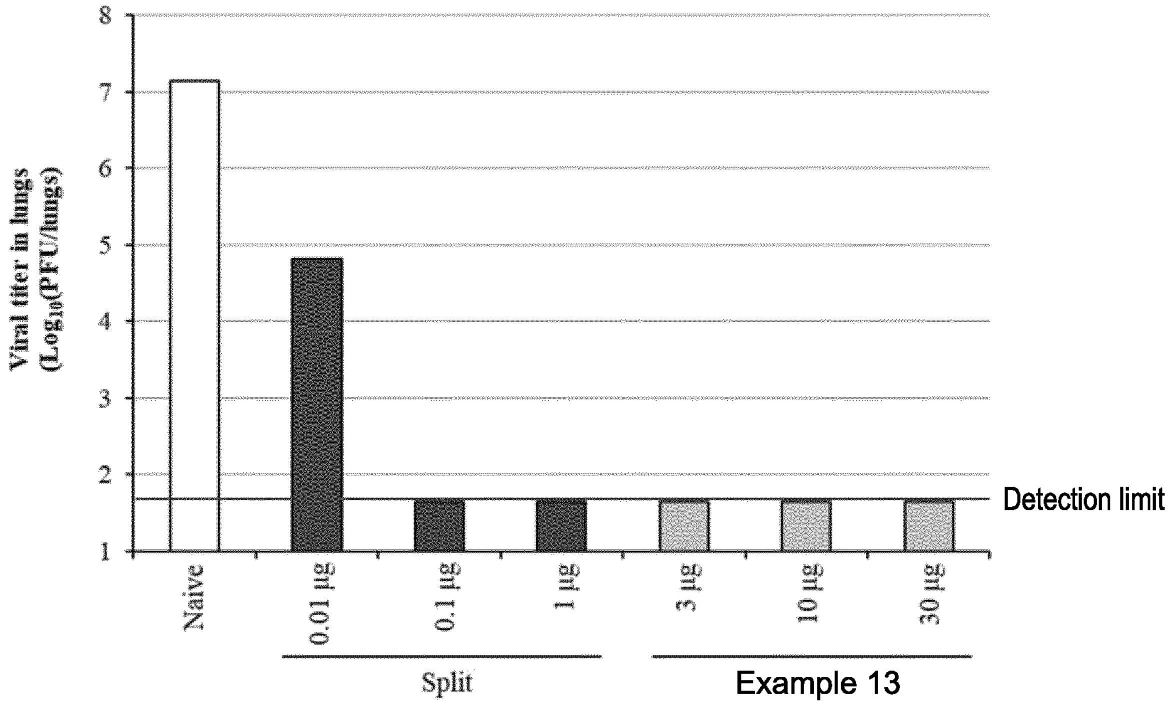

[Figure 3]

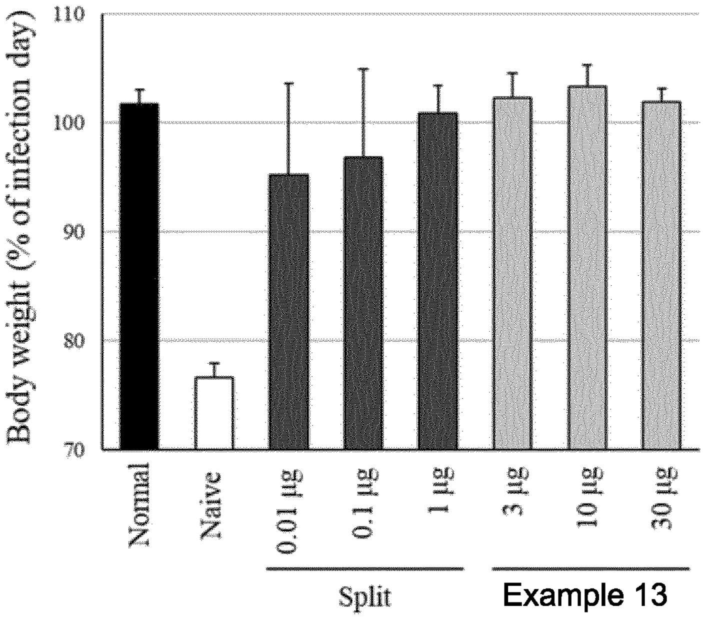


[Figure 4]

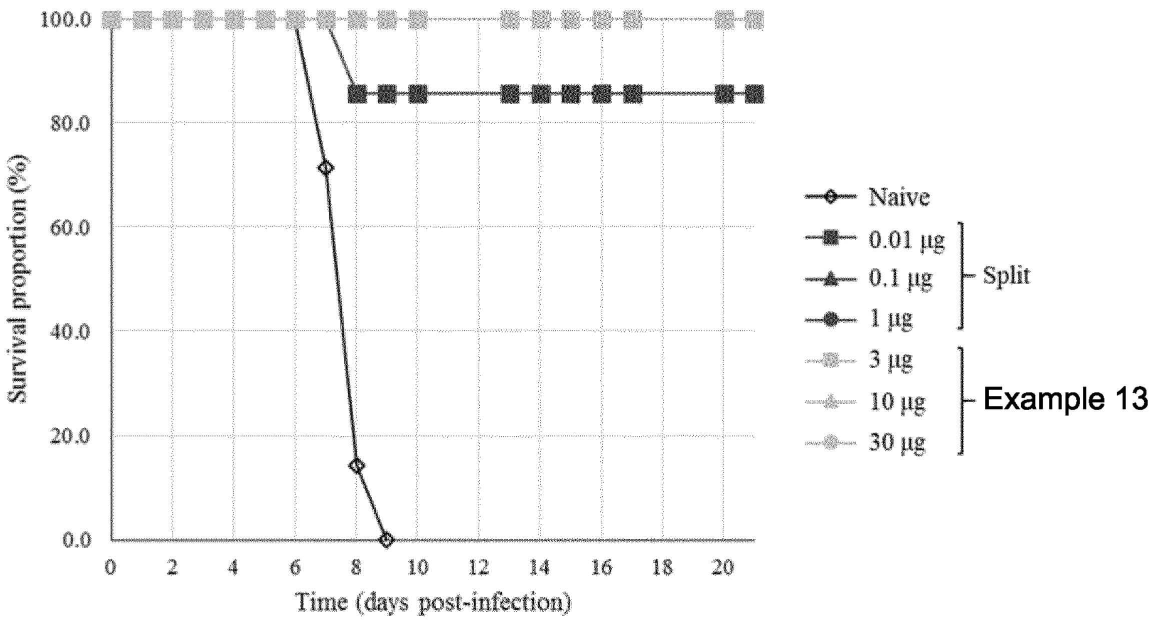

[Figure 5]

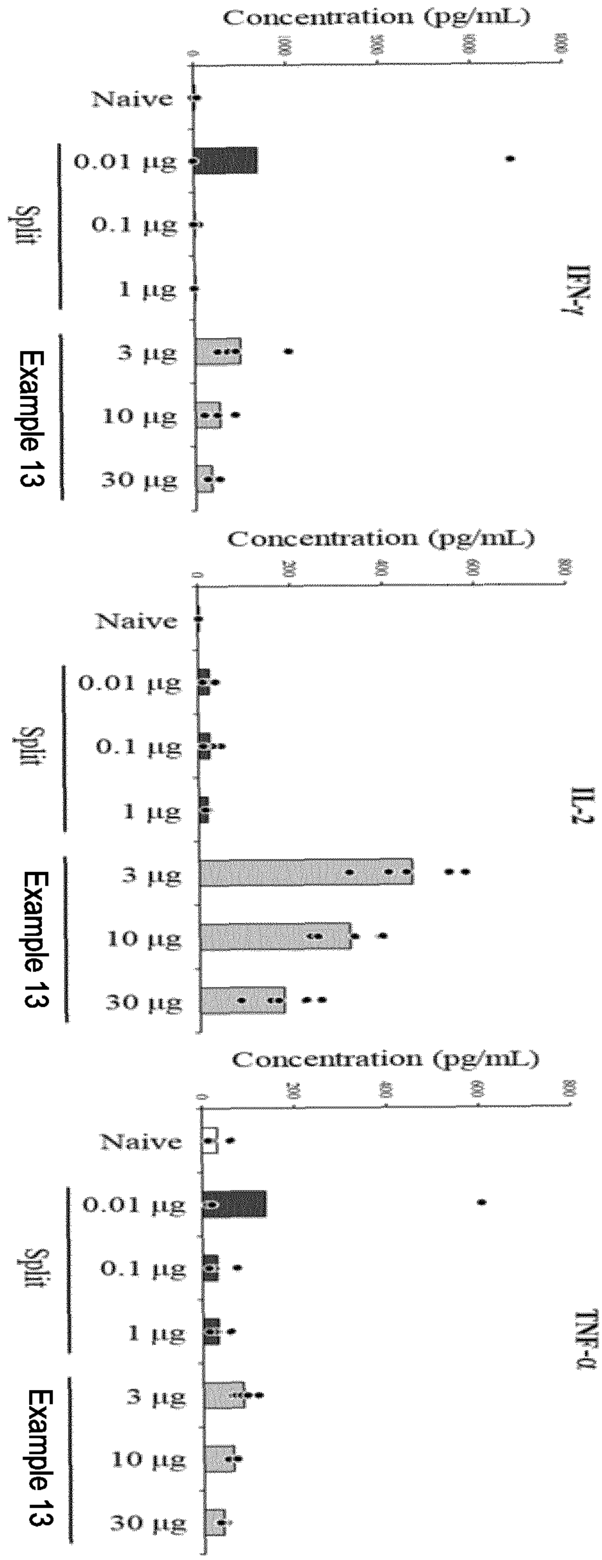
Concentration (pg/mL)
Naive
0.01 µg
0.1 µg
1 µg
3 µg
10 µg
30 µg
Split
Example 13
IFN-γ
IL-2
TNF-α

[Figure 6]

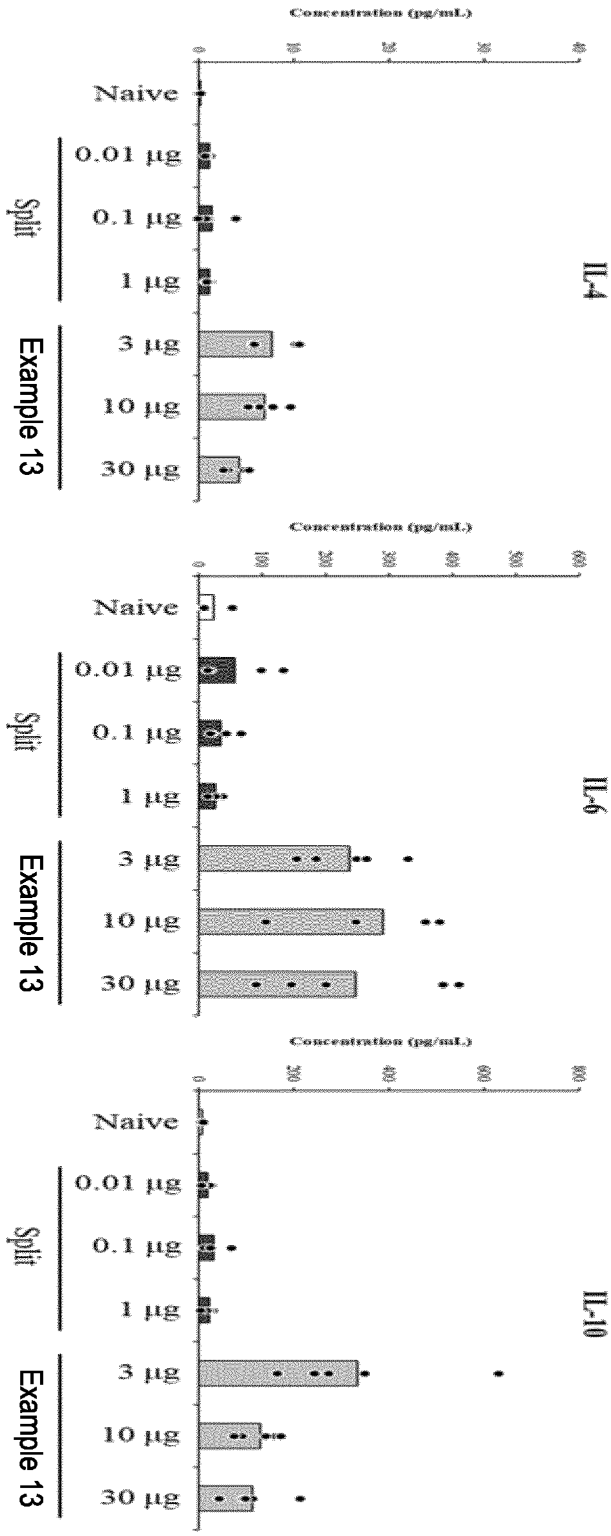
Concentration (pg/mL)
0
10
20
30
40
Naive
Split
0.01 µg
0.1 µg
1 µg
Example 13
3 µg
10 µg
30 µg
IL-4
Concentration (pg/mL)
0
100
200
300
400
500
600
Naive
Split
0.01 µg
0.1 µg
1 µg
Example 13
3 µg
10 µg
30 µg
IL-6
Concentration (pg/mL)
0
200
400
600
800
Naive
Split
0.01 µg
0.1 µg
1 µg
Example 13
3 µg
10 µg
30 µg
IL-10

[Figure 7]

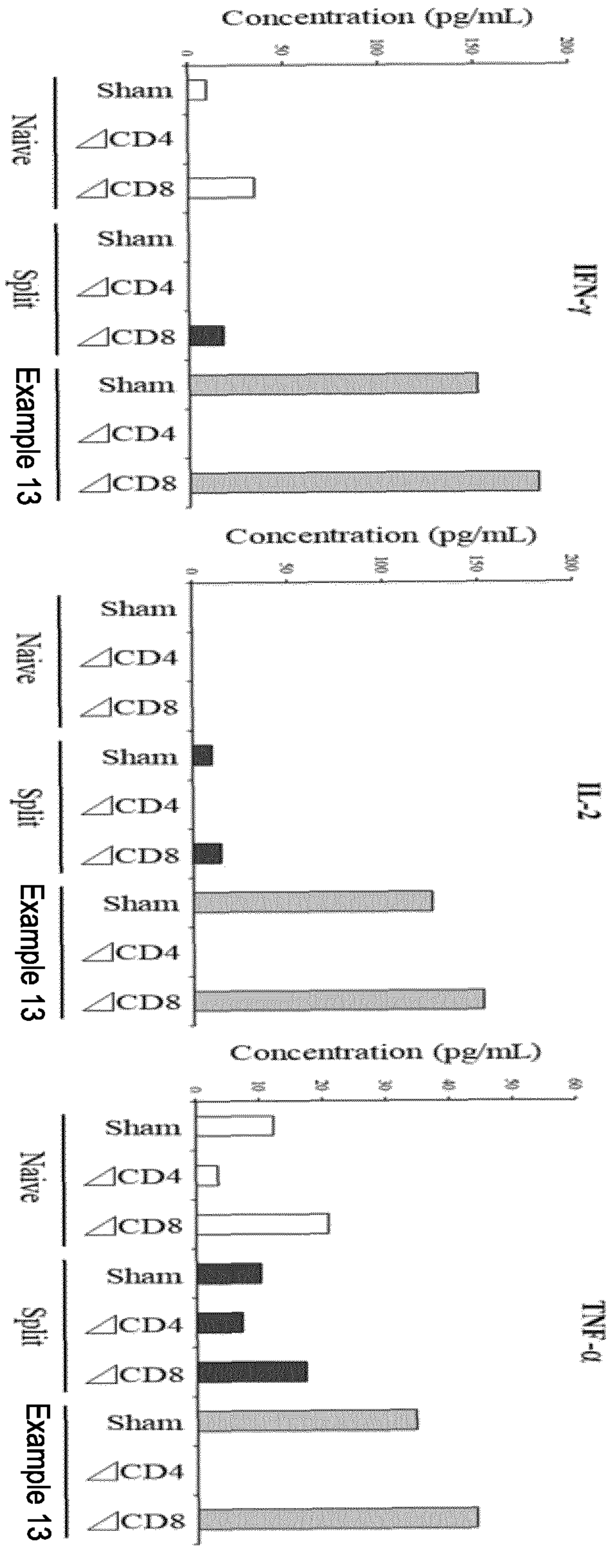
Concentration (pg/mL)
IFN-γ
IL-2
TNF-α
Naive
Split
Example 13
Sham
⊿CD4
⊿CD8

[Figure 8]

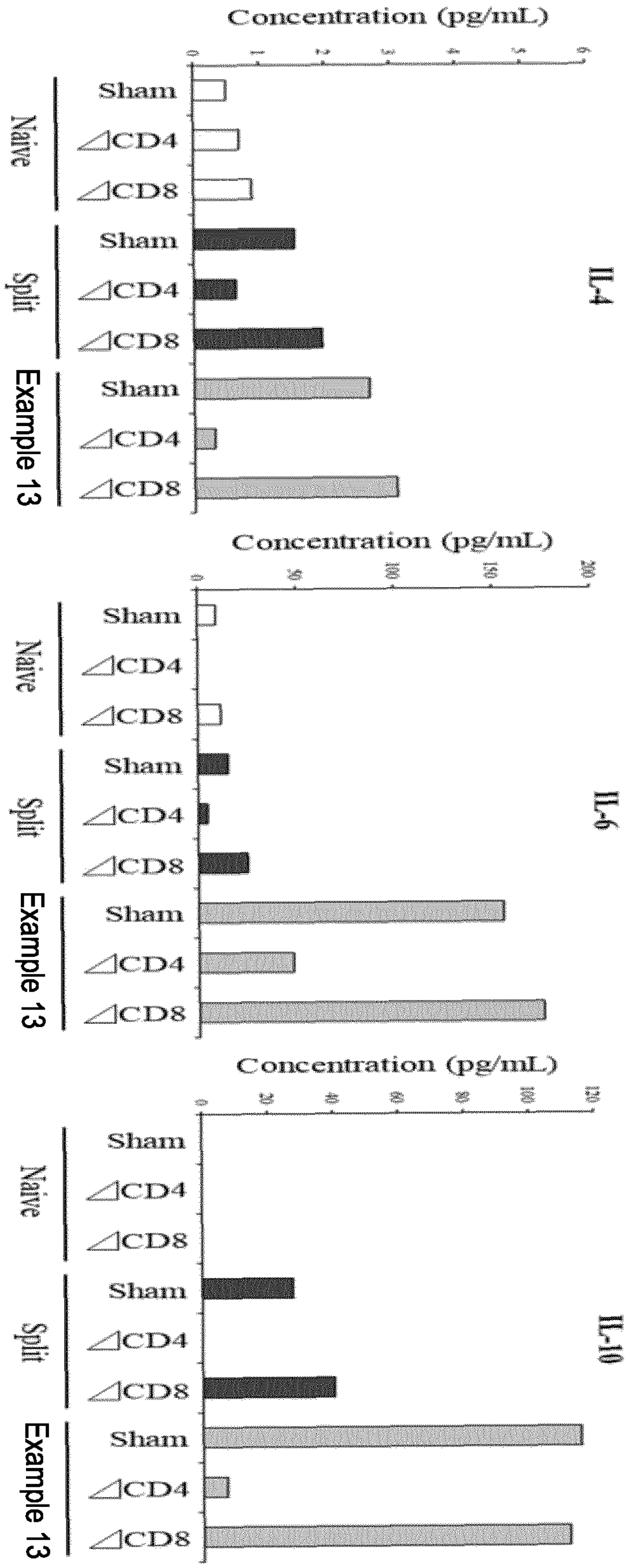
IL-4
IL-6
IL-10
Concentration (pg/mL)
Naive
Split
Example 13
Sham
⊿CD4
⊿CD8

[Figure 9]

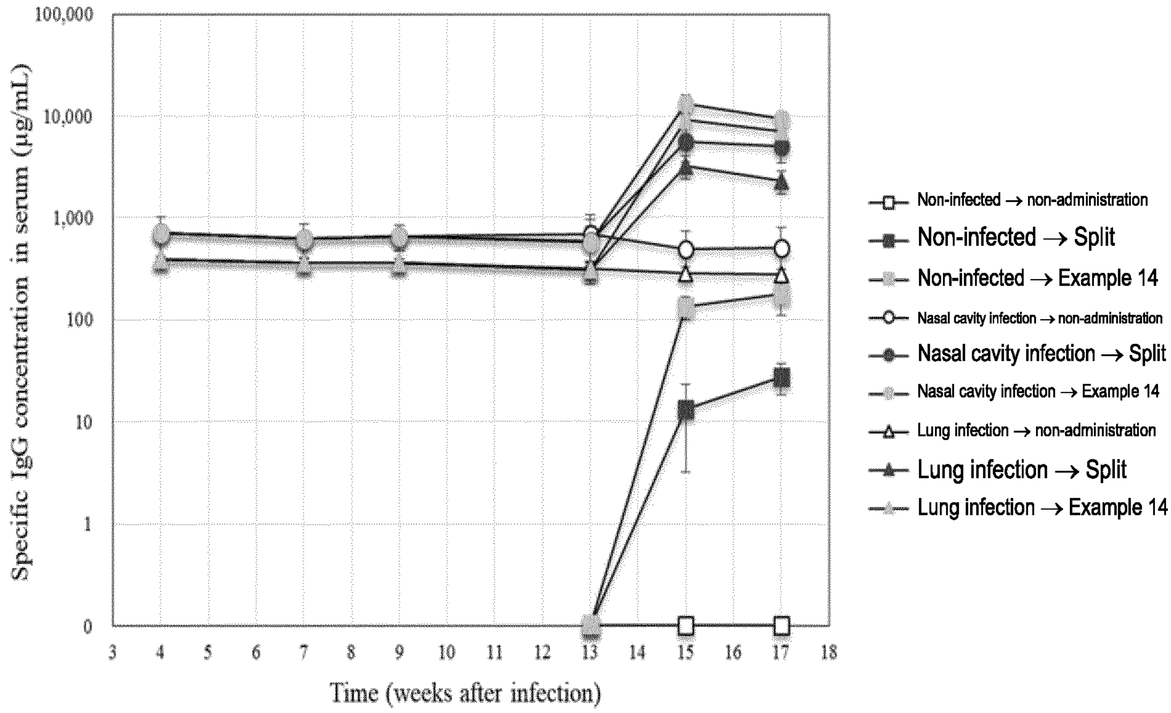


[Figure 10]

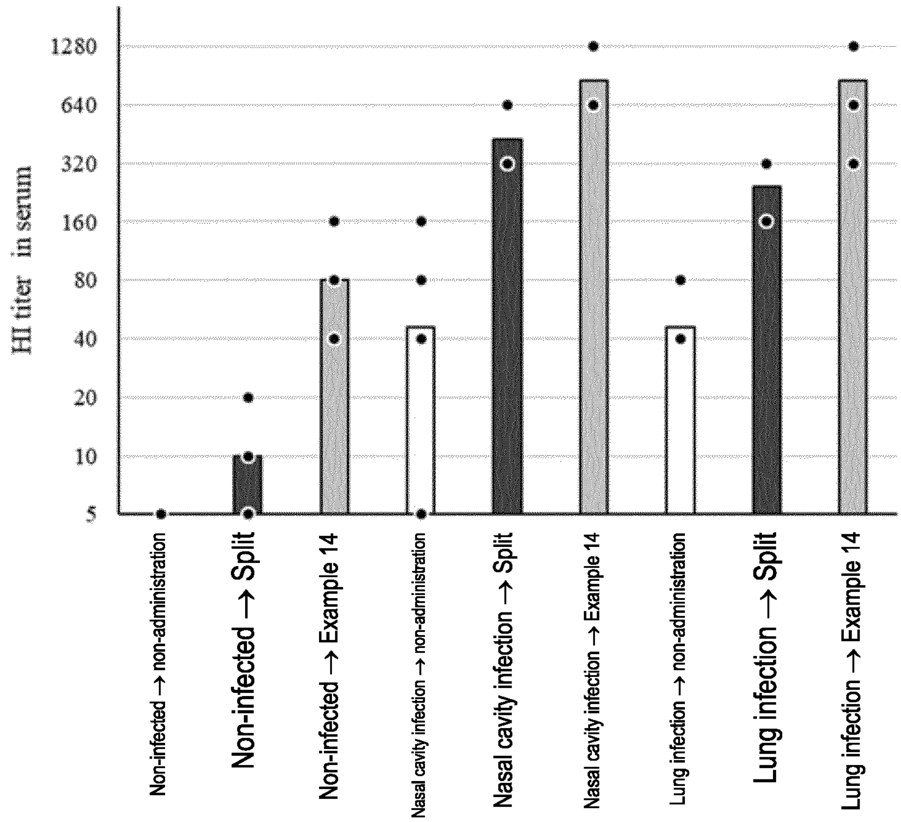

[Figure 11]

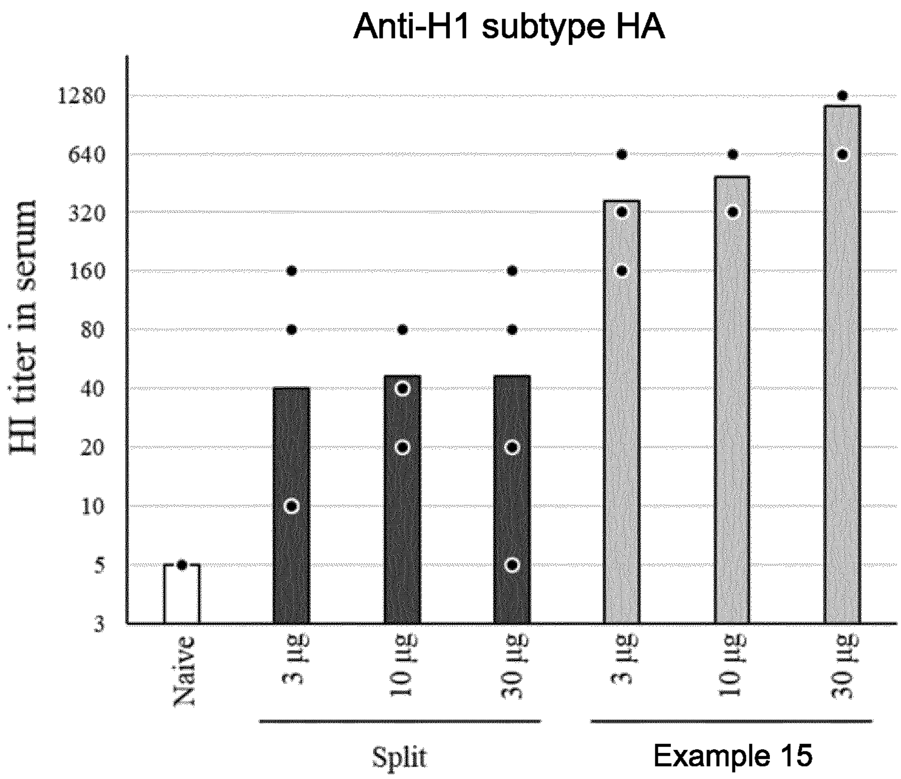


[Figure 12]

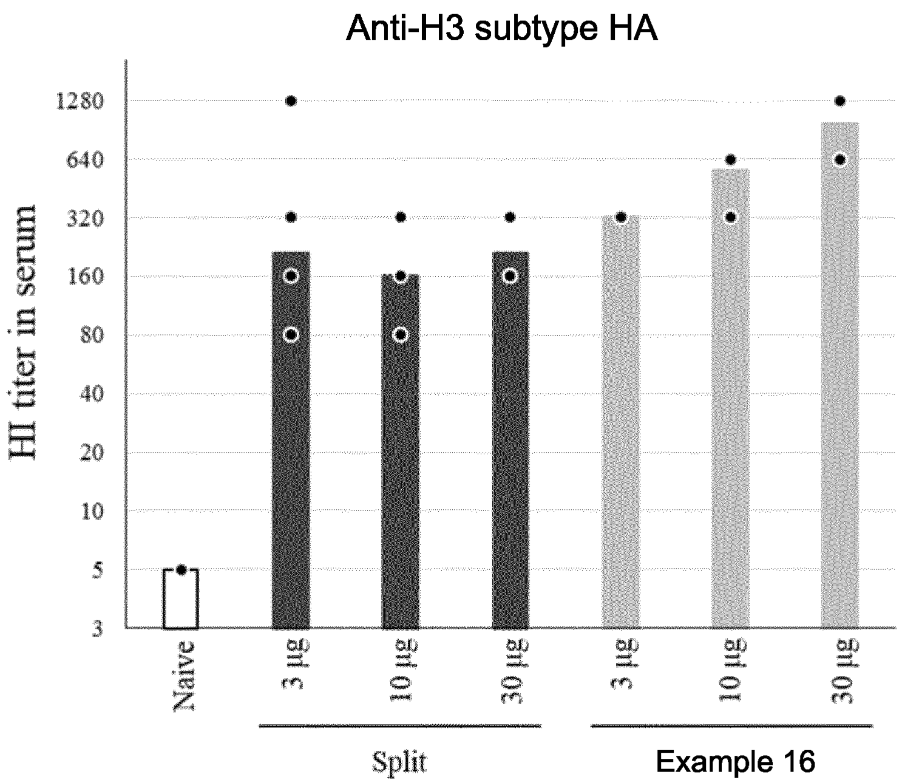

[Figure 13]

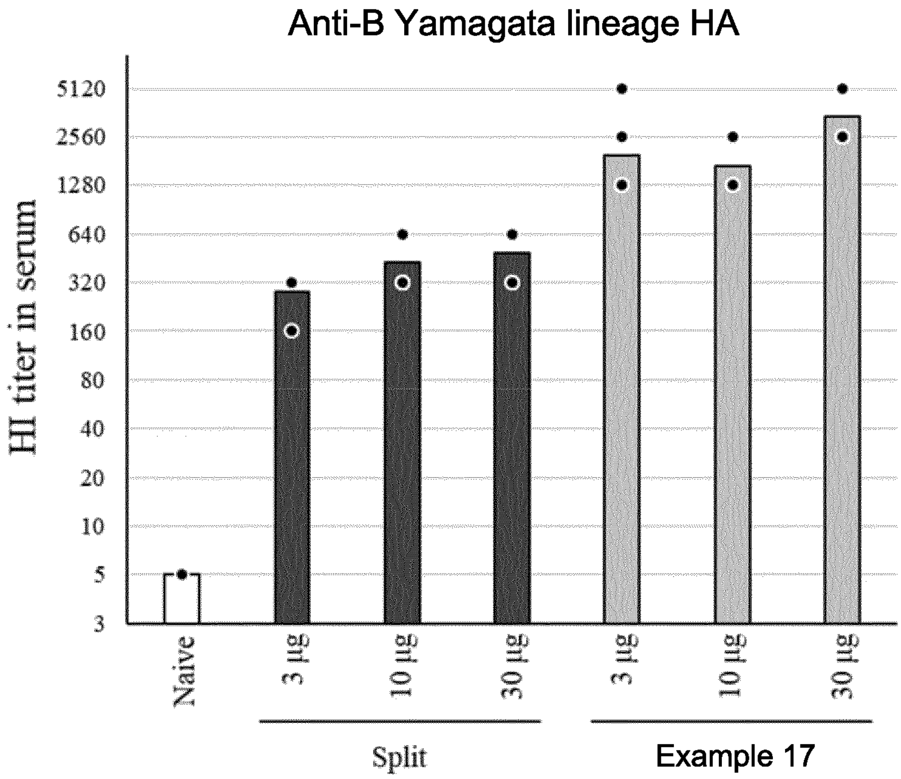


[Figure 14]

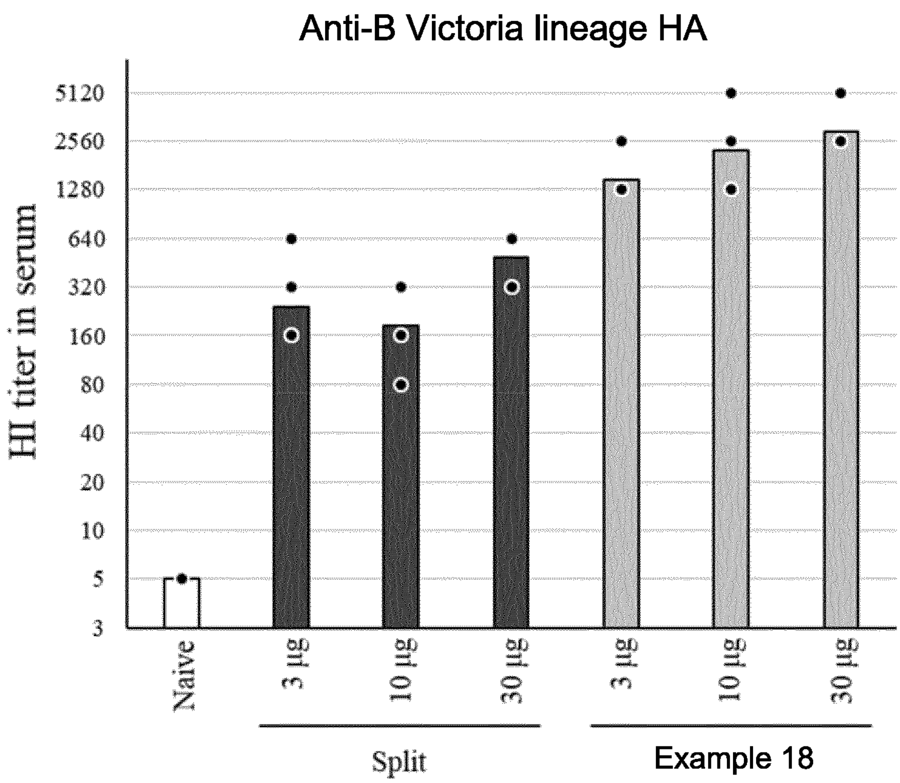

[Figure 15]

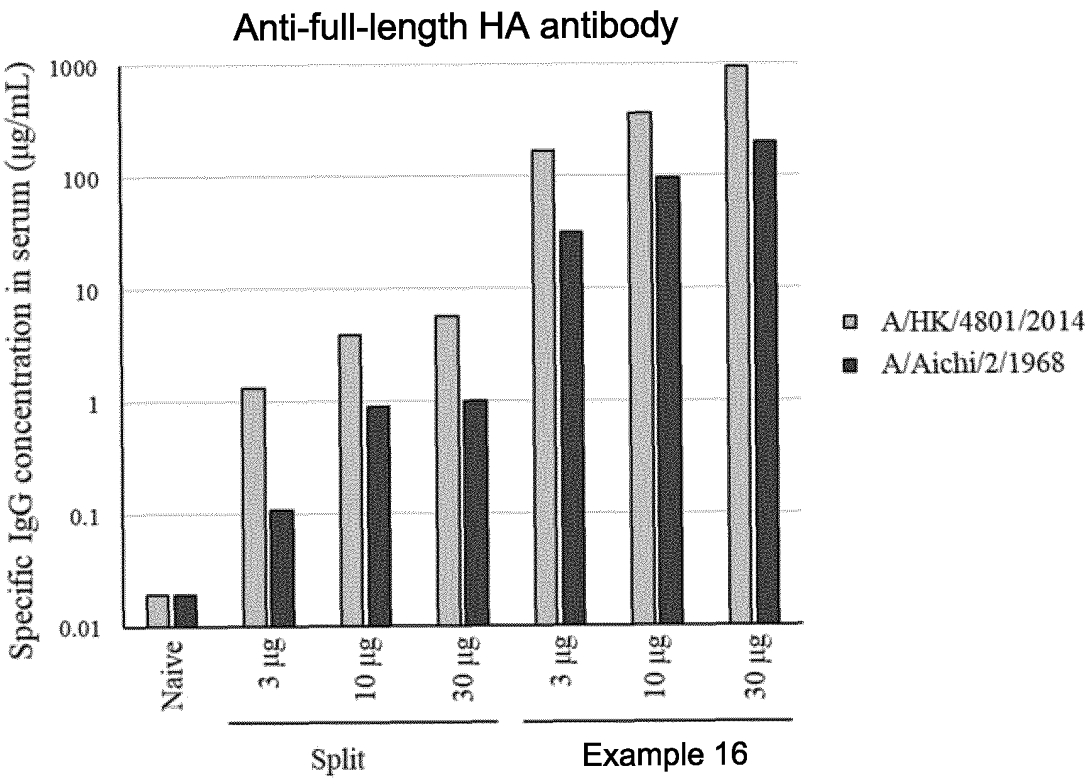


[Figure 16]

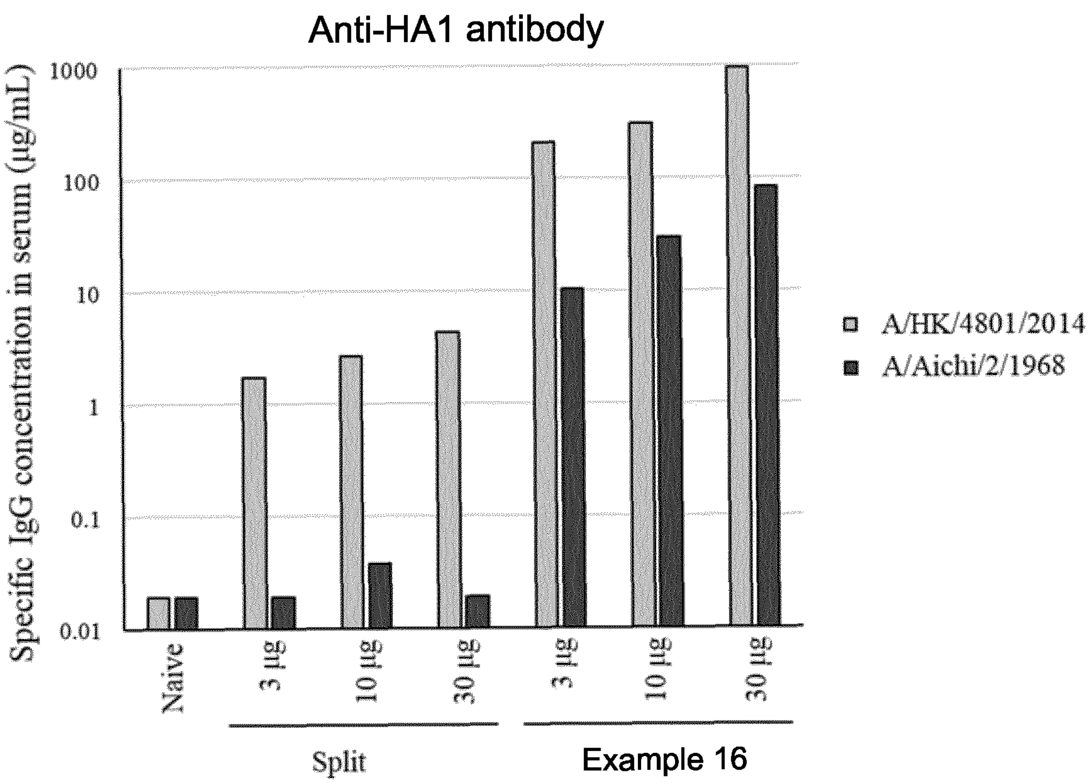

[Figure 17]

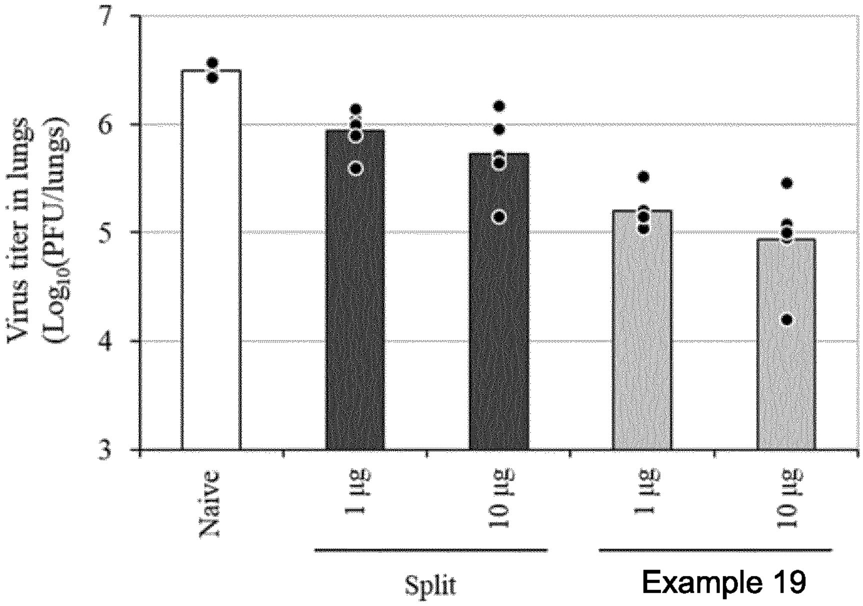


[Figure 18]

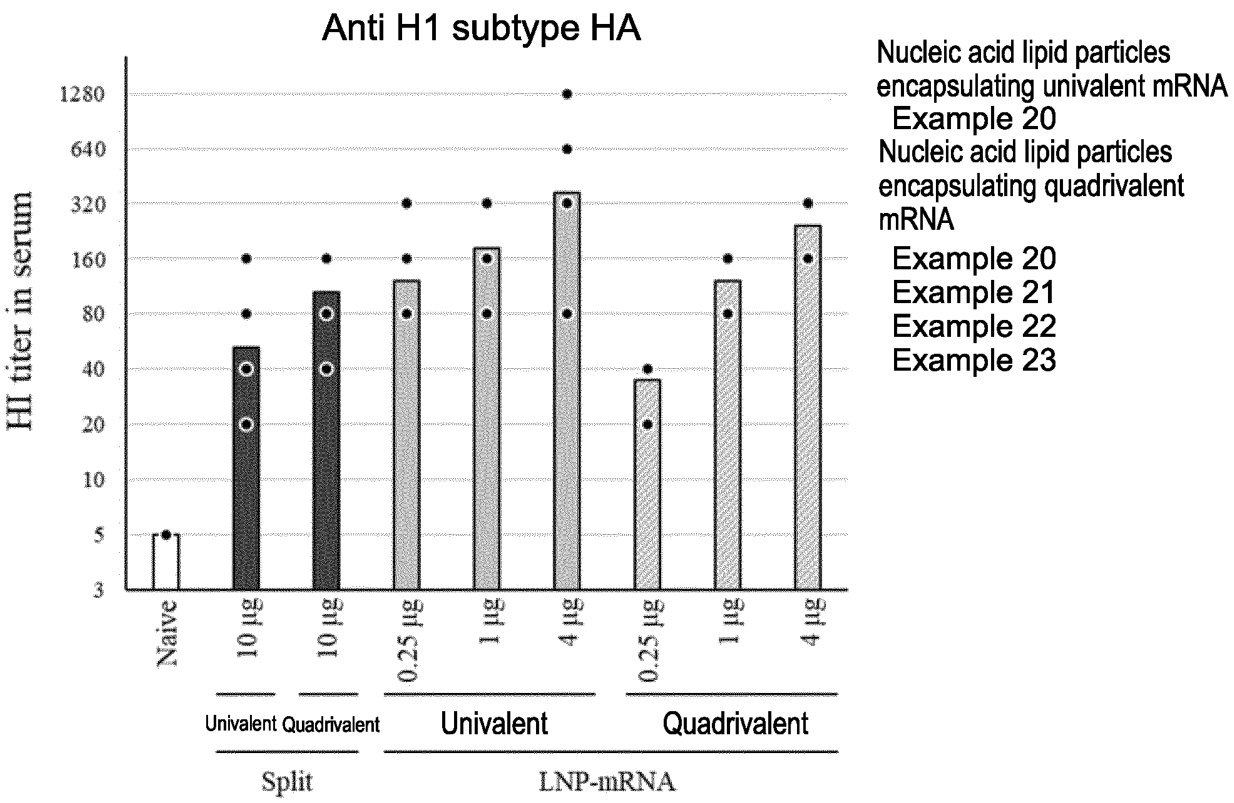

[Figure 19]

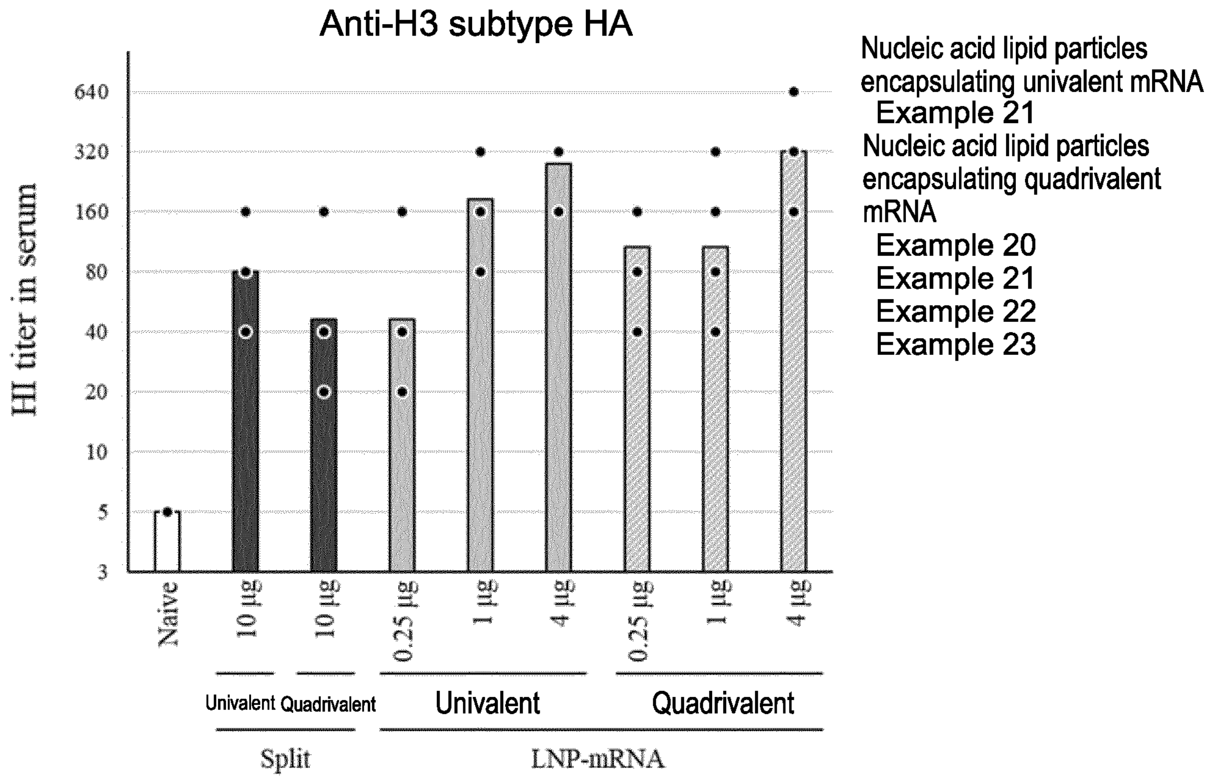


[Figure 20]

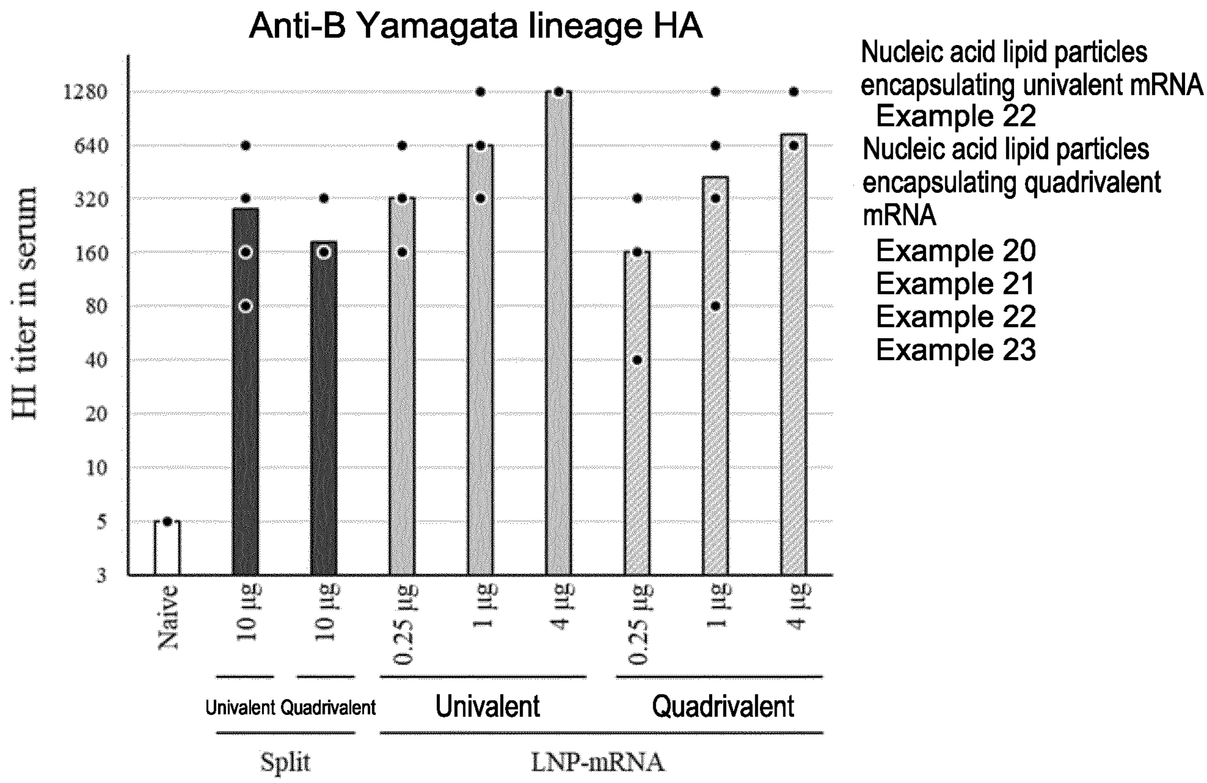

[Figure 21]

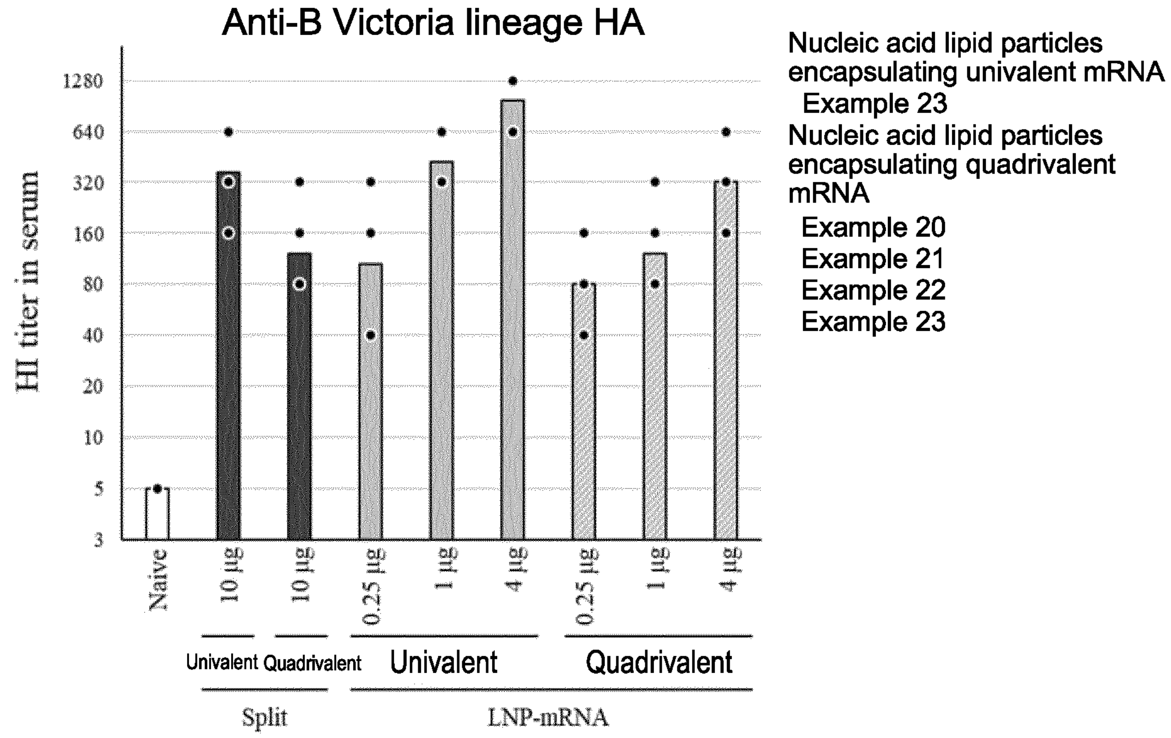


[Figure 22]

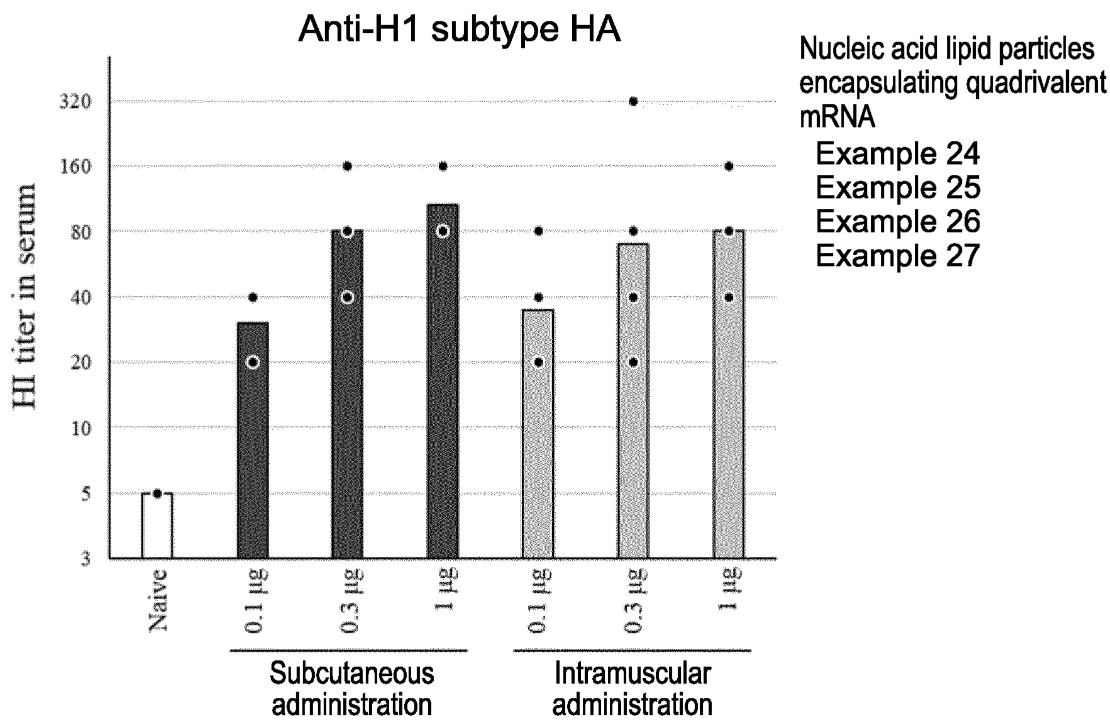

[Figure 23]

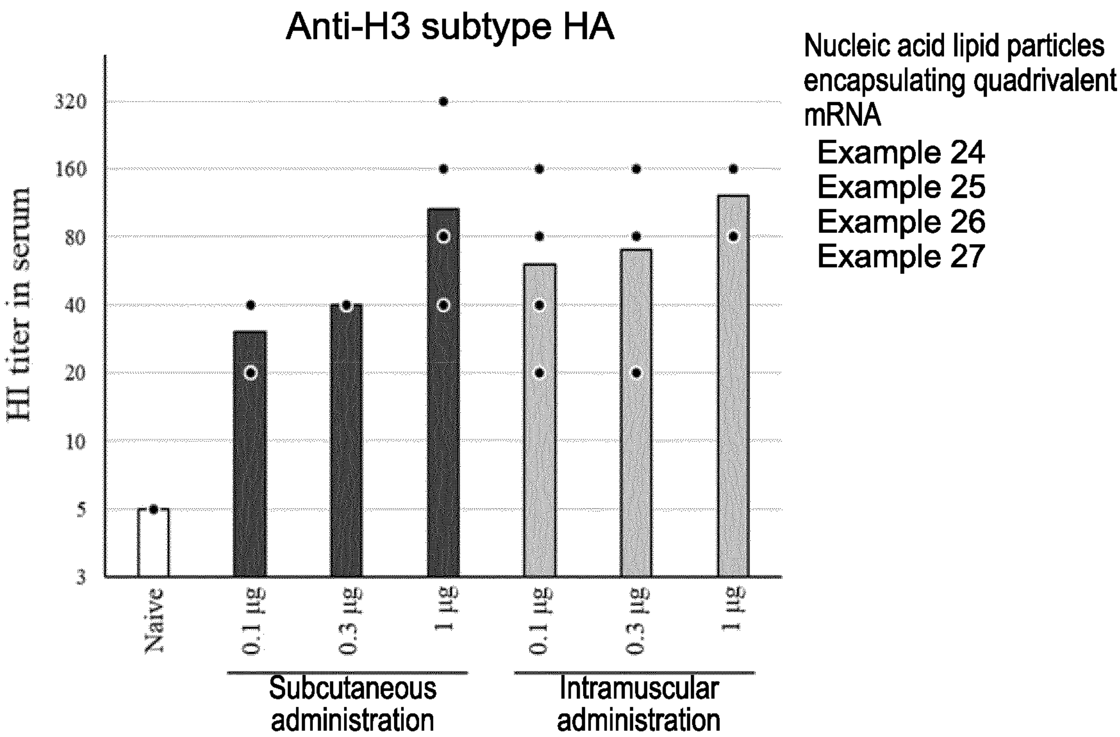


[Figure 24]

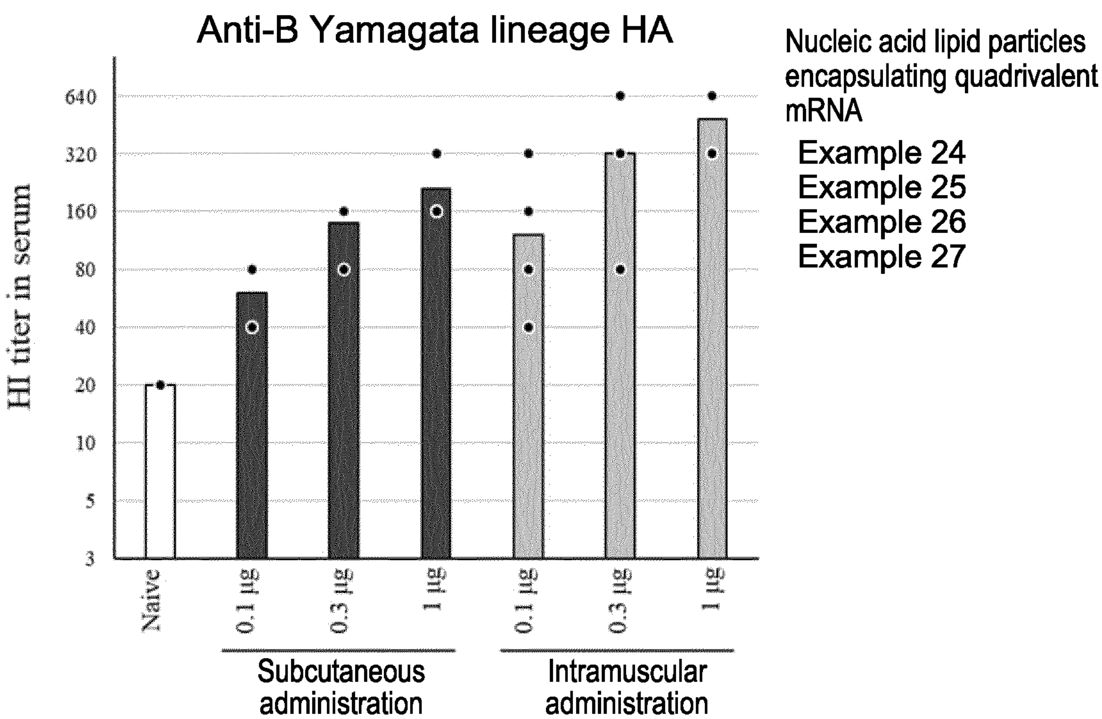

[Figure 25]

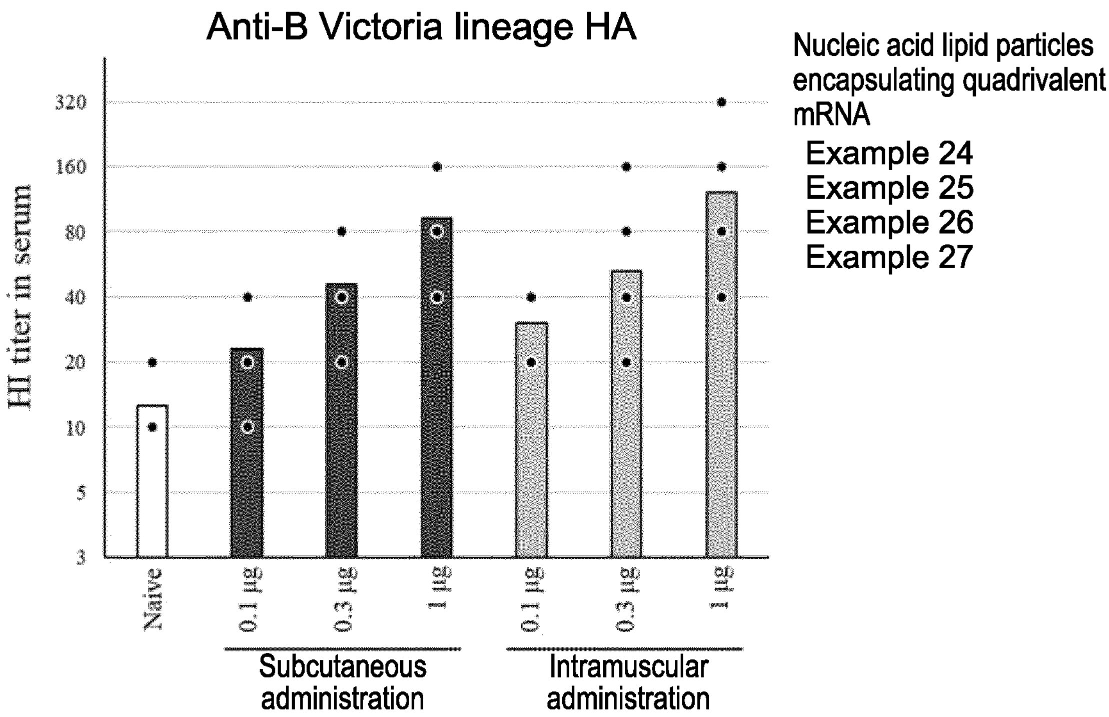


[Figure 26]

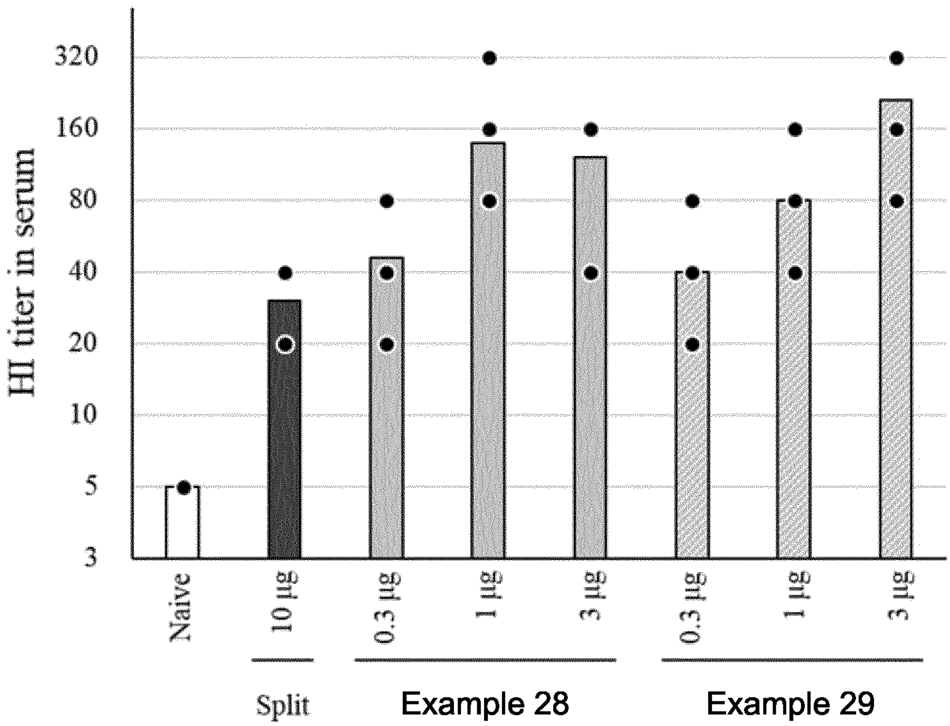

[Figure 27]

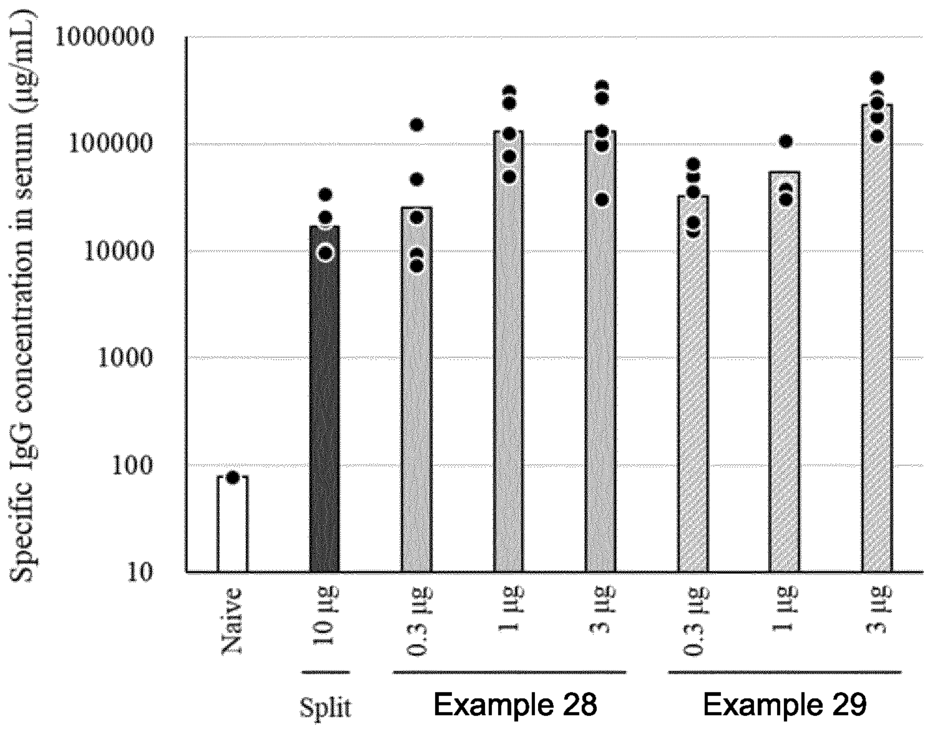


[Figure 28]

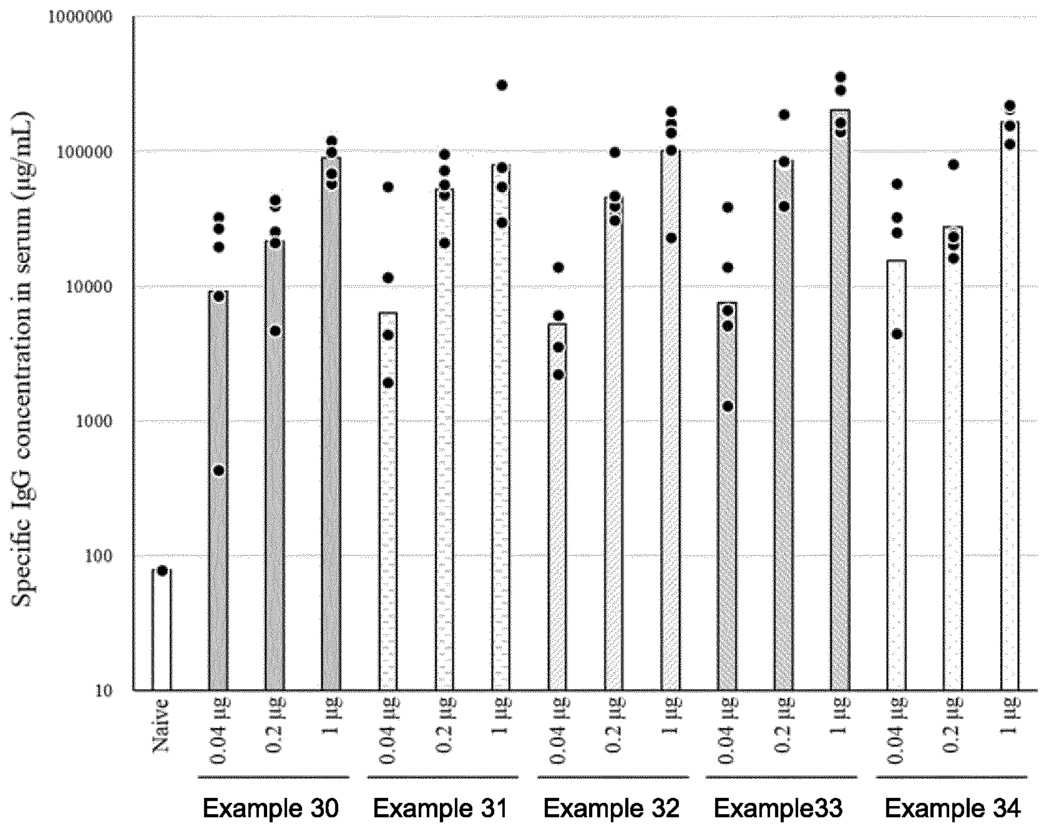

[Figure 29]

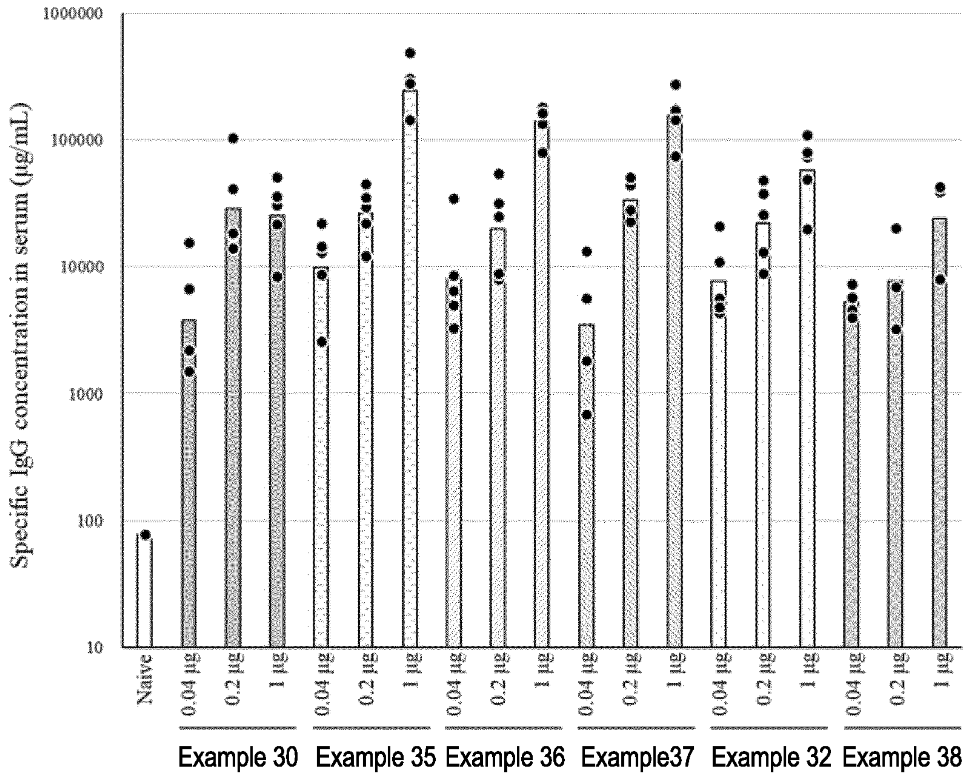


[Figure 30]

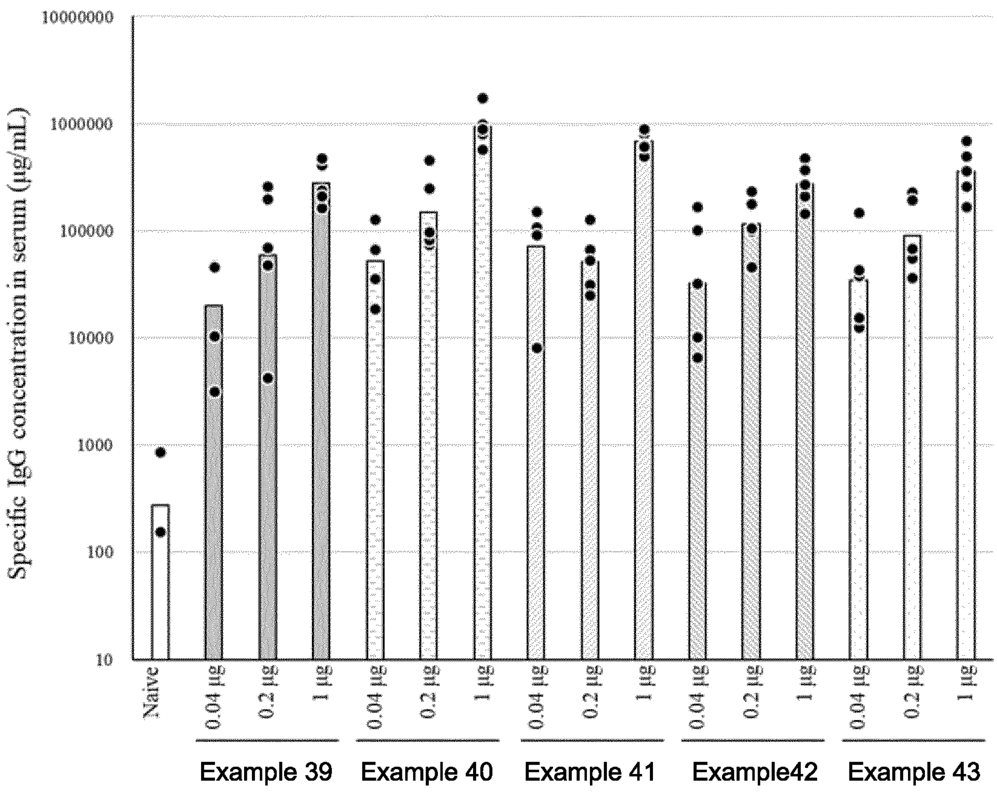

[Figure 31]

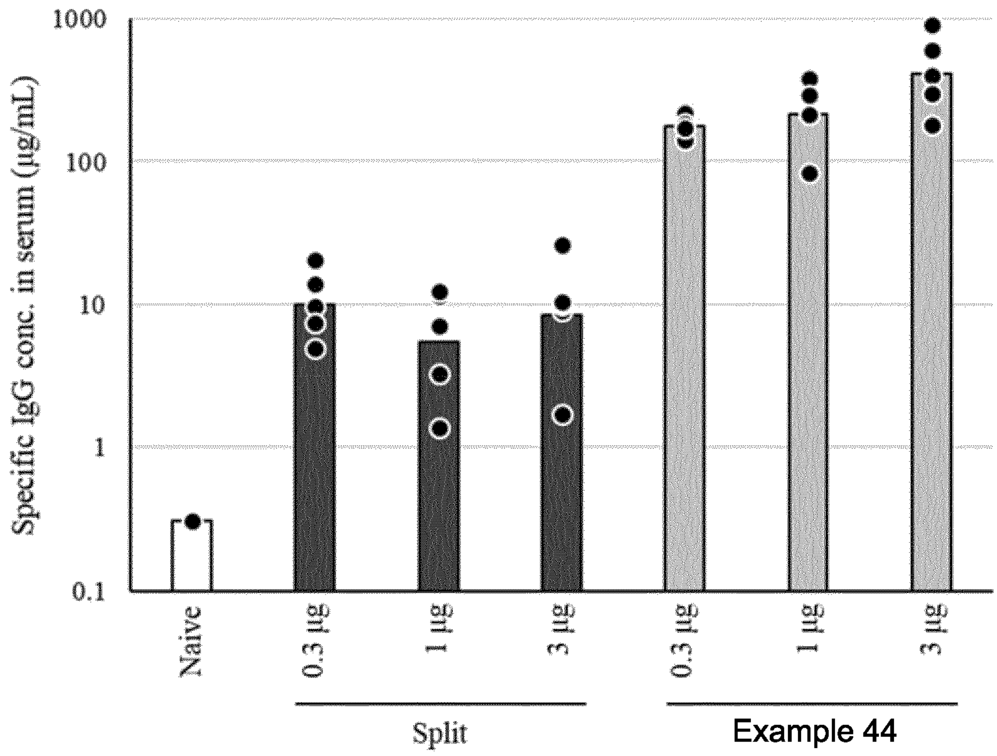


[Figure 32]

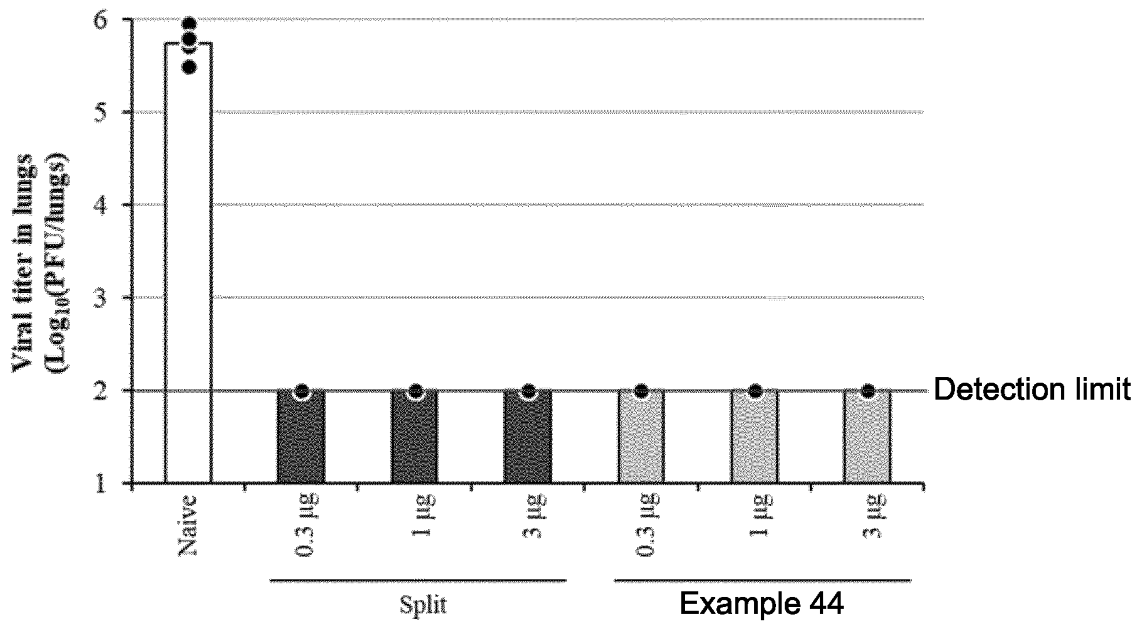

[Figure 33]

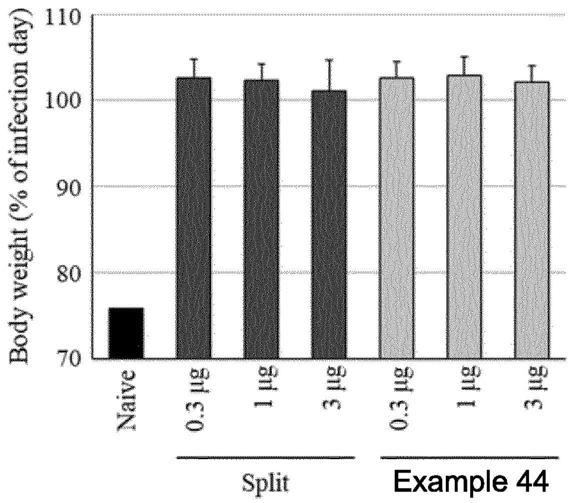


[Figure 34]

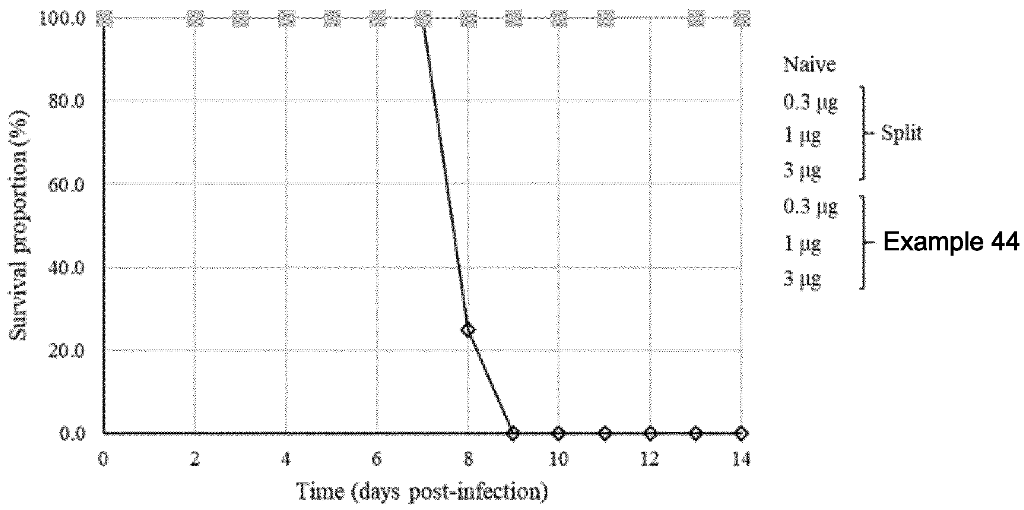


[Figure 35]

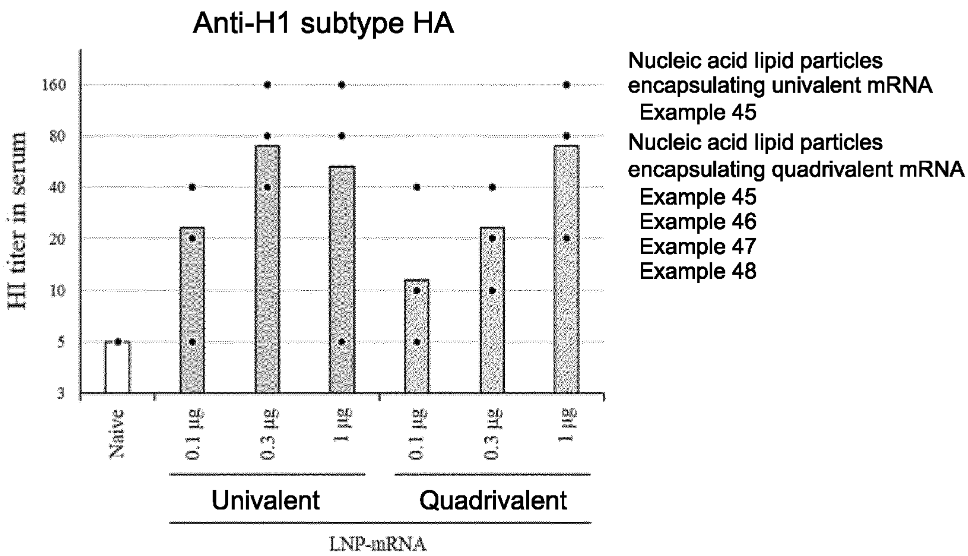

[Figure 36]

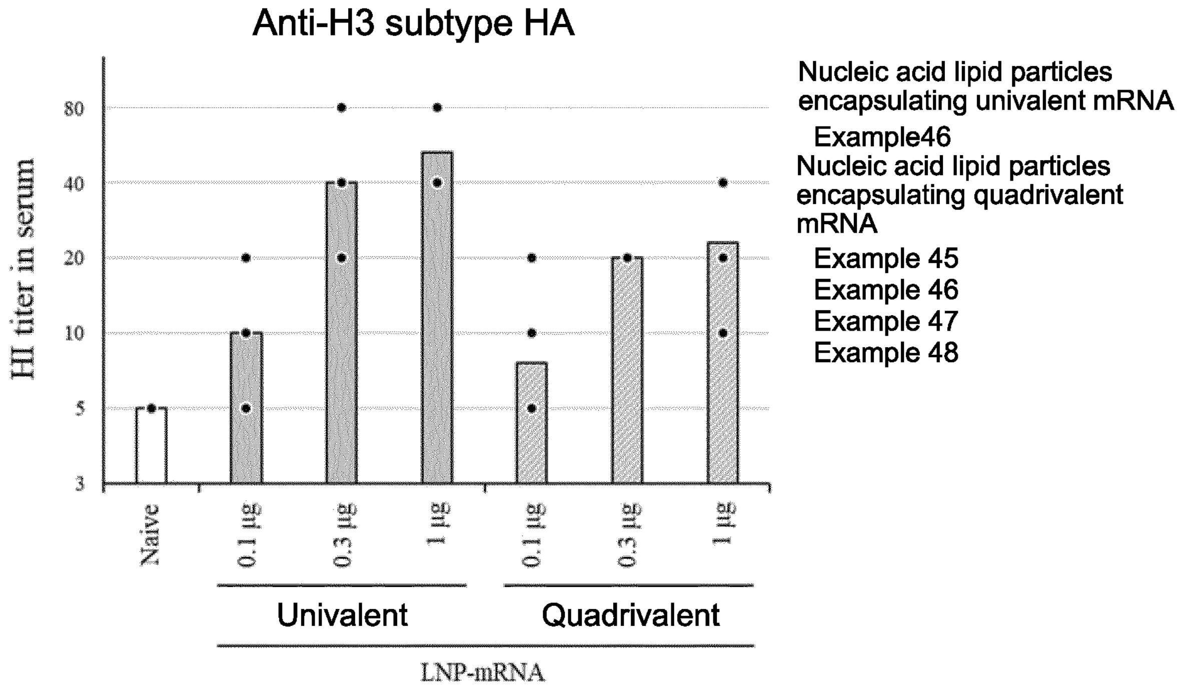


[Figure 37]

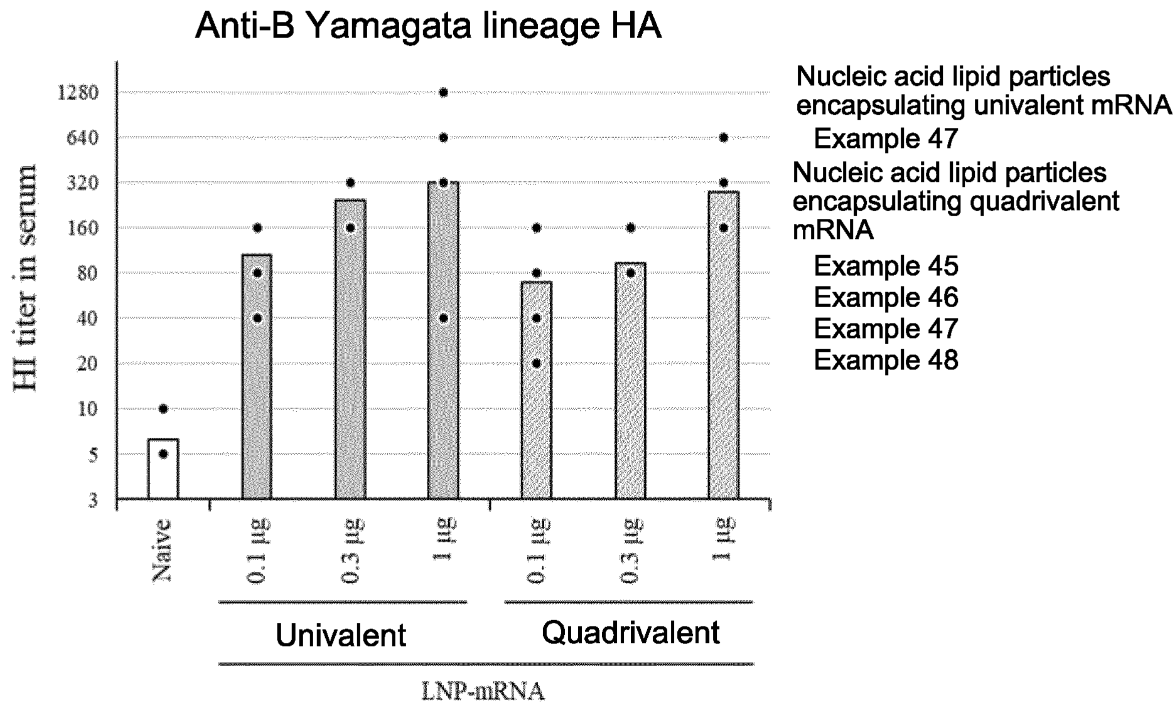

[Figure 38]

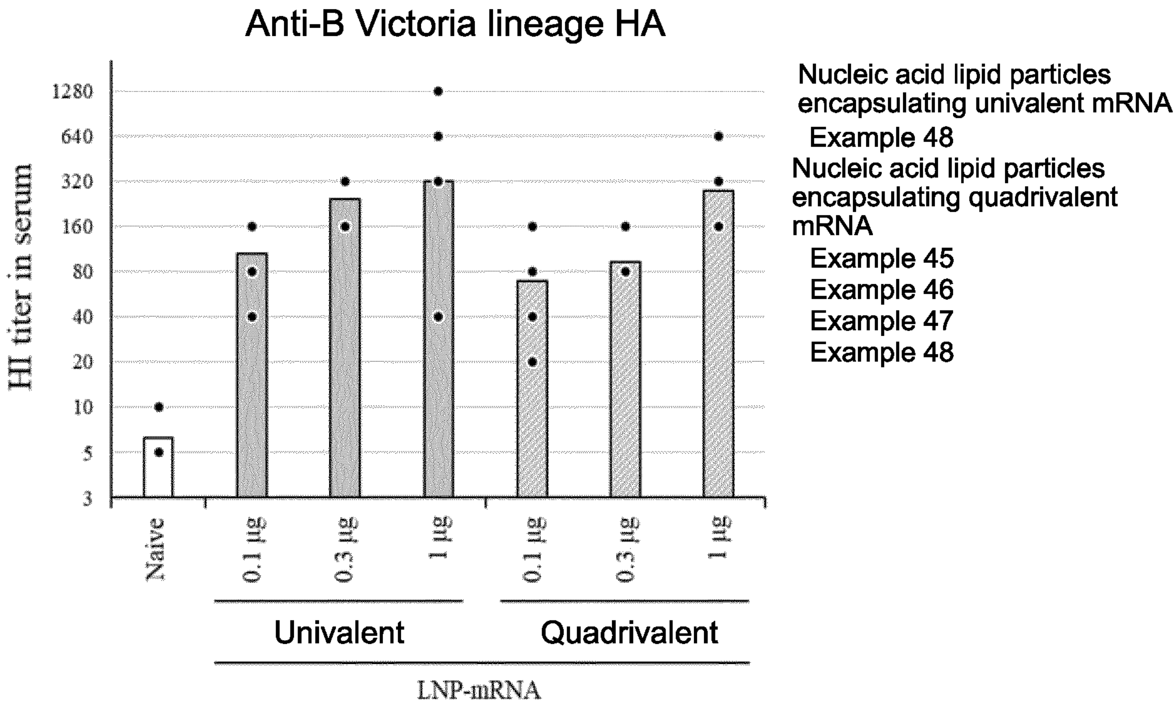
Anti-B Victoria lineage HA
HI titer in serum
1280
640
320
160
80
40
20
10
5
3
Naive
0.1 µg
0.3 µg
1 µg
0.1 µg
0.3 µg
1 µg
Univalent
Quadrivalent
LNP-mRNA
Nucleic acid lipid particles encapsulating univalent mRNA
Example 48
Nucleic acid lipid particles encapsulating quadrivalent mRNA
Example 45
Example 46
Example 47
Example 48

[Figure 39]

# Template DNA for IVT of A/Puerto Rico/8/34 (H1 subtype) HA mRNA-001

GCTAGCGTAATACGACTCACTATAGGGAGACCCAAGCTACATTTGCTTCTGACACAACTGTGTTCA
CTAGCAACCTCAAACAGACACCGCCACCATGAAGGCCAACCTGCTGGTGCTGCTGTGTGCTCTG
GCTGCCGCTGATGCCGATACCATCTGTATCGGCTACCACGCCAACAACAGCACCGACACCGTGGA
TACCGTGCTGGAAAAGAACGTGACCGTGACACACAGCGTGAACCTGCTCGAGGACAGCCACAA
TGGCAAGCTGTGCCGGCTGAAGGGAATTGCCCCTCTGCAGCTGGGCAAGTGCAATATCGCTGGA
TGGCTGCTGGGCAACCCCGAGTGTGATCCTCTGCTGCCTGTCAGATCCTGGTCCTACATCGTGGA
AACCCCTAACAGCGAGAACGGCATCTGCTACCCCGGCGACTTCATCGACTACGAGGAACTGAGA
GAACAGCTGAGCAGCGTGTCCAGCTTCGAGAGATTCGAGATCTTCCCCAAAGAGAGCAGCTGG
CCCAACCACAACACCAATGGCGTGACAGCCGCCTGTTCTCACGAGGGCAAGAGCAGCTTCTACC
GGAACCTGCTGTGGCTGACCGAGAAAGAGGGCAGCTACCCCAAGCTGAAGAACTCCTACGTGA
ACAAGAAAGGCAAAGAGGTGCTGGTCCTCTGGGGCATCCACCATCCTCCAAACAGCAAAGAGC
AGCAGAACCTGTACCAGAACGAGAATGCCTACGTGTCCGTGGTCACCAGCAACTACAACCGGCG
GTTCACACCTGAGATCGCCGAGAGGCCTAAAGTGCGCGATCAGGCCGGCAGAATGAACTACTAC
TGGACCCTGCTGAAGCCCGGCGACACCATCATCTTTGAGGCCAACGGCAACCTGATCGCCCCTAT
GTACGCCTTCGCTCTGAGCAGAGGCTTTGGCAGCGGCATCATCACCTCCAACGCCAGCATGCAC
GAGTGCAACACCAAGTGTCAGACACCCCTGGGCGCTATCAACAGCAGCCTGCCTTACCAGAACA
TTCACCCCGTGACCATCGGCGAGTGCCCCAAATACGTCAGATCCGCCAAGCTGAGAATGGTCACC
GGCCTGAGAAACATCCCCAGCATCCAGTCCAGAGGCCTGTTTGGCGCCATTGCCGGCTTTATCGA
AGGCGGCTGGACCGGCATGATCGACGGATGGTACGGATACCACCACCAGAATGAGCAAGGCAG
CGGCTACGCCGCCGATCAGAAAAGCACACAGAACGCCATCAACGGCATCACCAACAAAGTGAAC
ACCGTGATCGAGAAGATGAACATCCAGTTCACCGCCGTGGGCAAAGAGTTCAACAAGCTGGAA
AAACGGATGGAAAACCTCAACAAGAAGGTGGACGACGGCTTCCTGGACATCTGGACCTACAAT
GCCGAGCTGCTCGTGCTCCTGGAAAACGAGAGAACCCTGGACTTCCACGACAGCAACGTGAAG
AATCTGTACGAGAAAGTGAAGTCCCAGCTCAAGAACAACGCCAAAGAGATCGGCAACGGCTGC
TTCGAGTTCTACCACAAGTGCGACAACGAGTGCATGGAAAGCGTGCGGAACGGCACCTACGACT
ACCCTAAGTACAGCGAGGAAAGCAAGCTGAACCGCGAAAAAGTGGACGGCGTGAAGCTGGAA
TCCATGGGCATCTACCAGATTCTGGCCATCTACAGCACCGTGGCCTCTAGCCTGGTGCTTCTGGTT
TCTCTGGGCGCCATCAGCTTTTGGATGTGCAGCAATGGCAGCCTGCAGTGCCGGATCTGCATCTG
AGCTCGCTTTCTTGCTGTCCAATTTCTATTAAAGGTTCCTTTGTTCCCTAAGTCCAACTACTAAACT
GGGGGATATTATGAAGGGCCTTGAGCATCTGGATTCTGCCTAATAAAAAACATTTATTTTCATTGC
GAATTC

(SEQ ID NO: 1)

GCTAGC (NheI site): base Nos. 1 to 6
T7 Promoter sequence (before transcription start point): base Nos. 8 to 24
G (transcription start point): base No. 25
5'-UTR sequence (including transcription start point and KOZAK sequence of base Nos. 89 to 94): base Nos. 25 to 94
Sequence of translated region of A/Puerto Rico/8/34 (H1 subtype) HA: base Nos. 95 to 1792
3'-UTR sequence: base Nos. 1793 to 1924
GAATTC (EcoRI site): base Nos. 1925 to 1930

[Figure 40]

A/Puerto Rico/8/34 (H1 subtype) HA mRNA-001

GGGAGACCCAAGCUACAUUUGCUUCUGACACAACUGUGUUCACUAGCAACCUCAAACAGA
CACCGCCACCAUGAAGGCCAACCUGCUGGUGCUGCUGUGUGCUCUGGCUGCCGCUGAUGC
CGAUACCAUCUGUAUCGGCUACCACGCCAACAACAGCACCGACACCGUGGAUACCGUGCUG
GAAAAGAACGUGACCGUGACACACAGCGUGAACCUGCUCGAGGACAGCCACAAUGGCAAGC
UGUGCCGGCUGAAGGGAAUUGCCCCUCUGCAGCUGGGCAAGUGCAAUAUCGCUGGAUGG
CUGCUGGGCAACCCCGAGUGUGAUCCUCUGCUGCCUGUCAGAUCCUGGUCCUACAUCGUG
GAAACCCCUAACAGCGAGAACGGCAUCUGCUACCCCGGCGACUUCAUCGACUACGAGGAAC
UGAGAGAACAGCUGAGCAGCGUGUCCAGCUUCGAGAGAUUCGAGAUCUUCCCCAAAGAGA
GCAGCUGGCCCAACCACAACACCAAUGGCGUGACAGCCGCCUGUUCUCACGAGGGCAAGAG
CAGCUUCUACCGGAACCUGCUGUGGCUGACCGAGAAAGAGGGCAGCUACCCCAAGCUGAA
GAACUCCUACGUGAACAAGAAAGGCAAAGAGGUGCUGGUCCUCUGGGGCAUCCACCAUCC
UCCAAACAGCAAAGAGCAGCAGAACCUGUACCAGAACGAGAAUGCCUACGUGUCCGUGGU
CACCAGCAACUACAACCGGCGGUUCACACCUGAGAUCGCCGAGAGGCCUAAAGUGCGCGAU
CAGGCCGGCAGAAUGAACUACUACUGGACCCUGCUGAAGCCCGGCGACACCAUCAUCUUUG
AGGCCAACGGCAACCUGAUCGCCCCUAUGUACGCCUUCGCUCUGAGCAGAGGCUUUGGCA
GCGGCAUCAUCACCUCCAACGCCAGCAUGCACGAGUGCAACACCAAGUGUCAGACACCCCU
GGGCGCUAUCAACAGCAGCCUGCCUUACCAGAACAUUCACCCCGUGACCAUCGGCGAGUGC
CCCAAAUACGUCAGAUCCGCCAAGCUGAGAAUGGUCACCGGCCUGAGAAACAUCCCCAGCA
UCCAGUCCAGAGGCCUGUUUGGCGCCAUUGCCGGCUUUAUCGAAGGCGGCUGGACCGGCA
UGAUCGACGGAUGGUACGGAUACCACCACCAGAAUGAGCAAGGCAGCGGCUACGCCGCCGA
UCAGAAAAGCACACAGAACGCCAUCAACGGCAUCACCAACAAAGUGAACACCGUGAUCGAG
AAGAUGAACAUCCAGUUCACCGCCGUGGGCAAAGAGUUCAACAAGCUGGAAAAACGGAUG
GAAAACCUCAACAAGAAGGUGGACGACGGCUUCCUGGACAUCUGGACCUACAAUGCCGAG
CUGCUCGUGCUCCUGGAAAACGAGAGAACCCUGGACUUCCACGACAGCAACGUGAAGAAU
CUGUACGAGAAAGUGAAGUCCCAGCUCAAGAACAACGCCAAAGAGAUCGGCAACGGCUGC
UUCGAGUUCUACCACAAGUGCGACAACGAGUGCAUGGAAAGCGUGCGGAACGGCACCUAC
GACUACCCUAAGUACAGCGAGGAAAGCAAGCUGAACCGCGAAAAAGUGGACGGCGUGAAG
CUGGAAUCCAUGGGCAUCUACCAGAUUCUGGCCAUCUACAGCACCGUGGCCUCUAGCCUG
GUGCUUCUGGUUUCUCUGGGCGCCAUCAGCUUUUGGAUGUGCAGCAAUGGCAGCCUGCA
GUGCCGGAUCUGCAUCUGAGCUCGCUUUCUUGCUGUCCAAUUUCUAUUAAAGGUUCCUU
UGUUCCCUAAGUCCAACUACUAAACUGGGGGAUAUUAUGAAGGGCCUUGAGCAUCUGGA
UUCUGCCUAAUAAAAAACAUUUAUUUUCAUUGCGAAUU
(SEQ ID NO: 2)

[Figure 41]

## DNA fragment containing A/Puerto Rico/8/34 (H1 subtype) HA

GCTAGCGTAATACGACTCACTATAGGGAGACCCAAGCTACATTTGCTTCTGACACAACTGTGTTCA
CTAGCAACCTCAAACAGACACCGCCACCATGAAGGCCAACCTGCTGGTGCTGCTGTGTGCTCTG
GCTGCCGCTGATGCCGATACCATCTGTATCGGCTACCACGCCAACAACAGCACCGACACCGTGGA
TACCGTGCTGGAAAAGAACGTGACCGTGACACACAGCGTGAACCTGCTCGAGGACAGCCACAA
TGGCAAGCTGTGCCGGCTGAAGGGAATTGCCCCTCTGCAGCTGGGCAAGTGCAATATCGCTGGA
TGGCTGCTGGGCAACCCCGAGTGTGATCCTCTGCTGCCTGTCAGATCCTGGTCCTACATCGTGGA
AACCCCTAACAGCGAGAACGGCATCTGCTACCCCGGCGACTTCATCGACTACGAGGAACTGAGA
GAACAGCTGAGCAGCGTGTCCAGCTTCGAGAGATTCGAGATCTTCCCCAAAGAGAGCAGCTGG
CCCAACCACAACACCAATGGCGTGACAGCCGCCTGTTCTCACGAGGGCAAGAGCAGCTTCTACC
GGAACCTGCTGTGGCTGACCGAGAAAGAGGGCAGCTACCCCAAGCTGAAGAACTCCTACGTGA
ACAAGAAAGGCAAAGAGGTGCTGGTCCTCTGGGGCATCCACCATCCTCCAAACAGCAAAGAGC
AGCAGAACCTGTACCAGAACGAGAATGCCTACGTGTCCGTGGTCACCAGCAACTACAACCGGCG
GTTCACACCTGAGATCGCCGAGAGGCCTAAAGTGCGCGATCAGGCCGGCAGAATGAACTACTAC
TGGACCCTGCTGAAGCCCGGCGACACCATCATCTTTGAGGCCAACGGCAACCTGATCGCCCCTAT
GTACGCCTTCGCTCTGAGCAGAGGCTTTGGCAGCGGCATCATCACCTCCAACGCCAGCATGCAC
GAGTGCAACACCAAGTGTCAGACACCCCTGGGCGCTATCAACAGCAGCCTGCCTTACCAGAACA
TTCACCCCGTGACCATCGGCGAGTGCCCCAAATACGTCAGATCCGCCAAGCTGAGAATGGTCACC
GGCCTGAGAAACATCCCCAGCATCCAGTCCAGAGGCCTGTTTGGCGCCATTGCCGGCTTTATCGA
AGGCGGCTGGACCGGCATGATCGACGGATGGTACGGATACCACCACCAGAATGAGCAAGGCAG
CGGCTACGCCGCCGATCAGAAAAGCACACAGAACGCCATCAACGGCATCACCAACAAAGTGAAC
ACCGTGATCGAGAAGATGAACATCCAGTTCACCGCCGTGGGCAAAGAGTTCAACAAGCTGGAA
AAACGGATGGAAAACCTCAACAAGAAGGTGGACGACGGCTTCCTGGACATCTGGACCTACAAT
GCCGAGCTGCTCGTGCTCCTGGAAAACGAGAGAACCCTGGACTTCCACGACAGCAACGTGAAG
AATCTGTACGAGAAAGTGAAGTCCCAGCTCAAGAACAACGCCAAAGAGATCGGCAACGGCTGC
TTCGAGTTCTACCACAAGTGCGACAACGAGTGCATGGAAAGCGTGCGGAACGGCACCTACGACT
ACCCTAAGTACAGCGAGGAAAGCAAGCTGAACCGCGAAAAAGTGGACGGCGTGAAGCTGGAA
TCCATGGGCATCTACCAGATTCTGGCCATCTACAGCACCGTGGCCTCTAGCCTGGTGCTTCTGGTT
TCTCTGGGCGCCATCAGCTTTTGGATGTGCAGCAATGGCAGCCTGCAGTGCCGGATCTGCATCTG
AGCTCGCTTTCTTGCTGTCCAATTTCTATTAAAGGTTCCTTTGTTCCCTAAGTCCAACTACTAAACT
GGGGGATATTATGAAGGGCCTTGAGCATCTGGATTCTGCCTAATAAAAACATTTATTTTCATTGC
AAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAGAGCACTAGT
(SEQ ID NO: 3)

[Figure 42]

(Sense primer)
TAATACGACTCACTATAAGGAGAC
(SEQ ID NO: 4)

(Antisense primer)
TTTTTTTTTTTTTTTTTTTTTTTTTTTTTTTTTTTTTTTTTTTTTTTTTTTTTTTTTTTTTTTTTTTTTTT
TTTTTTTTTTTTTTTTTTTTTTTTTTTTTGCAATGAAAATAAATGTTTTTATTAGGC
(SEQ ID NO: 5)

[Figure 43]

## Template DNA of A/Puerto Rico/8/34 (H1 subtype) HA

TAATACGACTCACTATAAGGAGACCCAAGCTACATTTGCTTCTGACACAACTGTGTTCACTAGCAA
CCTCAAACAGACACCGCCACCATGAAGGCCAACCTGCTGGTGCTGCTGTGTGCTCTGGCTGCCG
CTGATGCCGATACCATCTGTATCGGCTACCACGCCAACAACAGCACCGACACCGTGGATACCGTG
CTGGAAAAGAACGTGACCGTGACACACAGCGTGAACCTGCTCGAGGACAGCCACAATGGCAAG
CTGTGCCGGCTGAAGGGAATTGCCCCTCTGCAGCTGGGCAAGTGCAATATCGCTGGATGGCTGC
TGGGCAACCCCGAGTGTGATCCTCTGCTGCCTGTCAGATCCTGGTCCTACATCGTGGAAACCCCT
AACAGCGAGAACGGCATCTGCTACCCCGGCGACTTCATCGACTACGAGGAACTGAGAGAACAG
CTGAGCAGCGTGTCCAGCTTCGAGAGATTCGAGATCTTCCCCAAAGAGAGCAGCTGGCCCAACC
ACAACACCAATGGCGTGACAGCCGCCTGTTCTCACGAGGGCAAGAGCAGCTTCTACCGGAACCT
GCTGTGGCTGACCGAGAAAGAGGGCAGCTACCCCAAGCTGAAGAACTCCTACGTGAACAAGAA
AGGCAAAGAGGTGCTGGTCCTCTGGGGCATCCACCATCCTCCAAACAGCAAAGAGCAGCAGAA
CCTGTACCAGAACGAGAATGCCTACGTGTCCGTGGTCACCAGCAACTACAACCGGCGGTTCACA
CCTGAGATCGCCGAGAGGCCTAAAGTGCGCGATCAGGCCGGCAGAATGAACTACTACTGGACCC
TGCTGAAGCCCGGCGACACCATCATCTTTGAGGCCAACGGCAACCTGATCGCCCCTATGTACGCC
TTCGCTCTGAGCAGAGGCTTTGGCAGCGGCATCATCACCTCCAACGCCAGCATGCACGAGTGCA
ACACCAAGTGTCAGACACCCCTGGGCGCTATCAACAGCAGCCTGCCTTACCAGAACATTCACCCC
GTGACCATCGGCGAGTGCCCCAAATACGTCAGATCCGCCAAGCTGAGAATGGTCACCGGCCTGA
GAAACATCCCCAGCATCCAGTCCAGAGGCCTGTTTGGCGCCATTGCCGGCTTTATCGAAGGCGG
CTGGACCGGCATGATCGACGGATGGTACGGATACCACCACCAGAATGAGCAAGGCAGCGGCTAC
GCCGCCGATCAGAAAAGCACACAGAACGCCATCAACGGCATCACCAACAAAGTGAACACCGTG
ATCGAGAAGATGAACATCCAGTTCACCGCCGTGGGCAAAGAGTTCAACAAGCTGGAAAAACGG
ATGGAAAACCTCAACAAGAAGGTGGACGACGGCTTCCTGGACATCTGGACCTACAATGCCGAGC
TGCTCGTGCTCCTGGAAAACGAGAGAACCCTGGACTTCCACGACAGCAACGTGAAGAATCTGTA
CGAGAAAGTGAAGTCCCAGCTCAAGAACAACGCCAAAGAGATCGGCAACGGCTGCTTCGAGTT
CTACCACAAGTGCGACAACGAGTGCATGGAAAGCGTGCGGAACGGCACCTACGACTACCCTAAG
TACAGCGAGGAAAGCAAGCTGAACCGCGAAAAAGTGGACGGCGTGAAGCTGGAATCCATGGG
CATCTACCAGATTCTGGCCATCTACAGCACCGTGGCCTCTAGCCTGGTGCTTCTGGTTTCTCTGGG
CGCCATCAGCTTTTGGATGTGCAGCAATGGCAGCCTGCAGTGCCGGATCTGCATCTGAGCTCGCT
TTCTTGCTGTCCAATTTCTATTAAAGGTTCCTTTGTTCCCTAAGTCCAACTACTAAACTGGGGGATA
TTATGAAGGGCCTTGAGCATCTGGATTCTGCCTAATAAAAAACATTTATTTTCATTGCAAAAAAAA
AAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAA
(SEQ ID NO: 6)

T7 Promoter sequence (before transcription start point): base Nos. 1 to 17
A (transcription start point): base No. 18
5'-UTR sequence (including transcription start point and KOZAK sequence of base Nos. 82 to 87): base Nos. 18 to 87
Sequence of translated region of A/Puerto Rico/8/34 (H1 subtype) HA: base Nos. 88 to 1785
3'-UTR sequence: base Nos. 1786 to 1917
polyA sequence (A100): base Nos. 1918 to 2017

[Figure 44]

A/Puerto Rico/8/34 (H1 subtype) HA mRNA-002 and 003

AGGAGACCCAAGCUACAUUUGCUUCUGACACAACUGUGUUCACUAGCAACCUCAAACAGAC
ACCGCCACCAUGAAGGCCAACCUGCUGGUGCUGCUGUGUGCUCUGGCUGCCGCUGAUGCC
GAUACCAUCUGUAUCGGCUACCACGCCAACAACAGCACCGACACCGUGGAUACCGUGCUGG
AAAAGAACGUGACCGUGACACACAGCGUGAACCUGCUCGAGGACAGCCACAAUGGCAAGCU
GUGCCGGCUGAAGGGAAUUGCCCCUCUGCAGCUGGGCAAGUGCAAUAUCGCUGGAUGGC
UGCUGGGCAACCCCGAGUGUGAUCCUCUGCUGCCUGUCAGAUCCUGGUCCUACAUCGUGG
AAACCCCUAACAGCGAGAACGGCAUCUGCUACCCCGGCGACUUCAUCGACUACGAGGAACU
GAGAGAACAGCUGAGCAGCGUGUCCAGCUUCGAGAGAUUCGAGAUCUUCCCCAAAGAGAG
CAGCUGGCCCAACCACAACACCAAUGGCGUGACAGCCGCCUGUUCUCACGAGGGCAAGAGC
AGCUUCUACCGGAACCUGCUGUGGCUGACCGAGAAAGAGGGCAGCUACCCCAAGCUGAAG
AACUCCUACGUGAACAAGAAAGGCAAAGAGGUGCUGGUCCUCUGGGGCAUCCACCAUCCU
CCAAACAGCAAAGAGCAGCAGAACCUGUACCAGAACGAGAAUGCCUACGUGUCCGUGGUCA
CCAGCAACUACAACCGGCGGUUCACACCUGAGAUCGCCGAGAGGCCUAAAGUGCGCGAUCA
GGCCGGCAGAAUGAACUACUACUGGACCCUGCUGAAGCCCGGCGACACCAUCAUCUUUGA
GGCCAACGGCAACCUGAUCGCCCCUAUGUACGCCUUCGCUCUGAGCAGAGGCUUUGGCAG
CGGCAUCAUCACCUCCAACGCCAGCAUGCACGAGUGCAACACCAAGUGUCAGACACCCCUG
GGCGCUAUCAACAGCAGCCUGCCUUACCAGAACAUUCACCCCGUGACCAUCGGCGAGUGCC
CCAAAUACGUCAGAUCCGCCAAGCUGAGAAUGGUCACCGGCCUGAGAAACAUCCCCAGCAU
CCAGUCCAGAGGCCUGUUUGGCGCCAUUGCCGGCUUUAUCGAAGGCGGCUGGACCGGCAU
GAUCGACGGAUGGUACGGAUACCACCACCAGAAUGAGCAAGGCAGCGGCUACGCCGCCGAU
CAGAAAAGCACACAGAACGCCAUCAACGGCAUCACCAACAAAGUGAACACCGUGAUCGAGA
AGAUGAACAUCCAGUUCACCGCCGUGGGCAAAGAGUUCAACAAGCUGGAAAAACGGAUGG
AAAACCUCAACAAGAAGGUGGACGACGGCUUCCUGGACAUCUGGACCUACAAUGCCGAGC
UGCUCGUGCUCCUGGAAAACGAGAGAACCCUGGACUUCCACGACAGCAACGUGAAGAAUC
UGUACGAGAAAGUGAAGUCCCAGCUCAAGAACAACGCCAAAGAGAUCGGCAACGGCUGCU
UCGAGUUCUACCACAAGUGCGACAACGAGUGCAUGGAAAGCGUGCGGAACGGCACCUACG
ACUACCCUAAGUACAGCGAGGAAAGCAAGCUGAACCGCGAAAAAGUGGACGGCGUGAAGC
UGGAAUCCAUGGGCAUCUACCAGAUUCUGGCCAUCUACAGCACCGUGGCCUCUAGCCUGG
UGCUUCUGGUUUCUCUGGGCGCCAUCAGCUUUUGGAUGUGCAGCAAUGGCAGCCUGCAG
UGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAU
UCUGCCUAAUAAAAAACAUUUAUUUCAUUGCAAAAAAAAAAAAAAAAAAAAAAAAAAAA
AAAAAAAAAAAAAAAAAAAAAAAAAAAAAAAAAAAAAAAAAAAAAAAAAAAAAAAAAAAAA
AAAAAAAAAAA
(SEQ ID NO: 7)

[Figure 45]

DNA fragment containing A/Singapore/GP1908/2015 (H1 subtype) HA

GCTAGCGTAATACGACTCACTATAAGGAGACCCAAGCTACATTTGCTTCTGACACAACTGTGTTCA
CTAGCAACCTCAAACAGACACCGCCACCATGAAGGCCATCCTGGTGGTGCTGCTGTACACCTTCA
CCACCGCCAACGCCGACACACTGTGTATCGGCTACCACGCCAACAACAGCACCGACACCGTGGA
TACCGTGCTGGAAAAGAACGTGACCGTGACACACAGCGTGAACCTGCTGGAAGATAAGCACAA
CGGCAAGCTGTGCAAGCTGAGAGGCGTGGCACCTCTGCACCTGGGCAAGTGTAATATCGCCGG
CTGGATCCTGGGCAACCCTGAGTGTGAAAGCCTGAGCACAGCCAGCAGCTGGTCCTACATCGTG
GAAACCAGCAACAGCGACAACGGCACATGCTACCCCGGCGACTTCATCAACTACGAGGAACTGC
GGGAACAGCTGAGCAGCGTGTCCAGCTTCGAGAGATTCGAGATCTTCCCCAAGGCCTCCAGCTG
GCCCAACCACGATTCTAACAAAGGCGTGACCGCCGCCTGTCCTCATGCCGGCGCTAAGAGCTTCT
ACAAGAACCTGATCTGGCTGGTCAAGAAGGGCAACAGCTACCCCAAGCTGAACCAGAGCTACAT
CAACGACAAGGGCAAAGAGGTGCTGGTCCTCTGGGGCATCCACCATCCTAGCACAACAGCCGAT
CAGCAGAGCCTGTACCAGAACGCCGATGCCTATGTGTTCGTGGGCACCAGCCGGTACAGCAAGA
AGTTCAAGCCCGAGATCGCCACCAGACCTAAAGTGCGGGATCAAGAGGCCCGGATGAACTACTA
CTGGACCCTGGTGGAACCCGGCGACAAGATCACATTTGAGGCCACCGGCAACCTGGTGGTCCCC
AGATACGCCTTCACCATGGAAAGAAATGCCGGCAGCGGCATCATCATCAGCGACACCCCTGTGCA
CGACTGCAACACCACCTGTCAGACACCTGAGGGCGCCATCAATACCAGCCTGCCTTTCCAGAACA
TTCACCCCATCACCATCGGCAAGTGCCCCAAATACGTGAAGTCCACCAAGCTGAGGCTGGCCACA
GGCCTGAGAAATGTGCCCTCCATCCAGAGCAGAGGCCTGTTTGGAGCCATTGCCGGCTTTATCG
AAGGCGGCTGGACAGGCATGGTGGACGGATGGTACGGATACCACCACCAGAACGAGCAAGGC
TCTGGCTATGCCGCCGACCTGAAGTCTACCCAGAATGCCATCGATAAGATCACCAACAAAGTGAA
CAGCGTGATCGAGAAGATGAACACCCAGTTCACCGCCGTGGGAAAAGAGTTCAACCACCTGGA
AAAGCGCATCGAGAACCTGAACAAGAAGGTGGACGACGGCTTCCTGGACATCTGGACCTACAA
TGCCGAGCTGCTGGTGCTCCTGGAAAACGAGAGAACCCTGGACTACCACGACAGCAACGTGAA
GAACCTGTACGAGAAAGTGCGCAACCAGCTGAAGAACAACGCCAAAGAGATCGGCAACGGCT
GCTTCGAGTTCTACCACAAGTGCGACAATACCTGCATGGAAAGCGTGAAGAATGGCACCTACGA
CTACCCTAAGTACAGCGAGGAAGCCAAACTGAACCGCGAGAAGATCGACGGCGTGAAGCTGGA
AAGCACCCGGATCTATCAGATCCTGGCCATCTACAGCACCGTGGCCAGTTCTCTGGTGCTGGTGG
TTTCTCTGGGCGCTATCAGCTTCTGGATGTGCAGCAATGGCAGCCTGCAGTGCCGGATCTGCATC
TGAGCTCGCTTTCTTGCTGTCCAATTTCTATTAAAGGTTCCTTTGTTCCCTAAGTCCAACTACTAAA
CTGGGGGATATTATGAAGGGCCTTGAGCATCTGGATTCTGCCTAATAAAAAACATTTATTTTCATT
GCAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAGAGCACTAGT
(SEQ ID NO: 8)

[Figure 46]

## Template DNA of A/Singapore/GP1908/2015 (H1 subtype) HA

TAATACGACTCACTATAAGGAGACCCAAGCTACATTTGCTTCTGACACAACTGTGTTCACTAGCAA
CCTCAAACAGACACCGCCACCATGAAGGCCATCCTGGTGGTGCTGCTGTACACCTTCACCACCGC
CAACGCCGACACACTGTGTATCGGCTACCACGCCAACAACAGCACCGACACCGTGGATACCGTG
CTGGAAAAGAACGTGACCGTGACACACAGCGTGAACCTGCTGGAAGATAAGCACAACGGCAAG
CTGTGCAAGCTGAGAGGCGTGGCACCTCTGCACCTGGGCAAGTGTAATATCGCCGGCTGGATCC
TGGGCAACCCTGAGTGTGAAAGCCTGAGCACAGCCAGCAGCTGGTCCTACATCGTGGAAACCA
GCAACAGCGACAACGGCACATGCTACCCCGGCGACTTCATCAACTACGAGGAACTGCGGGAAC
AGCTGAGCAGCGTGTCCAGCTTCGAGAGATTCGAGATCTTCCCCAAGGCCTCCAGCTGGCCCAA
CCACGATTCTAACAAAGGCGTGACCGCCGCCTGTCCTCATGCCGGCGCTAAGAGCTTCTACAAGA
ACCTGATCTGGCTGGTCAAGAAGGGCAACAGCTACCCCAAGCTGAACCAGAGCTACATCAACGA
CAAGGGCAAAGAGGTGCTGGTCCTCTGGGGCATCCACCATCCTAGCACAACAGCCGATCAGCAG
AGCCTGTACCAGAACGCCGATGCCTATGTGTTCGTGGGCACCAGCCGGTACAGCAAGAAGTTCA
AGCCCGAGATCGCCACCAGACCTAAAGTGCGGGATCAAGAGGCCCGGATGAACTACTACTGGAC
CCTGGTGGAACCCGGCGACAAGATCACATTTGAGGCCACCGGCAACCTGGTGGTCCCCAGATAC
GCCTTCACCATGGAAAGAAATGCCGGCAGCGGCATCATCATCAGCGACACCCCTGTGCACGACT
GCAACACCACCTGTCAGACACCTGAGGGCGCCATCAATACCAGCCTGCCTTTCCAGAACATTCAC
CCCATCACCATCGGCAAGTGCCCCAAATACGTGAAGTCCACCAAGCTGAGGCTGGCCACAGGCC
TGAGAAATGTGCCCTCCATCCAGAGCAGAGGCCTGTTTGGAGCCATTGCCGGCTTTATCGAAGG
CGGCTGGACAGGCATGGTGGACGGATGGTACGGATACCACCACCAGAACGAGCAAGGCTCTGG
CTATGCCGCCGACCTGAAGTCTACCCAGAATGCCATCGATAAGATCACCAACAAAGTGAACAGCG
TGATCGAGAAGATGAACACCCAGTTCACCGCCGTGGGAAAAGAGTTCAACCACCTGGAAAAGC
GCATCGAGAACCTGAACAAGAAGGTGGACGACGGCTTCCTGGACATCTGGACCTACAATGCCGA
GCTGCTGGTGCTCCTGGAAAACGAGAGAACCCTGGACTACCACGACAGCAACGTGAAGAACCT
GTACGAGAAAGTGCGCAACCAGCTGAAGAACAACGCCAAAGAGATCGGCAACGGCTGCTTCGA
GTTCTACCACAAGTGCGACAATACCTGCATGGAAAGCGTGAAGAATGGCACCTACGACTACCCTA
AGTACAGCGAGGAAGCCAAACTGAACCGCGAGAAGATCGACGGCGTGAAGCTGGAAAGCACC
CGGATCTATCAGATCCTGGCCATCTACAGCACCGTGGCCAGTTCTCTGGTGCTGGTGGTTTCTCTG
GGCGCTATCAGCTTCTGGATGTGCAGCAATGGCAGCCTGCAGTGCCGGATCTGCATCTGAGCTCG
CTTTCTTGCTGTCCAATTTCTATTAAAGGTTCCTTTGTTCCCTAAGTCCAACTACTAAACTGGGGGA
TATTATGAAGGGCCTTGAGCATCTGGATTCTGCCTAATAAAAAACATTTATTTTCATTGCAAAAAA
AAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAA
(SEQ ID NO: 9)

T7 Promoter sequence (before transcription start point): base Nos. 1 to 17
A (transcription start point): base No. 18
5'-UTR sequence (including transcription start point and KOZAK sequence of base Nos. 82 to 87): base Nos. 18 to 87
Sequence of translated region of A/Singapore/GP1908/2015 (H1 subtype) HA: base Nos. 88 to 1788
3'-UTR sequence: base Nos. 1789 to 1920
polyA sequence (A100): base Nos. 1921 to 2020

[Figure 47]

## A/Singapore/GP1908/2015 (H1 subtype) HA mRNA-001, 002 and 003

AGGAGACCCAAGCUACAUUUGCUUCUGACACAACUGUGUUCACUAGCAACCUCAAACAGAC
ACCGCCACCAUGAAGGCCAUCCUGGUGGUGCUGCUGUACACCUUCACCACCGCCAACGCCG
ACACACUGUGUAUCGGCUACCACGCCAACAACAGCACCGACACCGUGGAUACCGUGCUGGA
AAAGAACGUGACCGUGACACACAGCGUGAACCUGCUGGAAGAUAAGCACAACGGCAAGCU
GUGCAAGCUGAGAGGCGUGGCACCUCUGCACCUGGGCAAGUGUAAUAUCGCCGGCUGGA
UCCUGGGCAACCCUGAGUGUGAAAGCCUGAGCACAGCCAGCAGCUGGUCCUACAUCGUGG
AAACCAGCAACAGCGACAACGGCACAUGCUACCCCGGCGACUUCAUCAACUACGAGGAACU
GCGGGAACAGCUGAGCAGCGUGUCCAGCUUCGAGAGAUUCGAGAUCUUCCCCAAGGCCUC
CAGCUGGCCCAACCACGAUUCUAACAAAGGCGUGACCGCCGCCUGUCCUCAUGCCGGCGCU
AAGAGCUUCUACAAGAACCUGAUCUGGCUGGUCAAGAAGGGCAACAGCUACCCCAAGCUG
AACCAGAGCUACAUCAACGACAAGGGCAAAGAGGUGCUGGUCCUCUGGGGCAUCCACCAU
CCUAGCACAACAGCCGAUCAGCAGAGCCUGUACCAGAACGCCGAUGCCUAUGUGUUCGUG
GGCACCAGCCGGUACAGCAAGAAGUUCAAGCCCGAGAUCGCCACCAGACCUAAAGUGCGGG
AUCAAGAGGCCCGGAUGAACUACUACUGGACCCUGGUGGAACCCGGCGACAAGAUCACAU
UUGAGGCCACCGGCAACCUGGUGGUCCCCAGAUACGCCUUCACCAUGGAAAGAAAUGCCG
GCAGCGGCAUCAUCAUCAGCGACACCCCUGUGCACGACUGCAACACCACCUGUCAGACACC
UGAGGGCGCCAUCAAUACCAGCCUGCCUUUCCAGAACAUUCACCCCAUCACCAUCGGCAAG
UGCCCCAAAUACGUGAAGUCCACCAAGCUGAGGCUGGCCACAGGCCUGAGAAAUGUGCCC
UCCAUCCAGAGCAGAGGCCUGUUUGGAGCCAUUGCCGGCUUUAUCGAAGGCGGCUGGACA
GGCAUGGUGGACGGAUGGUACGGAUACCACCACCAGAACGAGCAAGGCUCUGGCUAUGCC
GCCGACCUGAAGUCUACCCAGAAUGCCAUCGAUAAGAUCACCAACAAAGUGAACAGCGUGA
UCGAGAAGAUGAACACCCAGUUCACCGCCGUGGGAAAAGAGUUCAACCACCUGGAAAAGC
GCAUCGAGAACCUGAACAAGAAGGUGGACGACGGCUUCCUGGACAUCUGGACCUACAAUG
CCGAGCUGCUGGUGCUCCUGGAAAACGAGAGAACCCUGGACUACCACGACAGCAACGUGA
AGAACCUGUACGAGAAAGUGCGCAACCAGCUGAAGAACAACGCCAAAGAGAUCGGCAACGG
CUGCUUCGAGUUCUACCACAAGUGCGACAAUACCUGCAUGGAAAGCGUGAAGAAUGGCAC
CUACGACUACCCUAAGUACAGCGAGGAAGCCAAACUGAACCGCGAGAAGAUCGACGGCGUG
AAGCUGGAAAGCACCCGGAUCUAUCAGAUCCUGGCCAUCUACAGCACCGUGGCCAGUUCUC
UGGUGCUGGUGGUUUCUCUGGGCGCUAUCAGCUUCUGGAUGUGCAGCAAUGGCAGCCUG
CAGUGCCGGAUCUGCAUCUGAGCUCGCUUUCUUGCUGUCCAAUUUCUAUUAAAGGUUCC
UUUGUUCCCUAAGUCCAACUACUAAACUGGGGGAUAUUAUGAAGGGCCUUGAGCAUCUG
GAUUCUGCCUAAUAAAAAACAUUUAUUUUCAUUGCAAAAAAAAAAAAAAAAAAAAAAAAA
AAAAAAAAAAAAAAAAAAAAAAAAAAAAAAAAAAAAAAAAAAAAAAAAAAAAAAAAAAAA
AAAAAAAAAAAAAA

(SEQ ID NO: 10)

[Figure 48]

## DNA fragment containing A/Singapore/INFIMH-16-0019/2016 (H3 subtype) HA

GCTAGCGTAATACGACTCACTATAAGGAGACCCAAGCTACATTTGCTTCTGACACAACTGTGTTCA
CTAGCAACCTCAAACAGACACCGCCACCATGAAGACCATCATTGCCCTGAGCTACATCCTGTGCC
TGGTGTTCGCCCAGAAGATCCCCGGCAACGACAATAGCACCGCCACACTGTGTCTGGGCCATCA
CGCTGTGCCTAACGGCACCATCGTGAAAACCATCACCAACGACCGGATCGAAGTGACCAACGCC
ACAGAGCTGGTGCAGAACAGCAGCATCGGCGAGATCTGCGATAGCCCTCACCAGATCCTGGACG
GCGAGAACTGCACACTGATCGATGCCCTGCTGGGCGATCCTCAGTGTGACGGCTTCCAGAACAA
GAAATGGGACCTGTTCGTGGAACGGTCCAAGGCCTACAGCAACTGCTACCCCTACGACGTGCCA
GACTACGCCAGCCTGAGATCTCTGGTGGCCTCTAGCGGCACCCTGGAATTCAAGAACGAGAGCT
TCAACTGGACCGGCGTGACCCAGAATGGCACAAGCAGCGCCTGTATCAGAGGCAGCAGCTCCA
GCTTCTTCAGCAGACTGAACTGGCTGACCCACCTGAACTACAAGTACCCCGCTCTGAACGTGACC
ATGCCTAACAAAGAGCAGTTCGACAAGCTGTACATCTGGGGCGTGCACCATCCTGGCACCGACA
AGGATCAGATCTTCCCATACGCTCAGAGCAGCGGCAGAATCACCGTGTCCACCAAGAGAAGCCA
GCAGGCCGTGATTCCCAACATCGGCAGCAGACCTAGAATCCGGGGCATCCCCAGCCGGATCAGC
ATCTACTGGACAATCGTGAAGCCCGGCGACATCCTGCTGATCAACAGCACCGGCAATCTGATCGC
CCCTCGGGGCTACTTCAAGATCAGAAGCGGCAAGAGCAGCATCATGCGGAGCGACGCCCCTATC
GGCAAGTGCAAGAGCGAGTGCATCACCCCAAACGGCAGCATCCCCAACGACAAGCCCTTCCAG
AATGTGAACCGGATCACCTACGGCGCCTGTCCTAGATACGTGAAGCACAGCACCCTGAAGCTGG
CCACCGGCATGAGAAATGTGCCCGAGAAGCAGACCAGAGGCATCTTCGGAGCCATTGCCGGCT
TCATCGAGAACGGCTGGGAAGGCATGGTGGACGGATGGTACGGCTTCAGACACCAGAACAGCG
AAGGCAGAGGACAGGCCGCTGACCTGAAATCTACACAGGCCGCCATCGACCAGATCAACGGCA
AGCTGAACCGGCTGATCGGCAAGACCAACGAGAAGTTCCACCAGATCGAGAAAGAGTTCAGCG
AGGTCGAGGGCAGAGTGCAGGACCTCGAGAAATACGTGGAAGATACCAAGATCGACCTGTGGT
CCTACAATGCCGAGCTGCTGGTGGCCCTGGAAAACCAGCACACCATCGACCTGACCGACAGCGA
GATGAACAAGCTGTTCGAAAAGACCAAGAAGCAGCTGCGCGAGAACGCCGAGGATATGGGCA
ACGGCTGCTTTAAGATCTACCACAAGTGCGACAACGCCTGCATCGAGAGCATCCGGAACGAGAC
ATACGACCACAACGTGTACAGAGATGAGGCCCTGAACAACCGGTTCCAGATCAAAGGCGTGGA
ACTGAAGTCCGGCTACAAGGACTGGATCCTGTGGATCAGCTTCGCCATCAGCTGCTTTCTGCTGT
GCGTGGCACTGCTGGGCTTCATCATGTGGGCCTGCCAGAAAGGCAACATCCGGTGCAACATCTG
CATCTGAGCTCGCTTTCTTGCTGTCCAATTTCTATTAAAGGTTCCTTTGTTCCCTAAGTCCAACTAC
TAAACTGGGGGATATTATGAAGGGCCTTGAGCATCTGGATTCTGCCTAATAAAAAACATTTATTTT
CATTGCAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAGAGCACTAGT
(SEQ ID NO: 11)

[Figure 49]

## Template DNA of A/Singapore/INFIMH-16-0019/2016 (H3 subtype) HA

TAATACGACTCACTATAAGGAGACCCAAGCTACATTTGCTTCTGACACAACTGTGTTCACTAGCAA
CCTCAAACAGACACCGCCACCATGAAGACCATCATTGCCCTGAGCTACATCCTGTGCCTGGTGTTC
GCCCAGAAGATCCCCGGCAACGACAATAGCACCGCCACACTGTGTCTGGGCCATCACGCTGTGC
CTAACGGCACCATCGTGAAAACCATCACCAACGACCGGATCGAAGTGACCAACGCCACAGAGCT
GGTGCAGAACAGCAGCATCGGCGAGATCTGCGATAGCCCTCACCAGATCCTGGACGGCGAGAA
CTGCACACTGATCGATGCCCTGCTGGGCGATCCTCAGTGTGACGGCTTCCAGAACAAGAAATGG
GACCTGTTCGTGGAACGGTCCAAGGCCTACAGCAACTGCTACCCCTACGACGTGCCAGACTACG
CCAGCCTGAGATCTCTGGTGGCCTCTAGCGGCACCCTGGAATTCAAGAACGAGAGCTTCAACTG
GACCGGCGTGACCCAGAATGGCACAAGCAGCGCCTGTATCAGAGGCAGCAGCTCCAGCTTCTTC
AGCAGACTGAACTGGCTGACCCACCTGAACTACAAGTACCCCGCTCTGAACGTGACCATGCCTAA
CAAAGAGCAGTTCGACAAGCTGTACATCTGGGGCGTGCACCATCCTGGCACCGACAAGGATCAG
ATCTTCCCATACGCTCAGAGCAGCGGCAGAATCACCGTGTCCACCAAGAGAAGCCAGCAGGCCG
TGATTCCCAACATCGGCAGCAGACCTAGAATCCGGGGCATCCCCAGCCGGATCAGCATCTACTGG
ACAATCGTGAAGCCCGGCGACATCCTGCTGATCAACAGCACCGGCAATCTGATCGCCCCTCGGG
GCTACTTCAAGATCAGAAGCGGCAAGAGCAGCATCATGCGGAGCGACGCCCCTATCGGCAAGTG
CAAGAGCGAGTGCATCACCCCAAACGGCAGCATCCCCAACGACAAGCCCTTCCAGAATGTGAAC
CGGATCACCTACGGCGCCTGTCCTAGATACGTGAAGCACAGCACCCTGAAGCTGGCCACCGGCA
TGAGAAATGTGCCCGAGAAGCAGACCAGAGGCATCTTCGGAGCCATTGCCGGCTTCATCGAGA
ACGGCTGGGAAGGCATGGTGGACGGATGGTACGGCTTCAGACACCAGAACAGCGAAGGCAGA
GGACAGGCCGCTGACCTGAAATCTACACAGGCCGCCATCGACCAGATCAACGGCAAGCTGAACC
GGCTGATCGGCAAGACCAACGAGAAGTTCCACCAGATCGAGAAAGAGTTCAGCGAGGTCGAG
GGCAGAGTGCAGGACCTCGAGAAATACGTGGAAGATACCAAGATCGACCTGTGGTCCTACAATG
CCGAGCTGCTGGTGGCCCTGGAAAACCAGCACACCATCGACCTGACCGACAGCGAGATGAACA
AGCTGTTCGAAAAGACCAAGAAGCAGCTGCGCGAGAACGCCGAGGATATGGGCAACGGCTGCT
TTAAGATCTACCACAAGTGCGACAACGCCTGCATCGAGAGCATCCGGAACGAGACATACGACCA
CAACGTGTACAGAGATGAGGCCCTGAACAACCGGTTCCAGATCAAAGGCGTGGAACTGAAGTC
CGGCTACAAGGACTGGATCCTGTGGATCAGCTTCGCCATCAGCTGCTTTCTGCTGTGCGTGGCAC
TGCTGGGCTTCATCATGTGGGCCTGCCAGAAAGGCAACATCCGGTGCAACATCTGCATCTGAGCT
CGCTTTCTTGCTGTCCAATTTCTATTAAAGGTTCCTTTGTTCCCTAAGTCCAACTACTAAACTGGGG
GATATTATGAAGGGCCTTGAGCATCTGGATTCTGCCTAATAAAAACATTTATTTTCATTGCAAAA
AAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAA
(SEQ ID NO: 12)

T7 Promoter sequence (before transcription start point): base Nos. 1 to 17
A (transcription start point): base No. 18
5'-UTR sequence (including transcription start point and KOZAK sequence of base Nos. 82 to 87): base Nos. 18 to 87
Sequence of translated region of A/Singapore/INFIMH-16-0019/2016 (H3 subtype) HA: base Nos. 88 to 1788
3'-UTR sequence: base Nos. 1789 to 1920
polyA sequence (A100): base Nos. 1921 to 2020

[Figure 50]

## A/Singapore/INFIMH-16-0019/2016 (H3 subtype) HA mRNA-001 and 002

AGGAGACCCAAGCUACAUUUGCUUCUGACACAACUGUGUUCACUAGCAACCUCAAACAGAC
ACCGCCACCAUGAAGACCAUCAUUGCCCUGAGCUACAUCCUGUGCCUGGUGUUCGCCCAGA
AGAUCCCCGGCAACGACAAUAGCACCGCCACACUGUGUCUGGGCCAUCACGCUGUGCCUAA
CGGCACCAUCGUGAAAACCAUCACCAACGACCGGAUCGAAGUGACCAACGCCACAGAGCUG
GUGCAGAACAGCAGCAUCGGCGAGAUCUGCGAUAGCCCUCACCAGAUCCUGGACGGCGAG
AACUGCACACUGAUCGAUGCCCUGCUGGGCGAUCCUCAGUGUGACGGCUUCCAGAACAAG
AAAUGGGACCUGUUCGUGGAACGGUCCAAGGCCUACAGCAACUGCUACCCCUACGACGUG
CCAGACUACGCCAGCCUGAGAUCUCUGGUGGCCUCUAGCGGCACCCUGGAAUUCAAGAAC
GAGAGCUUCAACUGGACCGGCGUGACCCAGAAUGGCACAAGCAGCGCCUGUAUCAGAGGC
AGCAGCUCCAGCUUCUUCAGCAGACUGAACUGGCUGACCCACCUGAACUACAAGUACCCCG
CUCUGAACGUGACCAUGCCUAACAAAGAGCAGUUCGACAAGCUGUACAUCUGGGGCGUGC
ACCAUCCUGGCACCGACAAGGAUCAGAUCUUCCCAUACGCUCAGAGCAGCGGCAGAAUCAC
CGUGUCCACCAAGAGAAGCCAGCAGGCCGUGAUUCCCAACAUCGGCAGCAGACCUAGAAUC
CGGGGCAUCCCCAGCCGGAUCAGCAUCUACUGGACAAUCGUGAAGCCCGGCGACAUCCUGC
UGAUCAACAGCACCGGCAAUCUGAUCGCCCCUCGGGGCUACUUCAAGAUCAGAAGCGGCAA
GAGCAGCAUCAUGCGGAGCGACGCCCCUAUCGGCAAGUGCAAGAGCGAGUGCAUCACCCCA
AACGGCAGCAUCCCCAACGACAAGCCCUUCCAGAAUGUGAACCGGAUCACCUACGGCGCCU
GUCCUAGAUACGUGAAGCACAGCACCCUGAAGCUGGCCACCGGCAUGAGAAAUGUGCCCG
AGAAGCAGACCAGAGGCAUCUUCGGAGCCAUUGCCGGCUUCAUCGAGAACGGCUGGGAAG
GCAUGGUGGACGGAUGGUACGGCUUCAGACACCAGAACAGCGAAGGCAGAGGACAGGCCG
CUGACCUGAAAUCUACACAGGCCGCCAUCGACCAGAUCAACGGCAAGCUGAACCGGCUGAU
CGGCAAGACCAACGAGAAGUUCCACCAGAUCGAGAAAGAGUUCAGCGAGGUCGAGGGCAG
AGUGCAGGACCUCGAGAAAUACGUGGAAGAUACCAAGAUCGACCUGUGGUCCUACAAUGC
CGAGCUGCUGGUGGCCCUGGAAAACCAGCACACCAUCGACCUGACCGACAGCGAGAUGAAC
AAGCUGUUCGAAAAGACCAAGAAGCAGCUGCGCGAGAACGCCGAGGAUAUGGGCAACGGC
UGCUUUAAGAUCUACCACAAGUGCGACAACGCCUGCAUCGAGAGCAUCCGGAACGAGACA
UACGACCACAACGUGUACAGAGAUGAGGCCCUGAACAACCGGUUCCAGAUCAAAGGCGUG
GAACUGAAGUCCGGCUACAAGGACUGGAUCCUGUGGAUCAGCUUCGCCAUCAGCUGCUUU
CUGCUGUGCGUGGCACUGCUGGGCUUCAUCAUGUGGGCCUGCCAGAAAGGCAACAUCCG
GUGCAACAUCUGCAUCUGAGCUCGCUUUCUUGCUGUCCAAUUUCUAUUAAAGGUUCCUU
UGUUCCCUAAGUCCAACUACUAAACUGGGGGAUAUUAUGAAGGGCCUUGAGCAUCUGGA
UUCUGCCUAAUAAAAAACAUUUAUUUUCAUUGCAAAAAAAAAAAAAAAAAAAAAAAAAAAA
AAAAAAAAAAAAAAAAAAAAAAAAAAAAAAAAAAAAAAAAAAAAAAAAAAAAAAAAAAAAA
AAAAAAAAAAAA

(SEQ ID NO: 13)

[Figure 51]

DNA fragment containing B/Phuket/3073/2013 (Yamagata lineage) HA

GCTAGCGTAATACGACTCACTATAAGGAGACCCAAGCTACATTTGCTTCTGACACAACTGTGTTCA
CTAGCAACCTCAAACAGACACCGCCACCATGAAGGCCATCATCGTGCTGCTGATGGTGGTCACCA
GCAACGCCGACAGAATCTGCACCGGCATCACCAGCAGCAACAGCCCTCACGTGGTCAAGACAG
CCACACAGGGCGAAGTGAATGTGACCGGCGTGATCCCTCTGACCACCACACCTACCAAGAGCTA
CTTCGCCAACCTGAAGGGCACCAGAACCAGAGGCAAGCTGTGCCCCGATTGCCTGAACTGCACC
GATCTGGATGTGGCCCTGGGCAGACCTATGTGCGTGGGAACAACACCTAGCGCCAAGGCCAGC
ATCCTGCATGAAGTGCGGCCTGTGACCAGCGGCTGCTTCCCTATTATGCACGACCGGACCAAGAT
CAGACAGCTGCCCAATCTGCTGCGGGGCTACGAGAAGATCAGGCTGAGCACCCAGAACGTGAT
CGACGCCGAAAAAGCTCCTGGCGGCCCTTACAGACTGGGCACATCTGGCTCTTGCCCCAACGCT
ACAAGCAAGATCGGCTTCTTCGCCACCATGGCCTGGGCCGTGCCTAAGGACAACTACAAGAACG
CCACCAATCCTCTGACCGTGGAAGTGCCCTACATCTGTACCGAAGGCGAGGACCAGATCACCGTG
TGGGGCTTTCACAGCGACGACAAGACCCAGATGAAGTCCCTGTACGGCGACAGCAACCCTCAG
AAGTTTACCAGCAGCGCCAACGGCGTGACCACACACTATGTGTCCCAGATCGGCGACTTCCCCG
ACCAGACAGAAGATGGCGGACTGCCTCAGAGCGGCAGAATCGTGGTGGACTACATGATGCAGA
AGCCCGGCAAGACCGGCACCATCGTGTATCAGAGAGGCGTCCTGCTGCCACAGAAAGTTTGGT
GCGCCAGCGGCCGGTCCAAAGTGATCAAAGGATCCCTGCCTCTGATCGGCGAGGCCGACTGTCT
GCACGAAGAATATGGCGGCCTGAACAAGAGCAAGCCCTACTACACAGGCAAGCACGCCAAAGC
CATCGGCAACTGCCCTATCTGGGTCAAGACCCCTCTGAAGCTGGCCAACGGCACCAAGTATAGAC
CTCCAGCCAAGCTGCTGAAAGAGCGGGGCTTCTTTGGAGCTATCGCCGGCTTTCTTGAAGGCGG
CTGGGAGGGAATGATTGCCGGCTGGCATGGCTACACATCTCATGGCGCACATGGCGTGGCAGTG
GCCGCTGATCTGAAGTCTACACAAGAGGCCATCAACAAGATCACCAAGAACCTGAACAGCCTGA
GCGAGCTGGAAGTGAAGAACCTGCAGAGACTGTCCGGCGCCATGGACGAGCTGCACAACGAG
ATCCTGGAACTGGACGAGAAGGTGGACGACCTGAGAGCCGATACCATCTCCAGCCAGATTGAGC
TGGCAGTGCTGCTGTCCAACGAGGGCATCATCAACAGCGAGGACGAGCATCTGCTGGCCCTGG
AACGGAAGCTGAAGAAGATGCTGGGACCCAGCGCCGTGGATATCGGCAATGGCTGCTTCGAGA
CAAAGCACAAGTGCAACCAGACCTGCCTGGACAGAATTGCCGCCGGAACCTTTAACGCCGGCG
AGTTTAGCCTGCCTACCTTCGACAGCCTGAACATCACAGCCGCCAGCCTGAATGACGACGGCCTG
GACAATCACACCATCCTGCTGTACTACTCCACCGCCGCCTCTTCTCTGGCCGTGACACTGATGCTG
GCTATCTTCATCGTGTACATGGTGTCCAGAGACAACGTGTCCTGCAGCATCTGTCTCTGAGCTCGC
TTTCTTGCTGTCCAATTTCTATTAAAGGTTCCTTTGTTCCCTAAGTCCAACTACTAAACTGGGGGAT
ATTATGAAGGGCCTTGAGCATCTGGATTCTGCCTAATAAAAAACATTTATTTTCATTGCAAAAAAA
AAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAGAGCACTAGT

(SEQ ID NO: 14)

[Figure 52]

Template DNA of B/Phuket/3073/2013 (Yamagata lineage) HA

TAATACGACTCACTATAAGGAGACCCAAGCTACATTTGCTTCTGACACAACTGTGTTCACTAGCAA
CCTCAAACAGACACCGCCACCATGAAGGCCATCATCGTGCTGCTGATGGTGGTCACCAGCAACG
CCGACAGAATCTGCACCGGCATCACCAGCAGCAACAGCCCTCACGTGGTCAAGACAGCCACACA
GGGCGAAGTGAATGTGACCGGCGTGATCCCTCTGACCACCACACCTACCAAGAGCTACTTCGCC
AACCTGAAGGGCACCAGAACCAGAGGCAAGCTGTGCCCCGATTGCCTGAACTGCACCGATCTG
GATGTGGCCCTGGGCAGACCTATGTGCGTGGGAACAACACCTAGCGCCAAGGCCAGCATCCTGC
ATGAAGTGCGGCCTGTGACCAGCGGCTGCTTCCCTATTATGCACGACCGGACCAAGATCAGACA
GCTGCCCAATCTGCTGCGGGGCTACGAGAAGATCAGGCTGAGCACCCAGAACGTGATCGACGC
CGAAAAAGCTCCTGGCGGCCCTTACAGACTGGGCACATCTGGCTCTTGCCCCAACGCTACAAGC
AAGATCGGCTTCTTCGCCACCATGGCCTGGGCCGTGCCTAAGGACAACTACAAGAACGCCACCA
ATCCTCTGACCGTGGAAGTGCCCTACATCTGTACCGAAGGCGAGGACCAGATCACCGTGTGGGG
CTTTCACAGCGACGACAAGACCCAGATGAAGTCCCTGTACGGCGACAGCAACCCTCAGAAGTTT
ACCAGCAGCGCCAACGGCGTGACCACACACTATGTGTCCCAGATCGGCGACTTCCCCGACCAGA
CAGAAGATGGCGGACTGCCTCAGAGCGGCAGAATCGTGGTGGACTACATGATGCAGAAGCCCG
GCAAGACCGGCACCATCGTGTATCAGAGAGGCGTCCTGCTGCCACAGAAAGTTTGGTGCGCCA
GCGGCCGGTCCAAAGTGATCAAAGGATCCCTGCCTCTGATCGGCGAGGCCGACTGTCTGCACGA
AGAATATGGCGGCCTGAACAAGAGCAAGCCCTACTACACAGGCAAGCACGCCAAAGCCATCGG
CAACTGCCCTATCTGGGTCAAGACCCCTCTGAAGCTGGCCAACGGCACCAAGTATAGACCTCCAG
CCAAGCTGCTGAAAGAGCGGGGCTTCTTTGGAGCTATCGCCGGCTTTCTTGAAGGCGGCTGGG
AGGGAATGATTGCCGGCTGGCATGGCTACACATCTCATGGCGCACATGGCGTGGCAGTGGCCGC
TGATCTGAAGTCTACACAAGAGGCCATCAACAAGATCACCAAGAACCTGAACAGCCTGAGCGAG
CTGGAAGTGAAGAACCTGCAGAGACTGTCCGGCGCCATGGACGAGCTGCACAACGAGATCCTG
GAACTGGACGAGAAGGTGGACGACCTGAGAGCCGATACCATCTCCAGCCAGATTGAGCTGGCA
GTGCTGCTGTCCAACGAGGGCATCATCAACAGCGAGGACGAGCATCTGCTGGCCCTGGAACGG
AAGCTGAAGAAGATGCTGGGACCCAGCGCCGTGGATATCGGCAATGGCTGCTTCGAGACAAAG
CACAAGTGCAACCAGACCTGCCTGGACAGAATTGCCGCCGGAACCTTTAACGCCGGCGAGTTTA
GCCTGCCTACCTTCGACAGCCTGAACATCACAGCCGCCAGCCTGAATGACGACGGCCTGGACAA
TCACACCATCCTGCTGTACTACTCCACCGCCGCCTCTTCTCTGGCCGTGACACTGATGCTGGCTATC
TTCATCGTGTACATGGTGTCCAGAGACAACGTGTCCTGCAGCATCTGTCTCTGAGCTCGCTTTCTT
GCTGTCCAATTTCTATTAAAGGTTCCTTTGTTCCCTAAGTCCAACTACTAAACTGGGGGATATTATG
AAGGGCCTTGAGCATCTGGATTCTGCCTAATAAAAAACATTTATTTTCATTGCAAAAAAAAAAAA
AAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAA
(SEQ ID NO: 15)

T7 Promoter sequence (before transcription start point): base Nos. 1 to 17
A (transcription start point): base No. 18
5'-UTR sequence (including transcription start point and KOZAK sequence of base Nos. 82 to 87): base Nos. 18 to 87
Sequence of translated region of B/Phuket/3073/2013 (Yamagata lineage) HA: base Nos. 88 to 1842
3'-UTR sequence: base Nos. 1843 to 1974
polyA sequence (A100): base Nos. 1975 to 2074

[Figure 53]

## B/Phuket/3073/2013 (Yamagata lineage) HA mRNA-001 and 002

AGGAGACCCAAGCUACAUUUGCUUCUGACACAACUGUGUUCACUAGCAACCUCAAACAGAC
ACCGCCACCAUGAAGGCCAUCAUCGUGCUGCUGAUGGUGGUCACCAGCAACGCCGACAGAA
UCUGCACCGGCAUCACCAGCAGCAACAGCCCUCACGUGGUCAAGACAGCCACACAGGGCGA
AGUGAAUGUGACCGGCGUGAUCCCUCUGACCACCACCUACCAAGAGCUACUUCGCCAAC
CUGAAGGGCACCAGAACCAGAGGCAAGCUGUGCCCCGAUUGCCUGAACUGCACCGAUCUG
GAUGUGGCCCUGGGCAGACCUAUGUGCGUGGGAACAACACCUAGCGCCAAGGCCAGCAUC
CUGCAUGAAGUGCGGCCUGUGACCAGCGGCUGCUUCCCUAUUAUGCACGACCGGACCAAG
AUCAGACAGCUGCCCAAUCUGCUGCGGGGCUACGAGAAGAUCAGGCUGAGCACCCAGAAC
GUGAUCGACGCCGAAAAAGCUCCUGGCGGCCCUUACAGACUGGGCACAUCUGGCUCUUGC
CCCAACGCUACAAGCAAGAUCGGCUUCUUCGCCACCAUGGCCUGGGCCGUGCCUAAGGACA
ACUACAAGAACGCCACCAAUCCUCUGACCGUGGAAGUGCCCUACAUCUGUACCGAAGGCGA
GGACCAGAUCACCGUGUGGGGCUUUCACAGCGACGACAAGACCCAGAUGAAGUCCCUGUA
CGGCGACAGCAACCCUCAGAAGUUUACCAGCAGCGCCAACGGCGUGACCACACACUAUGUG
UCCCAGAUCGGCGACUUCCCCGACCAGACAGAAGAUGGCGGACUGCCUCAGAGCGGCAGAA
UCGUGGUGGACUACAUGAUGCAGAAGCCCGGCAAGACCGGCACCAUCGUGUAUCAGAGAG
GCGUCCUGCUGCCACAGAAAGUUUGGUGCGCCAGCGGCCGGUCCAAAGUGAUCAAAGGAU
CCCUGCCUCUGAUCGGCGAGGCCGACUGUCUGCACGAAGAAUAUGGCGGCCUGAACAAGA
GCAAGCCCUACUACACAGGCAAGCACGCCAAAGCCAUCGGCAACUGCCCUAUCUGGGUCAA
GACCCCUCUGAAGCUGGCCAACGGCACCAAGUAUAGACCUCCAGCCAAGCUGCUGAAAGAG
CGGGGCUUCUUUGGAGCUAUCGCCGGCUUUCUUGAAGGCGGCUGGGAGGGAAUGAUUGC
CGGCUGGCAUGGCUACACAUCUCAUGGCGCACAUGGCGUGGCAGUGGCCGCUGAUCUGAA
GUCUACACAAGAGGCCAUCAACAAGAUCACCAAGAACCUGAACAGCCUGAGCGAGCUGGAA
GUGAAGAACCUGCAGAGACUGUCCGGCGCCAUGGACGAGCUGCACAACGAGAUCCUGGAA
CUGGACGAGAAGGUGGACGACCUGAGAGCCGAUACCAUCUCCAGCCAGAUUGAGCUGGCA
GUGCUGCUGUCCAACGAGGGCAUCAUCAACAGCGAGGACGAGCAUCUGCUGGCCCUGGAA
CGGAAGCUGAAGAAGAUGCUGGGACCCAGCGCCGUGGAUAUCGGCAAUGGCUGCUUCGA
GACAAAGCACAAGUGCAACCAGACCUGCCUGGACAGAAUUGCCGCCGGAACCUUUAACGCC
GGCGAGUUUAGCCUGCCUACCUUCGACAGCCUGAACAUCACAGCCGCCAGCCUGAAUGACG
ACGGCCUGGACAAUCACACCAUCCUGCUGUACUACUCCACCGCCGCCUCUUCUCUGGCCGU
GACACUGAUGCUGGCUAUCUUCAUCGUGUACAUGGUGUCCAGAGACAACGUGUCCUGCA
GCAUCUGUCUCUGAGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAUCUGGAUUCUG
CCUAAUAAAAAACAUUUAUUUUCAUUGCAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAA
AAAAAAA

(SEQ ID NO: 16)

[Figure 54]

## DNA fragment containing B/Maryland/15/2016 (Victoria lineage) HA

GCTAGCGTAATACGACTCACTATAAGGAGACCCAAGCTACATTTGCTTCTGACACAACTGTGTTCA
CTAGCAACCTCAAACAGACACCGCCACCATGAAGGCCATCATCGTGCTGCTGATGGTGGTCACCA
GCAACGCCGACAGAATCTGCACCGGCATCACCAGCAGCAACAGCCCTCACGTGGTCAAGACAG
CCACACAGGGCGAAGTGAATGTGACCGGCGTGATCCCTCTGACCACCACACCTACCAAGAGCCA
CTTCGCCAACCTGAAGGGCACCGAGACAAGAGGCAAGCTGTGCCCCAAGTGCCTGAACTGCAC
CGATCTGGATGTGGCCCTGGGCAGACCTAAGTGTACCGGCAAGATCCCTAGCGCCAGAGTGTCC
ATCCTGCACGAAGTGCGGCCTGTGACCAGCGGCTGCTTTCCCATTATGCACGACCGGACCAAGAT
CAGACAGCTGCCCAATCTGCTGCGGGGCTATGAACATGTGCGGCTGAGCACCCACAACGTGATC
AACGCCGAAGATGCTCCTGGCGGCCCTTACAAGATCGGCACATCTGGCTCTTGCCCCAACATTAC
CAACGGCAACGGCTTCTTCGCCACCATGGCTTGGGCTGTGCCCGACAAGAACAAGACCGCCACC
AATCCACTGACCATCGAGGTGCCCTACGTGTGCACAGAAGGCGAGGATCAGATCACCGTGTGGG
GCTTCCACAGCGACAACGAGATCCAGATGGCCAAGCTGTACGGCGACAGCAAGCCCCAGAAGT
TTACCAGCTCTGCCAACGGCGTGACCACACACTACGTGTCCCAGATCGGCGGCTTCCCCAATCAG
ACAGAAGATGGCGGACTGCCCCAGAGCGGAAGAATCGTGGTGGACTACATGGTGCAGAAGTCC
GGCAAGACCGGCACAATCACATACCAGCGGGGAATCCTGCTGCCTCAGAAAGTTTGGTGCGCCA
GCGGCCGGTCCAAAGTGATCAAAGGATCCCTGCCTCTGATCGGCGAGGCCGATTGTCTGCACGA
GAAATACGGCGGCCTGAACAAGAGCAAGCCCTACTACACAGGCGAGCACGCCAAGGCCATCGG
CAACTGTCCTATCTGGGTCAAGACCCCTCTGAAGCTGGCCAACGGCACCAAGTATAGACCTCCAG
CCAAGCTGCTGAAAGAGCGGGGCTTCTTTGGAGCTATCGCCGGCTTTCTTGAAGGCGGCTGGG
AGGGAATGATTGCCGGCTGGCATGGCTACACATCTCATGGCGCACATGGCGTGGCAGTGGCCGC
TGATCTGAAGTCTACACAAGAGGCCATCAACAAGATCACCAAGAACCTGAACAGCCTGAGCGAG
CTGGAAGTGAAGAACCTGCAGAGACTGTCCGGCGCCATGGACGAGCTGCACAATGAGATCCTG
GAACTGGACGAGAAGGTGGACGACCTGAGAGCCGATACCATCTCCAGCCAGATTGAGCTGGCA
GTGCTGCTGTCCAACGAGGGCATCATCAACAGCGAGGACGAGCATCTGCTGGCCCTGGAACGG
AAGCTGAAGAAGATGCTGGGCCCAAGCGCCGTGGAAATCGGCAATGGCTGCTTCGAGACAAAG
CACAAGTGCAACCAGACCTGCCTGGACAAGATTGCCGCCGGAACATTTGACGCCGGCGAGTTTA
GCCTGCCTACCTTCGACAGCCTGAACATCACAGCCGCCAGCCTGAATGACGACGGCCTGGATAAT
CACACCATCCTGCTGTACTACTCCACCGCCGCCTCTTCTCTGGCCGTGACACTGATGATCGCCATCT
TTGTGGTGTACATGGTGTCCAGAGACAACGTGTCCTGCAGCATCTGTCTCTGAGCTCGCTTTCTTG
CTGTCCAATTTCTATTAAAGGTTCCTTTGTTCCCTAAGTCCAACTACTAAACTGGGGGATATTATGA
AGGGCCTTGAGCATCTGGATTCTGCCTAATAAAAAACATTTATTTTCATTGCAAAAAAAAAAAAA
AAAAAAAAAAAAAAAAAAAAAAAAAAAAAAAAAAAAAAAAAAAAAAAAAAAAAAAAAAA
AAAAAAAAAAAAAAAAAAAAAAAAAAAAAGAGCACTAGT

(SEQ ID NO: 17)

[Figure 55]

## Template DNA of B/Maryland/15/2016 (Victoria lineage) HA

TAATACGACTCACTATAAGGAGACCCAAGCTACATTTGCTTCTGACACAACTGTGTTCACTAGCAA
CCTCAAACAGACACCGCCACCATGAAGGCCATCATCGTGCTGCTGATGGTGGTCACCAGCAACG
CCGACAGAATCTGCACCGGCATCACCAGCAGCAACAGCCCTCACGTGGTCAAGACAGCCACACA
GGGCGAAGTGAATGTGACCGGCGTGATCCCTCTGACCACCACACCTACCAAGAGCCACTTCGCC
AACCTGAAGGGCACCGAGACAAGAGGCAAGCTGTGCCCCAAGTGCCTGAACTGCACCGATCTG
GATGTGGCCCTGGGCAGACCTAAGTGTACCGGCAAGATCCCTAGCGCCAGAGTGTCCATCCTGC
ACGAAGTGCGGCCTGTGACCAGCGGCTGCTTTCCCATTATGCACGACCGGACCAAGATCAGACA
GCTGCCCAATCTGCTGCGGGGCTATGAACATGTGCGGCTGAGCACCCACAACGTGATCAACGCC
GAAGATGCTCCTGGCGGCCCTTACAAGATCGGCACATCTGGCTCTTGCCCCAACATTACCAACGG
CAACGGCTTCTTCGCCACCATGGCTTGGGCTGTGCCCGACAAGAACAAGACCGCCACCAATCCA
CTGACCATCGAGGTGCCCTACGTGTGCACAGAAGGCGAGGATCAGATCACCGTGTGGGGCTTCC
ACAGCGACAACGAGATCCAGATGGCCAAGCTGTACGGCGACAGCAAGCCCCAGAAGTTTACCA
GCTCTGCCAACGGCGTGACCACACACTACGTGTCCCAGATCGGCGGCTTCCCCAATCAGACAGA
AGATGGCGGACTGCCCCAGAGCGGAAGAATCGTGGTGGACTACATGGTGCAGAAGTCCGGCAA
GACCGGCACAATCACATACCAGCGGGGAATCCTGCTGCCTCAGAAAGTTTGGTGCGCCAGCGGC
CGGTCCAAAGTGATCAAAGGATCCCTGCCTCTGATCGGCGAGGCCGATTGTCTGCACGAGAAAT
ACGGCGGCCTGAACAAGAGCAAGCCCTACTACACAGGCGAGCACGCCAAGGCCATCGGCAACT
GTCCTATCTGGGTCAAGACCCCTCTGAAGCTGGCCAACGGCACCAAGTATAGACCTCCAGCCAA
GCTGCTGAAAGAGCGGGGCTTCTTTGGAGCTATCGCCGGCTTTCTTGAAGGCGGCTGGGAGGG
AATGATTGCCGGCTGGCATGGCTACACATCTCATGGCGCACATGGCGTGGCAGTGGCCGCTGATC
TGAAGTCTACACAAGAGGCCATCAACAAGATCACCAAGAACCTGAACAGCCTGAGCGAGCTGG
AAGTGAAGAACCTGCAGAGACTGTCCGGCGCCATGGACGAGCTGCACAATGAGATCCTGGAAC
TGGACGAGAAGGTGGACGACCTGAGAGCCGATACCATCTCCAGCCAGATTGAGCTGGCAGTGC
TGCTGTCCAACGAGGGCATCATCAACAGCGAGGACGAGCATCTGCTGGCCCTGGAACGGAAGC
TGAAGAAGATGCTGGGCCCAAGCGCCGTGGAAATCGGCAATGGCTGCTTCGAGACAAAGCACA
AGTGCAACCAGACCTGCCTGGACAAGATTGCCGCCGGAACATTTGACGCCGGCGAGTTTAGCCT
GCCTACCTTCGACAGCCTGAACATCACAGCCGCCAGCCTGAATGACGACGGCCTGGATAATCACA
CCATCCTGCTGTACTACTCCACCGCCGCCTCTTCTCTGGCCGTGACACTGATGATCGCCATCTTTGT
GGTGTACATGGTGTCCAGAGACAACGTGTCCTGCAGCATCTGTCTCTGAGCTCGCTTTCTTGCTG
TCCAATTTCTATTAAAGGTTCCTTTGTTCCCTAAGTCCAACTACTAAACTGGGGGATATTATGAAGG
GCCTTGAGCATCTGGATTCTGCCTAATAAAAAACATTTATTTTCATTGCAAAAAAAAAAAAAAAAA
AAAAAAAAAAAAAAAAAAAAAAAAAAAAAAAAAAAAAAAAAAAAAAAAAAAAAAAAAAAA
AAAAAAAAAAAAAAAAAAAAAAA

(SEQ ID NO: 18)

T7 Promoter sequence (before transcription start point): base Nos. 1 to 17
A (transcription start point): base No. 18
5'-UTR sequence (including transcription start point and KOZAK sequence of base Nos. 82 to 87): base Nos. 18 to 87
Sequence of translated region of B/Maryland/15/2016 (Victoria lineage) HA: base Nos. 88 to 1839
3'-UTR sequence: base Nos. 1840 to 1971
polyA sequence (A100): base Nos. 1972 to 2071

[Figure 56]

## B/Maryland/15/2016 (Victoria lineage) HA mRNA-001 and 002

AGGAGACCCAAGCUACAUUUGCUUCUGACACAACUGUGUUCACUAGCAACCUCAAACAGAC
ACCGCCACCAUGAAGGCCAUCAUCGUGCUGCUGAUGGUGGUCACCAGCAACGCCGACAGAA
UCUGCACCGGCAUCACCAGCAGCAACAGCCCUCACGUGGUCAAGACAGCCACACAGGGCGA
AGUGAAUGUGACCGGCGUGAUCCCUCUGACCACCACACCUACCAAGAGCCACUUCGCCAAC
CUGAAGGGCACCGAGACAAGAGGCAAGCUGUGCCCCAAGUGCCUGAACUGCACCGAUCUG
GAUGUGGCCCUGGGCAGACCUAAGUGUACCGGCAAGAUCCCUAGCGCCAGAGUGUCCAUC
CUGCACGAAGUGCGGCCUGUGACCAGCGGCUGCUUUCCCAUUAUGCACGACCGGACCAAG
AUCAGACAGCUGCCCAAUCUGCUGCGGGGCUAUGAACAUGUGCGGCUGAGCACCCACAAC
GUGAUCAACGCCGAAGAUGCUCCUGGCGGCCCUUACAAGAUCGGCACAUCUGGCUCUUGC
CCCAACAUUACCAACGGCAACGGCUUCUUCGCCACCAUGGCUUGGGCUGUGCCCGACAAGA
ACAAGACCGCCACCAAUCCACUGACCAUCGAGGUGCCCUACGUGUGCACAGAAGGCGAGGA
UCAGAUCACCGUGUGGGGCUUCCACAGCGACAACGAGAUCCAGAUGGCCAAGCUGUACGG
CGACAGCAAGCCCCAGAAGUUUACCAGCUCUGCCAACGGCGUGACCACACACUACGUGUCC
CAGAUCGGCGGCUUCCCCAAUCAGACAGAAGAUGGCGGACUGCCCCAGAGCGGAAGAAUC
GUGGUGGACUACAUGGUGCAGAAGUCCGGCAAGACCGGCACAAUCACAUACCAGCGGGGA
AUCCUGCUGCCUCAGAAAGUUUGGUGCGCCAGCGGCCGGUCCAAAGUGAUCAAAGGAUCC
CUGCCUCUGAUCGGCGAGGCCGAUUGUCUGCACGAGAAAUACGGCGGCCUGAACAAGAGC
AAGCCCUACUACACAGGCGAGCACGCCAAGGCCAUCGGCAACUGUCCUAUCUGGGUCAAGA
CCCCUCUGAAGCUGGCCAACGGCACCAAGUAUAGACCUCCAGCCAAGCUGCUGAAAGAGCG
GGGCUUCUUUGGAGCUAUCGCCGGCUUUCUUGAAGGCGGCUGGGAGGGAAUGAUUGCCG
GCUGGCAUGGCUACACAUCUCAUGGCGCACAUGGCGUGGCAGUGGCCGCUGAUCUGAAGU
CUACACAAGAGGCCAUCAACAAGAUCACCAAGAACCUGAACAGCCUGAGCGAGCUGGAAGU
GAAGAACCUGCAGAGACUGUCCGGCGCCAUGGACGAGCUGCACAAUGAGAUCCUGGAACU
GGACGAGAAGGUGGACGACCUGAGAGCCGAUACCAUCUCCAGCCAGAUUGAGCUGGCAGU
GCUGCUGUCCAACGAGGGCAUCAUCAACAGCGAGGACGAGCAUCUGCUGGCCCUGGAACG
GAAGCUGAAGAAGAUGCUGGGCCCAAGCGCCGUGGAAAUCGGCAAUGGCUGCUUCGAGAC
AAAGCACAAGUGCAACCAGACCUGCCUGGACAAGAUUGCCGCCGGAACAUUUGACGCCGGC
GAGUUUAGCCUGCCUACCUUCGACAGCCUGAACAUCACAGCCGCCAGCCUGAAUGACGACG
GCCUGGAUAAUCACACCAUCCUGCUGUACUACUCCACCGCCGCCUCUUCUCUGGCCGUGAC
ACUGAUGAUCGCCAUCUUUGUGGUGUACAUGGUGUCCAGAGACAACGUGUCCUGCAGCA
UCUGUCUCUGAGCUCGCUUUCUUGCUGUCCAAUUUCUAUUAAAGGUUCCUUUGUUCCCU
AAGUCCAACUACUAAACUGGGGGAUAUUAUGAAGGGCCUUGAGCAUCUGGAUUCUGCCU
AAUAAAAAACAUUUAUUUUCAUUGCAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAA
AAAA

(SEQ ID NO: 19)

[Figure 57]

Amino acid sequence of A/Puerto Rico/8/34 (H1 subtype) HA

MKANLLVLLCALAAADADTICIGYHANNSTDTVDTVLEKNVTVTHSVNLLEDSHNGKLCRLKGIAPL
QLGKCNIAGWLLGNPECDPLLPVRSWSYIVETPNSENGICYPGDFIDYEELREQLSSVSSFERFEIFPKE
SSWPNHNTNGVTAACSHEGKSSFYRNLLWLTEKEGSYPKLKNSYVNKKGKEVLVLWGIHHPPNSKE
QQNLYQNENAYVSVVTSNYNRRFTPEIAERPKVRDQAGRMNYYWTLLKPGDTIIFEANGNLIAPMY
AFALSRGFGSGIITSNASMHECNTKCQTPLGAINSSLPYQNIHPVTIGECPKYVRSAKLRMVTGLRNIP
SIQSRGLFGAIAGFIEGGWTGMIDGWYGYHHQNEQGSGYAADQKSTQNAINGITNKVNTVIEKM
NIQFTAVGKEFNKLEKRMENLNKKVDDGFLDIWTYNAELLVLLENERTLDFHDSNVKNLYEKVKSQL
KNNAKEIGNGCFEFYHKCDNECMESVRNGTYDYPKYSEESKLNREKVDGVKLESMGIYQILAIYSTVA
SSLVLLVSLGAISFWMCSNGSLQCRICI
(SEQ ID NO: 20)

[Figure 58]

Amino acid sequence of A/Singapore/GP1908/2015 (H1 subtype) HA

MKAILVVLLYTFTTANADTLCIGYHANNSTDTVDTVLEKNVTVTHSVNLLEDKHNGKLCKLRGVAPLH
LGKCNIAGWILGNPECESLSTASSWSYIVETSNSDNGTCYPGDFINYEELREQLSSVSSFERFEIFPKAS
SWPNHDSNKGVTAACPHAGAKSFYKNLIWLVKKGNSYPKLNQSYINDKGKEVLVLWGIHHPSTTAD
QQSLYQNADAYVFVGTSRYSKKFKPEIATRPKVRDQEARMNYYWTLVEPGDKITFEATGNLVVPRYA
FTMERNAGSGIIISDTPVHDCNTTCQTPEGAINTSLPFQNIHPITIGKCPKYVKSTKLRLATGLRNVPSI
QSRGLFGAIAGFIEGGWTGMVDGWYGYHHQNEQGSGYAADLKSTQNAIDKITNKVNSVIEKMNT
QFTAVGKEFNHLEKRIENLNKKVDDGFLDIWTYNAELLVLLENERTLDYHDSNVKNLYEKVRNQLKN
NAKEIGNGCFEFYHKCDNTCMESVKNGTYDYPKYSEEAKLNREKIDGVKLESTRIYQILAIYSTVASSLV
LVVSLGAISFWMCSNGSLQCRICI
(SEQ ID NO: 21)

[Figure 59]

Amino acid sequence of A/Singapore/INFIMH-16-0019/2016 (H3 subtype) HA

MKTIIALSYILCLVFAQKIPGNDNSTATLCLGHHAVPNGTIVKTITNDRIEVTNATELVQNSSIGEICDSP
HQILDGENCTLIDALLGDPQCDGFQNKKWDLFVERSKAYSNCYPYDVPDYASLRSLVASSGTLEFKNE
SFNWTGVTQNGTSSACIRGSSSSFFSRLNWLTHLNYKYPALNVTMPNKEQFDKLYIWGVHHPGTDK
DQIFPYAQSSGRITVSTKRSQQAVIPNIGSRPRIRGIPSRISIYWTIVKPGDILLINSTGNLIAPRGYFKIRS
GKSSIMRSDAPIGKCKSECITPNGSIPNDKPFQNVNRITYGACPRYVKHSTLKLATGMRNVPEKQTRG
IFGAIAGFIENGWEGMVDGWYGFRHQNSEGRGQAADLKSTQAAIDQINGKLNRLIGKTNEKFHQI
EKEFSEVEGRVQDLEKYVEDTKIDLWSYNAELLVALENQHTIDLTDSEMNKLFEKTKKQLRENAEDM
GNGCFKIYHKCDNACIESIRNETYDHNVYRDEALNNRFQIKGVELKSGYKDWILWISFAISCFLLCVAL
LGFIMWACQKGNIRCNICI
(SEQ ID NO: 22)

[Figure 60]

Amino acid sequence of B/Phuket/3073/2013 (Yamagata lineage) HA

MKAIIVLLMVVTSNADRICTGITSSNSPHVVKTATQGEVNVTGVIPLTTTPTKSYFANLKGTRTRGKLC
PDCLNCTDLDVALGRPMCVGTTPSAKASILHEVRPVTSGCFPIMHDRTKIRQLPNLLRGYEKIRLSTQ
NVIDAEKAPGGPYRLGTSGSCPNATSKIGFFATMAWAVPKDNYKNATNPLTVEVPYICTEGEDQITV
WGFHSDDKTQMKSLYGDSNPQKFTSSANGVTTHYVSQIGDFPDQTEDGGLPQSGRIVVDYMMQ
KPGKTGTIVYQRGVLLPQKVWCASGRSKVIKGSLPLIGEADCLHEEYGGLNKSKPYYTGKHAKAIGNC
PIWVKTPLKLANGTKYRPPAKLLKERGFFGAIAGFLEGGWEGMIAGWHGYTSHGAHGVAVAADLK
STQEAINKITKNLNSLSELEVKNLQRLSGAMDELHNEILELDEKVDDLRADTISSQIELAVLLSNEGIINS
EDEHLLALERKLKKMLGPSAVDIGNGCFETKHKCNQTCLDRIAAGTFNAGEFSLPTFDSLNITAASLN
DDGLDNHTILLYYSTAASSLAVTLMLAIFIVYMVSRDNVSCSICL
(SEQ ID NO: 23)

[Figure 61]

Amino acid sequence of B/Maryland/15/2016 (Victoria lineage) HA

MKAIIVLLMVVTSNADRICTGITSSNSPHVVKTATQGEVNVTGVIPLTTTPTKSHFANLKGTETRGKLC
PKCLNCTDLDVALGRPKCTGKIPSARVSILHEVRPVTSGCFPIMHDRTKIRQLPNLLRGYEHVRLSTHN
VINAEDAPGGPYKIGTSGSCPNITNGNGFFATMAWAVPDKNKTATNPLTIEVPYVCTEGEDQITVW
GFHSDNEIQMAKLYGDSKPQKFTSSANGVTTHYVSQIGGFPNQTEDGGLPQSGRIVVDYMVQKSG
KTGTITYQRGILLPQKVWCASGRSKVIKGSLPLIGEADCLHEKYGGLNKSKPYYTGEHAKAIGNCPIW
VKTPLKLANGTKYRPPAKLLKERGFFGAIAGFLEGGWEGMIAGWHGYTSHGAHGVAVAADLKSTQ
EAINKITKNLNSLSELEVKNLQRLSGAMDELHNEILELDEKVDDLRADTISSQIELAVLLSNEGIINSEDE
HLLALERKLKKMLGPSAVEIGNGCFETKHKCNQTCLDKIAAGTFDAGEFSLPTFDSLNITAASLNDDG
LDNHTILLYYSTAASSLAVTLMIAIFVVYMVSRDNVSCSICL
(SEQ ID NO: 24)

[Figure 62]

Amino acid sequence of protease cleavage sequence

RGRKRRS
(SEQ ID NO: 25)

[Figure 63]

## Template DNA sequence of A/Guangdong-Maonan/SWL1536/2019, polyA110

GCTAGCGTAATACGACTCACTATAAGGAGACCCAAGCTACATTTGCTTCTGACACAACTGTGTTCACTAGCA
ACCTCAAACAGACACCGCCACCATGAAGGCCATCCTGGTGGTGCTGCTGTACACCTTCACCACCGCCAACG
CCGACACACTGTGCATCGGCTACCACGCCAACAACAGCACCGACACCGTGGACACCGTGCTGGAAAAGA
ACGTGACCGTGACACACAGCGTGAACCTGCTGGAAGACAAGCACAACGGCAAGCTGTGCAAGCTGAGA
GGCGTGGCACCCCTGCACCTGGGCAAGTGCAACATCGCCGGCTGGATCCTGGGCAACCCCGAGTGCGAA
AGCCTGAGCACCGCCAGAAGCTGGAGCTACATCGTGGAAACCAGCAACAGCGACAACGGCACATGCTAC
CCCGGCGACTTCATCAACTACGAGGAACTGCGGGAACAGCTGAGCAGCGTGAGCAGCTTCGAGAGATTC
GAGATCTTCCCCAAGACCAGCAGCTGGCCCAACCACGACAGCGACAAAGGCGTGACAGCCGCCTGCCCC
CACGCCGGCGCCAAGAGCTTCTACAAGAACCTGATCTGGCTGGTCAAGAAGGGCAACAGCTACCCCAAG
CTGAACCAGACCTACATCAACGACAAGGGCAAAGAGGTGCTGGTCCTCTGGGGCATCCACCACCCCCCAA
CAATCGCCGCCCAAGAGAGCCTGTACCAGAACGCCGACGCCTACGTGTTCGTGGGCACCAGCAGATACAG
CAAGAAGTTCAAGCCCGAGATCGCCACCAGGCCCAAAGTGCGGGACCAAGAGGGCAGAATGAACTACTA
CTGGACCCTGGTGGAACCCGGCGACAAGATCACATTCGAGGCCACCGGCAACCTGGTGGTCCCCAGATAC
GCCTTCACCATGGAAAGAGACGCCGGCAGCGGCATCATCATCAGCGACACCCCCGTGCACGACTGCAACA
CCACCTGCCAGACACCCGAGGGCGCCATCAACACCAGCCTGCCCTTCCAGAACGTGCACCCCATCACCATC
GGCAAGTGCCCCAAATACGTGAAGTCCACCAAGCTGAGGCTGGCCACAGGCCTGAGAAACGTGCCCAGC
ATCCAGAGCAGAGGCCTGTTCGGAGCCATCGCCGGCTTCATCGAAGGCGGCTGGACAGGCATGGTGGAC
GGATGGTACGGATACCACCACCAGAACGAGCAAGGCAGCGGCTACGCCGCCGACCTGAAGTCCACCCAG
AACGCCATCGACAAGATCACCAACAAAGTGAACAGCGTGATCGAGAAGATGAACACCCAGTTCACCGCCG
TGGGAAAAGAGTTCAACCACCTGGAAAAGCGCATCGAGAACCTGAACAAGAAGGTGGACGACGGCTTC
CTGGACATCTGGACCTACAACGCCGAGCTGCTCGTGCTGCTCGAGAACGAGAGAACCCTGGACTACCACG
ACAGCAACGTGAAGAACCTGTACGAGAAAGTGCGCAACCAGCTGAAGAACAACGCCAAAGAGATCGGC
AACGGCTGCTTCGAGTTCTACCACAAGTGCGACAACACCTGCATGGAAAGCGTGAAGAACGGCACCTACG
ACTACCCCAAGTACAGCGAGGAAGCCAAACTGAACCGCGAGAAGATCGACGGCGTGAAGCTGGAAAGC
ACCCGGATCTACCAGATCCTGGCCATCTACAGCACCGTGGCCAGCAGCCTGGTGCTGGTGGTGAGCCTGG
GCGCCATCAGCTTCTGGATGTGCAGCAACGGCAGCCTGCAGTGCCGGATCTGCATCTGAGCTCGCTTTCTT
GCTGTCCAATTTCTATTAAAGGTTCCTTTGTTCCCTAAGTCCAACTACTAAACTGGGGGATATTATGAAGGG
CCTTGAGCATCTGGATTCTGCCTAATAAAAAACATTTATTTTCATTGCAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAA
AAAAAAAAAAAAAAAAAAAACGAAGAGC

(SEQ ID NO: 26)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of Guangdong-Maonan/SWL1536/2019 HA: base Nos. 95 to 1795
3'-UTR sequence: base Nos. 1796 to 1927
polyA sequence (A100): base Nos. 1928 to 2037

[Figure 64]

## Template DNA sequence of A/Guangdong-Maonan/SWL1536/2019, polyA95

GCTAGCGTAATACGACTCACTATAAGGAGACCCAAGCTACATTTGCTTCTGACACAACTGTGTTCACTAGCA
ACCTCAAACAGACACCGCCACCATGAAGGCCATCCTGGTGGTGCTGCTGTACACCTTCACCACCGCCAACG
CCGACACACTGTGCATCGGCTACCACGCCAACAACAGCACCGACACCGTGGACACCGTGCTGGAAAAGA
ACGTGACCGTGACACACAGCGTGAACCTGCTGGAAGACAAGCACAACGGCAAGCTGTGCAAGCTGAGA
GGCGTGGCACCCCTGCACCTGGGCAAGTGCAACATCGCCGGCTGGATCCTGGGCAACCCCGAGTGCGAA
AGCCTGAGCACCGCCAGAAGCTGGAGCTACATCGTGGAAACCAGCAACAGCGACAACGGCACATGCTAC
CCCGGCGACTTCATCAACTACGAGGAACTGCGGGAACAGCTGAGCAGCGTGAGCAGCTTCGAGAGATTC
GAGATCTTCCCCAAGACCAGCAGCTGGCCCAACCACGACAGCGACAAAGGCGTGACAGCCGCCTGCCCC
CACGCCGGCGCCAAGAGCTTCTACAAGAACCTGATCTGGCTGGTCAAGAAGGGCAACAGCTACCCCAAG
CTGAACCAGACCTACATCAACGACAAGGGCAAAGAGGTGCTGGTCCTCTGGGGCATCCACCACCCCCCAA
CAATCGCCGCCCAAGAGAGCCTGTACCAGAACGCCGACGCCTACGTGTTCGTGGGCACCAGCAGATACAG
CAAGAAGTTCAAGCCCGAGATCGCCACCAGGCCCAAAGTGCGGGACCAAGAGGGCAGAATGAACTACTA
CTGGACCCTGGTGGAACCCGGCGACAAGATCACATTCGAGGCCACCGGCAACCTGGTGGTCCCCAGATAC
GCCTTCACCATGGAAAGAGACGCCGGCAGCGGCATCATCATCAGCGACACCCCCGTGCACGACTGCAACA
CCACCTGCCAGACACCCGAGGGCGCCATCAACACCAGCCTGCCCTTCCAGAACGTGCACCCCATCACCATC
GGCAAGTGCCCCAAATACGTGAAGTCCACCAAGCTGAGGCTGGCCACAGGCCTGAGAAACGTGCCCAGC
ATCCAGAGCAGAGGCCTGTTCGGAGCCATCGCCGGCTTCATCGAAGGCGGCTGGACAGGCATGGTGGAC
GGATGGTACGGATACCACCACCAGAACGAGCAAGGCAGCGGCTACGCCGCCGACCTGAAGTCCACCCAG
AACGCCATCGACAAGATCACCAACAAAGTGAACAGCGTGATCGAGAAGATGAACACCCAGTTCACCGCC
GTGGGAAAAGAGTTCAACCACCTGGAAAAGCGCATCGAGAACCTGAACAAGAAGGTGGACGACGGCTT
CCTGGACATCTGGACCTACAACGCCGAGCTGCTCGTGCTGCTCGAGAACGAGAGAACCCTGGACTACCAC
GACAGCAACGTGAAGAACCTGTACGAGAAAGTGCGCAACCAGCTGAAGAACAACGCCAAAGAGATCGG
CAACGGCTGCTTCGAGTTCTACCACAAGTGCGACAACACCTGCATGGAAAGCGTGAAGAACGGCACCTAC
GACTACCCCAAGTACAGCGAGGAAGCCAAACTGAACCGCGAGAAGATCGACGGCGTGAAGCTGGAAAG
CACCCGGATCTACCAGATCCTGGCCATCTACAGCACCGTGGCCAGCAGCCTGGTGCTGGTGGTGAGCCTG
GGCGCCATCAGCTTCTGGATGTGCAGCAACGGCAGCCTGCAGTGCCGGATCTGCATCTGAGCTCGCTTTCT
TGCTGTCCAATTTCTATTAAAGGTTCCTTTGTTCCCTAAGTCCAACTACTAAACTGGGGGATATTATGAAGG
GCCTTGAGCATCTGGATTCTGCCTAATAAAAAACATTTATTTTCATTGCAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAA
AAAAAACGAAGAGC
(SEQ ID NO: 27)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of A/Guangdong-Maonan/SWL1536/2019 HA: base Nos. 95 to 1795
3'-UTR sequence: base Nos. 1796 to 1927
polyA sequence (A95): base Nos. 1928 to 2022

[Figure 65]

## Template DNA sequence of A/Guangdong-Maonan/SWL1536/2019, polyA80

GCTAGCGTAATACGACTCACTATAAGGAGACCCAAGCTACATTTGCTTCTGACACAACTGTGTTCACTAGCA
ACCTCAAACAGACACCGCCACCATGAAGGCCATCCTGGTGGTGCTGCTGTACACCTTCACCACCGCCAAC
GCCGACACACTGTGCATCGGCTACCACGCCAACAACAGCACCGACACCGTGGACACCGTGCTGGAAAAG
AACGTGACCGTGACACACAGCGTGAACCTGCTGGAAGACAAGCACAACGGCAAGCTGTGCAAGCTGAG
AGGCGTGGCACCCCTGCACCTGGGCAAGTGCAACATCGCCGGCTGGATCCTGGGCAACCCCGAGTGCGA
AAGCCTGAGCACCGCCAGAAGCTGGAGCTACATCGTGGAAACCAGCAACAGCGACAACGGCACATGCTA
CCCCGGCGACTTCATCAACTACGAGGAACTGCGGGAACAGCTGAGCAGCGTGAGCAGCTTCGAGAGATT
CGAGATCTTCCCCAAGACCAGCAGCTGGCCCAACCACGACAGCGACAAAGGCGTGACAGCCGCCTGCCC
CCACGCCGGCGCCAAGAGCTTCTACAAGAACCTGATCTGGCTGGTCAAGAAGGGCAACAGCTACCCCAA
GCTGAACCAGACCTACATCAACGACAAGGGCAAAGAGGTGCTGGTCCTCTGGGGCATCCACCACCCCCA
ACAATCGCCGCCCAAGAGAGCCTGTACCAGAACGCCGACGCCTACGTGTTCGTGGGCACCAGCAGATACA
GCAAGAAGTTCAAGCCCGAGATCGCCACCAGGCCCAAAGTGCGGGACCAAGAGGGCAGAATGAACTAC
TACTGGACCCTGGTGGAACCCGGCGACAAGATCACATTCGAGGCCACCGGCAACCTGGTGGTCCCCAGAT
ACGCCTTCACCATGGAAAGAGACGCCGGCAGCGGCATCATCATCAGCGACACCCCCGTGCACGACTGCAA
CACCACCTGCCAGACACCCGAGGGCGCCATCAACACCAGCCTGCCCTTCCAGAACGTGCACCCCATCACC
ATCGGCAAGTGCCCCAAATACGTGAAGTCCACCAAGCTGAGGCTGGCCACAGGCCTGAGAAACGTGCCC
AGCATCCAGAGCAGAGGCCTGTTCGGAGCCATCGCCGGCTTCATCGAAGGCGGCTGGACAGGCATGGTG
GACGGATGGTACGGATACCACCACCAGAACGAGCAAGGCAGCGGCTACGCCGCCGACCTGAAGTCCACC
CAGAACGCCATCGACAAGATCACCAACAAAGTGAACAGCGTGATCGAGAAGATGAACACCCAGTTCACC
GCCGTGGGAAAAGAGTTCAACCACCTGGAAAAGCGCATCGAGAACCTGAACAAGAAGGTGGACGACGG
CTTCCTGGACATCTGGACCTACAACGCCGAGCTGCTCGTGCTGCTCGAGAACGAGAGAACCCTGGACTAC
CACGACAGCAACGTGAAGAACCTGTACGAGAAAGTGCGCAACCAGCTGAAGAACAACGCCAAAGAGAT
CGGCAACGGCTGCTTCGAGTTCTACCACAAGTGCGACAACACCTGCATGGAAAGCGTGAAGAACGGCAC
CTACGACTACCCCAAGTACAGCGAGGAAGCCAAACTGAACCGCGAGAAGATCGACGGCGTGAAGCTGGA
AAGCACCCGGATCTACCAGATCCTGGCCATCTACAGCACCGTGGCCAGCAGCCTGGTGCTGGTGGTGAGC
CTGGGCGCCATCAGCTTCTGGATGTGCAGCAACGGCAGCCTGCAGTGCCGGATCTGCATCTGAGCTCGCT
TTCTTGCTGTCCAATTTCTATTAAAGGTTCCTTTGTTCCCTAAGTCCAACTACTAAACTGGGGGATATTATGA
AGGGCCTTGAGCATCTGGATTCTGCCTAATAAAAACATTTATTTTCATTGCAAAAAAAAAAAAAAAAAAAA
AAAAAAAAAAAAAAAAAAAAAAAAAAAAAAAAAAAAAAAAAAAAAAAAAAAAAAAAAAAACGAAG
AGC

(SEQ ID NO: 28)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of A/Guangdong-Maonan/SWL1536/2019 HA: base Nos. 95 to 1795
3'-UTR sequence: base Nos. 1796 to 1927
polyA sequence (A80): base Nos. 1928 to 2007

[Figure 66]

## Template DNA sequence of A/Guangdong-Maonan/SWL1536/2019, polyA60

GCTAGCGTAATACGACTCACTATAAGGAGACCCAAGCTACATTTGCTTCTGACACAACTGTGTTCACTAGCA
ACCTCAAACAGACACCGCCACCATGAAGGCCATCCTGGTGGTGCTGCTGTACACCTTCACCACCGCCAACG
CCGACACACTGTGCATCGGCTACCACGCCAACAACAGCACCGACACCGTGGACACCGTGCTGGAAAAGAA
CGTGACCGTGACACACAGCGTGAACCTGCTGGAAGACAAGCACAACGGCAAGCTGTGCAAGCTGAGAGG
CGTGGCACCCCTGCACCTGGGCAAGTGCAACATCGCCGGCTGGATCCTGGGCAACCCCGAGTGCGAAAG
CCTGAGCACCGCCAGAAGCTGGAGCTACATCGTGGAAACCAGCAACAGCGACAACGGCACATGCTACCCC
GGCGACTTCATCAACTACGAGGAACTGCGGGAACAGCTGAGCAGCGTGAGCAGCTTCGAGAGATTCGAG
ATCTTCCCCAAGACCAGCAGCTGGCCCAACCACGACAGCGACAAAGGCGTGACAGCCGCCTGCCCCCACG
CCGGCGCCAAGAGCTTCTACAAGAACCTGATCTGGCTGGTCAAGAAGGGCAACAGCTACCCCAAGCTGAA
CCAGACCTACATCAACGACAAGGGCAAAGAGGTGCTGGTCCTCTGGGGCATCCACCACCCCCCAACAATC
GCCGCCCAAGAGAGCCTGTACCAGAACGCCGACGCCTACGTGTTCGTGGGCACCAGCAGATACAGCAAG
AAGTTCAAGCCCGAGATCGCCACCAGGCCCAAAGTGCGGGACCAAGAGGGCAGAATGAACTACTACTGG
ACCCTGGTGGAACCCGGCGACAAGATCACATTCGAGGCCACCGGCAACCTGGTGGTCCCCAGATACGCCT
TCACCATGGAAAGAGACGCCGGCAGCGGCATCATCATCAGCGACACCCCCGTGCACGACTGCAACACCAC
CTGCCAGACACCCGAGGGCGCCATCAACACCAGCCTGCCCTTCCAGAACGTGCACCCCATCACCATCGGCA
AGTGCCCCAAATACGTGAAGTCCACCAAGCTGAGGCTGGCCACAGGCCTGAGAAACGTGCCCAGCATCCA
GAGCAGAGGCCTGTTCGGAGCCATCGCCGGCTTCATCGAAGGCGGCTGGACAGGCATGGTGGACGGATG
GTACGGATACCACCACCAGAACGAGCAAGGCAGCGGCTACGCCGCCGACCTGAAGTCCACCCAGAACGC
CATCGACAAGATCACCAACAAAGTGAACAGCGTGATCGAGAAGATGAACACCCAGTTCACCGCCGTGGGA
AAAGAGTTCAACCACCTGGAAAAGCGCATCGAGAACCTGAACAAGAAGGTGGACGACGGCTTCCTGGAC
ATCTGGACCTACAACGCCGAGCTGCTCGTGCTGCTCGAGAACGAGAGAACCCTGGACTACCACGACAGCA
ACGTGAAGAACCTGTACGAGAAAGTGCGCAACCAGCTGAAGAACAACGCCAAAGAGATCGGCAACGGCT
GCTTCGAGTTCTACCACAAGTGCGACAACACCTGCATGGAAAGCGTGAAGAACGGCACCTACGACTACCC
CAAGTACAGCGAGGAAGCCAAACTGAACCGCGAGAAGATCGACGGCGTGAAGCTGGAAAGCACCCGGA
TCTACCAGATCCTGGCCATCTACAGCACCGTGGCCAGCAGCCTGGTGCTGGTGGTGAGCCTGGGCGCCATC
AGCTTCTGGATGTGCAGCAACGGCAGCCTGCAGTGCCGGATCTGCATCTGAGCTCGCTTTCTTGCTGTCCA
ATTTCTATTAAAGGTTCCTTTGTTCCCTAAGTCCAACTACTAAACTGGGGGATATTATGAAGGGCCTTGAGC
ATCTGGATTCTGCCTAATAAAAAACATTTATTTTCATTGCAAAAAAAAAAAAAAAAAAAAAAAAAAAAAAA
AAAAAAAAAAAAAAAAAAAAAAAAAAAAACGAAGAGC
(SEQ ID NO: 29)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of A/Guangdong-Maonan/SWL1536/2019 HA: base Nos. 95 to 1795
3'-UTR sequence: base Nos. 1796 to 1927
polyA sequence (A60): base Nos. 1928 to 1987

[Figure 67]

## Template DNA sequence of A/Guangdong-Maonan/SWL1536/2019, polyA40

GCTAGCGTAATACGACTCACTATAAGGAGACCCAAGCTACATTTGCTTCTGACACAACTGTGTTCACTAGCA
ACCTCAAACAGACACCGCCACCATGAAGGCCATCCTGGTGGTGCTGCTGTACACCTTCACCACCGCCAACG
CCGACACACTGTGCATCGGCTACCACGCCAACAACAGCACCGACACCGTGGACACCGTGCTGGAAAAGA
ACGTGACCGTGACACACAGCGTGAACCTGCTGGAAGACAAGCACAACGGCAAGCTGTGCAAGCTGAGA
GGCGTGGCACCCCTGCACCTGGGCAAGTGCAACATCGCCGGCTGGATCCTGGGCAACCCCGAGTGCGAA
AGCCTGAGCACCGCCAGAAGCTGGAGCTACATCGTGGAAACCAGCAACAGCGACAACGGCACATGCTAC
CCCGGCGACTTCATCAACTACGAGGAACTGCGGGAACAGCTGAGCAGCGTGAGCAGCTTCGAGAGATTC
GAGATCTTCCCCAAGACCAGCAGCTGGCCCAACCACGACAGCGACAAAGGCGTGACAGCCGCCTGCCCC
CACGCCGGCGCCAAGAGCTTCTACAAGAACCTGATCTGGCTGGTCAAGAAGGGCAACAGCTACCCCAAG
CTGAACCAGACCTACATCAACGACAAGGGCAAAGAGGTGCTGGTCCTCTGGGGCATCCACCACCCCCAA
CAATCGCCGCCCAAGAGAGCCTGTACCAGAACGCCGACGCCTACGTGTTCGTGGGCACCAGCAGATACAG
CAAGAAGTTCAAGCCCGAGATCGCCACCAGGCCCAAAGTGCGGGACCAAGAGGGCAGAATGAACTACTA
CTGGACCCTGGTGGAACCCGGCGACAAGATCACATTCGAGGCCACCGGCAACCTGGTGGTCCCCAGATAC
GCCTTCACCATGGAAAGAGACGCCGGCAGCGGCATCATCATCAGCGACACCCCCGTGCACGACTGCAACA
CCACCTGCCAGACACCCGAGGGCGCCATCAACACCAGCCTGCCCTTCCAGAACGTGCACCCCATCACCATC
GGCAAGTGCCCCAAATACGTGAAGTCCACCAAGCTGAGGCTGGCCACAGGCCTGAGAAACGTGCCCAGC
ATCCAGAGCAGAGGCCTGTTCGGAGCCATCGCCGGCTTCATCGAAGGCGGCTGGACAGGCATGGTGGAC
GGATGGTACGGATACCACCACCAGAACGAGCAAGGCAGCGGCTACGCCGCCGACCTGAAGTCCACCCAG
AACGCCATCGACAAGATCACCAACAAAGTGAACAGCGTGATCGAGAAGATGAACACCCAGTTCACCGCC
GTGGGAAAAGAGTTCAACCACCTGGAAAAGCGCATCGAGAACCTGAACAAGAAGGTGGACGACGGCTT
CCTGGACATCTGGACCTACAACGCCGAGCTGCTCGTGCTGCTCGAGAACGAGAGAACCCTGGACTACCAC
GACAGCAACGTGAAGAACCTGTACGAGAAAGTGCGCAACCAGCTGAAGAACAACGCCAAAGAGATCGG
CAACGGCTGCTTCGAGTTCTACCACAAGTGCGACAACACCTGCATGGAAAGCGTGAAGAACGGCACCTAC
GACTACCCCAAGTACAGCGAGGAAGCCAAACTGAACCGCGAGAAGATCGACGGCGTGAAGCTGGAAAG
CACCCGGATCTACCAGATCCTGGCCATCTACAGCACCGTGGCCAGCAGCCTGGTGCTGGTGGTGAGCCTG
GGCGCCATCAGCTTCTGGATGTGCAGCAACGGCAGCCTGCAGTGCCGGATCTGCATCTGAGCTCGCTTTC
TTGCTGTCCAATTTCTATTAAAGGTTCCTTTGTTCCCTAAGTCCAACTACTAAACTGGGGGATATTATGAAGG
GCCTTGAGCATCTGGATTCTGCCTAATAAAAAACATTTATTTTCATTGCAAAAAAAAAAAAAAAAAAAAA
AAAAAAAAAAAAAAAAAAACGAAGAGC
(SEQ ID NO: 30)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of A/Guangdong-Maonan/SWL 1536/2019 HA: base Nos. 95 to 1795
3'-UTR sequence: base Nos. 1796 to 1927
polyA sequence (A40): base Nos. 1928 to 1967

[Figure 68]

## Template DNA sequence of A/Guangdong-Maonan/SWL1536/2019, polyA20

GCTAGCGTAATACGACTCACTATAAGGAGACCCAAGCTACATTTGCTTCTGACACAACTGTGTTCACTAGCA
ACCTCAAACAGACACCGCCACCATGAAGGCCATCCTGGTGGTGCTGCTGTACACCTTCACCACCGCCAACG
CCGACACACTGTGCATCGGCTACCACGCCAACAACAGCACCGACACCGTGGACACCGTGCTGGAAAAGA
ACGTGACCGTGACACACAGCGTGAACCTGCTGGAAGACAAGCACAACGGCAAGCTGTGCAAGCTGAGA
GGCGTGGCACCCCTGCACCTGGGCAAGTGCAACATCGCCGGCTGGATCCTGGGCAACCCCGAGTGCGAA
AGCCTGAGCACCGCCAGAAGCTGGAGCTACATCGTGGAAACCAGCAACAGCGACAACGGCACATGCTAC
CCCGGCGACTTCATCAACTACGAGGAACTGCGGGAACAGCTGAGCAGCGTGAGCAGCTTCGAGAGATTC
GAGATCTTCCCCAAGACCAGCAGCTGGCCCAACCACGACAGCGACAAAGGCGTGACAGCCGCCTGCCCC
CACGCCGGCGCCAAGAGCTTCTACAAGAACCTGATCTGGCTGGTCAAGAAGGGCAACAGCTACCCCAAG
CTGAACCAGACCTACATCAACGACAAGGGCAAAGAGGTGCTGGTCCTCTGGGGCATCCACCACCCCCAA
CAATCGCCGCCCAAGAGAGCCTGTACCAGAACGCCGACGCCTACGTGTTCGTGGGCACCAGCAGATACAG
CAAGAAGTTCAAGCCCGAGATCGCCACCAGGCCCAAAGTGCGGGACCAAGAGGGCAGAATGAACTACTA
CTGGACCCTGGTGGAACCCGGCGACAAGATCACATTCGAGGCCACCGGCAACCTGGTGGTCCCCAGATAC
GCCTTCACCATGGAAAGAGACGCCGGCAGCGGCATCATCATCAGCGACACCCCCGTGCACGACTGCAACA
CCACCTGCCAGACACCCGAGGGCGCCATCAACACCAGCCTGCCCTTCCAGAACGTGCACCCCATCACCATC
GGCAAGTGCCCCAAATACGTGAAGTCCACCAAGCTGAGGCTGGCCACAGGCCTGAGAAACGTGCCCAGC
ATCCAGAGCAGAGGCCTGTTCGGAGCCATCGCCGGCTTCATCGAAGGCGGCTGGACAGGCATGGTGGAC
GGATGGTACGGATACCACCACCAGAACGAGCAAGGCAGCGGCTACGCCGCCGACCTGAAGTCCACCCAG
AACGCCATCGACAAGATCACCAACAAAGTGAACAGCGTGATCGAGAAGATGAACACCCAGTTCACCGCC
GTGGGAAAAGAGTTCAACCACCTGGAAAAGCGCATCGAGAACCTGAACAAGAAGGTGGACGACGGCTT
CCTGGACATCTGGACCTACAACGCCGAGCTGCTCGTGCTGCTCGAGAACGAGAGAACCCTGGACTACCAC
GACAGCAACGTGAAGAACCTGTACGAGAAAGTGCGCAACCAGCTGAAGAACAACGCCAAAGAGATCGG
CAACGGCTGCTTCGAGTTCTACCACAAGTGCGACAACACCTGCATGGAAAGCGTGAAGAACGGCACCTAC
GACTACCCCAAGTACAGCGAGGAAGCCAAACTGAACCGCGAGAAGATCGACGGCGTGAAGCTGGAAAG
CACCCGGATCTACCAGATCCTGGCCATCTACAGCACCGTGGCCAGCAGCCTGGTGCTGGTGGTGAGCCTG
GGCGCCATCAGCTTCTGGATGTGCAGCAACGGCAGCCTGCAGTGCCGGATCTGCATCTGAGCTCGCTTTC
TTGCTGTCCAATTTCTATTAAAGGTTCCTTTGTTCCCTAAGTCCAACTACTAAACTGGGGGATATTATGAAGG
GCCTTGAGCATCTGGATTCTGCCTAATAAAAAACATTTATTTTCATTGCAAAAAAAAAAAAAAAAAAAACG
AAGAGC

(SEQ ID NO: 31)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of A/Guangdong-Maonan/SWL 1536/2019 HA: base Nos. 95 to 1795
3'-UTR sequence: base Nos. 1796 to 1927
polyA sequence (A20): base Nos. 1928 to 1947

[Figure 69]

## Template DNA sequence of B/Phuket/3073/2013 (Yamagata lineage), polyA110

GCTAGCGTAATACGACTCACTATAAGGAGACCCAAGCTACATTTGCTTCTGACACAACTGTGTTCACTAGC
AACCTCAAACAGACACCGCCACCATGAAGGCCATCATCGTGCTGCTGATGGTGGTCACCAGCAACGCCG
ACAGAATCTGCACCGGCATCACCAGCAGCAACAGCCCCCACGTGGTCAAGACAGCCACACAGGGCGAA
GTGAACGTGACCGGCGTGATCCCCCTGACCACCACACCCACCAAGAGCTACTTCGCCAACCTGAAGGGC
ACCAGAACCAGAGGCAAGCTGTGCCCCGACTGCCTGAACTGCACCGACCTGGACGTGGCCCTGGGCAG
ACCCATGTGCGTGGGAACAACACCCAGCGCCAAGGCCAGCATCCTGCACGAAGTGCGGCCCGTGACCA
GCGGCTGCTTCCCCATCATGCACGACCGGACCAAGATCAGACAGCTGCCCAACCTGCTGCGGGGCTACG
AGAAGATCAGGCTGAGCACCCAGAACGTGATCGACGCCGAAAAAGCCCCCGGCGGCCCCTACAGACTG
GGCACAAGCGGCAGCTGCCCCAACGCCACAAGCAAGATCGGCTTCTTCGCCACCATGGCCTGGGCCGT
GCCCAAGGACAACTACAAGAACGCCACCAACCCCCTGACCGTGGAAGTGCCCTACATCTGCACCGAAG
GCGAGGACCAGATCACCGTGTGGGGCTTCCACAGCGACAACAAGACCCAGATGAAGTCCCTGTACGGC
GACAGCAACCCCCAGAAGTTCACCAGCAGCGCCAACGGCGTGACCACACACTACGTGAGCCAGATCGG
CGACTTCCCCGACCAGACAGAAGACGGCGGACTGCCCCAGAGCGGCAGAATCGTGGTGGACTACATGA
TGCAGAAGCCCGGCAAGACCGGCACCATCGTGTACCAGAGAGGCGTCCTGCTGCCACAGAAAGTGTGG
TGCGCCAGCGGCCGGAGCAAAGTGATCAAAGGAAGCCTGCCCCTGATCGGCGAGGCCGACTGCCTGC
ACGAAGAATACGGCGGCCTGAACAAGAGCAAGCCCTACTACACAGGCAAGCACGCCAAAGCCATCGGC
AACTGCCCCATCTGGGTCAAGACCCCCCTGAAGCTGGCCAACGGCACCAAGTACAGACCCCCAGCCAA
GCTGCTGAAAGAGCGGGGCTTCTTCGGAGCCATCGCCGGCTTCCTGGAAGGCGGCTGGGAGGGAATG
ATCGCCGGCTGGCACGGCTACACAAGCCACGGCGCACACGGCGTGGCAGTGGCCGCCGACCTGAAGTC
CACACAAGAGGCCATCAACAAGATCACCAAGAACCTGAACAGCCTGAGCGAGCTGGAAGTGAAGAAC
CTGCAGAGACTGAGCGGCGCCATGGACGAGCTGCACAACGAGATCCTGGAACTGGACGAGAAGGTGG
ACGACCTGAGAGCCGACACCATCAGCAGCCAGATCGAGCTGGCAGTGCTGCTGAGCAACGAGGGCATC
ATCAACAGCGAGGACGAGCACCTGCTGGCCCTGGAACGGAAGCTGAAGAAGATGCTGGGACCCAGCG
CCGTGGACATCGGCAACGGCTGCTTCGAGACAAAGCACAAGTGCAACCAGACCTGCCTGGACAGAATC
GCCGCCGGAACCTTCAACGCCGGCGAGTTCAGCCTGCCCACCTTCGACAGCCTGAACATCACAGCCGCC
AGCCTGAACGACGACGGCCTGGACAACCACACCATCCTGCTGTACTACAGCACCGCCGCCAGCAGCCTG
GCCGTGACACTGATGCTGGCCATCTTCATCGTGTACATGGTGAGCAGAGACAACGTGAGCTGCAGCATC
TGCCTGTGAGCTCGCTTTCTTGCTGTCCAATTTCTATTAAAGGTTCCTTTGTTCCCTAAGTCCAACTACTAA
ACTGGGGGATATTATGAAGGGCCTTGAGCATCTGGATTCTGCCTAATAAAAAACATTTATTTTCATTGCAA
AAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAACGAAGAGC
(SEQ ID NO: 32)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of B/Phuket/3073/2013 (Yamagata lineage) HA: base Nos. 95 to 1849
3'-UTR sequence: base Nos. 1850 to 1981
polyA sequence (A110): base Nos. 1982 to 2091

[Figure 70]

## Template DNA sequence of B/Phuket/3073/2013 (Yamagata lineage), polyA95

GCTAGCGTAATACGACTCACTATAAGGAGACCCAAGCTACATTTGCTTCTGACACAACTGTGTTCACTAG
CAACCTCAAACAGACACCGCCACCATGAAGGCCATCATCGTGCTGCTGATGGTGGTCACCAGCAACGCC
GACAGAATCTGCACCGGCATCACCAGCAGCAACAGCCCCCACGTGGTCAAGACAGCCACACAGGGCGA
AGTGAACGTGACCGGCGTGATCCCCCTGACCACCACACCCACCAAGAGCTACTTCGCCAACCTGAAGG
GCACCAGAACCAGAGGCAAGCTGTGCCCCGACTGCCTGAACTGCACCGACCTGGACGTGGCCCTGGGC
AGACCCATGTGCGTGGGAACAACACCCAGCGCCAAGGCCAGCATCCTGCACGAAGTGCGGCCCGTGAC
CAGCGGCTGCTTCCCCATCATGCACGACCGGACCAAGATCAGACAGCTGCCCAACCTGCTGCGGGGCTA
CGAGAAGATCAGGCTGAGCACCCAGAACGTGATCGACGCCGAAAAAGCCCCCGGCGGCCCCTACAGA
CTGGGCACAAGCGGCAGCTGCCCCAACGCCACAAGCAAGATCGGCTTCTTCGCCACCATGGCCTGGGC
CGTGCCCAAGGACAACTACAAGAACGCCACCAACCCCCTGACCGTGGAAGTGCCCTACATCTGCACCGA
AGGCGAGGACCAGATCACCGTGTGGGGCTTCCACAGCGACAACAAGACCCAGATGAAGTCCCTGTACG
GCGACAGCAACCCCCAGAAGTTCACCAGCAGCGCCAACGGCGTGACCACACACTACGTGAGCCAGATC
GGCGACTTCCCCGACCAGACAGAAGACGGCGGACTGCCCCAGAGCGGCAGAATCGTGGTGGACTACA
TGATGCAGAAGCCCGGCAAGACCGGCACCATCGTGTACCAGAGAGGCGTCCTGCTGCCACAGAAAGTG
TGGTGCGCCAGCGGCCGGAGCAAAGTGATCAAAGGAAGCCTGCCCCTGATCGGCGAGGCCGACTGCC
TGCACGAAGAATACGGCGGCCTGAACAAGAGCAAGCCCTACTACACAGGCAAGCACGCCAAAGCCATC
GGCAACTGCCCCATCTGGGTCAAGACCCCCCTGAAGCTGGCCAACGGCACCAAGTACAGACCCCCAGC
CAAGCTGCTGAAAGAGCGGGGCTTCTTCGGAGCCATCGCCGGCTTCCTGGAAGGCGGCTGGGAGGGA
ATGATCGCCGGCTGGCACGGCTACACAAGCCACGGCGCACACGGCGTGGCAGTGGCCGCCGACCTGA
AGTCCACACAAGAGGCCATCAACAAGATCACCAAGAACCTGAACAGCCTGAGCGAGCTGGAAGTGAA
GAACCTGCAGAGACTGAGCGGCGCCATGGACGAGCTGCACAACGAGATCCTGGAACTGGACGAGAAG
GTGGACGACCTGAGAGCCGACACCATCAGCAGCCAGATCGAGCTGGCAGTGCTGCTGAGCAACGAGG
GCATCATCAACAGCGAGGACGAGCACCTGCTGGCCCTGGAACGGAAGCTGAAGAAGATGCTGGGACC
CAGCGCCGTGGACATCGGCAACGGCTGCTTCGAGACAAAGCACAAGTGCAACCAGACCTGCCTGGACA
GAATCGCCGCCGGAACCTTCAACGCCGGCGAGTTCAGCCTGCCCACCTTCGACAGCCTGAACATCACAG
CCGCCAGCCTGAACGACGACGGCCTGGACAACCACACCATCCTGCTGTACTACAGCACCGCCGCCAGCA
GCCTGGCCGTGACACTGATGCTGGCCATCTTCATCGTGTACATGGTGAGCAGAGACAACGTGAGCTGCA
GCATCTGCCTGTGAGCTCGCTTTCTTGCTGTCCAATTTCTATTAAAGGTTCCTTTGTTCCCTAAGTCCAACT
ACTAAACTGGGGGATATTATGAAGGGCCTTGAGCATCTGGATTCTGCCTAATAAAAAACATTTATTTTCAT
TGCAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAACGAAGAGC

(SEQ ID NO: 33)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of B/Phuket/3073/2013 (Yamagata lineage) HA: base Nos. 95 to 1849
3'-UTR sequence: base Nos. 1850 to 1981
polyA sequence (A95): base Nos. 1982 to 2076

[Figure 71]

## Template DNA sequence of B/Phuket/3073/2013 (Yamagata lineage), polyA80

GCTAGCGTAATACGACTCACTATAAGGAGACCCAAGCTACATTTGCTTCTGACACAACTGTGTTCACTAGC
AACCTCAAACAGACACCGCCACCATGAAGGCCATCATCGTGCTGCTGATGGTGGTCACCAGCAACGCCGA
CAGAATCTGCACCGGCATCACCAGCAGCAACAGCCCCCACGTGGTCAAGACAGCCACACAGGGCGAAGT
GAACGTGACCGGCGTGATCCCCCTGACCACCACACCCACCAAGAGCTACTTCGCCAACCTGAAGGGCAC
CAGAACCAGAGGCAAGCTGTGCCCCGACTGCCTGAACTGCACCGACCTGGACGTGGCCCTGGGCAGAC
CCATGTGCGTGGGAACAACACCCAGCGCCAAGGCCAGCATCCTGCACGAAGTGCGGCCCGTGACCAGC
GGCTGCTTCCCCATCATGCACGACCGGACCAAGATCAGACAGCTGCCCAACCTGCTGCGGGGCTACGAG
AAGATCAGGCTGAGCACCCAGAACGTGATCGACGCCGAAAAAGCCCCCGGCGGCCCCTACAGACTGGG
CACAAGCGGCAGCTGCCCCAACGCCACAAGCAAGATCGGCTTCTTCGCCACCATGGCCTGGGCCGTGCC
CAAGGACAACTACAAGAACGCCACCAACCCCCTGACCGTGGAAGTGCCCTACATCTGCACCGAAGGCGA
GGACCAGATCACCGTGTGGGGCTTCCACAGCGACAACAAGACCCAGATGAAGTCCCTGTACGGCGACAG
CAACCCCCAGAAGTTCACCAGCAGCGCCAACGGCGTGACCACACACTACGTGAGCCAGATCGGCGACTT
CCCCGACCAGACAGAAGACGGCGGACTGCCCCAGAGCGGCAGAATCGTGGTGGACTACATGATGCAGA
AGCCCGGCAAGACCGGCACCATCGTGTACCAGAGAGGCGTCCTGCTGCCACAGAAAGTGTGGTGCGCC
AGCGGCCGGAGCAAAGTGATCAAAGGAAGCCTGCCCCTGATCGGCGAGGCCGACTGCCTGCACGAAGA
ATACGGCGGCCTGAACAAGAGCAAGCCCTACTACACAGGCAAGCACGCCAAAGCCATCGGCAACTGCCC
CATCTGGGTCAAGACCCCCCTGAAGCTGGCCAACGGCACCAAGTACAGACCCCCAGCCAAGCTGCTGAA
AGAGCGGGGCTTCTTCGGAGCCATCGCCGGCTTCCTGGAAGGCGGCTGGGAGGGAATGATCGCCGGCT
GGCACGGCTACACAAGCCACGGCGCACACGGCGTGGCAGTGGCCGCCGACCTGAAGTCCACACAAGAG
GCCATCAACAAGATCACCAAGAACCTGAACAGCCTGAGCGAGCTGGAAGTGAAGAACCTGCAGAGACT
GAGCGGCGCCATGGACGAGCTGCACAACGAGATCCTGGAACTGGACGAGAAGGTGGACGACCTGAGA
GCCGACACCATCAGCAGCCAGATCGAGCTGGCAGTGCTGCTGAGCAACGAGGGCATCATCAACAGCGA
GGACGAGCACCTGCTGGCCCTGGAACGGAAGCTGAAGAAGATGCTGGGACCCAGCGCCGTGGACATCG
GCAACGGCTGCTTCGAGACAAAGCACAAGTGCAACCAGACCTGCCTGGACAGAATCGCCGCCGGAACC
TTCAACGCCGGCGAGTTCAGCCTGCCCACCTTCGACAGCCTGAACATCACAGCCGCCAGCCTGAACGAC
GACGGCCTGGACAACCACACCATCCTGCTGTACTACAGCACCGCCGCCAGCAGCCTGGCCGTGACACTG
ATGCTGGCCATCTTCATCGTGTACATGGTGAGCAGAGACAACGTGAGCTGCAGCATCTGCCTGTGAGCTC
GCTTTCTTGCTGTCCAATTTCTATTAAAGGTTCCTTTGTTCCCTAAGTCCAACTACTAAACTGGGGGATATTA
TGAAGGGCCTTGAGCATCTGGATTCTGCCTAATAAAAAACATTTATTTTCATTGCAAAAAAAAAAAAAAA
AAAAAAAAAAAAAAAAAAAAAAAAAAAAAAAAAAAAAAAAAAAAAAAAAAAAAAAAAAAAAAAAACG
AAGAGC
(SEQ ID NO: 34)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of B/Phuket/3073/2013 (Yamagata lineage) HA: base Nos. 95 to 1849
3'-UTR sequence: base Nos. 1850 to 1981
polyA sequence (A80): base Nos. 1982 to 2061

[Figure 72]

## Template DNA sequence of B/Phuket/3073/2013 (Yamagata lineage), polyA60

GCTAGCGTAATACGACTCACTATAAGGAGACCCAAGCTACATTTGCTTCTGACACAACTGTGTTCACTA
GCAACCTCAAACAGACACCGCCACCATGAAGGCCATCATCGTGCTGCTGATGGTGGTCACCAGCAAC
GCCGACAGAATCTGCACCGGCATCACCAGCAGCAACAGCCCCCACGTGGTCAAGACAGCCACACAG
GGCGAAGTGAACGTGACCGGCGTGATCCCCCTGACCACCACACCCACCAAGAGCTACTTCGCCAACC
TGAAGGGCACCAGAACCAGAGGCAAGCTGTGCCCCGACTGCCTGAACTGCACCGACCTGGACGTGG
CCCTGGGCAGACCCATGTGCGTGGGAACAACACCCAGCGCCAAGGCCAGCATCCTGCACGAAGTGC
GGCCCGTGACCAGCGGCTGCTTCCCCATCATGCACGACCGGACCAAGATCAGACAGCTGCCCAACCT
GCTGCGGGGCTACGAGAAGATCAGGCTGAGCACCCAGAACGTGATCGACGCCGAAAAAGCCCCCGG
CGGCCCCTACAGACTGGGCACAAGCGGCAGCTGCCCCAACGCCACAAGCAAGATCGGCTTCTTCGCC
ACCATGGCCTGGGCCGTGCCCAAGGACAACTACAAGAACGCCACCAACCCCCTGACCGTGGAAGTG
CCCTACATCTGCACCGAAGGCGAGGACCAGATCACCGTGTGGGGCTTCCACAGCGACAACAAGACCC
AGATGAAGTCCCTGTACGGCGACAGCAACCCCCAGAAGTTCACCAGCAGCGCCAACGGCGTGACCA
CACACTACGTGAGCCAGATCGGCGACTTCCCCGACCAGACAGAAGACGGCGGACTGCCCCAGAGCG
GCAGAATCGTGGTGGACTACATGATGCAGAAGCCCGGCAAGACCGGCACCATCGTGTACCAGAGAG
GCGTCCTGCTGCCACAGAAAGTGTGGTGCGCCAGCGGCCGGAGCAAAGTGATCAAAGGAAGCCTGC
CCCTGATCGGCGAGGCCGACTGCCTGCACGAAGAATACGGCGGCCTGAACAAGAGCAAGCCCTACTA
CACAGGCAAGCACGCCAAAGCCATCGGCAACTGCCCCATCTGGGTCAAGACCCCCCTGAAGCTGGCC
AACGGCACCAAGTACAGACCCCCAGCCAAGCTGCTGAAAGAGCGGGGCTTCTTCGGAGCCATCGCC
GGCTTCCTGGAAGGCGGCTGGGAGGGAATGATCGCCGGCTGGCACGGCTACACAAGCCACGGCGC
ACACGGCGTGGCAGTGGCCGCCGACCTGAAGTCCACACAAGAGGCCATCAACAAGATCACCAAGAA
CCTGAACAGCCTGAGCGAGCTGGAAGTGAAGAACCTGCAGAGACTGAGCGGCGCCATGGACGAGC
TGCACAACGAGATCCTGGAACTGGACGAGAAGGTGGACGACCTGAGAGCCGACACCATCAGCAGCC
AGATCGAGCTGGCAGTGCTGCTGAGCAACGAGGGCATCATCAACAGCGAGGACGAGCACCTGCTGG
CCCTGGAACGGAAGCTGAAGAAGATGCTGGGACCCAGCGCCGTGGACATCGGCAACGGCTGCTTCG
AGACAAAGCACAAGTGCAACCAGACCTGCCTGGACAGAATCGCCGCCGGAACCTTCAACGCCGGCG
AGTTCAGCCTGCCCACCTTCGACAGCCTGAACATCACAGCCGCCAGCCTGAACGACGACGGCCTGGA
CAACCACACCATCCTGCTGTACTACAGCACCGCCGCCAGCAGCCTGGCCGTGACACTGATGCTGGCCA
TCTTCATCGTGTACATGGTGAGCAGAGACAACGTGAGCTGCAGCATCTGCCTGTGAGCTCGCTTTCTT
GCTGTCCAATTTCTATTAAAGGTTCCTTTGTTCCCTAAGTCCAACTACTAAACTGGGGGATATTATGAAG
GGCCTTGAGCATCTGGATTCTGCCTAATAAAAACATTTATTTTCATTGCAAAAAAAAAAAAAAAAA
AAAAAAAAAAAAAAAAAAAAAAAAAAAAAAAAAAAAAAAAAAACGAAGAGC
(SEQ ID NO: 35)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of B/Phuket/3073/2013 (Yamagata lineage) HA: base Nos. 95 to 1849
3'-UTR sequence: base Nos. 1850 to 1981
polyA sequence (A60): base Nos. 1982 to 2041

[Figure 73]

## Template DNA sequence of B/Phuket/3073/2013 (Yamagata lineage), polyA40

GCTAGCGTAATACGACTCACTATAAGGAGACCCAAGCTACATTTGCTTCTGACACAACTGTGTTCACTA
GCAACCTCAAACAGACACCGCCACCATGAAGGCCATCATCGTGCTGCTGATGGTGGTCACCAGCAAC
GCCGACAGAATCTGCACCGGCATCACCAGCAGCAACAGCCCCCACGTGGTCAAGACAGCCACACAG
GGCGAAGTGAACGTGACCGGCGTGATCCCCCTGACCACCACACCCACCAAGAGCTACTTCGCCAACC
TGAAGGGCACCAGAACCAGAGGCAAGCTGTGCCCCGACTGCCTGAACTGCACCGACCTGGACGTGG
CCCTGGGCAGACCCATGTGCGTGGGAACAACACCCAGCGCCAAGGCCAGCATCCTGCACGAAGTGC
GGCCCGTGACCAGCGGCTGCTTCCCCATCATGCACGACCGGACCAAGATCAGACAGCTGCCCAACCT
GCTGCGGGGCTACGAGAAGATCAGGCTGAGCACCCAGAACGTGATCGACGCCGAAAAGCCCCCGG
CGGCCCCTACAGACTGGGCACAAGCGGCAGCTGCCCCAACGCCACAAGCAAGATCGGCTTCTTCGCC
ACCATGGCCTGGGCCGTGCCCAAGGACAACTACAAGAACGCCACCAACCCCCTGACCGTGGAAGTGC
CCTACATCTGCACCGAAGGCGAGGACCAGATCACCGTGTGGGGCTTCCACAGCGACAACAAGACCCA
GATGAAGTCCCTGTACGGCGACAGCAACCCCCAGAAGTTCACCAGCAGCGCCAACGGCGTGACCACA
CACTACGTGAGCCAGATCGGCGACTTCCCCGACCAGACAGAAGACGGCGGACTGCCCCAGAGCGGC
AGAATCGTGGTGGACTACATGATGCAGAAGCCCGGCAAGACCGGCACCATCGTGTACCAGAGAGGC
GTCCTGCTGCCACAGAAAGTGTGGTGCGCCAGCGGCCGGAGCAAAGTGATCAAAGGAAGCCTGCCC
CTGATCGGCGAGGCCGACTGCCTGCACGAAGAATACGGCGGCCTGAACAAGAGCAAGCCCTACTACA
CAGGCAAGCACGCCAAAGCCATCGGCAACTGCCCCATCTGGGTCAAGACCCCCCTGAAGCTGGCCAA
CGGCACCAAGTACAGACCCCCAGCCAAGCTGCTGAAAGAGCGGGGCTTCTTCGGAGCCATCGCCGG
CTTCCTGGAAGGCGGCTGGGAGGGAATGATCGCCGGCTGGCACGGCTACACAAGCCACGGCGCACA
CGGCGTGGCAGTGGCCGCCGACCTGAAGTCCACACAAGAGGCCATCAACAAGATCACCAAGAACCT
GAACAGCCTGAGCGAGCTGGAAGTGAAGAACCTGCAGAGACTGAGCGGCGCCATGGACGAGCTGC
ACAACGAGATCCTGGAACTGGACGAGAAGGTGGACGACCTGAGAGCCGACACCATCAGCAGCCAGA
TCGAGCTGGCAGTGCTGCTGAGCAACGAGGGCATCATCAACAGCGAGGACGAGCACCTGCTGGCCC
TGGAACGGAAGCTGAAGAAGATGCTGGGACCCAGCGCCGTGGACATCGGCAACGGCTGCTTCGAGA
CAAAGCACAAGTGCAACCAGACCTGCCTGGACAGAATCGCCGCCGGAACCTTCAACGCCGGCGAGT
TCAGCCTGCCCACCTTCGACAGCCTGAACATCACAGCCGCCAGCCTGAACGACGACGGCCTGGACAA
CCACACCATCCTGCTGTACTACAGCACCGCCGCCAGCAGCCTGGCCGTGACACTGATGCTGGCCATCT
TCATCGTGTACATGGTGAGCAGAGACAACGTGAGCTGCAGCATCTGCCTGTGAGCTCGCTTTCTTGCT
GTCCAATTTCTATTAAAGGTTCCTTTGTTCCCTAAGTCCAACTACTAAACTGGGGGATATTATGAAGGGC
CTTGAGCATCTGGATTCTGCCTAATAAAAAACATTTATTTTCATTGCAAAAAAAAAAAAAAAAAAAAAA
AAAAAAAAAAAAAAAAAACGAAGAGC

(SEQ ID NO: 36)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of B/Phuket/3073/2013 (Yamagata lineage) HA: base Nos. 95 to 1849
3'-UTR sequence: base Nos. 1850 to 1981
polyA sequence (A40): base Nos. 1982 to 2021

[Figure 74]

## Template DNA sequence of B/Phuket/3073/2013 (Yamagata lineage), polyA20

GCTAGCGTAATACGACTCACTATAAGGAGACCCAAGCTACATTTGCTTCTGACACAACTGTGTTCACTAG
CAACCTCAAACAGACACCGCCACCATGAAGGCCATCATCGTGCTGCTGATGGTGGTCACCAGCAACGCC
GACAGAATCTGCACCGGCATCACCAGCAGCAACAGCCCCCACGTGGTCAAGACAGCCACACAGGGCG
AAGTGAACGTGACCGGCGTGATCCCCCTGACCACCACACCCACCAAGAGCTACTTCGCCAACCTGAAG
GGCACCAGAACCAGAGGCAAGCTGTGCCCCGACTGCCTGAACTGCACCGACCTGGACGTGGCCCTGG
GCAGACCCATGTGCGTGGGAACAACACCCAGCGCCAAGGCCAGCATCCTGCACGAAGTGCGGCCCGT
GACCAGCGGCTGCTTCCCCATCATGCACGACCGGACCAAGATCAGACAGCTGCCCAACCTGCTGCGGG
GCTACGAGAAGATCAGGCTGAGCACCCAGAACGTGATCGACGCCGAAAAAGCCCCCGGCGGCCCCTAC
AGACTGGGCACAAGCGGCAGCTGCCCCAACGCCACAAGCAAGATCGGCTTCTTCGCCACCATGGCCTG
GGCCGTGCCCAAGGACAACTACAAGAACGCCACCAACCCCCTGACCGTGGAAGTGCCCTACATCTGCA
CCGAAGGCGAGGACCAGATCACCGTGTGGGGCTTCCACAGCGACAACAAGACCCAGATGAAGTCCCT
GTACGGCGACAGCAACCCCCAGAAGTTCACCAGCAGCGCCAACGGCGTGACCACACACTACGTGAGCC
AGATCGGCGACTTCCCCGACCAGACAGAAGACGGCGGACTGCCCCAGAGCGGCAGAATCGTGGTGGA
CTACATGATGCAGAAGCCCGGCAAGACCGGCACCATCGTGTACCAGAGAGGCGTCCTGCTGCCACAGA
AAGTGTGGTGCGCCAGCGGCCGGAGCAAAGTGATCAAAGGAAGCCTGCCCCTGATCGGCGAGGCCGA
CTGCCTGCACGAAGAATACGGCGGCCTGAACAAGAGCAAGCCCTACTACACAGGCAAGCACGCCAAAG
CCATCGGCAACTGCCCCATCTGGGTCAAGACCCCCCTGAAGCTGGCCAACGGCACCAAGTACAGACCCC
CAGCCAAGCTGCTGAAAGAGCGGGGCTTCTTCGGAGCCATCGCCGGCTTCCTGGAAGGCGGCTGGGA
GGGAATGATCGCCGGCTGGCACGGCTACACAAGCCACGGCGCACACGGCGTGGCAGTGGCCGCCGAC
CTGAAGTCCACACAAGAGGCCATCAACAAGATCACCAAGAACCTGAACAGCCTGAGCGAGCTGGAAGT
GAAGAACCTGCAGAGACTGAGCGGCGCCATGGACGAGCTGCACAACGAGATCCTGGAACTGGACGAG
AAGGTGGACGACCTGAGAGCCGACACCATCAGCAGCCAGATCGAGCTGGCAGTGCTGCTGAGCAACG
AGGGCATCATCAACAGCGAGGACGAGCACCTGCTGGCCCTGGAACGGAAGCTGAAGAAGATGCTGGG
ACCCAGCGCCGTGGACATCGGCAACGGCTGCTTCGAGACAAAGCACAAGTGCAACCAGACCTGCCTG
GACAGAATCGCCGCCGGAACCTTCAACGCCGGCGAGTTCAGCCTGCCCACCTTCGACAGCCTGAACAT
CACAGCCGCCAGCCTGAACGACGACGGCCTGGACAACCACACCATCCTGCTGTACTACAGCACCGCCGC
CAGCAGCCTGGCCGTGACACTGATGCTGGCCATCTTCATCGTGTACATGGTGAGCAGAGACAACGTGAG
CTGCAGCATCTGCCTGTGAGCTCGCTTTCTTGCTGTCCAATTTCTATTAAAGGTTCCTTTGTTCCCTAAGTC
CAACTACTAAACTGGGGGATATTATGAAGGGCCTTGAGCATCTGGATTCTGCCTAATAAAAAACATTTATT
TTCATTGCAAAAAAAAAAAAAAAAAAAACGAAGAGC
(SEQ ID NO: 37)

GCTAGC (NheI site): base Nos. 1 to 6
T7 Promoter sequence (before transcription start point): base Nos. 8 to 24
A (transcription start point): base No. 25
5'-UTR sequence (including transcription start point and KOZAK sequence of base Nos. 89 to 94): base Nos. 25 to 94
Sequence of translated region of B/Phuket/3073/2013 (Yamagata lineage) HA: base Nos. 95 to 1849
3'-UTR sequence: base Nos. 1850 to 1981
polyA sequence (A20): base Nos. 1982 to 2001

[Figure 75]

mRNA sequence of A/Guangdong-Maonan/SWL1536/2019, polyA110

AGGAGACCCAAGCUACAUUUGCUUCUGACACAACUGUGUUCACUAGCAACCUCAAACAGACACCG
CCACCAUGAAGGCCAUCCUGGUGGUGCUGCUGUACACCUUCACCACCGCCAACGCCGACACACUG
UGCAUCGGCUACCACGCCAACAACAGCACCGACACCGUGGACACCGUGCUGGAAAAGAACGUGAC
CGUGACACACAGCGUGAACCUGCUGGAAGACAAGCACAACGGCAAGCUGUGCAAGCUGAGAGGC
GUGGCACCCCUGCACCUGGGCAAGUGCAACAUCGCCGGCUGGAUCCUGGGCAACCCCGAGUGCGA
AAGCCUGAGCACCGCCAGAAGCUGGAGCUACAUCGUGGAAACCAGCAACAGCGACAACGGCACAU
GCUACCCCGGCGACUUCAUCAACUACGAGGAACUGCGGGAACAGCUGAGCAGCGUGAGCAGCUU
CGAGAGAUUCGAGAUCUUCCCCAAGACCAGCAGCUGGCCCAACCACGACAGCGACAAAGGCGUGA
CAGCCGCCUGCCCCCACGCCGGCGCCAAGAGCUUCUACAAGAACCUGAUCUGGCUGGUCAAGAAG
GGCAACAGCUACCCCAAGCUGAACCAGACCUACAUCAACGACAAGGGCAAAGAGGUGCUGGUCCU
CUGGGGCAUCCACCACCCCCCAACAAUCGCCGCCCAAGAGAGCCUGUACCAGAACGCCGACGCCUA
CGUGUUCGUGGGCACCAGCAGAUACAGCAAGAAGUUCAAGCCCGAGAUCGCCACCAGGCCCAAAG
UGCGGGACCAAGAGGGCAGAAUGAACUACUACUGGACCCUGGUGGAACCCGGCGACAAGAUCAC
AUUCGAGGCCACCGGCAACCUGGUGGUCCCCAGAUACGCCUUCACCAUGGAAAGAGACGCCGGCA
GCGGCAUCAUCAUCAGCGACACCCCCGUGCACGACUGCAACACCACCUGCCAGACACCCGAGGGCG
CCAUCAACACCAGCCUGCCCUUCCAGAACGUGCACCCCAUCACCAUCGGCAAGUGCCCCAAAUACG
UGAAGUCCACCAAGCUGAGGCUGGCCACAGGCCUGAGAAACGUGCCCAGCAUCCAGAGCAGAGGC
CUGUUCGGAGCCAUCGCCGGCUUCAUCGAAGGCGGCUGGACAGGCAUGGUGGACGGAUGGUAC
GGAUACCACCACCAGAACGAGCAAGGCAGCGGCUACGCCGCCGACCUGAAGUCCACCCAGAACGCC
AUCGACAAGAUCACCAACAAAGUGAACAGCGUGAUCGAGAAGAUGAACACCCAGUUCACCGCCGU
GGGAAAAGAGUUCAACCACCUGGAAAAGCGCAUCGAGAACCUGAACAAGAAGGUGGACGACGGC
UUCCUGGACAUCUGGACCUACAACGCCGAGCUGCUCGUGCUGCUCGAGAACGAGAGAACCCUGG
ACUACCACGACAGCAACGUGAAGAACCUGUACGAGAAAGUGCGCAACCAGCUGAAGAACAACGCC
AAAGAGAUCGGCAACGGCUGCUUCGAGUUCUACCACAAGUGCGACAACACCUGCAUGGAAAGCG
UGAAGAACGGCACCUACGACUACCCCAAGUACAGCGAGGAAGCCAAACUGAACCGCGAGAAGAUC
GACGGCGUGAAGCUGGAAAGCACCCGGAUCUACCAGAUCCUGGCCAUCUACAGCACCGUGGCCAG
CAGCCUGGUGCUGGUGGUGAGCCUGGGCGCCAUCAGCUUCUGGAUGUGCAGCAACGGCAGCCU
GCAGUGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAUUCUG
CCUAAUAAAAAACAUUUAUUUUCAUUGCAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAA
AAAAAAAA

(SEQ ID NO: 38)

[Figure 76]

## mRNA sequence of A/Guangdong-Maonan/SWL1536/2019, polyA95

AGGAGACCCAAGCUACAUUUGCUUCUGACACAACUGUGUUCACUAGCAACCUCAAACAGACACCG
CCACCAUGAAGGCCAUCCUGGUGGUGCUGCUGUACACCUUCACCACCGCCAACGCCGACACACUG
UGCAUCGGCUACCACGCCAACAACAGCACCGACACCGUGGACACCGUGCUGGAAAAGAACGUGAC
CGUGACACACAGCGUGAACCUGCUGGAAGACAAGCACAACGGCAAGCUGUGCAAGCUGAGAGGC
GUGGCACCCCUGCACCUGGGCAAGUGCAACAUCGCCGGCUGGAUCCUGGGCAACCCCGAGUGCGA
AAGCCUGAGCACCGCCAGAAGCUGGAGCUACAUCGUGGAAACCAGCAACAGCGACAACGGCACAU
GCUACCCCGGCGACUUCAUCAACUACGAGGAACUGCGGGAACAGCUGAGCAGCGUGAGCAGCUU
CGAGAGAUUCGAGAUCUUCCCCAAGACCAGCAGCUGGCCCAACCACGACAGCGACAAAGGCGUGA
CAGCCGCCUGCCCCCACGCCGGCGCCAAGAGCUUCUACAAGAACCUGAUCUGGCUGGUCAAGAAG
GGCAACAGCUACCCCAAGCUGAACCAGACCUACAUCAACGACAAGGGCAAAGAGGUGCUGGUCCU
CUGGGGCAUCCACCACCCCCCAACAAUCGCCGCCCAAGAGAGCCUGUACCAGAACGCCGACGCCUA
CGUGUUCGUGGGCACCAGCAGAUACAGCAAGAAGUUCAAGCCCGAGAUCGCCACCAGGCCCAAAG
UGCGGGACCAAGAGGGCAGAAUGAACUACUACUGGACCCUGGUGGAACCCGGCGACAAGAUCAC
AUUCGAGGCCACCGGCAACCUGGUGGUCCCCAGAUACGCCUUCACCAUGGAAAGAGACGCCGGCA
GCGGCAUCAUCAUCAGCGACACCCCCGUGCACGACUGCAACACCACCUGCCAGACACCCGAGGGCG
CCAUCAACACCAGCCUGCCCUUCCAGAACGUGCACCCCAUCACCAUCGGCAAGUGCCCCAAAUACG
UGAAGUCCACCAAGCUGAGGCUGGCCACAGGCCUGAGAAACGUGCCCAGCAUCCAGAGCAGAGGC
CUGUUCGGAGCCAUCGCCGGCUUCAUCGAAGGCGGCUGGACAGGCAUGGUGGACGGAUGGUAC
GGAUACCACCACCAGAACGAGCAAGGCAGCGGCUACGCCGCCGACCUGAAGUCCACCCAGAACGCC
AUCGACAAGAUCACCAACAAAGUGAACAGCGUGAUCGAGAAGAUGAACACCCAGUUCACCGCCGU
GGGAAAAGAGUUCAACCACCUGGAAAAGCGCAUCGAGAACCUGAACAAGAAGGUGGACGACGGC
UUCCUGGACAUCUGGACCUACAACGCCGAGCUGCUCGUGCUGCUCGAGAACGAGAGAACCCUGG
ACUACCACGACAGCAACGUGAAGAACCUGUACGAGAAAGUGCGCAACCAGCUGAAGAACAACGCC
AAAGAGAUCGGCAACGGCUGCUUCGAGUUCUACCACAAGUGCGACAACACCUGCAUGGAAAGCG
UGAAGAACGGCACCUACGACUACCCCAAGUACAGCGAGGAAGCCAAACUGAACCGCGAGAAGAUC
GACGGCGUGAAGCUGGAAAGCACCCGGAUCUACCAGAUCCUGGCCAUCUACAGCACCGUGGCCAG
CAGCCUGGUGCUGGUGGUGAGCCUGGGCGCCAUCAGCUUCUGGAUGUGCAGCAACGGCAGCCU
GCAGUGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAUUCUG
CCUAAUAAAAAACAUUUAUUUUCAUUGCAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAA

(SEQ ID NO: 39)

[Figure 77]

## mRNA sequence of A/Guangdong-Maonan/SWL1536/2019, polyA80

AGGAGACCCAAGCUACAUUUGCUUCUGACACAACUGUGUUCACUAGCAACCUCAAACAGACACCG
CCACCAUGAAGGCCAUCCUGGUGGUGCUGCUGUACACCUUCACCACCGCCAACGCCGACACACUG
UGCAUCGGCUACCACGCCAACAACAGCACCGACACCGUGGACACCGUGCUGGAAAAGAACGUGAC
CGUGACACACAGCGUGAACCUGCUGGAAGACAAGCACAACGGCAAGCUGUGCAAGCUGAGAGGC
GUGGCACCCCUGCACCUGGGCAAGUGCAACAUCGCCGGCUGGAUCCUGGGCAACCCCGAGUGCGA
AAGCCUGAGCACCGCCAGAAGCUGGAGCUACAUCGUGGAAACCAGCAACAGCGACAACGGCACAU
GCUACCCCGGCGACUUCAUCAACUACGAGGAACUGCGGGAACAGCUGAGCAGCGUGAGCAGCUU
CGAGAGAUUCGAGAUCUUCCCCAAGACCAGCAGCUGGCCCAACCACGACAGCGACAAAGGCGUGA
CAGCCGCCUGCCCCCACGCCGGCGCCAAGAGCUUCUACAAGAACCUGAUCUGGCUGGUCAAGAAG
GGCAACAGCUACCCCAAGCUGAACCAGACCUACAUCAACGACAAGGGCAAAGAGGUGCUGGUCCU
CUGGGGCAUCCACCACCCCCCAACAAUCGCCGCCCAAGAGAGCCUGUACCAGAACGCCGACGCCUA
CGUGUUCGUGGGCACCAGCAGAUACAGCAAGAAGUUCAAGCCCGAGAUCGCCACCAGGCCCAAAG
UGCGGGACCAAGAGGGCAGAAUGAACUACUACUGGACCCUGGUGGAACCCGGCGACAAGAUCAC
AUUCGAGGCCACCGGCAACCUGGUGGUCCCCAGAUACGCCUUCACCAUGGAAAGAGACGCCGGCA
GCGGCAUCAUCAUCAGCGACACCCCCGUGCACGACUGCAACACCACCUGCCAGACACCCGAGGGCG
CCAUCAACACCAGCCUGCCCUUCCAGAACGUGCACCCCAUCACCAUCGGCAAGUGCCCCAAAUACG
UGAAGUCCACCAAGCUGAGGCUGGCCACAGGCCUGAGAAACGUGCCCAGCAUCCAGAGCAGAGGC
CUGUUCGGAGCCAUCGCCGGCUUCAUCGAAGGCGGCUGGACAGGCAUGGUGGACGGAUGGUAC
GGAUACCACCACCAGAACGAGCAAGGCAGCGGCUACGCCGCCGACCUGAAGUCCACCCAGAACGCC
AUCGACAAGAUCACCAACAAAGUGAACAGCGUGAUCGAGAAGAUGAACACCCAGUUCACCGCCGU
GGGAAAAGAGUUCAACCACCUGGAAAAGCGCAUCGAGAACCUGAACAAGAAGGUGGACGACGGC
UUCCUGGACAUCUGGACCUACAACGCCGAGCUGCUCGUGCUGCUCGAGAACGAGAGAACCCUGG
ACUACCACGACAGCAACGUGAAGAACCUGUACGAGAAAGUGCGCAACCAGCUGAAGAACAACGCC
AAAGAGAUCGGCAACGGCUGCUUCGAGUUCUACCACAAGUGCGACAACACCUGCAUGGAAAGCG
UGAAGAACGGCACCUACGACUACCCCAAGUACAGCGAGGAAGCCAAACUGAACCGCGAGAAGAUC
GACGGCGUGAAGCUGGAAAGCACCCGGAUCUACCAGAUCCUGGCCAUCUACAGCACCGUGGCCAG
CAGCCUGGUGCUGGUGGUGAGCCUGGGCGCCAUCAGCUUCUGGAUGUGCAGCAACGGCAGCCU
GCAGUGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAUUCUG
CCUAAUAAAAAACAUUUAUUUCAUUGCAAAAAAAAAAAAAAAAAAAAAAAAAAAAAAAAAAAAA
AAAAAAAAAAAAAAAAAAAAAAAAAAAAAAAAAAAAAAAAAAA

(SEQ ID NO: 40)

[Figure 78]

## mRNA sequence of A/Guangdong-Maonan/SWL1536/2019, polyA60

AGGAGACCCAAGCUACAUUUGCUUCUGACACAACUGUGUUCACUAGCAACCUCAAACAGACACCG
CCACCAUGAAGGCCAUCCUGGUGGUGCUGCUGUACACCUUCACCACCGCCAACGCCGACACACUG
UGCAUCGGCUACCACGCCAACAACAGCACCGACACCGUGGACACCGUGCUGGAAAAGAACGUGAC
CGUGACACACAGCGUGAACCUGCUGGAAGACAAGCACAACGGCAAGCUGUGCAAGCUGAGAGGC
GUGGCACCCCUGCACCUGGGCAAGUGCAACAUCGCCGGCUGGAUCCUGGGCAACCCCGAGUGCGA
AAGCCUGAGCACCGCCAGAAGCUGGAGCUACAUCGUGGAAACCAGCAACAGCGACAACGGCACAU
GCUACCCCGGCGACUUCAUCAACUACGAGGAACUGCGGGAACAGCUGAGCAGCGUGAGCAGCUU
CGAGAGAUUCGAGAUCUUCCCCAAGACCAGCAGCUGGCCCAACCACGACAGCGACAAAGGCGUGA
CAGCCGCCUGCCCCCACGCCGGCGCCAAGAGCUUCUACAAGAACCUGAUCUGGCUGGUCAAGAAG
GGCAACAGCUACCCCAAGCUGAACCAGACCUACAUCAACGACAAGGGCAAAGAGGUGCUGGUCCU
CUGGGGCAUCCACCACCCCCCAACAAUCGCCGCCCAAGAGAGCCUGUACCAGAACGCCGACGCCUA
CGUGUUCGUGGGCACCAGCAGAUACAGCAAGAAGUUCAAGCCCGAGAUCGCCACCAGGCCCAAAG
UGCGGGACCAAGAGGGCAGAAUGAACUACUACUGGACCCUGGUGGAACCCGGCGACAAGAUCAC
AUUCGAGGCCACCGGCAACCUGGUGGUCCCCAGAUACGCCUUCACCAUGGAAAGAGACGCCGGCA
GCGGCAUCAUCAUCAGCGACACCCCCGUGCACGACUGCAACACCACCUGCCAGACACCCGAGGGCG
CCAUCAACACCAGCCUGCCCUUCCAGAACGUGCACCCCAUCACCAUCGGCAAGUGCCCCAAAUACG
UGAAGUCCACCAAGCUGAGGCUGGCCACAGGCCUGAGAAACGUGCCCAGCAUCCAGAGCAGAGGC
CUGUUCGGAGCCAUCGCCGGCUUCAUCGAAGGCGGCUGGACAGGCAUGGUGGACGGAUGGUAC
GGAUACCACCACCAGAACGAGCAAGGCAGCGGCUACGCCGCCGACCUGAAGUCCACCCAGAACGCC
AUCGACAAGAUCACCAACAAAGUGAACAGCGUGAUCGAGAAGAUGAACACCCAGUUCACCGCCGU
GGGAAAAGAGUUCAACCACCUGGAAAAGCGCAUCGAGAACCUGAACAAGAAGGUGGACGACGGC
UUCCUGGACAUCUGGACCUACAACGCCGAGCUGCUCGUGCUGCUCGAGAACGAGAGAACCCUGG
ACUACCACGACAGCAACGUGAAGAACCUGUACGAGAAAGUGCGCAACCAGCUGAAGAACAACGCC
AAAGAGAUCGGCAACGGCUGCUUCGAGUUCUACCACAAGUGCGACAACACCUGCAUGGAAAGCG
UGAAGAACGGCACCUACGACUACCCCAAGUACAGCGAGGAAGCCAAACUGAACCGCGAGAAGAUC
GACGGCGUGAAGCUGGAAAGCACCCGGAUCUACCAGAUCCUGGCCAUCUACAGCACCGUGGCCAG
CAGCCUGGUGCUGGUGGUGAGCCUGGGCGCCAUCAGCUUCUGGAUGUGCAGCAACGGCAGCCU
GCAGUGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAUUCUG
CCUAAUAAAAAACAUUUAUUUCAUUGCAAAAAAAAAAAAAAAAAAAAAAAAAAAAAAAAAAAAAA
AAAAAAAAAAAAAAAAAAAAAA

(SEQ ID NO: 41)

[Figure 79]

## mRNA sequence of A/Guangdong-Maonan/SWL1536/2019, polyA40

AGGAGACCCAAGCUACAUUUGCUUCUGACACAACUGUGUUCACUAGCAACCUCAAACAGACACCG
CCACCAUGAAGGCCAUCCUGGUGGUGCUGCUGUACACCUUCACCACCGCCAACGCCGACACACUG
UGCAUCGGCUACCACGCCAACAACAGCACCGACACCGUGGACACCGUGCUGGAAAAGAACGUGAC
CGUGACACACAGCGUGAACCUGCUGGAAGACAAGCACAACGGCAAGCUGUGCAAGCUGAGAGGC
GUGGCACCCCUGCACCUGGGCAAGUGCAACAUCGCCGGCUGGAUCCUGGGCAACCCCGAGUGCGA
AAGCCUGAGCACCGCCAGAAGCUGGAGCUACAUCGUGGAAACCAGCAACAGCGACAACGGCACAU
GCUACCCCGGCGACUUCAUCAACUACGAGGAACUGCGGGAACAGCUGAGCAGCGUGAGCAGCUU
CGAGAGAUUCGAGAUCUUCCCCAAGACCAGCAGCUGGCCCAACCACGACAGCGACAAAGGCGUGA
CAGCCGCCUGCCCCCACGCCGGCGCCAAGAGCUUCUACAAGAACCUGAUCUGGCUGGUCAAGAAG
GGCAACAGCUACCCCAAGCUGAACCAGACCUACAUCAACGACAAGGGCAAAGAGGUGCUGGUCCU
CUGGGGCAUCCACCACCCCCCAACAAUCGCCGCCCAAGAGAGCCUGUACCAGAACGCCGACGCCUA
CGUGUUCGUGGGCACCAGCAGAUACAGCAAGAAGUUCAAGCCCGAGAUCGCCACCAGGCCCAAAG
UGCGGGACCAAGAGGGCAGAAUGAACUACUACUGGACCCUGGUGGAACCCGGCGACAAGAUCAC
AUUCGAGGCCACCGGCAACCUGGUGGUCCCCAGAUACGCCUUCACCAUGGAAAGAGACGCCGGCA
GCGGCAUCAUCAUCAGCGACACCCCCGUGCACGACUGCAACACCACCUGCCAGACACCCGAGGGCG
CCAUCAACACCAGCCUGCCCUUCCAGAACGUGCACCCCAUCACCAUCGGCAAGUGCCCCAAAUACG
UGAAGUCCACCAAGCUGAGGCUGGCCACAGGCCUGAGAAACGUGCCCAGCAUCCAGAGCAGAGGC
CUGUUCGGAGCCAUCGCCGGCUUCAUCGAAGGCGGCUGGACAGGCAUGGUGGACGGAUGGUAC
GGAUACCACCACCAGAACGAGCAAGGCAGCGGCUACGCCGCCGACCUGAAGUCCACCCAGAACGCC
AUCGACAAGAUCACCAACAAAGUGAACAGCGUGAUCGAGAAGAUGAACACCCAGUUCACCGCCGU
GGGAAAAGAGUUCAACCACCUGGAAAAGCGCAUCGAGAACCUGAACAAGAAGGUGGACGACGGC
UUCCUGGACAUCUGGACCUACAACGCCGAGCUGCUCGUGCUGCUCGAGAACGAGAGAACCCUGG
ACUACCACGACAGCAACGUGAAGAACCUGUACGAGAAAGUGCGCAACCAGCUGAAGAACAACGCC
AAAGAGAUCGGCAACGGCUGCUUCGAGUUCUACCACAAGUGCGACAACACCUGCAUGGAAAGCG
UGAAGAACGGCACCUACGACUACCCCAAGUACAGCGAGGAAGCCAAACUGAACCGCGAGAAGAUC
GACGGCGUGAAGCUGGAAAGCACCCGGAUCUACCAGAUCCUGGCCAUCUACAGCACCGUGGCCAG
CAGCCUGGUGCUGGUGGUGAGCCUGGGCGCCAUCAGCUUCUGGAUGUGCAGCAACGGCAGCCU
GCAGUGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAUUCUG
CCUAAUAAAAAACAUUUAUUUUCAUUGCAAAAAAAAAAAAAAAAAAAAAAAAAAAAAAAAAAAAA
AAA

(SEQ ID NO: 42)

[Figure 80]

## mRNA sequence of A/Guangdong-Maonan/SWL1536/2019, polyA20

AGGAGACCCAAGCUACAUUUGCUUCUGACACAACUGUGUUCACUAGCAACCUCAAACAGACACCG
CCACCAUGAAGGCCAUCCUGGUGGUGCUGCUGUACACCUUCACCACCGCCAACGCCGACACACUG
UGCAUCGGCUACCACGCCAACAACAGCACCGACACCGUGGACACCGUGCUGGAAAAGAACGUGAC
CGUGACACACAGCGUGAACCUGCUGGAAGACAAGCACAACGGCAAGCUGUGCAAGCUGAGAGGC
GUGGCACCCCUGCACCUGGGCAAGUGCAACAUCGCCGGCUGGAUCCUGGGCAACCCCGAGUGCGA
AAGCCUGAGCACCGCCAGAAGCUGGAGCUACAUCGUGGAAACCAGCAACAGCGACAACGGCACAU
GCUACCCCGGCGACUUCAUCAACUACGAGGAACUGCGGGAACAGCUGAGCAGCGUGAGCAGCUU
CGAGAGAUUCGAGAUCUUCCCCAAGACCAGCAGCUGGCCCAACCACGACAGCGACAAAGGCGUGA
CAGCCGCCUGCCCCCACGCCGGCGCCAAGAGCUUCUACAAGAACCUGAUCUGGCUGGUCAAGAAG
GGCAACAGCUACCCCAAGCUGAACCAGACCUACAUCAACGACAAGGGCAAAGAGGUGCUGGUCCU
CUGGGGCAUCCACCACCCCCCAACAAUCGCCGCCCAAGAGAGCCUGUACCAGAACGCCGACGCCUA
CGUGUUCGUGGGCACCAGCAGAUACAGCAAGAAGUUCAAGCCCGAGAUCGCCACCAGGCCCAAAG
UGCGGGACCAAGAGGGCAGAAUGAACUACUACUGGACCCUGGUGGAACCCGGCGACAAGAUCAC
AUUCGAGGCCACCGGCAACCUGGUGGUCCCCAGAUACGCCUUCACCAUGGAAAGAGACGCCGGCA
GCGGCAUCAUCAUCAGCGACACCCCCGUGCACGACUGCAACACCACCUGCCAGACACCCGAGGGCG
CCAUCAACACCAGCCUGCCCUUCCAGAACGUGCACCCCAUCACCAUCGGCAAGUGCCCCAAAUACG
UGAAGUCCACCAAGCUGAGGCUGGCCACAGGCCUGAGAAACGUGCCCAGCAUCCAGAGCAGAGGC
CUGUUCGGAGCCAUCGCCGGCUUCAUCGAAGGCGGCUGGACAGGCAUGGUGGACGGAUGGUAC
GGAUACCACCACCAGAACGAGCAAGGCAGCGGCUACGCCGCCGACCUGAAGUCCACCCAGAACGCC
AUCGACAAGAUCACCAACAAAGUGAACAGCGUGAUCGAGAAGAUGAACACCCAGUUCACCGCCGU
GGGAAAAGAGUUCAACCACCUGGAAAAGCGCAUCGAGAACCUGAACAAGAAGGUGGACGACGGC
UUCCUGGACAUCUGGACCUACAACGCCGAGCUGCUCGUGCUGCUCGAGAACGAGAGAACCCUGG
ACUACCACGACAGCAACGUGAAGAACCUGUACGAGAAAGUGCGCAACCAGCUGAAGAACAACGCC
AAAGAGAUCGGCAACGGCUGCUUCGAGUUCUACCACAAGUGCGACAACACCUGCAUGGAAAGCG
UGAAGAACGGCACCUACGACUACCCCAAGUACAGCGAGGAAGCCAAACUGAACCGCGAGAAGAUC
GACGGCGUGAAGCUGGAAAGCACCCGGAUCUACCAGAUCCUGGCCAUCUACAGCACCGUGGCCAG
CAGCCUGGUGCUGGUGGUGAGCCUGGGCGCCAUCAGCUUCUGGAUGUGCAGCAACGGCAGCCU
GCAGUGCCGGAUCUGCAUCUGAGCUCGCUUUCUUGCUGUCCAAUUUCUAUUAAAGGUUCCUUU
GUUCCCUAAGUCCAACUACUAAACUGGGGGAUAUUAUGAAGGGCCUUGAGCAUCUGGAUUCUG
CCUAAUAAAAAACAUUUAUUUUCAUUGCAAAAAAAAAAAAAAAAAAAA

(SEQ ID NO: 43)

[Figure 81]

## mRNA sequence of B/Phuket/3073/2013 (Yamagata lineage), polyA110

AGGAGACCCAAGCUACAUUUGCUUCUGACACAACUGUGUUCACUAGCAACCUCAAACAGACACCG
CCACCAUGAAGGCCAUCAUCGUGCUGCUGAUGGUGGUCACCAGCAACGCCGACAGAAUCUGCACC
GGCAUCACCAGCAGCAACAGCCCCCACGUGGUCAAGACAGCCACACAGGGCGAAGUGAACGUGAC
CGGCGUGAUCCCCCUGACCACCACACCCACCAAGAGCUACUUCGCCAACCUGAAGGGCACCAGAAC
CAGAGGCAAGCUGUGCCCCGACUGCCUGAACUGCACCGACCUGGACGUGGCCCUGGGCAGACCCA
UGUGCGUGGGAACAACACCCAGCGCCAAGGCCAGCAUCCUGCACGAAGUGCGGCCCGUGACCAGC
GGCUGCUUCCCCAUCAUGCACGACCGGACCAAGAUCAGACAGCUGCCCAACCUGCUGCGGGGCUA
CGAGAAGAUCAGGCUGAGCACCCAGAACGUGAUCGACGCCGAAAAGCCCCCGGCGGCCCCUACA
GACUGGGCACAAGCGGCAGCUGCCCCAACGCCACAAGCAAGAUCGGCUUCUUCGCCACCAUGGCC
UGGGCCGUGCCCAAGGACAACUACAAGAACGCCACCAACCCCCUGACCGUGGAAGUGCCCUACAU
CUGCACCGAAGGCGAGGACCAGAUCACCGUGUGGGGCUUCCACAGCGACAACAAGACCCAGAUGA
AGUCCCUGUACGGCGACAGCAACCCCCAGAAGUUCACCAGCAGCGCCAACGGCGUGACCACACACU
ACGUGAGCCAGAUCGGCGACUUCCCCGACCAGACAGAAGACGGCGGACUGCCCCAGAGCGGCAGA
AUCGUGGUGGACUACAUGAUGCAGAAGCCCGGCAAGACCGGCACCAUCGUGUACCAGAGAGGCG
UCCUGCUGCCACAGAAAGUGUGGUGCGCCAGCGGCCGGAGCAAAGUGAUCAAAGGAAGCCUGCC
CCUGAUCGGCGAGGCCGACUGCCUGCACGAAGAAUACGGCGGCCUGAACAAGAGCAAGCCCUACU
ACACAGGCAAGCACGCCAAAGCCAUCGGCAACUGCCCCAUCUGGGUCAAGACCCCCCUGAAGCUG
GCCAACGGCACCAAGUACAGACCCCCAGCCAAGCUGCUGAAAGAGCGGGGCUUCUUCGGAGCCAU
CGCCGGCUUCCUGGAAGGCGGCUGGGAGGGAAUGAUCGCCGGCUGGCACGGCUACACAAGCCAC
GGCGCACACGGCGUGGCAGUGGCCGCCGACCUGAAGUCCACACAAGAGGCCAUCAACAAGAUCAC
CAAGAACCUGAACAGCCUGAGCGAGCUGGAAGUGAAGAACCUGCAGAGACUGAGCGGCGCCAUG
GACGAGCUGCACAACGAGAUCCUGGAACUGGACGAGAAGGUGGACGACCUGAGAGCCGACACCA
UCAGCAGCCAGAUCGAGCUGGCAGUGCUGCUGAGCAACGAGGGCAUCAUCAACAGCGAGGACGA
GCACCUGCUGGCCCUGGAACGGAAGCUGAAGAAGAUGCUGGGACCCAGCGCCGUGGACAUCGGC
AACGGCUGCUUCGAGACAAAGCACAAGUGCAACCAGACCUGCCUGGACAGAAUCGCCGCCGGAAC
CUUCAACGCCGGCGAGUUCAGCCUGCCCACCUUCGACAGCCUGAACAUCACAGCCGCCAGCCUGA
ACGACGACGGCCUGGACAACCACACCAUCCUGCUGUACUACAGCACCGCCGCCAGCAGCCUGGCCG
UGACACUGAUGCUGGCCAUCUUCAUCGUGUACAUGGUGAGCAGAGACAACGUGAGCUGCAGCAU
CUGCCUGUGAGCUCGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUCUGCCUAAUAAAAAAC
AUUUAUUUCAUUGCAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAAAAAAAAAAA
(SEQ ID NO: 44)

[Figure 82]

## mRNA sequence of B/Phuket/3073/2013 (Yamagata lineage), polyA95

AGGAGACCCAAGCUACAUUUGCUUCUGACACAACUGUGUUCACUAGCAACCUCAAACAGACACCG
CCACCAUGAAGGCCAUCAUCGUGCUGCUGAUGGUGGUCACCAGCAACGCCGACAGAAUCUGCACC
GGCAUCACCAGCAGCAACAGCCCCCACGUGGUCAAGACAGCCACACAGGGCGAAGUGAACGUGAC
CGGCGUGAUCCCCCUGACCACCACACCCACCAAGAGCUACUUCGCCAACCUGAAGGGCACCAGAAC
CAGAGGCAAGCUGUGCCCCGACUGCCUGAACUGCACCGACCUGGACGUGGCCCUGGGCAGACCCA
UGUGCGUGGGAACAACACCCAGCGCCAAGGCCAGCAUCCUGCACGAAGUGCGGCCCGUGACCAGC
GGCUGCUUCCCCAUCAUGCACGACCGGACCAAGAUCAGACAGCUGCCCAACCUGCUGCGGGGCUA
CGAGAAGAUCAGGCUGAGCACCCAGAACGUGAUCGACGCCGAAAAGCCCCCGGCGGCCCCUACA
GACUGGGCACAAGCGGCAGCUGCCCCAACGCCACAAGCAAGAUCGGCUUCUUCGCCACCAUGGCC
UGGGCCGUGCCCAAGGACAACUACAAGAACGCCACCAACCCCCUGACCGUGGAAGUGCCCUACAU
CUGCACCGAAGGCGAGGACCAGAUCACCGUGUGGGGCUUCCACAGCGACAACAAGACCCAGAUGA
AGUCCCUGUACGGCGACAGCAACCCCCAGAAGUUCACCAGCAGCGCCAACGGCGUGACCACACACU
ACGUGAGCCAGAUCGGCGACUUCCCCGACCAGACAGAAGACGGCGGACUGCCCCAGAGCGGCAGA
AUCGUGGUGGACUACAUGAUGCAGAAGCCCGGCAAGACCGGCACCAUCGUGUACCAGAGAGGCG
UCCUGCUGCCACAGAAAGUGUGGUGCGCCAGCGGCCGGAGCAAAGUGAUCAAAGGAAGCCUGCC
CCUGAUCGGCGAGGCCGACUGCCUGCACGAAGAAUACGGCGGCCUGAACAAGAGCAAGCCCUACU
ACACAGGCAAGCACGCCAAAGCCAUCGGCAACUGCCCCAUCUGGGUCAAGACCCCCCUGAAGCUG
GCCAACGGCACCAAGUACAGACCCCCAGCCAAGCUGCUGAAAGAGCGGGGCUUCUUCGGAGCCAU
CGCCGGCUUCCUGGAAGGCGGCUGGGAGGGAAUGAUCGCCGGCUGGCACGGCUACACAAGCCAC
GGCGCACACGGCGUGGCAGUGGCCGCCGACCUGAAGUCCACACAAGAGGCCAUCAACAAGAUCAC
CAAGAACCUGAACAGCCUGAGCGAGCUGGAAGUGAAGAACCUGCAGAGACUGAGCGGCGCCAUG
GACGAGCUGCACAACGAGAUCCUGGAACUGGACGAGAAGGUGGACGACCUGAGAGCCGACACCA
UCAGCAGCCAGAUCGAGCUGGCAGUGCUGCUGAGCAACGAGGGCAUCAUCAACAGCGAGGACGA
GCACCUGCUGGCCCUGGAACGGAAGCUGAAGAAGAUGCUGGGACCCAGCGCCGUGGACAUCGGC
AACGGCUGCUUCGAGACAAAGCACAAGUGCAACCAGACCUGCCUGGACAGAAUCGCCGCCGGAAC
CUUCAACGCCGGCGAGUUCAGCCUGCCCACCUUCGACAGCCUGAACAUCACAGCCGCCAGCCUGA
ACGACGACGGCCUGGACAACCACACCAUCCUGCUGUACUACAGCACCGCCGCCAGCAGCCUGGCCG
UGACACUGAUGCUGGCCAUCUUCAUCGUGUACAUGGUGAGCAGAGACAACGUGAGCUGCAGCAU
CUGCCUGUGAGCUCGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUCUGCCUAAUAAAAAAC
AUUUAUUUCAUUGCAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAA

(SEQ ID NO: 45)

[Figure 83]

mRNA sequence of B/Phuket/3073/2013 (Yamagata lineage), polyA80

AGGAGACCCAAGCUACAUUUGCUUCUGACACAACUGUGUUCACUAGCAACCUCAAACAGACACCG
CCACCAUGAAGGCCAUCAUCGUGCUGCUGAUGGUGGUCACCAGCAACGCCGACAGAAUCUGCACC
GGCAUCACCAGCAGCAACAGCCCCCACGUGGUCAAGACAGCCACACAGGGCGAAGUGAACGUGAC
CGGCGUGAUCCCCCUGACCACCACACCCACCAAGAGCUACUUCGCCAACCUGAAGGGCACCAGAAC
CAGAGGCAAGCUGUGCCCCGACUGCCUGAACUGCACCGACCUGGACGUGGCCCUGGGCAGACCCA
UGUGCGUGGGAACAACACCCAGCGCCAAGGCCAGCAUCCUGCACGAAGUGCGGCCCGUGACCAGC
GGCUGCUUCCCCAUCAUGCACGACCGGACCAAGAUCAGACAGCUGCCCAACCUGCUGCGGGGCUA
CGAGAAGAUCAGGCUGAGCACCCAGAACGUGAUCGACGCCGAAAAGCCCCCGGCGGCCCCUACA
GACUGGGCACAAGCGGCAGCUGCCCCAACGCCACAAGCAAGAUCGGCUUCUUCGCCACCAUGGCC
UGGGCCGUGCCCAAGGACAACUACAAGAACGCCACCAACCCCCUGACCGUGGAAGUGCCCUACAU
CUGCACCGAAGGCGAGGACCAGAUCACCGUGUGGGGCUUCCACAGCGACAACAAGACCCAGAUGA
AGUCCCUGUACGGCGACAGCAACCCCCAGAAGUUCACCAGCAGCGCCAACGGCGUGACCACACACU
ACGUGAGCCAGAUCGGCGACUUCCCCGACCAGACAGAAGACGGCGGACUGCCCCAGAGCGGCAGA
AUCGUGGUGGACUACAUGAUGCAGAAGCCCGGCAAGACCGGCACCAUCGUGUACCAGAGAGGCG
UCCUGCUGCCACAGAAAGUGUGGUGCGCCAGCGGCCGGAGCAAAGUGAUCAAAGGAAGCCUGCC
CCUGAUCGGCGAGGCCGACUGCCUGCACGAAGAAUACGGCGGCCUGAACAAGAGCAAGCCCUACU
ACACAGGCAAGCACGCCAAAGCCAUCGGCAACUGCCCCAUCUGGGUCAAGACCCCCCUGAAGCUG
GCCAACGGCACCAAGUACAGACCCCCAGCCAAGCUGCUGAAAGAGCGGGGCUUCUUCGGAGCCAU
CGCCGGCUUCCUGGAAGGCGGCUGGGAGGGAAUGAUCGCCGGCUGGCACGGCUACACAAGCCAC
GGCGCACACGGCGUGGCAGUGGCCGCCGACCUGAAGUCCACACAAGAGGCCAUCAACAAGAUCAC
CAAGAACCUGAACAGCCUGAGCGAGCUGGAAGUGAAGAACCUGCAGAGACUGAGCGGCGCCAUG
GACGAGCUGCACAACGAGAUCCUGGAACUGGACGAGAAGGUGGACGACCUGAGAGCCGACACCA
UCAGCAGCCAGAUCGAGCUGGCAGUGCUGCUGAGCAACGAGGGCAUCAUCAACAGCGAGGACGA
GCACCUGCUGGCCCUGGAACGGAAGCUGAAGAAGAUGCUGGGACCCAGCGCCGUGGACAUCGGC
AACGGCUGCUUCGAGACAAAGCACAAGUGCAACCAGACCUGCCUGGACAGAAUCGCCGCCGGAAC
CUUCAACGCCGGCGAGUUCAGCCUGCCCACCUUCGACAGCCUGAACAUCACAGCCGCCAGCCUGA
ACGACGACGGCCUGGACAACCACACCAUCCUGCUGUACUACAGCACCGCCGCCAGCAGCCUGGCCG
UGACACUGAUGCUGGCCAUCUUCAUCGUGUACAUGGUGAGCAGAGACAACGUGAGCUGCAGCAU
CUGCCUGUGAGCUCGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUCUGCCUAAUAAAAAAC
AUUUAUUUCAUUGCAAAAAAAAAAAAAAAAAAAAAAAAAAAAAAAAAAAAAAAAAAAAAAAAAAA
AAAAAAAAAAAAAAAAAAAAAAAAAAAAA

(SEQ ID NO: 46)

[Figure 84]

## mRNA sequence of B/Phuket/3073/2013 (Yamagata lineage), polyA60

AGGAGACCCAAGCUACAUUUGCUUCUGACACAACUGUGUUCACUAGCAACCUCAAACAGACACCG
CCACCAUGAAGGCCAUCAUCGUGCUGCUGAUGGUGGUCACCAGCAACGCCGACAGAAUCUGCACC
GGCAUCACCAGCAGCAACAGCCCCCACGUGGUCAAGACAGCCACACAGGGCGAAGUGAACGUGAC
CGGCGUGAUCCCCCUGACCACCACACCCACCAAGAGCUACUUCGCCAACCUGAAGGGCACCAGAAC
CAGAGGCAAGCUGUGCCCCGACUGCCUGAACUGCACCGACCUGGACGUGGCCCUGGGCAGACCCA
UGUGCGUGGGAACAACACCCAGCGCCAAGGCCAGCAUCCUGCACGAAGUGCGGCCCGUGACCAGC
GGCUGCUUCCCCAUCAUGCACGACCGGACCAAGAUCAGACAGCUGCCCAACCUGCUGCGGGGCUA
CGAGAAGAUCAGGCUGAGCACCCAGAACGUGAUCGACGCCGAAAAGCCCCCGGCGGCCCCUACA
GACUGGGCACAAGCGGCAGCUGCCCCAACGCCACAAGCAAGAUCGGCUUCUUCGCCACCAUGGCC
UGGGCCGUGCCCAAGGACAACUACAAGAACGCCACCAACCCCCUGACCGUGGAAGUGCCCUACAU
CUGCACCGAAGGCGAGGACCAGAUCACCGUGUGGGGCUUCCACAGCGACAACAAGACCCAGAUGA
AGUCCCUGUACGGCGACAGCAACCCCCAGAAGUUCACCAGCAGCGCCAACGGCGUGACCACACACU
ACGUGAGCCAGAUCGGCGACUUCCCCGACCAGACAGAAGACGGCGGACUGCCCCAGAGCGGCAGA
AUCGUGGUGGACUACAUGAUGCAGAAGCCCGGCAAGACCGGCACCAUCGUGUACCAGAGAGGCG
UCCUGCUGCCACAGAAAGUGUGGUGCGCCAGCGGCCGGAGCAAAGUGAUCAAAGGAAGCCUGCC
CCUGAUCGGCGAGGCCGACUGCCUGCACGAAGAAUACGGCGGCCUGAACAAGAGCAAGCCCUACU
ACACAGGCAAGCACGCCAAAGCCAUCGGCAACUGCCCCAUCUGGGUCAAGACCCCCCUGAAGCUG
GCCAACGGCACCAAGUACAGACCCCCAGCCAAGCUGCUGAAAGAGCGGGGCUUCUUCGGAGCCAU
CGCCGGCUUCCUGGAAGGCGGCUGGGAGGGAAUGAUCGCCGGCUGGCACGGCUACACAAGCCAC
GGCGCACACGGCGUGGCAGUGGCCGCCGACCUGAAGUCCACACAAGAGGCCAUCAACAAGAUCAC
CAAGAACCUGAACAGCCUGAGCGAGCUGGAAGUGAAGAACCUGCAGAGACUGAGCGGCGCCAUG
GACGAGCUGCACAACGAGAUCCUGGAACUGGACGAGAAGGUGGACGACCUGAGAGCCGACACCA
UCAGCAGCCAGAUCGAGCUGGCAGUGCUGCUGAGCAACGAGGGCAUCAUCAACAGCGAGGACGA
GCACCUGCUGGCCCUGGAACGGAAGCUGAAGAAGAUGCUGGGACCCAGCGCCGUGGACAUCGGC
AACGGCUGCUUCGAGACAAAGCACAAGUGCAACCAGACCUGCCUGGACAGAAUCGCCGCCGGAAC
CUUCAACGCCGGCGAGUUCAGCCUGCCCACCUUCGACAGCCUGAACAUCACAGCCGCCAGCCUGA
ACGACGACGGCCUGGACAACCACACCAUCCUGCUGUACUACAGCACCGCCGCCAGCAGCCUGGCCG
UGACACUGAUGCUGGCCAUCUUCAUCGUGUACAUGGUGAGCAGAGACAACGUGAGCUGCAGCAU
CUGCCUGUGAGCUCGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUCUGCCUAAUAAAAAAC
AUUUAUUUCAUUGCAAAAAAAAAAAAAAAAAAAAAAAAAAAAAAAAAAAAAAAAAAAAAAAAA
AAAAAAAAAAA
(SEQ ID NO: 47)

[Figure 85]

## mRNA sequence of B/Phuket/3073/2013 (Yamagata lineage), polyA40

AGGAGACCCAAGCUACAUUUGCUUCUGACACAACUGUGUUCACUAGCAACCUCAAACAGACACCG
CCACCAUGAAGGCCAUCAUCGUGCUGCUGAUGGUGGUCACCAGCAACGCCGACAGAAUCUGCACC
GGCAUCACCAGCAGCAACAGCCCCCACGUGGUCAAGACAGCCACACAGGGCGAAGUGAACGUGAC
CGGCGUGAUCCCCCUGACCACCACACCCACCAAGAGCUACUUCGCCAACCUGAAGGGCACCAGAAC
CAGAGGCAAGCUGUGCCCCGACUGCCUGAACUGCACCGACCUGGACGUGGCCCUGGGCAGACCCA
UGUGCGUGGGAACAACACCCAGCGCCAAGGCCAGCAUCCUGCACGAAGUGCGGCCCGUGACCAGC
GGCUGCUUCCCCAUCAUGCACGACCGGACCAAGAUCAGACAGCUGCCCAACCUGCUGCGGGGCUA
CGAGAAGAUCAGGCUGAGCACCCAGAACGUGAUCGACGCCGAAAAGCCCCCGGCGGCCCCUACA
GACUGGGCACAAGCGGCAGCUGCCCCAACGCCACAAGCAAGAUCGGCUUCUUCGCCACCAUGGCC
UGGGCCGUGCCCAAGGACAACUACAAGAACGCCACCAACCCCCUGACCGUGGAAGUGCCCUACAU
CUGCACCGAAGGCGAGGACCAGAUCACCGUGUGGGGCUUCCACAGCGACAACAAGACCCAGAUGA
AGUCCCUGUACGGCGACAGCAACCCCCAGAAGUUCACCAGCAGCGCCAACGGCGUGACCACACACU
ACGUGAGCCAGAUCGGCGACUUCCCCGACCAGACAGAAGACGGCGGACUGCCCCAGAGCGGCAGA
AUCGUGGUGGACUACAUGAUGCAGAAGCCCGGCAAGACCGGCACCAUCGUGUACCAGAGAGGCG
UCCUGCUGCCACAGAAAGUGUGGUGCGCCAGCGGCCGGAGCAAAGUGAUCAAAGGAAGCCUGCC
CCUGAUCGGCGAGGCCGACUGCCUGCACGAAGAAUACGGCGGCCUGAACAAGAGCAAGCCCUACU
ACACAGGCAAGCACGCCAAAGCCAUCGGCAACUGCCCCAUCUGGGUCAAGACCCCCCUGAAGCUG
GCCAACGGCACCAAGUACAGACCCCCAGCCAAGCUGCUGAAAGAGCGGGGCUUCUUCGGAGCCAU
CGCCGGCUUCCUGGAAGGCGGCUGGGAGGGAAUGAUCGCCGGCUGGCACGGCUACACAAGCCAC
GGCGCACACGGCGUGGCAGUGGCCGCCGACCUGAAGUCCACACAAGAGGCCAUCAACAAGAUCAC
CAAGAACCUGAACAGCCUGAGCGAGCUGGAAGUGAAGAACCUGCAGAGACUGAGCGGCGCCAUG
GACGAGCUGCACAACGAGAUCCUGGAACUGGACGAGAAGGUGGACGACCUGAGAGCCGACACCA
UCAGCAGCCAGAUCGAGCUGGCAGUGCUGCUGAGCAACGAGGGCAUCAUCAACAGCGAGGACGA
GCACCUGCUGGCCCUGGAACGGAAGCUGAAGAAGAUGCUGGGACCCAGCGCCGUGGACAUCGGC
AACGGCUGCUUCGAGACAAAGCACAAGUGCAACCAGACCUGCCUGGACAGAAUCGCCGCCGGAAC
CUUCAACGCCGGCGAGUUCAGCCUGCCCACCUUCGACAGCCUGAACAUCACAGCCGCCAGCCUGA
ACGACGACGGCCUGGACAACCACACCAUCCUGCUGUACUACAGCACCGCCGCCAGCAGCCUGGCCG
UGACACUGAUGCUGGCCAUCUUCAUCGUGUACAUGGUGAGCAGAGACAACGUGAGCUGCAGCAU
CUGCCUGUGAGCUCGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUCUGCCUAAUAAAAAAC
AUUUAUUUCAUUGCAAAAAAAAAAAAAAAAAAAAAAAAAAAAAAAAAAAAAAAA

(SEQ ID NO: 48)

[Figure 86]

## mRNA sequence of B/Phuket/3073/2013 (Yamagata lineage), polyA20

AGGAGACCCAAGCUACAUUUGCUUCUGACACAACUGUGUUCACUAGCAACCUCAAACAGACACCG
CCACCAUGAAGGCCAUCAUCGUGCUGCUGAUGGUGGUCACCAGCAACGCCGACAGAAUCUGCACC
GGCAUCACCAGCAGCAACAGCCCCCACGUGGUCAAGACAGCCACACAGGGCGAAGUGAACGUGAC
CGGCGUGAUCCCCCUGACCACCACACCCACCAAGAGCUACUUCGCCAACCUGAAGGGCACCAGAAC
CAGAGGCAAGCUGUGCCCCGACUGCCUGAACUGCACCGACCUGGACGUGGCCCUGGGCAGACCCA
UGUGCGUGGGAACAACACCCAGCGCCAAGGCCAGCAUCCUGCACGAAGUGCGGCCCGUGACCAGC
GGCUGCUUCCCCAUCAUGCACGACCGGACCAAGAUCAGACAGCUGCCCAACCUGCUGCGGGGCUA
CGAGAAGAUCAGGCUGAGCACCCAGAACGUGAUCGACGCCGAAAAGCCCCCGGCGGCCCCUACA
GACUGGGCACAAGCGGCAGCUGCCCCAACGCCACAAGCAAGAUCGGCUUCUUCGCCACCAUGGCC
UGGGCCGUGCCCAAGGACAACUACAAGAACGCCACCAACCCCCUGACCGUGGAAGUGCCCUACAU
CUGCACCGAAGGCGAGGACCAGAUCACCGUGUGGGGCUUCCACAGCGACAACAAGACCCAGAUGA
AGUCCCUGUACGGCGACAGCAACCCCCAGAAGUUCACCAGCAGCGCCAACGGCGUGACCACACACU
ACGUGAGCCAGAUCGGCGACUUCCCCGACCAGACAGAAGACGGCGGACUGCCCCAGAGCGGCAGA
AUCGUGGUGGACUACAUGAUGCAGAAGCCCGGCAAGACCGGCACCAUCGUGUACCAGAGAGGCG
UCCUGCUGCCACAGAAAGUGUGGUGCGCCAGCGGCCGGAGCAAAGUGAUCAAAGGAAGCCUGCC
CCUGAUCGGCGAGGCCGACUGCCUGCACGAAGAAUACGGCGGCCUGAACAAGAGCAAGCCCUACU
ACACAGGCAAGCACGCCAAAGCCAUCGGCAACUGCCCCAUCUGGGUCAAGACCCCCCUGAAGCUG
GCCAACGGCACCAAGUACAGACCCCCAGCCAAGCUGCUGAAAGAGCGGGGCUUCUUCGGAGCCAU
CGCCGGCUUCCUGGAAGGCGGCUGGGAGGGAAUGAUCGCCGGCUGGCACGGCUACACAAGCCAC
GGCGCACACGGCGUGGCAGUGGCCGCCGACCUGAAGUCCACACAAGAGGCCAUCAACAAGAUCAC
CAAGAACCUGAACAGCCUGAGCGAGCUGGAAGUGAAGAACCUGCAGAGACUGAGCGGCGCCAUG
GACGAGCUGCACAACGAGAUCCUGGAACUGGACGAGAAGGUGGACGACCUGAGAGCCGACACCA
UCAGCAGCCAGAUCGAGCUGGCAGUGCUGCUGAGCAACGAGGGCAUCAUCAACAGCGAGGACGA
GCACCUGCUGGCCCUGGAACGGAAGCUGAAGAAGAUGCUGGGACCCAGCGCCGUGGACAUCGGC
AACGGCUGCUUCGAGACAAAGCACAAGUGCAACCAGACCUGCCUGGACAGAAUCGCCGCCGGAAC
CUUCAACGCCGGCGAGUUCAGCCUGCCCACCUUCGACAGCCUGAACAUCACAGCCGCCAGCCUGA
ACGACGACGGCCUGGACAACCACACCAUCCUGCUGUACUACAGCACCGCCGCCAGCAGCCUGGCCG
UGACACUGAUGCUGGCCAUCUUCAUCGUGUACAUGGUGAGCAGAGACAACGUGAGCUGCAGCAU
CUGCCUGUGAGCUCGCUUUCUUGCUGUCCAAUUUCUAUUAAAGGUUCCUUUGUUCCCUAAGUC
CAACUACUAAACUGGGGGAUAUUAUGAAGGGCCUUGAGCAUCUGGAUUCUGCCUAAUAAAAAAC
AUUUAUUUUCAUUGCAAAAAAAAAAAAAAAAAAAA

(SEQ ID NO: 49)

[Figure 87]

## Amino acid sequence of A/Guangdong-Maonan/SWL1536/2019 HA

MKAILVVLLYTFTTANADTLCIGYHANNSTDTVDTVLEKNVTVTHSVNLLEDKHNGKLCKLRGVAPLHLGKC
NIAGWILGNPECESLSTARSWSYIVETSNSDNGTCYPGDFINYEELREQLSSVSSFERFEIFPKTSSWPNHDSD
KGVTAACPHAGAKSFYKNLIWLVKKGNSYPKLNQTYINDKGKEVLVLWGIHHPPTIAAQESLYQNADAYVFV
GTSRYSKKFKPEIATRPKVRDQEGRMNYYWTLVEPGDKITFEATGNLVVPRYAFTMERDAGSGIIISDTPVH
DCNTTCQTPEGAINTSLPFQNVHPITIGKCPKYVKSTKLRLATGLRNVPSIQSRGLFGAIAGFIEGGWTGMV
DGWYGYHHQNEQGSGYAADLKSTQNAIDKITNKVNSVIEKMNTQFTAVGKEFNHLEKRIENLNKKVDDGF
LDIWTYNAELLVLLENERTLDYHDSNVKNLYEKVRNQLKNNAKEIGNGCFEFYHKCDNTCMESVKNGTYDY
PKYSEEAKLNREKIDGVKLESTRIYQILAIYSTVASSLVLVVSLGAISFWMCSNGSLQCRICI

(SEQ ID NO: 50)

[Figure 88]

## DNA fragment containing A/Astrakhan/3212/2020 (H5 subtype) HA

GCTAGCGTAATACGACTCACTATAAGGAGACCCAAGCTACATTTGCTTCTGACACAACTGTGTTCACTAG
CAACCTCAAACAGACACCGCCACCATGGAGAACATAGTACTTCTTCTTGCAATAGTTAGCCTTGTTAAAA
GTGATCAGATTTGCATTGGTTATCATGCAAACAATTCGACAGAGCAAGTTGACACGATAATGGAAAAGA
ACGTCACTGTTACACATGCCCAAGACATACTGGAAAAACACACAACGGGAAGCTCTGTGATCTAAATG
GGGTGAAGCCTCTGATTTTAAAGGATTGTAGTGTAGCTGGATGGCTCCTCGGAAACCCAATGTGCGACG
AATTCATCAGAGTGCCGGAATGGTCCTACATAGTGGAGAGGGCTAATCCAGCTAATGACCTCTGCTACCC
AGGGAGCCTCAATGACTATGAAGAACTGAAACACCTGTTGAGCAGAATAAATCATTTTGAGAAGATTCT
GATTATCCCCAAGAGTTCCTGGCCAAACCATGAAACATCACTAGGGGTGAGCGCAGCTTGTCCATACCA
GGGAGCGCCCTCCTTTTTCAGAAATGTGGTGTGGCTTATCAAAAAGAACGATGCATACCCAACGATAAA
GATAAGCTACAATAATACCAATCGGGAAGATCTCTTGATACTGTGGGGGATTCATCATTCCAACAATGCA
GAAGAGCAGACAAATCTCTATAAAAACCCAACCACCTACATTTCAGTTGGAACATCAACTTTAAACCAGA
GGTTGGTACCAAAAATAGCTACTAGATCCCAAGTAAACGGGCAACGTGGAAGAATGGACTTCTTCTGGA
CAATTTTAAAACCGGATGATGCAATCCATTTCGAGAGTAATGGAAATTTCATTGCTCCAGAATATGCATAC
AAAATTGTCAAGAAAGGGGACTCAACAATTATGAAAAGTGGAGTGGAATATGGCCACTGCAACACCAA
ATGTCAAACCCCAGTAGGAGCGATAAATTCTAGTATGCCATTCCACAACATACATCCTCTCACCATTGGGG
AATGCCCCAAATACGTGAAGTCAAACAAGTTGGTCCTTGCGACTGGGCTCAGAAATAGTCCTCTAAGAG
AAAAGAGAAGAAAAAGAGGCCTGTTTGGGGCGATAGCAGGGTTTATAGAGGGAGGATGGCAGGGAA
TGGTTGATGGTTGGTATGGGTACCACCATAGCAATGAGCAGGGGAGTGGGTACGCTGCAGACAAAGAA
TCCACCCAAAAGGCAATAGATGGAGTTACCAATAAGGTCAACTCAATCATTGACAAAATGAACACTCAAT
TTGAGGCAGTTGGAAGGGAGTTTAATAACTTAGAAAGGAGGATAGAGAATTTGAACAAGAAAATGGA
AGACGGATTCCTAGATGTCTGGACCTATAATGCTGAACTTCTAGTTCTCATGGAAAACGAGAGGACTCTA
GATTTCCATGATTCAAATGTCAAGAACCTTTACGACAAAGTCAGACTACAGCTTAGGGATAATGCAAAG
GAGCTGGGTAACGGCTGTTTCGAATTCTACCACAAATGCGATAATGAATGTATGGAAAGTGTGAGAAAT
GGGACGTATGACTACCCTCAGTATTCAGAAGAAGCAAGATTAAAAAGAGAAGAAATAAGCGGAGTGAA
ATTAGAATCAATAGGAACTTACCAGATACTGTCAATTTATTCAACAGCGGCGAGTTCCCTAGCACTGGCA
ATCATGATGGCTGGTCTATCTTTATGGATGTGCTCCAATGGGTCGTTACAGTGCAGAATTTGCATTTGAGC
TCGCTTTCTTGCTGTCCAATTTCTATTAAAGGTTCCTTTGTTCCCTAAGTCCAACTACTAAACTGGGGGAT
ATTATGAAGGGCCTTGAGCATCTGGATTCTGCCTAATAAAAAACATTTATTTTCATTGC
(SEQ ID NO: 51)

[Figure 89]

## Template DNA of A/Astrakhan/3212/2020 (H5 subtype) HA

TAATACGACTCACTATAAGGAGACCCAAGCTACATTTGCTTCTGACACAACTGTGTTCACTAGCAACCTCA
AACAGACACCGCCACCATGGAGAACATAGTACTTCTTCTTGCAATAGTTAGCCTTGTTAAAAGTGATCAG
ATTTGCATTGGTTATCATGCAAACAATTCGACAGAGCAAGTTGACACGATAATGGAAAAGAACGTCACT
GTTACACATGCCCAAGACATACTGGAAAAAACACACAACGGGAAGCTCTGTGATCTAAATGGGGTGAA
GCCTCTGATTTTAAAGGATTGTAGTGTAGCTGGATGGCTCCTCGGAAACCCAATGTGCGACGAATTCATC
AGAGTGCCGGAATGGTCCTACATAGTGGAGAGGGCTAATCCAGCTAATGACCTCTGCTACCCAGGGAGC
CTCAATGACTATGAAGAACTGAAACACCTGTTGAGCAGAATAAATCATTTTGAGAAGATTCTGATTATCC
CCAAGAGTTCCTGGCCAAACCATGAAACATCACTAGGGGTGAGCGCAGCTTGTCCATACCAGGGAGCG
CCCTCCTTTTTCAGAAATGTGGTGTGGCTTATCAAAAAGAACGATGCATACCCAACGATAAAGATAAGCT
ACAATAATACCAATCGGGAAGATCTCTTGATACTGTGGGGGATTCATCATTCCAACAATGCAGAAGAGCA
GACAAATCTCTATAAAAACCCAACCACCTACATTTCAGTTGGAACATCAACTTTAAACCAGAGGTTGGTA
CCAAAAATAGCTACTAGATCCCAAGTAAACGGGCAACGTGGAAGAATGGACTTCTTCTGGACAATTTTA
AAACCGGATGATGCAATCCATTTCGAGAGTAATGGAAATTTCATTGCTCCAGAATATGCATACAAAATTGT
CAAGAAAGGGGACTCAACAATTATGAAAAGTGGAGTGGAATATGGCCACTGCAACACCAAATGTCAAA
CCCCAGTAGGAGCGATAAATTCTAGTATGCCATTCCACAACATACATCCTCTCACCATTGGGGAATGCCCC
AAATACGTGAAGTCAAACAAGTTGGTCCTTGCGACTGGGCTCAGAAATAGTCCTCTAAGAGAAAAGAG
AAGAAAAAGAGGCCTGTTTGGGGCGATAGCAGGGTTTATAGAGGGAGGATGGCAGGGAATGGTTGAT
GGTTGGTATGGGTACCACCATAGCAATGAGCAGGGGAGTGGGTACGCTGCAGACAAAGAATCCACCCA
AAAGGCAATAGATGGAGTTACCAATAAGGTCAACTCAATCATTGACAAAATGAACACTCAATTTGAGGC
AGTTGGAAGGGAGTTTAATAACTTAGAAAGGAGGATAGAGAATTTGAACAAGAAAATGGAAGACGGA
TTCCTAGATGTCTGGACCTATAATGCTGAACTTCTAGTTCTCATGGAAAACGAGAGGACTCTAGATTTCCA
TGATTCAAATGTCAAGAACCTTTACGACAAAGTCAGACTACAGCTTAGGGATAATGCAAAGGAGCTGGG
TAACGGCTGTTTCGAATTCTACCACAAATGCGATAATGAATGTATGGAAAGTGTGAGAAATGGGACGTAT
GACTACCCTCAGTATTCAGAAGAAGCAAGATTAAAAAGAGAAGAAATAAGCGGAGTGAAATTAGAATC
AATAGGAACTTACCAGATACTGTCAATTTATTCAACAGCGGCGAGTTCCCTAGCACTGGCAATCATGATG
GCTGGTCTATCTTTATGGATGTGCTCCAATGGGTCGTTACAGTGCAGAATTTGCATTTGAGCTCGCTTTCT
TGCTGTCCAATTTCTATTAAAGGTTCCTTTGTTCCCTAAGTCCAACTACTAAACTGGGGGATATTATGAAG
GGCCTTGAGCATCTGGATTCTGCCTAATAAAAACATTTATTTTCATTGCAAAAAAAAAAAAAAAAAAAA
AAAAAAAAAAAAAAAAAAAAAAAAAAAAAAAAAAAAAAAAAAAAAAAAAAAAAAAAAAAAAAAAA
AAAAAAAAAAAAAAA
(SEQ ID NO: 52)

T7 Promoter sequence (before transcription start point): base Nos. 1 to 17
A (transcription start point): base No. 18
5'-UTR sequence (including transcription start point and KOZAK sequence of base Nos. 82 to 87): base Nos. 18 to 87
Sequence of translated region of A/Astrakhan/3212/2020 (H5 subtype) HA: base Nos. 88 to 1791
3'-UTR sequence: base Nos. 1792 to 1923
polyA sequence (A100): base Nos. 1924 to 2023

[Figure 90]

## A/Astrakhan/3212/2020 (H5 subtype) HA mRNA-001

AGGAGACCCAAGCUACAUUUGCUUCUGACACAACUGUGUUCACUAGCAACCUCAAACAGAC
ACCGCCACCAUGGAGAACAUAGUACUUCUUCUUGCAAUAGUUAGCCUUGUUAAAAGUGAU
CAGAUUUGCAUUGGUUAUCAUGCAAACAAUUCGACAGAGCAAGUUGACACGAUAAUGGAA
AAGAACGUCACUGUUACACAUGCCCAAGACAUACUGGAAAAACACACAACGGGAAGCUCU
GUGAUCUAAAUGGGGUGAAGCCUCUGAUUUUAAAGGAUUGUAGUGUAGCUGGAUGGCU
CCUCGGAAACCCAAUGUGCGACGAAUUCAUCAGAGUGCCGGAAUGGUCCUACAUAGUGGA
GAGGGCUAAUCCAGCUAAUGACCUCUGCUACCCAGGGAGCCUCAAUGACUAUGAAGAACU
GAAACACCUGUUGAGCAGAAUAAAUCAUUUUGAGAAGAUUCUGAUUAUCCCCAAGAGUUC
CUGGCCAAACCAUGAAACAUCACUAGGGGUGAGCGCAGCUUGUCCAUACCAGGGAGCGCC
CUCCUUUUUCAGAAAUGUGGUGUGGCUUAUCAAAAAGAACGAUGCAUACCCAACGAUAAA
GAUAAGCUACAAUAAUACCAAUCGGGAAGAUCUCUUGAUACUGUGGGGGAUUCAUCAUU
CCAACAAUGCAGAAGAGCAGACAAAUCUCUAUAAAAACCCAACCACCUACAUUUCAGUUGG
AACAUCAACUUUAAACCAGAGGUUGGUACCAAAAAUAGCUACUAGAUCCCAAGUAAACGG
GCAACGUGGAAGAAUGGACUUCUUCUGGACAAUUUUAAAACCGGAUGAUGCAAUCCAUU
UCGAGAGUAAUGGAAAUUUCAUUGCUCCAGAAUAUGCAUACAAAAUUGUCAAGAAAGGG
GACUCAACAAUUAUGAAAAGUGGAGUGGAAUAUGGCCACUGCAACACCAAAUGUCAAACC
CCAGUAGGAGCGAUAAAUUCUAGUAUGCCAUUCCACAACAUACAUCCUCUCACCAUUGGG
GAAUGCCCCAAAUACGUGAAGUCAAACAAGUUGGUCCUUGCGACUGGGCUCAGAAAUAGU
CCUCUAAGAGAAAAGAGAAGAAAAAGAGGCCUGUUUGGGGCGAUAGCAGGGUUUAUAGA
GGGAGGAUGGCAGGGAAUGGUUGAUGGUUGGUAUGGGUACCACCAUAGCAAUGAGCAG
GGGAGUGGGUACGCUGCAGACAAAGAAUCCACCCAAAAGGCAAUAGAUGGAGUUACCAAU
AAGGUCAACUCAAUCAUUGACAAAAUGAACACUCAAUUUGAGGCAGUUGGAAGGGAGUU
UAAUAACUUAGAAAGGAGGAUAGAGAAUUUGAACAAGAAAAUGGAAGACGGAUUCCUAG
AUGUCUGGACCUAUAAUGCUGAACUUCUAGUUCUCAUGGAAAACGAGAGGACUCUAGAU
UUCCAUGAUUCAAAUGUCAAGAACCUUUACGACAAAGUCAGACUACAGCUUAGGGAUAAU
GCAAAGGAGCUGGGUAACGGCUGUUUCGAAUUCUACCACAAAUGCGAUAAUGAAUGUAU
GGAAAGUGUGAGAAAUGGGACGUAUGACUACCCUCAGUAUUCAGAAGAAGCAAGAUUAA
AAAGAGAAGAAAUAAGCGGAGUGAAAUUAGAAUCAAUAGGAACUUACCAGAUACUGUCAA
UUUAUUCAACAGCGGCGAGUUCCCUAGCACUGGCAAUCAUGAUGGCUGGUCUAUCUUUA
UGGAUGUGCUCCAAUGGGUCGUUACAGUGCAGAAUUUGCAUUUGAGCUCGCUUUCUUGC
UGUCCAAUUUCUAUUAAAGGUUCCUUUGUUCCCUAAGUCCAACUACUAAACUGGGGGAU
AUUAUGAAGGGCCUUGAGCAUCUGGAUUCUGCCUAAUAAAAACAUUUAUUUUCAUUGC
AAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAA

(SEQ ID NO: 53)

[Figure 91]

## Amino acid sequence of A/Astrakhan/3212/2020 (H5 subtype) HA

MENIVLLLAIVSLVKSDQICIGYHANNSTEQVDTIMEKNVTVTHAQDILEKTHNGKLCDLNGVKPLILK
DCSVAGWLLGNPMCDEFIRVPEWSYIVERANPANDLCYPGSLNDYEELKHLLSRINHFEKILIIPKSSW
PNHETSLGVSAACPYQGAPSFFRNVVWLIKKNDAYPTIKISYNNTNREDLLILWGIHHSNNAEEQTNL
YKNPTTYISVGTSTLNQRLVPKIATRSQVNGQRGRMDFFWTILKPDDAIHFESNGNFIAPEYAYKIVK
KGDSTIMKSGVEYGHCNTKCQTPVGAINSSMPFHNIHPLTIGECPKYVKSNKLVLATGLRNSPLREKR
RKRGLFGAIAGFIEGGWQGMVDGWYGYHHSNEQGSGYAADKESTQKAIDGVTNKVNSIIDKMNT
QFEAVGREFNNLERRIENLNKKMEDGFLDVWTYNAELLVLMENERTLDFHDSNVKNLYDKVRLQLR
DNAKELGNGCFEFYHKCDNECMESVRNGTYDYPQYSEEARLKREEISGVKLESIGTYQILSIYSTAASS
LALAIMMAGLSLWMCSNGSLQCRICI
(SEQ ID NO: 54)

[Figure 92]

## Sequence in which KOZAK sequence and A/Astrakhan/3212/2020 (H5N8) HA gene translation region are connected

GCCACCATGGAGAACATAGTACTTCTTCTTGCAATAGTTAGCCTTGTTAAAAGTGATCAGATTTGCATTG
GTTATCATGCAAACAATTCGACAGAGCAAGTTGACACGATAATGGAAAAGAACGTCACTGTTACACATG
CCCAAGACATACTGGAAAAAACACACAACGGGAAGCTCTGTGATCTAAATGGGGTGAAGCCTCTGATTT
TAAAGGATTGTAGTGTAGCTGGATGGCTCCTCGGAAACCCAATGTGCGACGAATTCATCAGAGTGCCGG
AATGGTCCTACATAGTGGAGAGGGCTAATCCAGCTAATGACCTCTGCTACCCAGGGAGCCTCAATGACTA
TGAAGAACTGAAACACCTGTTGAGCAGAATAAATCATTTTGAGAAGATTCTGATTATCCCCAAGAGTTCC
TGGCCAAACCATGAAACATCACTAGGGGTGAGCGCAGCTTGTCCATACCAGGGAGCGCCCTCCTTTTTC
AGAAATGTGGTGTGGCTTATCAAAAAGAACGATGCATACCCAACGATAAAGATAAGCTACAATAATACCA
ATCGGGAAGATCTCTTGATACTGTGGGGGATTCATCATTCCAACAATGCAGAAGAGCAGACAAATCTCTA
TAAAAACCCAACCACCTACATTTCAGTTGGAACATCAACTTTAAACCAGAGGTTGGTACCAAAAATAGCT
ACTAGATCCCAAGTAAACGGGCAACGTGGAAGAATGGACTTCTTCTGGACAATTTTAAAACCGGATGAT
GCAATCCATTTCGAGAGTAATGGAAATTTCATTGCTCCAGAATATGCATACAAAATTGTCAAGAAAGGGG
ACTCAACAATTATGAAAAGTGGAGTGGAATATGGCCACTGCAACACCAAATGTCAAACCCCAGTAGGAG
CGATAAATTCTAGTATGCCATTCCACAACATACATCCTCTCACCATTGGGGAATGCCCCAAATACGTGAAG
TCAAACAAGTTGGTCCTTGCGACTGGGCTCAGAAATAGTCCTCTAAGAGAAAAGAGAAGAAAAAGAG
GCCTGTTTGGGGCGATAGCAGGGTTTATAGAGGGAGGATGGCAGGGAATGGTTGATGGTTGGTATGG
GTACCACCATAGCAATGAGCAGGGGAGTGGGTACGCTGCAGACAAAGAATCCACCCAAAAGGCAATAG
ATGGAGTTACCAATAAGGTCAACTCAATCATTGACAAAATGAACACTCAATTTGAGGCAGTTGGAAGGG
AGTTTAATAACTTAGAAAGGAGGATAGAGAATTTGAACAAGAAAATGGAAGACGGATTCCTAGATGTCT
GGACCTATAATGCTGAACTTCTAGTTCTCATGGAAAACGAGAGGACTCTAGATTTCCATGATTCAAATGT
CAAGAACCTTTACGACAAAGTCAGACTACAGCTTAGGGATAATGCAAAGGAGCTGGGTAACGGCTGTT
TCGAATTCTACCACAAATGCGATAATGAATGTATGGAAAGTGTGAGAAATGGGACGTATGACTACCCTCA
GTATTCAGAAGAAGCAAGATTAAAAAGAGAAGAAATAAGCGGAGTGAAATTAGAATCAATAGGAACTT
ACCAGATACTGTCAATTTATTCAACAGCGGCGAGTTCCCTAGCACTGGCAATCATGATGGCTGGTCTATCT
TTATGGATGTGCTCCAATGGGTCGTTACAGTGCAGAATTTGCATTTAA
(SEQ ID NO: 55)

KOZAK sequence (1-6), translated region of A/Astrakhan/3212/2020 (H5N8) HA (7-1710)

[Figure 93]

Sequence in which KOZAK sequence and A/Laos/2121/2020 (H5N1) HA gene translation region are connected

GCCACCATGAAGAAAATAGTTCTTCTCTTTGCAACAATCAGCCTTGTCAAAAGCGATCATATTTGCATTG
GTTACCATGCAAATAATTCGACAGAGCAGGTTGACACAATAATGGAAAAAAACGTTACTGTTACACAAG
CCCAAGACATACTGGAAAAGACACACAACGGGAAGCTCTGCGATCTAAATGGAGTGAAGCCTCTGATT
TTAAAAGATTGTAGTGTAGCAGGATGGCTCCTCGGAAATCCACTGTGTGACGAATTCACCAATGTGCCA
GAATGGTCTTACATAGTAGAGAAGGCCAATCCAGCCAATGACCTCTGTTACCCAGGGAATTTCAATGATT
ATGAAGAATTGAAACACCTATTGAGCAGGATAAACCATTTTGAGAAAATACAGATCATCCCCAAAAACTC
TTGGTCAGATCATGAAGCCTCATTGGGGGTAAGCGCCGCGTGTTCATACCAGGGAAATTCCTCCTTCTTC
AGAAATGTGGTGTGGCTTATCAAAAAGGACAATGCATACCCAACAATAAAGAAAGGCTACAATAACACC
AATCGAGAAGATCTCCTGATACTGTGGGGGATCCACCATCCTAATGATGAGGCAGAGCAGACGAGGCTC
TACCAAAACCCAACTACCTATATTTCCATTGGAACTTCAACATTAAACCAGAGGTTGGTGCCAAGAATAG
CCACTAGACCCAAAATAAACGGGCAAAGTGGCAGGATAGATTTCTTCTGGACAATTTTAAAACCGAATG
ACGCAATCCACTTCGAGAGTAATGGAAATTTCATTGCTCCAGAATATGCATACAAAATTGTCAAGAAGGG
AGACTCCACAATCATGAGAAGTGAAGTAAAATATGGTAACTGCAACACCAGGTGTCAGACTCCAATAGG
AGCGATAAACTCTAGTATGCCATTCCACAACATACACCCTCTCACTATCGGAGAATGTCCCAAATATGTGA
AATCAAGCAAATTAGTCCTTGCAACCGGGCTCAGAAATAGTCCTCAAAAAGAGAGAAGAAGAAAAAGA
GGACTGTTTGGGGCTATAGCAGGCTTTATAGAGGGAGGTTGGCAAGGAATGGTAGATGGTTGGTATGG
GTACCACCACAGTAATGAACAGGGGAGTGGTTACGCTGCAGACAAAGAATCTACTCAAAAGGCGATAG
ACGGGGTCACCAATAAGGTCAATTCGATCATTGACAAAATGAACACTCAGTTTGAGGCTGTAGGAAGG
GAATTTAATAACTTAGAGAGGAGAATAGAAAACCTAAACAAGAAAATGGAAGATGGATTCCTAGATGTC
TGGACTTATAATGCTGAACTCCTGGTTCTCATGGAGAATGAGAGAACTCTAGACTTCCATGATTCAAATG
TCAAGAACCTTTATGATAAGGTCCGACTACAGCTCAAGGATAATGCAAAAGAACTGGGAAACGGTTGTT
TCGAGTTCTATCACAAATGTAATGATGAATGTATGGAAAGTGTAAGAAACGGGACGTATGACTACCCGCA
GTATTCAGAAGAAGCAAGATTAAAAAGAGAGGAAATAAGTGGAGTAAAATTGGAATCAATGGGAATCT
ATCAAATACTGTCAATTTATTCAACAGTGGCGAGTTCCCTAGTGCTGGCAATCATGATGGCTGGCCTATCT
TTATGGATGTGTTCCAACGGGTCGTTACAGTGCAGAATTTGCATTTAG
(SEQ ID NO: 56)

KOZAK sequence (1-6), translated region of A/Laos/2121/2020 (H5N1) HA (7-1710)

[Figure 94]

## Base sequence of translated region of A/Laos/2121/2020 (H5N1) HA

ATGAAGAAAATAGTTCTTCTCTTTGCAACAATCAGCCTTGTCAAAAGCGATCATATTTGCATTGGTTACCA
TGCAAATAATTCGACAGAGCAGGTTGACACAATAATGGAAAAAAACGTTACTGTTACACAAGCCCAAGA
CATACTGGAAAAGACACACAACGGGAAGCTCTGCGATCTAAATGGAGTGAAGCCTCTGATTTTAAAAG
ATTGTAGTGTAGCAGGATGGCTCCTCGGAAATCCACTGTGTGACGAATTCACCAATGTGCCAGAATGGT
CTTACATAGTAGAGAAGGCCAATCCAGCCAATGACCTCTGTTACCCAGGGAATTTCAATGATTATGAAGA
ATTGAAACACCTATTGAGCAGGATAAACCATTTTGAGAAAATACAGATCATCCCCAAAAACTCTTGGTCA
GATCATGAAGCCTCATTGGGGGTAAGCGCCGCGTGTTCATACCAGGGAAATTCCTCCTTCTTCAGAAAT
GTGGTGTGGCTTATCAAAAAGGACAATGCATACCCAACAATAAAGAAAGGCTACAATAACACCAATCGA
GAAGATCTCCTGATACTGTGGGGGATCCACCATCCTAATGATGAGGCAGAGCAGACGAGGCTCTACCAA
AACCCAACTACCTATATTTCCATTGGTTCAACATTAAACCAGAGGTTGGTGCCAAGAATAGCCACTAGAC
CCAAAATAAACGGGCAAAGTGGCAGGATAGATTTCTTCTGGACAATTTTAAAACCGAATGACGCAATCC
ACTTCGAGAGTAATGGAAATTTCATTGCTCCAGAATATGCATACAAAATTGTCAAGAAGGGAGACTCCAC
AATCATGAGAAGTGAAGTAAAATATGGTAACTGCAACACCAGGTGTCAGACTCCAATAGGAGCGATAAA
CTCTAGTATGCCATTCCACAACATACACCCTCTCACTATCGGAGAATGTCCCAAATATGTGAAATCAAGCA
AATTAGTCCTTGCAACCGGGCTCAGAAATAGTCCTCAAAAAGAGAGAAGAAGAAAAAGAGGACTGTTT
GGGGCTATAGCAGGCTTTATAGAGGGAGGTTGGCAAGGAATGGTAGATGGTTGGTATGGGTACCACCA
CAGTAATGAACAGGGGAGTGGTTACGCTGCAGACAAAGAATCTACTCAAAAGGCGATAGACGGGGTC
ACCAATAAGGTCAATTCGATCATTGACAAAATGAACACTCAGTTTGAGGCTGTAGGAAGGGAATTTAAT
AACTTAGAGAGGAGAATAGAAAACCTAAACAAGAAAATGGAAGATGGATTCCTAGATGTCTGGACTTAT
AATGCTGAACTCCTGGTTCTCATGGAGAATGAGAGAACTCTAGACTTCCATGATTCAAATGTCAAGAACC
TTTATGATAAGGTCCGACTACAGCTCAAGGATAATGCAAAAGAACTGGGAAACGGTTGTTTCGAGTTCT
ATCACAAATGTAATGATGAATGTATGGAAAGTGTAAGAAACGGGACGTATGACTACCCGCAGTATTCAGA
AGAAGCAAGATTAAAAAGAGAGGAAATAAGTGGAGTAAAATTGGAATCAATGGGAATCTATCAAATAC
TGTCAATTTATTCAACAGTGGCGAGTTCCCTAGTGCTGGCAATCATGATGGCTGGCCTATCTTTATGGATG
TGTTCCAACGGGTCGTTACAGTGCAGAATTTGCATTTAG
(SEQ ID NO: 57)

[Figure 95]

## Amino acid sequence of translated region of A/Laos/2121/2020 (H5N1) HA

MKKIVLLFATISLVKSDHICIGYHANNSTEQVDTIMEKNVTVTQAQDILEKTHNGKLCDLNGVKPLILKDCSVA
GWLLGNPLCDEFTNVPEWSYIVEKANPANDLCYPGNFNDYEELKHLLSRINHFEKIQIIPKNSWSDHEASLG
VSAACSYQGNSSFFRNVVWLIKKDNAYPTIKKGYNNTNREDLLILWGIHHPNDEAEQTRLYQNPTTYISIGTS
TLNQRLVPRIATRPKINGQSGRIDFFWTILKPNDAIHFESNGNFIAPEYAYKIVKKGDSTIMRSEVKYGNCNTR
CQTPIGAINSSMPFHNIHPLTIGECPKYVKSSKLVLATGLRNSPQKERRRKRGLFGAIAGFIEGGWQGMVDG
WYGYHHSNEQGSGYAADKESTQKAIDGVTNKVNSIIDKMNTQFEAVGREFNNLERRIENLNKKMEDGFLD
VWTYNAELLVLMENERTLDFHDSNVKNLYDKVRLQLKDNAKELGNGCFEFYHKCNDECMESVRNGTYDYP
QYSEEARLKREEISGVKLESMGIYQILSIYSTVASSLVLAIMMAGLSLWMCSNGSLQCRICI
(SEQ ID NO: 58)

[Figure 96]

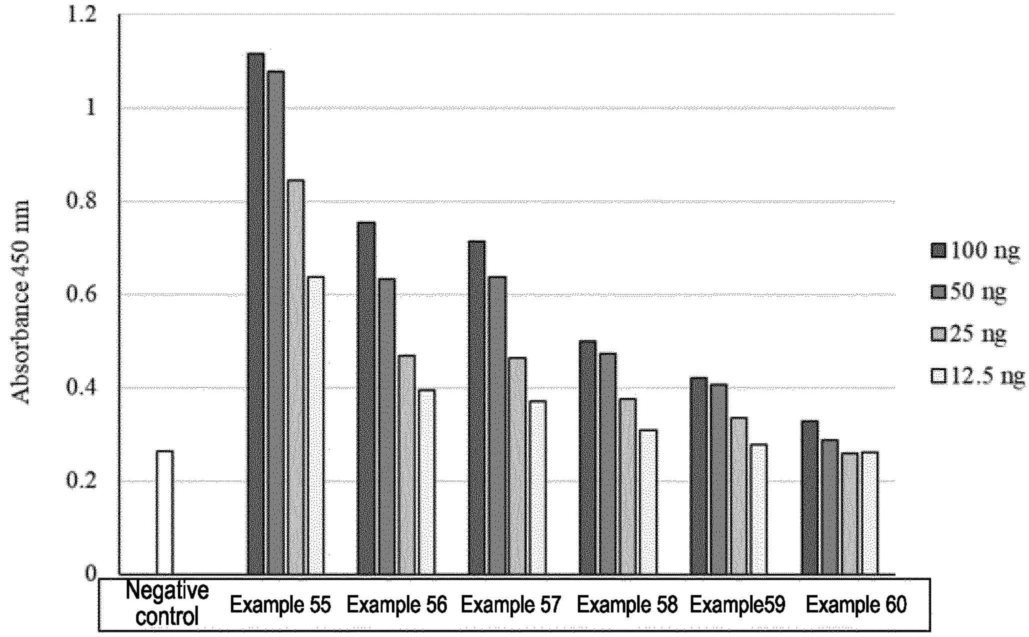


[Figure 97]

| pseudovirus | Neutralizing titers of mouse serum against LNP-mRNA-Astrakhan/3212/2020 HA(H5) |
|---|---|
| Astrakhan/3212/2020(H5) | **1280** |
| Laos/2121/2020(H5) | **<80** |

## INTERNATIONAL SEARCH REPORT

International application No. **PCT/JP2022/020745**

**A. CLASSIFICATION OF SUBJECT MATTER**

***A61K 39/145***(2006.01)i; ***A61K 9/127***(2006.01)i; ***A61K 47/10***(2006.01)i; ***A61K 47/18***(2006.01)i; ***A61K 47/24***(2006.01)i; ***A61K 47/28***(2006.01)i; ***A61K 48/00***(2006.01)i; ***A61P 31/16***(2006.01)i; ***A61P 37/04***(2006.01)i; ***C12N 15/44***(2006.01)i; ***C12N 15/88***(2006.01)i

FI: A61K39/145 ZNA; A61K48/00; A61K9/127; A61K47/18; A61K47/28; A61K47/10; A61K47/24; A61P31/16; A61P37/04; C12N15/88 Z; C12N15/44

According to International Patent Classification (IPC) or to both national classification and IPC

**B. FIELDS SEARCHED**

Minimum documentation searched (classification system followed by classification symbols)

A61K39/145; A61K9/127; A61K47/10; A61K47/18; A61K47/24; A61K47/28; A61K48/00; A61P31/16; A61P37/04; C12N15/44; C12N15/88

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2018-537521 A (MODERNATX, INC.) 20 December 2018 (2018-12-20) claims, paragraphs [0209]-[0494], examples, each table | 1-56 |
| Y | WO 2015/005253 A1 (DAIICHI SANKYO COMPANY, LIMITED) 15 January 2015 (2015-01-15) claims, paragraphs [0004], [0005], [0091], [0092], [0201]-[0337], examples | 1-56 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2022** | **21 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/020745**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/020745**

| Patent document cited in search report | Publication date (day/month/year) | Patent family member(s) | Publication date (day/month/year) |
|---|---|---|---|
| JP 2018-537521 A | 20 December 2018 | WO 2017/070620 A2<br>claims, p. 38, line 13 to p. 120, line 19, examples, tables<br>US 2018/0311336 A1<br>EP 3718565 B1<br>CN 109310751 A<br>KR 10-2018-0096591 A | |
| WO 2015/005253 A1 | 15 January 2015 | US 2016/0257951 A1<br>claims, paragraphs [0004], [0005], [0242]-[0377], table 1, examples<br>EP 3020701 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015164674 A
- WO 2018078053 A
- WO 2015005253 A


### Non-patent literature cited in the description


- *Nature Reviews Disease Primers,* 2018, vol. 4
- *American Family Physician,* 2020, vol. 102 (8), 505-507
- *Nature Reviews Drug Discovery,* 2015, vol. 14, 167-182
- *PLOS ONE,* 2017, vol. 12 (1), e0169528
- *The New England Journal of Medicine,* 2018, vol. 378, 7-9
- *Nature Communications,* 2018, vol. 9
- *J. Biol. Chem.,* 1992, vol. 267, 16396
- *J. Biol. Chem.,* 1991, vol. 266, 12127
- **SAMBROOK, J. et al.** Molecular Cloning a Laboratory Manual. 1989
- **RASHTCHIAN, A.** *Current Opinion in Biotechnology,* 1995, vol. 6 (1), 30-36
- **GIBSON D.G. et al.** *Science,* 2008, vol. 319 (5867), 1215-1220
- **KORMANN, M.** *Nature Biotechnology,* 2011, vol. 29, 154-157